# EUROPEAN PATENT SPECIFICATION

(11) **EP 1 084 239 B1**
(45) Date of publication and mention of the grant of the patent: **24.09.2008**
(21) Application number: 99922437.1
(22) Date of filing: 04.06.1999
(51) Int. Cl.: C12N 15/12, C07K 7/00, C07K 14/435, C07K 16/30, C12Q 1/68, A01K 67/027, G06F 19/00, G11B 23/00

(54) **POLYMORPHIC MARKERS OF PROSTATE CARCINOMA TUMOR ANTIGEN-1 (PCTA-1)**
POLYMORPHE MARKER VON PROSTATAKARZINOM TUMORANTIGEN-1 (PCTA-1)
MARQUEURS POLYMORPHES DE L'ANTIGENE-1 DE LA TUMEUR CANCEREUSE DE LA PROSTATE (PCTA-1)

(30) Priority: 05.06.1998 US 88187 P; 28.09.1998 US 102324 P
(43) Date of publication of application: 21.03.2001
(73) Proprietor: Serono Genetics Institute S.A., 91030 Evry Cedex (FR)
(72) Inventor: BLUMENFELD, Marta, F-75013 Paris (FR); BOUGUELERET, Lydie, F-75015 Paris (FR); CHUMAKOV, Ilya, F-77000 Vaux-le-Penil (FR)
(74) Representative: Warcoin, Jacques
(86) International application number: PCT/IB1999/001072
(87) International publication number: WO 1999/064590

(56) References cited:
- WO-A-96/21671
- WO-A-98/07830
- WO-A-98/18967
- WO-A-98/20165
- SU, Z.-Z ET AL: "Selective expression of the prostate carcinoma tumor antigen, PCTA-1, in human prostate cell lines and tissues" PROCEEDINGS OF THE AMERICAN ASSOCIATION FOR CANCER RESEARCH ANNUAL MEETING, vol. 39, March 1998 (1998-03), page 414 XP002116175
- WANG D ET AL: "Large-scale identification, mapping, and genotyping of single-nucleotide polymorphisms in the human genome" SCIENCE, vol. 280, 15 May 1998 (1998-05-15), pages 1077-82, XP002116081
- KRUGLYAK L: "PASSAGE TEXT. THE USE OF A GENETIC MAP OF BIALLELIC MARKERS IN LINKAGE STUDIES" NATURE GENETICS, vol. 17, no. 1, 1 September 1997 (1997-09-01), pages 22-24, XP002050647 ISSN: 1061-4036
- SCHORK N J ET AL: "Linkage disequilibrium mapping for quantitative traits within case/control settings" AMERICAN JOURNAL OF HUMAN GENETICS, vol. 61, no. 4, SUPPL, 1 January 1997 (1997-01-01), page A293 XP002089399 ISSN: 0002-9297

## Description

### FIELD OF THE INVENTION

The invention concerns the genomic and cDNA sequences of the *PCTA-1* gene, biallelic markers of the *PCTA-1* gene and the association established between these markers and prostate cancer. The invention provides means to determine the predisposition of individuals to prostate cancer as well as means for the diagnosis of this cancer and for the prognosis/detection of an eventual treatment response to therapeutic agents acting against prostate cancer.

### BACKGROUND OF THE INVENTION

### Prostate Cancer

The incidence of prostate cancer has dramatically increased over the last decades. It averages 30-50/100,000 males in Western European countries as well as within the US White male population. In these countries, it has recently become the most commonly diagnosed malignancy, being one of every four cancers diagnosed in American males. Prostate cancer's incidence is very much population specific, since it varies from 2/100,000 in China, to over 80/100,000 among African-American males.

In France, the incidence of prostate cancer is 35/100,000 males and it is increasing by 10/100,000 per decade. Mortality due to prostate cancer is also growing accordingly. It is the second cause of cancer death among French males, and the first one among French males aged over 70. This makes prostate cancer a serious burden in terms of public health.

Prostate cancer is a latent disease. Many men carry prostate cancer cells without overt signs of disease. Autopsies of individuals dying of other causes show prostate cancer cells in 30 % of men at age 50 and in 60 % of men at age 80. Furthermore, prostate cancer can take up to 10 years to kill a patient after the initial diagnosis.

The progression of the disease usually goes from a well-defined mass within the prostate to a breakdown and invasion of the lateral margins of the prostate, followed by metastasis to regional lymph nodes, and metastasis to the bone marrow. Cancer metastasis to bone is common and often associated with uncontrollable pain. ,

Unfortunately, in 80 % of cases, diagnosis of prostate cancer is established when the disease has already metastasized to the bones. Of special interest is the observation that prostate cancers frequently grow more rapidly in sites of metastasis than within the prostate itself.

Early-stage diagnosis of prostate cancer mainly relies today on Prostate Specific Antigen (PSA) dosage, and allows the detection of prostate cancer seven years before clinical symptoms become apparent. The effectiveness of PSA dosage diagnosis is however limited, due to its inability to discriminate between malignant and non-malignant affections of the organ and because not all prostate cancers give rise to an elevated serum PSA concentration. Furthermore, PSA dosage and other currently available approaches such as physical examination, tissue biopsy and bone scans are of limited value in predicting disease progression.

Therefore, there is a strong need for a reliable diagnostic procedure which would enable a more systematic early-stage prostate cancer prognosis.

Although an early-stage prostate cancer prognosis is important, the possibility of measuring the period of time during which treatment can be deferred is also interesting as currently available medicaments are expensive and generate important adverse effects. However, the aggressiveness of prostate tumors varies widely. Some tumors are relatively aggressive, doubling every six months whereas others are slow-growing, doubling once every five years. In fact, the majority of prostate cancers grows relatively slowly and never becomes clinically manifest. Very often, affected patients are among the elderly and die from another disease before prostate cancer actually develops. Thus, a significant question in treating prostate carcinoma is how to discriminate between tumors that will progress and those that will not progress during the expected lifetime of the patient.

Hence, there is also a strong need for detection means which may be used to evaluate the aggressiveness or the development potential of prostate cancer tumors once diagnosed.

Furthermore, at the present time, there is no means to predict prostate cancer susceptibility. It would also be very beneficial to detect individual susceptibility to prostate cancer. This could allow preventive treatment and a careful follow up of the development of the tumor.

A further consequence of the slow growth rate of prostate cancer is that few cancer cells are actively dividing at any one time, rendering prostate cancer generally resistant to radiation and chemotherapy. Surgery is the mainstay of treatment but it is largely ineffective and removes the ejaculatory ducts, resulting in impotence. Oral oestrogens and luteinizing releasing hormone analogs are also used for treatment of prostate cancer. These hormonal treatments provide marked improvement for many patients, but they only provide temporary relief. Indeed, most of these cancers soon relapse with the development of hormone-resistant tumor cells and the oestrogen treatment can lead to serious cardiovascular complications. Consequently, there is a strong need for preventive and curative treatment of prostate cancer.

Efficacy/tolerance prognosis could be precious in prostate cancer therapy. Indeed, hormonal therapy, the main treatment currently available, presents important side effects. The use of chemotherapy is limited because of the small number of patients with chemosensitive tumors. Furthermore the age profile of the prostate cancer patient and intolerance to chemotherapy make the systematic use of this treatment very difficult.

Therefore, a valuable assessment of the eventual efficacy of a medicament to be administered to a prostate cancer patent as well as the patent's eventual tolerance to it may permit to enhance the benefit/risk ratio of prostate cancer treatment.

### Prostate Carcinoma Tumor Antigen -1 (PCTA-1)

WO 96/21671 describes a new protein, named PCTA-1. The document describes the cloning and sequencing of a cDNA encoding PCTA-1 (GenBank L78132). This cDNA has 3.85 kb in length and presents about 80 % sequence homology with rat galectin-8.

WO 96/21671 mentions that the PCTA-1 protein retains a number of conserved structural motifs that are found in most members of the galectin gene family. On the basis of its predicted amino acid sequence, PCTA-1 is said to appear to be a human homologue of rat galectin-8. The galectins display wide tissue distribution, clear developmental regulation, and differential levels in specific tissues, supporting the hypothesis that they contribute to many physiologically important processes in mammalian cells. Of direct relevance to cancer is the finding that the galectins can mediate both cell-cell and cell-matrix interactions.

### SUMMARY OF THE INVENTION

The inventors have characterized the genomic sequence of the *PCTA-1* gene, including its regulatory regions, and, through an association study, have shown that alleles of some biallelic markers of *PCTA-1* are associated with prostate cancer.

Therefore, the present invention discloses the identification and characterization of the genomic sequence of the *PCTA-1* gene, of new cDNAs sequence and the proteins encoded by these cDNAs. The invention concerns biallelic markers located in such sequences, as well as the selection of significant polymorphisms associated with prostate cancer.

Oligonucleotide probes and primers hybridizing specifically with a genomic sequence of *PCTA-1* are also part of the invention.

The selected polymorphisms are used in the design of assays for the reliable detection of genetic susceptibility to prostate cancer, of an early onset of prostate cancer, of the aggressiveness of prostate cancer tumors, of a modified or forthcoming expression of the *PCTA-1* gene, of a modified or forthcoming production of the PCTA-1 protein, or of the production of a modified PCTA-1 protein. They can be used for diagnosis, staging, prognosis, and monitoring of such a disease, which processes can be further included within treatment approaches. The selected polymorphisms can also be used in the design of drug screening protocols to provide an accurate and efficient evaluation of the therapeutic and side-effect potential of new or already existing medicaments.

### BRIEF DESCRIPTION OF THE DRAWINGS

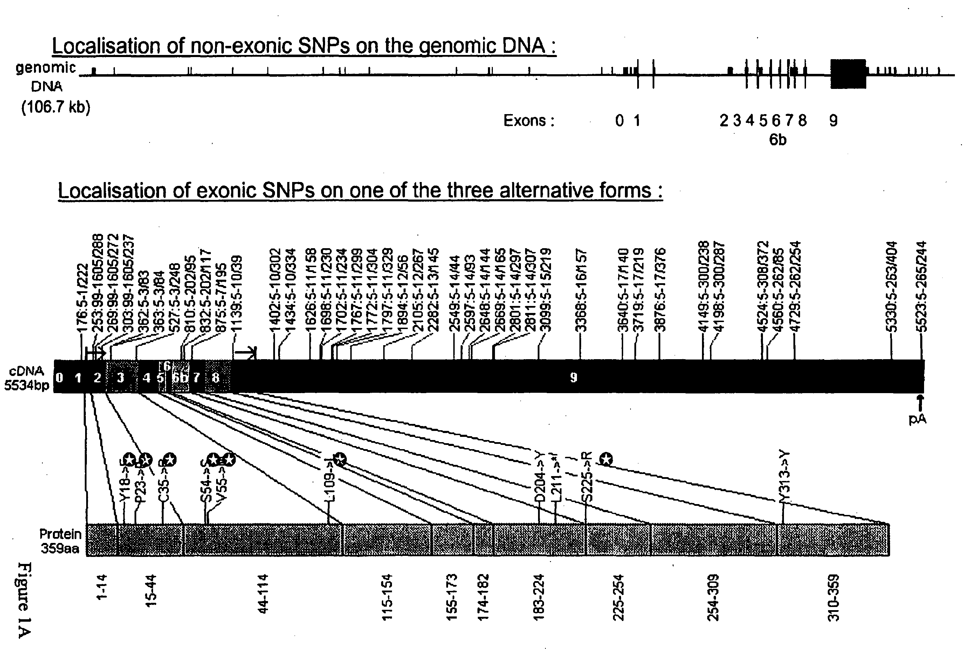

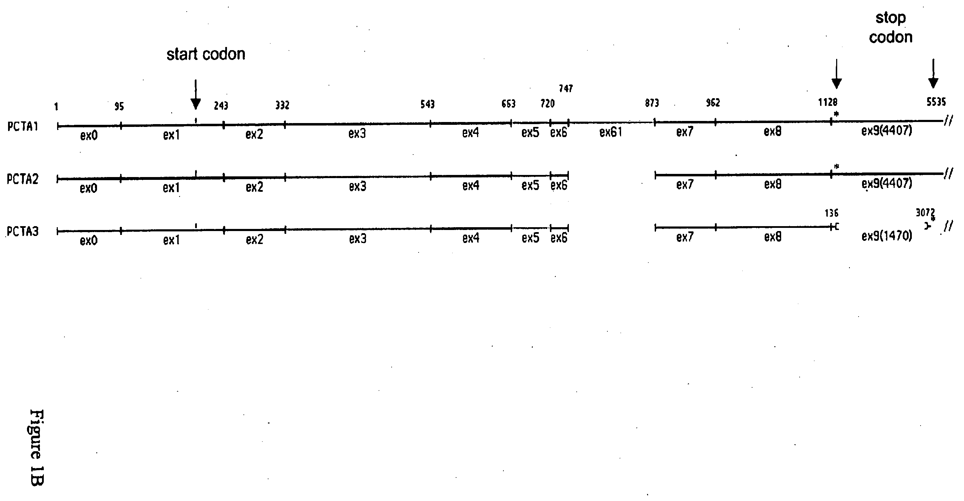

Figure 1A is a diagram of the *PCTA-1* gene with an indication of the relative position of the biallelic markers of the present invention. The upper line refers to the genomic sequence of *PCTA-1*. The middle line refers to the alternative cDNA comprising the exon 6bis with the biallelic markers localization. The lower line refers the PCTA-1 protein with the polymorphic amino acids due to the biallelic markers. ⊛ refers to frequent SNP (detected on pool of hundred DNA). Figure 1B is a diagram of the 3 alternative cDNAs of *PCTA-1*.

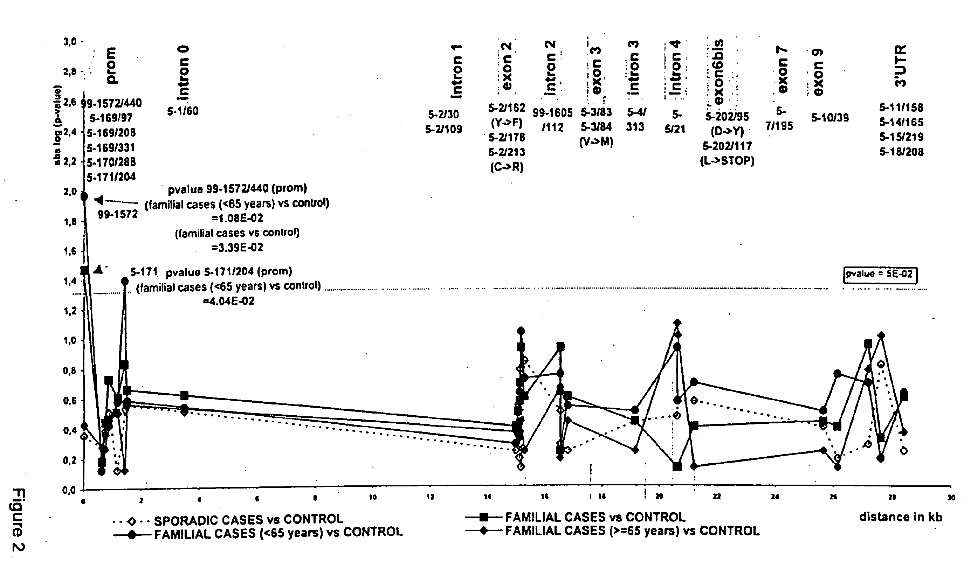

Figure 2 is a graph demonstrating the association between some of the biallelic markers of the invention and prostate cancer with the absolute value of the logarithm (base 10) of the p-value of the chi-square values for each marker shown on the y-axis and a rough estimate of the position of each marker with respect to the *PCTA-1* gene elements on the x-axis.

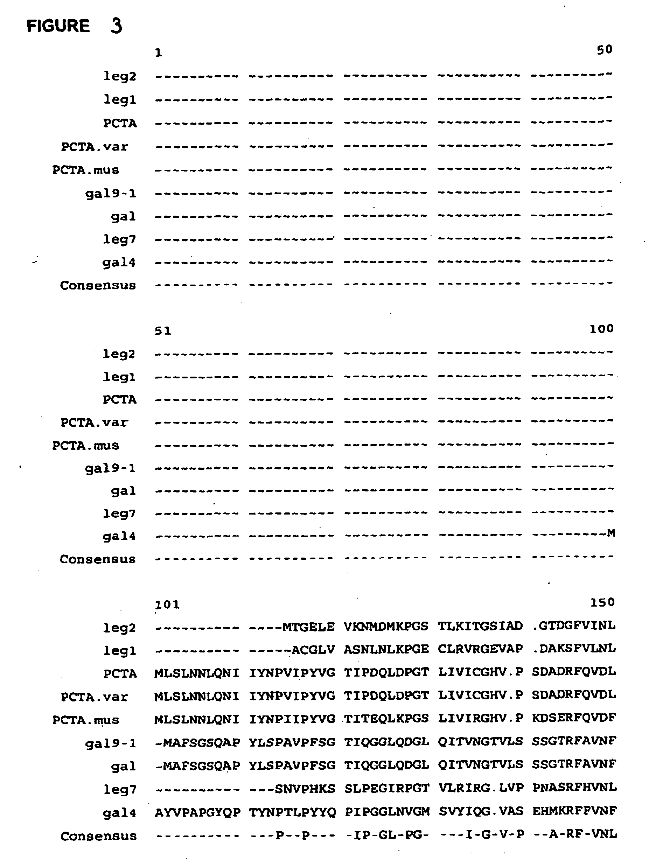

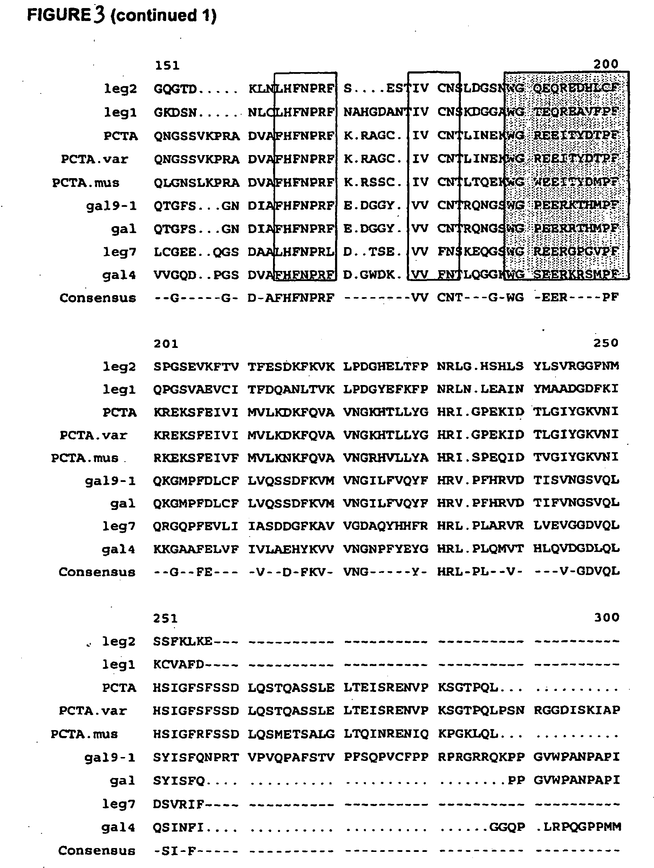

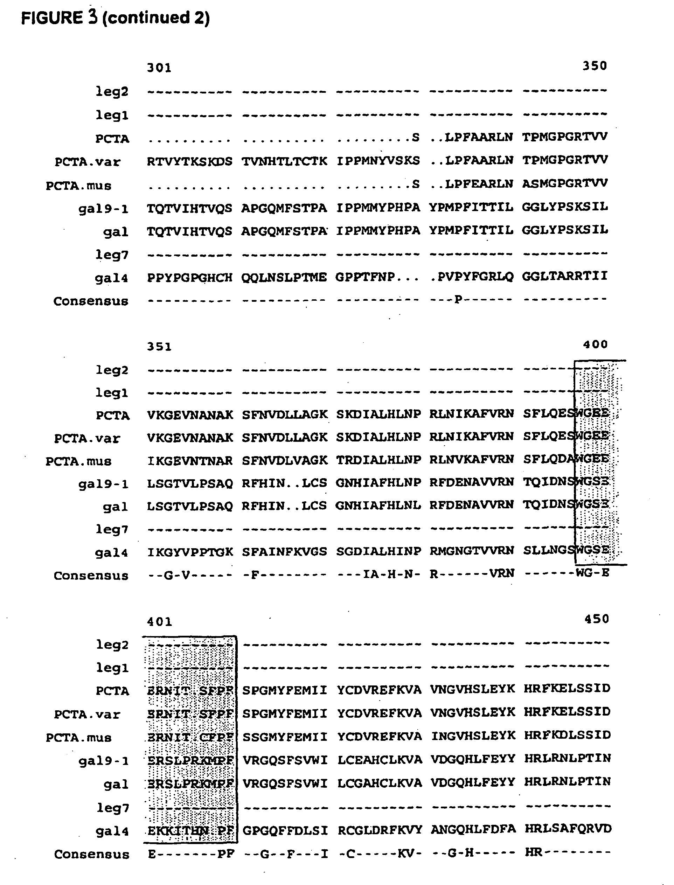

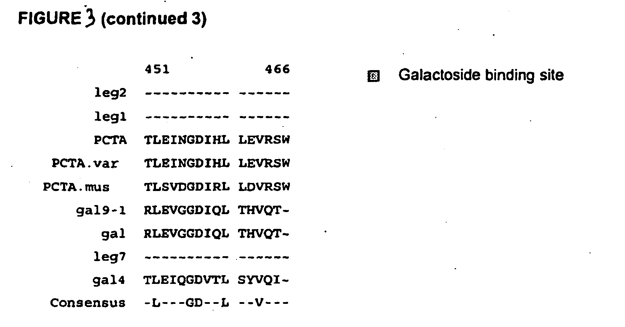

Figure 3 is an alignment of the mouse and human PCTA-1 proteins.

### BRIEF DESCRIPTION OF THE SEQUENCES PROVIDED IN THE SEQUENCE LISTING

SEQ ID No 1 contains a genomic sequence of *PCTA-1* comprising the 5' regulatory region (upstream untranscribed region), the exons (0, 1, 2, 3, 4, 5, 6, 6bis, 7, 8, 9, 9bis, and 9ter) and introns, and the 3' regulatory region (downstream untranscribed region).
SEQ ID No 2 contains a cDNA sequence of *PCTA-1* comprising the exons 0, 1, 2, 3, 4, 5, 6, 7, 8, and 9.
SEQ ID No 3 contains a cDNA sequence of *PCTA-1* comprising the exons 0, 1, 2, 3, 4, 5, 6, 6bis, 7, 8, and 9.
SEQ ID No 4 contains a cDNA sequence of *PCTA-1* comprising the exons 0, 1, 2, 3, 4, 5, 6, 7, 8, 9bis and 9ter.
SEQ ID No 5 contains the amino acid sequence encoded by the cDNA of SEQ ID No 2.
SEQ ID No 6 contains the amino acid sequence encoded by the cDNA of SEQ ID No 3.
SEQ ID No 7 contains the amino acid sequence encoded by the cDNA of SEQ ID No 4.
SEQ ID No 8 contains a murine cDNA sequence of *PCTA-1.*
SEQ ID No 9 contains the amino acid sequence encoded by the cDNA of SEQ ID No 8.
SEQ ID No 10 contains a primer containing the additional PU 5' sequence described further in Example 2.
SEQ ID No 11 contains a primer containing the additional RP 5' sequence described further in Example 2.

### DETAILED DESCRIPTION OF THE INVENTION

### Definitions

Before describing the invention in greater detail, the following definitions are set forth to illustrate and define the meaning and scope of the terms used to describe the invention herein.

The term "*PCTA-1* gene" is intended to define an entity which can comprise some or all the following elements : exons, introns, promoter, regulatory regions, 5'UTR, 3' UTR and regions never transcribed and located either upstream or downstream of the coding sequence of *PCTA-1.* The term *"PCTA-1* gene", when used herein, encompasses genomic, mRNA and cDNA sequences encoding a PCTA-1 protein.

The term "heterologous protein", when used herein, is intended to designate any protein or polypeptide other than the PCTA-1 protein. More particularly, the heterologous protein is a compound which can be used as a marker in further experiments with a *PCTA-1* regulatory region or as a toxin to certain cells in which it is intended to be produced, preferably a toxin to prostate cancer cells.

As used herein, the term "toxin gene" refers to a polynucleotide sequence which encodes a polypeptide that, when expressed in a eukaryotic cell, typically a mammalian cell, kills or disables the cell or causes the cell to exhibit apoptosis, cytostasis or senescence.

The term "isolated" requires that the material be removed from its original environment (e.g., the natural environment if it is naturally occurring). For example, a naturally-occurring polynucleotide or polypeptide present in a living animal is not isolated, but the same polynucleotide or DNA or polypeptide, separated from some or all of the coexisting materials in the natural system, is isolated. Such polynucleotide could be part of a vector and/or such polynucleotide or polypeptide could be part of a composition, and still be isolated in that the vector or composition is not part of its natural environment.

The term "purified" does not require absolute purity; rather, it is intended as a relative definition. Purification of starting material or natural material to at least one order of magnitude, preferably two or three orders, and more preferably four or five orders of magnitude is expressly contemplated. As an example, purification from 0.1 % concentration to 10 % concentration is two orders of magnitude. The term "purified" is used herein to describe a polynucleotide or polynucleotide vector of the invention which has been separated from other compounds including, but not limited to other nucleic acids, carbohydrates, lipids and proteins (such as the enzymes used in the synthesis of the polynucleotide), or the separation of covalently closed polynucleotides from linear polynucleotides. A polynucleotide is substantially pure when at least about 50%, preferably 60 to 75% of a sample exhibits a single polynucleotide sequence and conformation (linear versus covalently close). A substantially pure polynucleotide typically comprises about 50%, preferably 60 to 90% weight/weight of a nucleic acid sample, more usually about 95%, and preferably is over about 99% pure. Polynucleotide purity or homogeneity is indicated by a number of means well known in the art, such as agarose or polyacrylamide gel electrophoresis of a sample, followed by visualizing a single polynucleotide band upon staining the gel. For certain purposes higher resolution can be provided by using HPLC or other means well known in the art.

As used interchangeably herein, the terms "nucleic acids", "oligonucleotides", and "polynucleotides" include RNA, DNA, or RNA/DNA hybrid sequences of more than one nucleotide in either single chain or duplex form. The term "nucleotide" as used herein as an adjective to describe molecules comprising RNA, DNA, or RNA/DNA hybrid sequences of any length in single-stranded or duplex form. The term "nucleotide" is also used herein as a noun to refer to individual nucleotides or varieties of nucleotides, meaning a molecule, or individual unit in a larger nucleic acid molecule, comprising a purine or pyrimidine, a ribose or deoxyribose sugar moiety, and a phosphate group, or phosphodiester linkage in the case of nucleotides within an oligonucleotide or polynucleotide. Although the term "nucleotide" is also used herein to encompass "modified nucleotides" which comprise at least one modifications (a) an alternative linking group, (b) an analogous form of purine, (c) an analogous form of pyrimidine, or (d) an analogous sugar, for examples of analogous linking groups, purine, pyrimidines, and sugars see for example PCT publication No. WO 95/04064. This may be especially oligonucleotides with α or β anomers, oligonucleotides with inter-nucleotide linkage of the phosphorothioate or methyl phosphonate type, or alternatively oligothionucleotide. The polynucleotide sequences of the invention may be prepared by any known method, including synthetic, recombinant, *ex vivo* generation, or a combination thereof, as well as utilizing any purification methods known in the art.

Throughout the present specification, the expression "nucleotide sequence" may be employed to designate indifferently a polynucleotide or a nucleic acid. More precisely, the expression "nucleotide sequence" encompasses the nucleic material itself and is thus not restricted to the sequence information (i.e. the succession of letters chosen among the four base letters) that biochemically characterizes a specific DNA or RNA molecule.

A "promoter" refers to a DNA sequence recognized by the synthetic machinery of the cell required to initiate the specific transcription of a gene.

A sequence which is "operably linked" to a regulatory sequence such as a promoter means that said regulatory element is in the correct location and orientation in relation to the nucleic acid to control RNA polymerase initiation and expression of the nucleic acid of interest. As used herein, the term "operably linked" refers to a linkage of polynucleotide elements in a functional relationship. For instance, a promoter or enhancer is operably linked to a coding sequence if it affects the transcription of the coding sequence. More precisely, two DNA molecules (such as a polynucleotide containing a promoter region and a polynucleotide encoding a desired polypeptide or polynucleotide) are said to be "operably linked" if the nature of the linkage between the two polynucleotides does not (1) result in the introduction of a frame-shift mutation or (2) interfere with the ability of the polynucleotide containing the promoter to direct the transcription of the coding polynucleotide.

The term "primer" denotes a specific oligonucleotide sequence which is complementary to a target nucleotide sequence and used to hybridize to the target nucleotide sequence. A primer serves as an initiation point for nucleotide polymerization catalyzed by either DNA polymerase, RNA polymerase or reverse transcriptase.

The term "probe" denotes a defined nucleic acid segment (or nucleotide analog segment, e.g., polynucleotide as defined herein) which can be used to identify a specific polynucleotide sequence present in samples, said nucleic acid segment comprising a nucleotide sequence complementary of the specific polynucleotide sequence to be identified.

The terms "base paired" and "Watson & Crick base paired" are used interchangeably herein to refer to nucleotides which can be hydrogen bonded to one another be virtue of their sequence identities in a manner like that found in double-helical DNA with thymine or uracil residues linked to adenine residues by two hydrogen bonds and cytosine and guanine residues linked by three hydrogen bonds (See Stryer, L., Biochemistry, 4th edition, 1995).

The terms "complementary" or "complement thereof" are used herein to refer to the sequences of polynucleotides which is capable of forming Watson & Crick base pairing with another specified polynucleotide throughout the entirety of the complementary region. For the purpose of the present invention, a first polynucleotide is deemed to be complementary to a second polynucleotide when each base in the first polynucleotide is paired with its complementary base. Complementary bases are, generally, A and T (or A and U), or C and G. "Complement" is used herein as a synonym from "complementary polynucleotide", "complementary nucleic acid" and "complementary nucleotide sequence". These terms are applied to pairs of polynucleotides based solely upon their sequences and not any particular set of conditions under which the two polynucleotides would actually bind.

The term "polypeptide" refers to a polymer of amino acids without regard to the length of the polymer; thus, peptides, oligopeptides, and proteins are included within the definition of polypeptide. This term also does not specify or exclude post-expression modifications of polypeptides, for example, polypeptides which include the covalent attachment of glycosyl groups, acetyl groups, phosphate groups, lipid groups and the like are expressly encompassed by the term polypeptide. Also included within the definition are polypeptides which contain one or more analogs of an amino acid (including, for example, non-naturally occurring amino acids, amino acids which only occur naturally in an unrelated biological system, modified amino acids from mammalian systems etc.), polypeptides with substituted linkages, as well as other modifications known in the art, both naturally occurring and non-naturally occurring.

The term "recombinant polypeptide" is used herein to refer to polypeptides that have been artificially designed and which comprise at least two polypeptide sequences that are not found as contiguous polypeptide sequences in their initial natural environment, or to refer to polypeptides which have been expressed from a recombinant polynucleotide.

The term "purified" is used herein to describe a polypeptide of the invention which has been separated from other compounds including, but not limited to nucleic acids, lipids, carbohydrates and other proteins. A polypeptide is substantially pure when at least about 50%, preferably 60 to 75% of a sample exhibits a single polypeptide sequence. A substantially pure polypeptide typically comprises about 50%, preferably 60 to 90% weight/weight of a protein sample, more usually about 95%, and preferably is over about 99% pure. Polypeptide purity or homogeneity is indicated by a number of means well known in the art, such as agarose or polyacrylamide gel electrophoresis of a sample, followed by visualizing a single polypeptide band upon staining the gel. For certain purposes higher resolution can be provided by using HPLC or other means well known in the art.

As used herein, the term "non-human animal" refers to any non-human vertebrate, birds and more usually mammals, preferably primates, farm animals such as swine, goats, sheep, donkeys, and horses, rabbits or rodents, more preferably rats or mice. As used herein, the term "animal" is used to refer to any vertebrate, preferable a mammal. Both the terms "animal" and "mammal" expressly embrace human subjects unless preceded with the term "non-human".

As used herein, the term "antibody" refers to a polypeptide or group of polypeptides which are comprised of at least one binding domain, where an antibody binding domain is formed from the folding of variable domains of an antibody molecule to form three-dimensional binding spaces with an internal surface shape and charge distribution complementary to the features of an antigenic determinant of an antigen, which allows an immunological reaction with the antigen. Antibodies include recombinant proteins comprising the binding domains, as wells as fragments, including Fab, Fab', F(ab)₂, and F(ab')₂ fragments.

As used herein, an "antigenic determinant" is the portion of an antigen molecule, in this case a PCTA-1 polypeptide, that determines the specificity of the antigen-antibody reaction. An "epitope" refers to an antigenic determinant of a polypeptide. An epitope can comprise as few as 3 amino acids in a spatial conformation which is unique to the epitope. Generally an epitope consists of at least 6 such amino acids, and more usually at least 8-10 such amino acids. Methods for determining the amino acids which make up an epitope include x-ray crystallography, 2-dimensional nuclear magnetic resonance, and epitope mapping e.g. the Pepscan method described by Geysen et al. 1984; PCT Publication No. WO 84/03564; and PCT Publication No. WO 84/03506.

The term "allele" is used herein to refer to variants of a nucleotide sequence. A biallelic polymorphism has two forms. Diploid organisms may be homozygous or heterozygous for an allelic form.

The term "heterozygosity rate" is used herein to refer to the incidence of individuals in a population which are heterozygous at a particular allele. In a biallelic system, the heterozygosity rate is on average equal to 2Pₐ(1-Pₐ), where Pₐ is the frequency of the least common allele. In order to be useful in genetic studies, a genetic marker should have an adequate level of heterozygosity to allow a reasonable probability that a randomly selected person will be heterozygous.

The term "genotype" as used herein refers the identity of the alleles present in an individual or a sample. In the context of the present invention, a genotype preferably refers to the description of the biallelic marker alleles present in an individual or a sample. The term "genotyping" a sample or an individual for a biallelic marker consists of determining the specific allele or the specific nucleotide carried by an individual at a biallelic marker.

The term "mutation" as used herein refers to a difference in DNA sequence between or among different genomes or individuals which has a frequency below 1%.

The term "haplotype" refers to a combination of alleles present in an individual or a sample. In the context of the present invention, a haplotype preferably refers to a combination of biallelic marker alleles found in a given individual and which may be associated with a phenotype.

The term "polymorphism" as used herein refers to the occurrence of two or more alternative genomic sequences or alleles between or among different genomes or individuals. "Polymorphic" refers to the condition in which two or more variants of a specific genomic sequence can be found in a population. A "polymorphic site" is the locus at which the variation occurs. A single nucleotide polymorphism is a single base pair change. Typically a single nucleotide polymorphism is the replacement of one nucleotide by another nucleotide at the polymorphic site. Deletion of a single nucleotide or insertion of a single nucleotide, also give rise to single nucleotide polymorphisms. In the context of the present invention "single nucleotide polymorphism" preferably refers to a single nucleotide substitution. However, the polymorphism can also involve an insertion or a deletion of at least one nucleotide, preferably between 1 and 5 nucleotides. The nucleotide modification can also involve the presence of several adjacent single base polymorphisms. This type of nucleotide modification is usually called a "variable motif". Generally, a "variable motif" involves the presence of 2 to 10 adjacent single base polymorphisms. In some instances, series of two or more single base polymorphisms can be interrupted by single bases which are not polymorphic. This is also globally considered to be a "variable motif". Typically, between different genomes or between different individuals, the polymorphic site may be occupied by two different nucleotides.

The term "biallelic polymorphism" and "biallelic marker" are used interchangeably herein to refer to a polymorphism, usually a single nucleotide, having two alleles at a fairly high frequency in the population. A "biallelic marker allele" refers to the nucleotide variants present at a biallelic marker site. Typically, the frequency of the less common allele of the biallelic markers of the present invention has been validated to be greater than 1%, preferably the frequency is greater than 10%, more preferably the frequency is at least 20% (i.e. heterozygosity rate of at least 0.32), even more preferably the frequency is at least 30% (i.e. heterozygosity rate of at least 0.42). A biallelic marker wherein the frequency of the less common allele is 30% or more is termed a "high quality biallelic marker".

As used herein the terminology "defining a biallelic marker" means that a sequence includes a polymorphic base from a biallelic marker. The sequences defming a biallelic marker may be of any length consistent with their intended use, provided that they contain a polymorphic base from a biallelic marker. The sequence has between 2 and 100, preferably between 20, 30, or 40 and 60, and more preferably about 47 nucleotides in length. Likewise, the term "marker" or "biallelic marker" requires that the sequence is of sufficient length to practically (although not necessarily unambiguously) identify the polymorphic allele, which usually implies a length of at least 4, 5, 6, 10, 15, 20, 25, or 40 nucleotides.

As used herein the term "*PCTA-1*-related biallelic marker " or "biallelic marker of the *PCTA-1* gene" relates to a set of biallelic markers in linkage disequilibrium with the *PCTA-1* gene. The term *PCTA-1*-related biallelic marker encompasses all of the biallelic markers A1 to A125 disclosed in Table 2.

The location of nucleotides in a polynucleotide with respect to the center of the polynucleotide are described herein in the following manner. When a polynucleotide has an odd number of nucleotides, the nucleotide at an equal distance from the 3' and 5' ends of the polynucleotide is considered to be "at the center" of the polynucleotide, and any nucleotide immediately adjacent to the nucleotide at the center, or the nucleotide at the center itself is considered to be "within 1 nucleotide of the center." With an odd number of nucleotides in a polynucleotide any of the five nucleotides positions in the middle of the polynucleotide would be considered to be within 2 nucleotides of the center, and so on. When a polynucleotide has an even number of nucleotides, there would be a bond and not a nucleotide at the center of the polynucleotide. Thus, either of the two central nucleotides would be considered to be "within I nucleotide of the center" and any of the four nucleotides in the middle of the polynucleotide would be considered to be "within 2 nucleotides of the center", and so on. For polymorphisms which involve the substitution, insertion or deletion of 1 or more nucleotides, the polymorphism, allele or biallelic marker is "at the center" of a polynucleotide if the difference between the distance from the substituted, inserted, or deleted polynucleotides of the polymorphism and the 3' end of the polynucleotide, and the distance from the substituted, inserted, or deleted polynucleotides of the polymorphism and the 5' end of the polynucleotide is zero or one nucleotide. If this difference is 0 to 3, then the polymorphism is considered to be "within 1 nucleotide of the center." If the difference is 0 to 5, the polymorphism is considered to be "within 2 nucleotides of the center." If the difference is 0 to 7, the polymorphism is considered to be "within 3 nucleotides of the center," and so on.

The terms "trait" and "phenotype" are used interchangeably herein and refer to any visible, detectable or otherwise measurable property of an organism such as symptoms of, or susceptibility to a disease for example. Preferably, the term "trait" or "phenotype", when used herein, encompasses, but is not limited to, prostate cancer, an early onset of prostate cancer, a beneficial response to or side effects related to treatment or a vaccination against prostate cancer, a susceptibility to prostate cancer, the level of aggressiveness of prostate cancer tumors, a modified or forthcoming expression of the *PCTA-1* gene, a modified or forthcoming production of the PCTA-1 protein, or the production of a modified PCTA-1 protein. However, the term "trait" or "phenotype" can refer to other types of cancer.

The term "susceptibility to prostate cancer" is used herein to designate a strong likelihood for an individual to develop in his lifetime a form of prostate cancer, particularly a form of prostate cancer in which a PCTA-1 protein is expressed. This likelihood is strongly related to the association established between the biallelic markers of the present invention and prostate cancer or other more specific characteristics which can lead to the development of the prostate cancer such as the modified expression of the *PCTA-1 gene,* the modified production of the PCTA-1 protein or the production of a modified PCTA-1 protein.

The term "aggressiveness" of prostate cancer tumors refers to the metastatic potential of these tumors.

The term "treatment of prostate cancer" when used herein is intended to designate the administration of substances either for prophylactic or curative purposes. When administered for prophylactic purposes, the treatment is provided in advance of the appearance of biologically or clinically significant cancer symptoms. When administered for curative purposes, the treatment is provided to attenuate the pathological symptoms of prostate cancer, to decrease the size or growth of cancer tumors or metastases or to remove them.

The terms "an agent acting against prostate cancer" refers to any drug or compound that is capable of reducing the growth rate, rate of metastasis, or viability of tumor cells in a mammal, is capable of reducing the size or eliminating tumors in a mammal, or is capable of increasing the average life span of a mammal or human with cancer. Agents acting against prostate cancer also include compounds which are able to reduce the risk of cancer developing in a population, particularly a high risk population. Examples of agents acting against prostate cancer include hormonal therapeutic agents (for example, medroxyprogesterone acetate, estramustine phosphate, gonadotrophin releasing hormone (GnRH) agonists, anti-androgens such as flutamide, nilutamide, groserelin, and cyprosterone acetate, anti-gonadotropic agents such as stilboestrol and other oestrogenic agents, progestogens such as megestrol acetate) or chemotherapeutic agents (for example, carboplatin, cisplatin, methotrexate, mitomycin, epirubicin, vinblastine, 5-fluorouracyl, mitozantrone, cyclophosphamide, interferon, N-(4-hydroxyphenyl) retinamide (4HPR)). These agents can be used in combination.

The term "side effects to an agent acting against prostate cancer" refers to adverse effects of therapy resulting from extensions of the principal pharmacological action of the drug or to idiosyncratic adverse reactions resulting from an interaction of the drug with unique host factors. These side effects include, but are not limited to, adverse reactions such as dermatological, hematological or hepatological toxicities and further includes gastric and intestinal ulceration, disturbance in platelet function, renal injury, nephritis, vasomotor rhinitis with profuse watery secretions, angioneurotic edema, generalized urticaria, and bronchial asthma to laryngeal edema and bronchoconstriction, hypotension, sexual dysfunction, and shock. More particularly, the side effects can be nausea/vomiting, cardiovascular side effects such as deep vein thrombosis and fluid retention, and gynaecomastia.

The term "response to an agent acting against prostate cancer" refers to drug efficacy, including but not limited to ability to metabolize a compound, to the ability to convert a pro-drug to an active drug, and to the pharmacokinetics (absorption, distribution, elimination) and the pharmacodynamics (receptor-related) of a drug in an individual.

In the context of the present invention, a "positive response" to a medicament can be defined as comprising a reduction of the symptoms related to the disease, an increase of survival time or condition to be treated.

In the context of the present invention, a "negative response" to a medicament can be defined as comprising either a lack of positive response to the medicament which does not lead to a symptom reduction or an increase of survival time, or which leads to a side-effect observed following administration of the medicament.

### Variants and fragments

### 1- Polynucleotides

The invention also relates to variants and fragments of the polynucleotides described herein, particularly of a *PCTA-1* gene containing one or more biallelic markers according to the invention.

Variants of polynucleotides, as the term is used herein, are polynucleotides that differ from a reference polynucleotide. A variant of a polynucleotide may be a naturally occurring variant such as a naturally occurring allelic variant, or it may be a variant that is not known to occur naturally. Such non-naturally occurring variants of the polynucleotide may be made by mutagenesis techniques, including those applied to polynucleotides, cells or organisms. Generally, differences are limited so that the nucleotide sequences of the reference and the variant are closely similar overall and, in many regions, identical.

A preferred embodiment is directed to an isolated, purified of recombinant polypeptide consisting of a fragment of 15 to 50 nucleotides of SEQ ID NO:1 or the complement thereof, wherein said fragment includes the A2, A30, A41, A55 or A57 biallelic marker.

A polynucleotide fragment is a polynucleotide having a sequence that entirely is the same as part but not all of a given nucleotide sequence, preferably the nucleotide sequence of a *PCTA-1* gene, and variants thereof. The fragment can be a portion of an exon or of an intron of a *PCTA-1* gene. It can also be a portion of the regulatory sequences of the *PCTA-1* gene, preferably of the promoter. Such fragments comprise at least one of the biallelic markers A2, A30, A41, A55 or A57, and the complements thereof.

Such fragments may be "free-standing", i.e. not part of or fused to other polynucleotides, or they may be comprised within a single larger polynucleotide of which they form a part or region. However, several fragments may be comprised within a single larger polynucleotide.

As representative examples of polynucleotide fragments of the invention, there may be mentioned those which have from about 4, 6, 8, 15, 20, 25, 40, 10 to 30, nucleotides in length. Preferred are those fragments having about 47 nucleotides in length, such as those of P1 to P125 and the complementary sequences thereto, and containing at least one of the biallelic markers of the *PCTA-1* gene which are described herein. It will of course be understood that the polynucleotides P1 to P125 and the complementary sequences thereto can be shorter or longer, although it is preferred that they at least contain the biallelic marker of the primer which can be located at one end of the fragment.

### Genomic Sequence Of The PCTA-1 Gene

The present invention discloses a purified and/or isolated nucleic acid corresponding to the genomic sequence of the *PCTA-1* gene. Preferably, this genomic *PCTA-1* sequence comprises the nucleotide sequence of SEQ ID No 1, a sequence complementary thereto, a fragment or a variant thereof.

The present invention discloses the genomic sequence of *PCTA-1*. The *PTCA-1* gene sequence comprises a coding sequence including 13 exons included in SEQ ID No 1, namely exon 0, exon 1, exon 2, exon 3, exon 4, exon 5, exon 6, exon 6bis, exon 7, exon 8, exon 9, exon 9bis and exon 9ter, the intronic regions, the promoter, the 5'UTR, the 3'UTR, and regulatory regions located upstream and downstream of the coding region.

The localization of the exons and introns of the *PCTA-1* gene is detailed in Table A and is described as feature in SEQ ID No 1.

**Table A**

| Exon | Position range in SEQ ID No 1 | | Intron | Position range in SEQ ID No 1 | |
|---|---|---|---|---|---|
| | Beginning | End | | Beginning | End |
| 0 | 68648 | 68741 | 0 | 68742 | 70646 |
| 1 | 70647 | 70794 | 1 | 70795 | 82207 |
| 2 | 82208 | 82296 | 2 | 82297 | 83612 |
| 3 | 83613 | 83823 | 3 | 83824 | 85297 |
| 4 | 85298 | 85417 | 4 | 85418 | 86388 |
| 5 | 86389 | 86445 | 5 | 86446 | 87495 |
| 6 | 87496 | 87522 | 6 | 87523 | 87649 |
| 6bis | 87650 | 87775 | 6bis | 87776 | 88294 |
| 7 | 88295 | 88383 | 7 | 88384 | 89483 |
| 8 | 89484 | 89649 | 8 | 89650 | 92748 |
| 9 | 92749 | 97155 | 9bis | 92884 | 95820 |
| 9bis | 92749 | 92883 | | | |
| 9ter | 95821 | 97155 | | | |

Intron 0 refers to the nucleotide sequence located between Exon 0 and Exon 1, and so on. The intron 6 refers to the nucleotide sequence located between Exon 6 and Exon 6bis. The intron 6bis refers to the nucleotide sequence located between Exon 6bis and Exon 7. The intron 8 refers to the nucleotide sequence located between Exon 8 and Exon 9 or 9bis. The intron 9bis refers to the nucleotide sequence located between Exon 9bis and Exon 9ter.

The invention also discloses a purified, isolated, or recombinant polynucleotide comprising a nucleotide sequence having at least 70, 75, 80, 85, 90, or 95% nucleotide identity with a nucleotide sequence of SEQ ID No 1 or a complementary sequence thereto or a fragment thereof. The nucleotide differences as regards to the nucleotide sequence of SEQ ID No 1 may be generally randomly distributed throughout the entire nucleic acid. Nevertheless, preferred nucleic acids are those wherein the nucleotide differences as regards to the nucleotide sequence of SEQ ID No 1 are predominantly located outside the coding sequences contained in the exons. These nucleic acids, as well as their fragments and variants, may be used as oligonucleotide primers or probes in order to detect the presence of a copy of the *PCTA-1* gene in a test sample, or alternatively in order to amplify a target nucleotide sequence within the *PCTA-1* sequences.

The invention discloses a purified, isolated, or recombinant nucleic acid that hybridizes with the nucleotide sequence of SEQ ID No 1 or a complementary sequence thereto or a variant thereof, under the stringent hybridization conditions as defined above.

Nucleic acids disclosed herein include isolated, purified, or recombinant polynucleotides comprising a contiguous span of at least 12, 15, 18, 20, 25, 30, 35, 40, 50, 60, 70, 80, 90, 100, 150, 200, 500, or 1000 nucleotides of SEQ ID No 1 or the complements thereof, wherein said contiguous span comprises at least 1, 2, 3, 5, or 10 of the following nucleotide positions of SEQ ID No 1: 1-70715, 70795-82207, 82297-83612, 83824-85297, 85418-86388, 86446-87495, 87523-88294, 88384-89483, 89650-92748, 97156-98309, 98476-99329, 99491-100026, 100212-100281, 100396-100538, 100682-100833, 100995-101920, 102087-102970, 103264-103724, and 103753-106746. Disclosed herein are isolated, purified, or recombinant polynucleotides comprising a contiguous span of at least 12, 15, 18, 20, 25, 30, 35, 40, 50, 60, 70, 80, 90, 100, 150, 200, 500, or 1000 nucleotides of SEQ ID No 1 or the complements thereof, wherein said contiguous span comprises at least one nucleotide selected from the group consisting of a nucleotide G at positions 70728, 87860, 88297, 94432, and 95340 of SEQ ID No 1; a nucleotide A at positions 82218, 83644, 83808, 87787, 87806, 94218, and 97144 of SEQ ID No 1; a nucleotide C at positions 87902, 88215, 88283, 92760, 93726, and 94422 of SEQ ID No 1; and a nucleotide T at positions 93903, and 94170 of SEQ ID No 1. Disclosed herein are isolated, purified, or recombinant polynucleotides comprising a contiguous span of at least 12, 15, 18, 20, 25, 30, 35, 40, 50, 60, 70, 80, 90, 100, 150, 200, 500, or 1000 nucleotides of SEQ ID No 1 or the complements thereof, wherein said contiguous span comprises at least one nucleotide selected from the group consisting of a nucleotide G at positions 86435, 93592, 93680, 93681, 93682, 93728, 93761, and 95445 of SEQ ID No 1; a nucleotide A at positions 86434, 88355, 93240, 93471, and 93747of SEQ ID No 1; a nucleotide C at positions 93683, 95126, and 95444 of SEQ ID No 1; and a nucleotide T at positions 94154, and 94430 of SEQ ID No 1. Disclosed herein are isolated, purified, or recombinant polynucleotides comprising a contiguous span of at least 12, 15, 18, 20, 25, 30, 35, 40, 50, 60, 70, 80, 90, 100, 150, 200, 500, or 1000 nucleotides of SEQ ID No 1 or the complements thereof, wherein said contiguous span comprises nucleotide positions selected from the group consisting of the nucleotide positions of SEQ ID No 1: 92975-92977, 93711-93715, 94151-94153, 94240-94243, 94770-94773, 94804-94808, 95121-95122, 95129-95135, 95148-95153, 95154-95159, 95173-95178, 95367-95374, 95410-95413, 95418-95420, 95430-95436, 95533-95535, and 95677-95677. It should be noted that nucleic acid fragments of any size and sequence may also be comprised by the polynucleotides described in this section.

A preferred aspect of the present invention is a purified and/or isolated and/or recombined *PCT-1* gene or a fragment thereof comprising at least one of the A2, A30, A41, A55 and A57 biallelic polymorphisms described below, a sequence complementary thereto, a fragment or a variant thereof. In some embodiments, the *PCTA-1* gene or a fragment thereof may comprise at least one of the nucleotide sequences of P1 to P125, a sequence complementary thereto, a fragment or a variant thereof.

While this section is entitled "Genomic Sequences of The *PCTA-1* Gene", it should be noted that nucleic acid fragments of any size and sequence may also be comprised by the polynucleotides described in this section, flanking the genomic sequences of *PCTA-1* on either side or between two or more such genomic sequences.

### PCTA-1 cDNA Sequences

The invention also discloses a purified and/or isolated cDNA encoding a PCTA-1 protein. Preferably, the cDNA comprises a nucleotide sequence selected from the group consisting of SEQ ID Nos 2, 3, 4, sequences complementary thereto and functional fragments and variants thereof. Moreover, purified, isolated, or recombinant *PCTA-1* cDNAs consisting of, consisting essentially of, or comprising a sequence selected from the group consisting of SEQ ID Nos 2, 3, 4 and the complementary sequence thereto are disclosed herein.

The invention also discloses a purified and/or isolated cDNA sequence encoding a mouse PCTA-1 protein, particularly a cDNA comprising the nucleotide sequence of SEQ ID No 8, a sequence complementary thereto or a fragment and variant thereof. The main characteristics of the murine cDNA are detailed in Table B. Moreover, purified, isolated, or recombinant *PCTA-1* cDNAs consisting of, consisting essentially of, or comprising the sequence of SEQ ID No 8 and the complementary sequence thereto are disclosed herein.

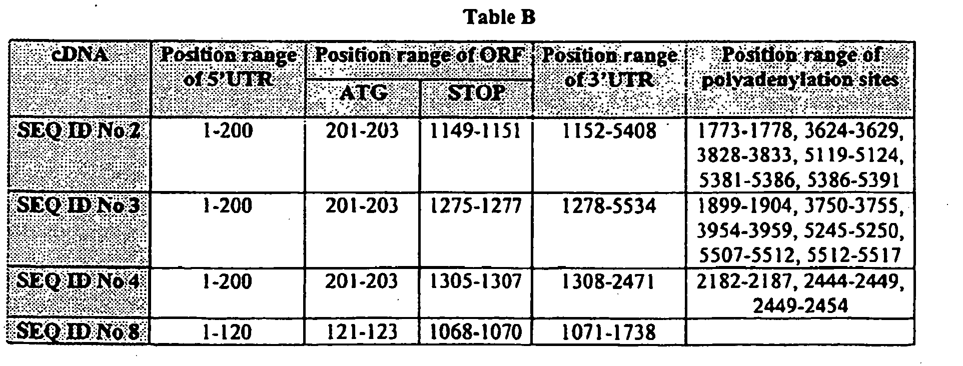

While this section is entitled "*PCTA-1* cDNA Sequences," it should be noted that nucleic acid fragments of any size and sequence may also be comprised by the polynucleotides described in this section, flanking the genomic sequences of *PCTA-1* on either side or between two or more such genomic sequences.

### Coding Regions

The invention also discloses a nucleotide sequence encoding the human PCTA-1 protein selected from the group consisting of SEQ ID No 5, 6, 7, sequences complementary thereto and fragments and variants thereof. Disclosed herein are isolated, purified, and recombinant polynucleotides which encode a polypeptides comprising a contiguous span of at least 6 amino acids, preferably at least 8 or 10 amino acids, more preferably at least 12, 15, 20, 25, 30, 40, 50, or 100 amino acids of SEQ ID No 5, wherein said contiguous span includes:
- a serine residue at amino acid position 170 and/or a lysine residue at amino acid position 203 in SEQ ID No 5; and/or
- at least one residue selected in the group consisting of a tyrosine residue at amino acid position 18, a cysteine residue at amino acid position 35, a methionine residue at amino acid position 55 and an arginine residue at amino acid position 183 in SEQ ID No 5.

### Oligonucleotide Probes And Primers

Polynucleotides derived from the *PCTA-1* gene are useful in order to detect the presence of at least a copy of a nucleotide sequence of SEQ ID No 1, or a fragment, complement, or variant thereof in a test sample.

Probes and primers of the present invention are used in a hybridization assay, a sequencing assay or an enzyme-based mismatch detection assay for determining the identity of a nucleotide at the A2, A30, A41, A55 or A57 biallelic marker.

The present invention also concerns oligonucleotides and groups of oligonucleotides for the detection of alleles of biallelic markers of the *PCTA-1* gene, preferably those associated with cancer, preferably with prostate cancer, with an early onset of prostate cancer, with a susceptibility to prostate cancer, with the level of aggressiveness of prostate cancer tumors, with a modified or forthcoming expression of the *PCTA-1* gene, with a modified or forthcoming production of the PCTA-1 protein, or with the production of a modified PCTA-1 protein. These oligonucleotides are characterized in that they can hybridize with a *PCTA-1* gene, preferably with a polymorphic *PCTA-1* gene and more preferably with a region of a *PCTA-1* gene comprising a polymorphic site containing a specific allele associated with prostate cancer, with the level of aggressiveness of prostate cancer tumors or with modifications in the regulation of expression of the *PCTA-1* gene. These oligonucleotides are useful either as primers for use in various processes such as DNA amplification and microsequencing or as probes for DNA recognition in hybridization analyses.

Therefore, probe according to the invention consists of a nucleic acid comprising a biallelic marker selected from the group consisting of A2, A30, A41, A55 and/or A57 or the complements thereof, for which the respective locations in the sequence listing are provided in Table 2. In some embodiments, the oligonucleotides comprise the polymorphic base of a sequence selected from P1 to P125, and the complementary sequences thereto. In other embodiments, the oligonucleotides have a 3' terminus immediately adjacent to a polymorphic base in the *PCTA-1* gene, such as a polymorphic base comprised in one of the sequences P1 to P125, and the complementary sequence thereto. In other embodiments, the oligonucleotide is capable of discriminating between different alleles of a biallelic marker in the *PCTA-1* gene, including the biallelic markers A2, A30, A41, A55 and/or A57 and the complements thereof.

In one embodiment the invention encompasses isolated, purified, and recombinant polynucleotides consisting of, or consisting essentially of a contiguous span of 8 to 50 nucleotides of any one of SEQ ID Nos 1, 2. 3, 4 and the complement thereof, wherein said span includes a *PCTA-1-*related biallelic markers in said sequence; wherein said *PCTA*-*1*-related biallelic marker is selected from the group consisting of A2, A30, A41, A55 and/or A57, and the complements thereof; optionally, wherein said contiguous span is 18 to 47 nucleotides in length and said biallelic marker is within 4 nucleotides of the center of said polynucleotide; optionally, wherein said polynucleotide consists of said contiguous span and said contiguous span is 25 nucleotides in length and said biallelic marker is at the center of said polynucleotide; optionally, wherein said polynucleotide consists of said contiguous span and said contiguous span is 47 nucleotides in length and said biallelic marker is at the center of said polynucleotide; optionally, wherein the 3' end of said contiguous span is present at the 3' end of said polynucleotide; and optionally, wherein the 3' end of said contiguous span is located at the 3' end of said polynucleotide and said biallelic marker is present at the 3' end of said polynucleotide. In a preferred embodiment, said probes comprises, consists of, or consists essentially of a sequence selected from the following sequences: P1 to P125 and the complementary sequences thereto.

In another embodiment the invention encompasses isolated, purified and recombinant polynucleotides comprising, consisting of, or consisting essentially of a contiguous span of 8 to 50 nucleotides of SEQ ID Nos 1, 2, 3, 4, or the complements thereof, wherein the 3' end of said contiguous span is located at the 3' end of said polynucleotide, and wherein the 3' end of said polynucleotide is located within 20 nucleotides upstream of a *PCTA-1*-related biallelic marker in said sequence; wherein said *PCTA-1*-related biallelic marker is selected from the group consisting of A2, A30, A41, A55 and/or A57, and the complements thereof.

The invention discloses isolated, purified, or recombinant polynucleotides comprising, consisting of, or consisting essentially of a sequence selected from the following sequences: B1 to B47 and C1 to C47.

In an additional embodiment, the invention encompasses polynucleotides for use in hybridization assays, sequencing assays, and enzyme-based mismatch detection assays for determining the identity of the nucleotide at a *PCTA-1*-related biallelic marker in SEQ ID Nos 1, 2, 3, 4, or the complements thereof, as well as polynucleotides for use in amplifying segments of nucleotides comprising a *PCTA-1*-related biallelic marker in SEQ ID Nos 1, 2, 3, 4, or the complements thereof; wherein said *PCTA-1*-related biallelic marker is selected from the group consisting of A2, A30, A41, A55 and/or A57, and the complements thereof.

The formation of stable hybrids depends on the melting temperature (Tm) of the DNA. The Tm depends on the length of the primer or probe, the ionic strength of the solution and the G+C content. The higher the G+C content of the primer or probe, the higher is the melting temperature because G:C pairs are held by three H bonds whereas A:T pairs have only two. The GC content in the probes of the invention usually ranges between 10 and 75 %, preferably between 35 and 60 %, and more preferably between 40 and 55 %.

A probe or a primer according to the invention has between 8 and 1000 nucleotides in length, or is specified to be at least 12, 15, 18, 20, 25, 35, 40, 50, 60, 70, 80, 100, 250, 500 or 1000 nucleotides in length. More particularly, the length of these probes and primers can range from 8, 10, 15, 20, or 30 to 100 nucleotides, preferably from 10 to 50, more preferably from 15 to 30 nucleotides. Shorter probes and primers tend to lack specificity for a target nucleic acid sequence and generally require cooler temperatures to form sufficiently stable hybrid complexes with the template. Longer probes and primers are expensive to produce and can sometimes self-hybridize to form hairpin structures. The appropriate length for primers and probes under a particular set of assay conditions may be empirically determined by one of skill in the art. A preferred probe or primer consists of a nucleic acid comprising a polynucleotide selected from the group of the nucleotide sequences of P1 to P125 and the complementary sequence thereto, B1 to B47, C1 to C47, D1 to D125, E1 to E125, for which the respective locations in the sequence listing are provided in Tables 1, 2, 3 and 4.

The primers and probes can be prepared by any suitable method, including, for example, cloning and restriction of appropriate sequences and direct chemical synthesis by a method such as the phosphodiester method of Narang et al.(1979), the phosphodiester method of Brown et al.(1979), the diethylphosphoramidite method of Beaucage et al.(1981) and the solid support method described in EP 0 707 592.

Detection probes are generally nucleic acid sequences or uncharged nucleic acid analogs such as, for example peptide nucleic acids which are disclosed in International Patent Application WO 92/20702, morpholino analogs which are described in U.S. Patents Numbered 5,185,444; 5,034,506 and 5,142,047. The probe may have to be rendered "non-extendable" in that additional dNTPs cannot be added to the probe. In and of themselves analogs usually are non-extendable and nucleic acid probes can be rendered non-extendable by modifying the 3' end of the probe such that the hydroxyl group is no longer capable of participating in elongation. For example, the 3' end of the probe can be functionalized with the capture or detection label to thereby consume or otherwise block the hydroxyl group. Alternatively, the 3' hydroxyl group simply can be cleaved, replaced or modified, U.S. Patent Application Serial No. 07/049,061 filed April 19, 1993 describes modifications, which can be used to render a probe non-extendable.

Any of the polynucleotides of the present invention can be labeled, if desired, by incorporating any label known in the art to be detectable by spectroscopic, photochemical, biochemical, immunochemical, or chemical means. For example, useful labels include radioactive substances (including, ³²P, ³⁵S, ³H, ¹²⁵I), fluorescent dyes (including, 5-bromodesoxyuridin, fluorescein, acetylaminofluorene, digoxigenin) or biotin. Preferably, polynucleotides are labeled at their 3' and 5' ends. Examples of non-radioactive labeling of nucleic acid fragments are described in the French patent No. FR-7810975 or by Urdea et al (1988) or Sanchez-Pescador et al (1988). In addition, the probes according to the present invention may have structural characteristics such that they allow the signal amplification, such structural characteristics being, for example, branched DNA probes as those described by Urdea et al. in 1991 or in the European patent No: EP 0 225 807 (Chiron).

A label can also be used to capture the primer, so as to facilitate the immobilization of either the primer or a primer extension product, such as amplified DNA, on a solid support. A capture label is attached to the primers or probes and can be a specific binding member which forms a binding pair with the solid's phase reagent's specific binding member (e.g. biotin and streptavidin). Therefore depending upon the type of label carried by a polynucleotide or a probe, it may be employed to capture or to detect the target DNA. Further, it will be understood that the polynucleotides, primers or probes provided herein, may, themselves, serve as the capture label. For example, in the case where a solid phase reagent's binding member is a nucleic acid sequence, it may be selected such that it binds a complementary portion of a primer or probe to thereby immobilize the primer or probe to the solid phase. In cases where a polynucleotide probe itself serves as the binding member, those skilled in the art will recognize that the probe will contain a sequence or "tail" that is not complementary to the target. In the case where a polynucleotide primer itself serves as the capture label, at least a portion of the primer will be free to hybridize with a nucleic acid on a solid phase. DNA Labeling techniques are well known to the skilled technician.

The probes of the present invention are useful for a number of purposes. They can be notably used in Southern hybridization to genomic DNA. The probes can also be used to detect PCR amplification products. They may also be used to detect mismatches in the *PCTA-1* gene or mRNA using other techniques.

Any of the polynucleotides, primers and probes of the present invention can be conveniently immobilized on a solid support. Solid supports are known to those skilled in the art and include the walls of wells of a reaction tray, test tubes, polystyrene beads, magnetic beads, nitrocellulose strips, membranes, microparticles such as latex particles, sheep (or other animal) red blood cells, duracytes and others. The solid support is not critical and can be selected by one skilled in the art. Thus, latex particles, microparticles, magnetic or non-magnetic beads, membranes, plastic tubes, walls of microtiter wells, glass or silicon chips, sheep (or other suitable animal's) red blood cells and duracytes are all suitable examples. Suitable methods for immobilizing nucleic acids on solid phases include ionic, hydrophobic, covalent interactions and the like. A solid support, as used herein, refers to any material which is insoluble, or can be made insoluble by a subsequent reaction. The solid support can be chosen for its intrinsic ability to attract and immobilize the capture reagent. Alternatively, the solid phase can retain an additional receptor which has the ability to attract and immobilize the capture reagent. The additional receptor can include a charged substance that is oppositely charged with respect to the capture reagent itself or to a charged substance conjugated to the capture reagent. As yet another alternative, the receptor molecule can be any specific binding member which is immobilized upon (attached to) the solid support and which has the ability to immobilize the capture reagent through a specific binding reaction. The receptor molecule enables the indirect binding of the capture reagent to a solid support material before the performance of the assay or during the performance of the assay. The solid phase thus can be a plastic, derivatized plastic, magnetic or non-magnetic metal, glass or silicon surface of a test tube, microtiter well, sheet, bead, microparticle, chip, sheep (or other suitable animal's) red blood cells, duracytes® and other configurations known to those of ordinary skill in the art. The polynucleotides of the invention can be attached to or immobilized on a solid support individually or in groups of at least 2, 5, 8, 10, 12, 15, 20, or 25 distinct polynucleotides of the invention to a single solid support. In addition, polynucleotides other than those of the invention may be attached to the same solid support as one or more polynucleotides of the invention.

Consequently, the invention also deals with a method for detecting the presence of a nucleic acid comprising a nucleotide sequence selected from a group consisting of SEQ ID Nos 1, 2, 3, 4, 8, a fragment or a variant thereof and a complementary sequence thereto in a sample wherein said nucleotide sequence comprises a A2, A30, A41, A55, A57 biallelic marker or the complement thereof, said method comprising the following steps of:
a) bringing into contact a nucleic acid probe or a plurality of nucleic acid probes which can hybridize with a nucleotide sequence included in a nucleic acid selected form the group consisting of the nucleotide sequences of SEQ ID Nos 1, 2, 3, 4, 8, a fragment or a variant thereof and a complementary sequence thereto and the sample to be assayed wherein said nucleotide sequence comprises a A2, A30, A41, A55, A57 biallelic marker or the complement thereof; and
b) detecting the hybrid complex formed between the probe and a nucleic acid in the sample.

The invention further concerns a kit for detecting the presence of a nucleic acid comprising a nucleotide sequence selected from a group consisting of SEQ ID Nos 1, 2, 3, 4, 8, a fragment or a variant thereof and a complementary sequence thereto in a sample wherein said nucleotide sequence comprises a A2, A30, A41, A55, A57 biallelic marker or the complement thereof, said kit comprising:
a) a nucleic acid probe or a plurality of nucleic acid probes which can hybridize with a nucleotide sequence included in a nucleic acid selected form the group consisting of the nucleotide sequences of SEQ ID Nos 1, 2, 3, 4, 8, a fragment or a variant thereof and a complementary sequence thereto wherein said nucleotide sequence comprises a A2, A30, A41 A55, A57 biallelic marker or the complement thereof; and
b) optionally, the reagents necessary for performing the hybridization reaction.

In a first preferred embodiment of this detection method and kit, said nucleic acid probe or the plurality of nucleic acid probes are labeled with a detectable molecule. In a second preferred embodiment of said method and kit, said nucleic acid probe or the plurality of nucleic acid probes has been immobilized on a substrate. The nucleic acid probe or the plurality of nucleic acid probes may comprise either a sequence which is selected from the group consisting of the nucleotide sequences of P1 to P125 and the complementary sequence thereto, B 1 to B47, C1 to C47, D1 to D125, E1 to E125 or a biallelic marker selected from the group consisting of A2, A30, A41, A55 and/or A57 and the complements thereto.

### Oligonucleotide Arrays

A substrate comprising a plurality of oligonucleotide primers or probes of the invention may be used either for detecting or amplifying targeted sequences in the *PCTA-1* gene and may also be used for detecting mutations in the coding or in the non-coding sequences of the *PCTA-1* gene.

Any polynucleotide provided herein may be attached in overlapping areas or at random locations on the solid support. Alternatively the polynucleotides of the invention may be attached in an ordered array wherein each polynucleotide is attached to a distinct region of the solid support which does not overlap with the attachment site of any other polynucleotide. Preferably, such an ordered array of polynucleotides is designed to be "addressable" where the distinct locations are recorded and can be accessed as part of an assay procedure. Addressable polynucleotide arrays typically comprise a plurality of different oligonucleotide probes that are coupled to a surface of a substrate in different known locations. The knowledge of the precise location of each polynucleotides location makes these "addressable" arrays particularly useful in hybridization assays. Any addressable array technology known in the art can be employed with the polynucleotides of the invention. One particular embodiment of these polynucleotide arrays is known as the Genechips^{™}, and has been generally described in US Patent 5,143,854; PCT publications WO 90/15070 and 92/10092. These arrays may generally be produced using mechanical synthesis methods or light directed synthesis methods which incorporate a combination of photolithographic methods and solid phase oligonucleotide synthesis (Fodor et al., 1991). The immobilization of arrays of oligonucleotides on solid supports has been rendered possible by the development of a technology generally identified as "Very Large Scale Immobilized Polymer Synthesis" (VLSIPS^{™}) in which, typically, probes are immobilized in a high density array on a solid surface of a chip. Examples of VLSIPS^{™} technologies are provided in US Patents 5,143,854; and 5,412,087 and in PCT Publications WO 90/15070, WO 92/10092 and WO 95/11995, which describe methods for forming oligonucleotide arrays through techniques such as light-directed synthesis techniques. In designing strategies aimed at providing arrays of nucleotides immobilized on solid supports, further presentation strategies were developed to order and display the oligonucleotide arrays on the chips in an attempt to maximize hybridization patterns and sequence information. Examples of such presentation strategies are disclosed in PCT Publications WO 94/12305, WO 94/11530, WO 97/29212 and WO 97/31256.

In another embodiment of the oligonucleotide arrays of the invention, an oligonucleotide probe matrix may advantageously be used to detect mutations occurring in the *PCTA-1* gene and preferably in its regulatory region. For this particular purpose, probes are specifically designed to have a nucleotide sequence allowing their hybridization to the genes that carry known mutations (either by deletion, insertion or substitution of one or several nucleotides). By known mutations, it is meant, mutations on the *PCTA-1* gene that have been identified according, for example to the technique used by Huang et al.(1996) or Samson et al.(1996).

Another technique that is used to detect mutations in the *PCTA-1* gene is the use of a high-density DNA array. Each oligonucleotide probe constituting a unit element of the high density DNA array is designed to match a specific subsequence of the *PCTA-1* genomic DNA or cDNA. Thus, an array consisting of oligonucleotides complementary to subsequences of the target gene sequence is used to determine the identity of the target sequence with the wild gene sequence, measure its amount, and detect differences between the target sequence and the reference wild gene sequence of the *PCTA-1* gene. In one such design, termed 4L tiled array, is implemented a set of four probes (A, C, G, T), preferably 15-nucleotide oligomers. In each set of four probes, the perfect complement will hybridize more strongly than mismatched probes. Consequently, a nucleic acid target of length L is scanned for mutations with a tiled array containing 4L probes, the whole probe set containing all the possible mutations in the known wild reference sequence. The hybridization signals of the 15-mer probe set tiled array are perturbed by a single base change in the target sequence. As a consequence, there is a characteristic loss of signal or a "footprint" for the probes flanking a mutation position. This technique was described by Chee et al. in 1996.

Consequently, the invention discloses an array of nucleic acid molecules comprising at least one polynucleotide described above as probes and primers. Preferably, the invention concerns an array of nucleic acid comprising at least two polynucleotides described above as probes and primers.

### PCTA-1-Related Biallelic markers

### Advantages Of The Biallelic Markers Of The Present Invention

The *PCTA-1*-related biallelic markers of the present invention offer a number of important advantages over other genetic markers such as RFLP (Restriction fragment length polymorphism) and VNTR (Variable Number of Tandem Repeats) markers.

The first generation of markers, were RFLPs, which are variations that modify the length of a restriction fragment. But methods used to identify and to type RFLPs are relatively wasteful of materials, effort, and time. The second generation of genetic markers were VNTRs, which can be categorized as either minisatellites or microsatellites. Minisatellites are tandemly repeated DNA sequences present in units of 5-50 repeats which are distributed along regions of the human chromosomes ranging from 0.1 to 20 kilobases in length. Since they present many possible alleles, their informative content is very high. Minisatellites are scored by performing Southern blots to identify the number of tandem repeats present in a nucleic acid sample from the individual being tested. However, there are only 10⁴ potential VNTRs that can be typed by Southern blotting. Moreover, both RFLP and VNTR markers are costly and time-consuming to develop and assay in large numbers.

Single nucleotide polymorphism or biallelic markers can be used in the same manner as RFLPs and VNTRs but offer several advantages. SNP are densely spaced in the human genome and represent the most frequent type of variation. An estimated number of more than 10⁷ sites are scattered along the 3x10⁹ base pairs of the human genome. Therefore, SNP occur at a greater frequency and with greater uniformity than RFLP or VNTR markers which means that there is a greater probability that such a marker will be found in close proximity to a genetic locus of interest. SNP are less variable than VNTR markers but are mutationally more stable.

Also, the different forms of a characterized single nucleotide polymorphism, such as the biallelic markers of the present invention, are often easier to distinguish and can therefore be typed easily on a routine basis. Biallelic markers have single nucleotide based alleles and they have only two common alleles, which allows highly parallel detection and automated scoring. The biallelic markers of the present invention offer the possibility of rapid, high throughput genotyping of a large number of individuals.

Biallelic markers are densely spaced in the genome, sufficiently informative and can be assayed in large numbers. The combined effects of these advantages make biallelic markers extremely valuable in genetic studies. Biallelic markers can be used in linkage studies in families, in allele sharing methods, in linkage disequilibrium studies in populations, in association studies of case-control populations or of trait positive and trait negative populations. An important aspect of the present invention is that biallelic markers allow association studies to be performed to identify genes involved in complex traits. Association studies examine the frequency of marker alleles in unrelated case- and control-populations and are generally employed in the detection of polygenic or sporadic traits. Association studies maybe conducted within the general population and are not limited to studies performed on related individuals in affected families (linkage studies). Bialielic markers in different genes can be screened in parallel for direct association with disease or response to a treatment. This multiple gene approach is a powerful tool for a variety of human genetic studies as it provides the necessary statistical power to examine the synergistic effect of multiple genetic factors on a particular phenotype, drug response, sporadic trait, or disease state with a complex genetic etiology.

### PCTA-1-Related Biallelic Markers And Polynucleotides Related Thereto

The invention also discloses a purified and/or isolated *PCTA-1*-related biallelic marker located in the sequence of the *PCTA-1* gene, preferably a biallelic marker comprising an allele associated with prostate cancer, with an early onset of prostate cancer, with a response to a prophylactic or therapeutic agent administered for cancer treatment, particularly prostate cancer, with the level of aggressiveness of prostate cancer tumors, with a modified or forthcoming expression of the *PCTA-1* gene, with a modified or forthcoming production of the PCTA-1 protein, or with the production of a modified PCTA-1 protein. The term *PCTA-1*-related biallelic marker includes the biallelic markers designated A1 to A125. The invention also concerns sets of these biallelic markers.

125 biallelic markers were identified. They include 3 deletions, 6 insertions and 2 variable motifs. 40 biallelic markers, namely A45, A54 to A56, A59 to A61, A75, A76, A85, A93 to A122, were located in exonic region. 39 biallelic markers, namely A44, A46 to A53, A57 to A58, A62 to A74, A77 to A84, A86 to A92, were localized in intronic region of the *PCTA-1* gene. 3 biallelic markers A123, A124 and A125 were in the 3' regulatory region. 43 biallelic markers, namely A1 to A43, were located in the 5' regulatory region. More particularly, 16 of them, namely A28 to A43, were in the promoter of the *PCTA-1* gene.

Among the exonic biallelic markers, 6 of them change the amino acid sequence of a PCTA-1 protein. First, the biallelic marker A54 encodes either a residue tyrosine or phenylalanine. The biallelic marker A56 encodes either a residue cysteine or arginine. The marker A60 encodes either a residue valine or methionine. The marker A75 encodes either a residue aspartic acid or tyrosine. The marker A76 encodes either a leucine residue or a STOP. Finally, the biallelic marker A85 encodes either a residue serine or arginine.

The invention also relates to a purified and/or isolated nucleotide sequence comprising a polymorphic base of a *PCTA-1*-related biallelic marker, selected from the group consisting of A2, A30, A41, A55 and/or A57, and the complements thereof. The sequence has between 8 and 1000 nucleotides in length, and preferably comprises at least 8, 10, 12, 15, 18, 20, 25, 35, 40, 50, 60, 70, 80, 100, 250, 500 or 1000 contiguous nucleotides, to the extent that such lengths are consistent with the specific sequence, of a nucleotide sequence selected from the group consisting of SEQ ID Nos 1, 2, 3, 4, or a variant thereof or a complementary sequence thereto. These nucleotide sequences comprise the polymorphic base of either allele 1 or allele 2 of the considered biallelic marker. Optionally, said biallelic marker may be within 6, 5, 4, 3, 2, or 1 nucleotides of the center of said polynucleotide or at the center of said polynucleotide. Optionally, the 3' end of said contiguous span may be present at the 3' end of said polynucleotide. Optionally, biallelic marker may be present at the 3' end of said polynucleotide. Optionally, the 3' end of said polynucleotide may be located within or at least 2, 4, 6, 8, 10, 12, 15, 18, 20, 25, 50, 100, 250, 500, or 1000 nucleotides upstream of a *PCTA-1*-related biallelic marker in said sequence. Optionally, the 3' end of said polynucleotide may be located 1 nucleotide upstream of a *PCTA-1*-related biallelic marker in said sequence. Optionally, said polynucleotide may further comprise a label. Optionally, said polynucleotide can be attached to solid support. In a further embodiment, the polynucleotides defined above can be used alone or in any combination.

In a preferred embodiment, the sequences comprising a polymorphic base of one of the biallelic markers A2, A30, A41, A55 and/or A57 listed in Table 2 are selected from the group consisting of the nucleotide sequences that have a contiguous span of, that consist of, that are comprised in, or that comprises a polynucleotide having one of the sequences set forth as the amplicons listed in Table 1 or a variant thereof or a complementary sequence thereto.

The invention also relates to a purified and/or isolated nucleotide sequence comprising a sequence defining a biallelic marker selected from the group consisting of A2, A30, A41, A55 and A57 located in the sequence of the *PCTA-1* gene. Preferably, the sequences defining a biallelic marker include the polymorphic base of one of the sequences P1 to P125 or the complementary sequence thereto. In some embodiments, the sequences defining a biallelic marker comprise one of the sequences selected from the group consisting of P1 to P125, or a fragment or variant thereof or a complementary sequence thereto, said fragment comprising the polymorphic base.

The invention also concerns a set of the purified and/or isolated nucleotide sequences defined above. More particularly, the set of purified and/or isolated nucleotide sequences comprises a group of sequences defining a combination of biallelic markers located in the sequence of the *PCTA-1* gene, preferably wherein alleles of said biallelic markers or the combinations thereof are associated with prostate cancer, with the level of aggressiveness of prostate cancer tumors, or with a level of expression of the *PCTA-1* gene.

The invention also encompasses the use of any polynucleotide for, or any polynucleotide for use in, determining the identity of one or more nucleotides at a *PCTA-1*-related biallelic marker. In addition, the polynucleotides of the invention for use in determining the identity of one or more nucleotides at a *PCTA-1*-related biallelic marker encompass polynucleotides with any further limitation described in this disclosure, or those following, specified alone or in any combination, wherein said *PCTA-1*-related biallelic marker is selected from the group consisting of A2, A30, A41, A55 and/or A57, and the complements thereof. Optionally, said polynucleotide may comprise a sequence disclosed in the present specification; Optionally, said polynucleotide may consist of, or consist essentially of any polynucleotide described in the present specification; Optionally, said determining may be performed in a hybridization assay, a sequencing assay, a microsequencing assay, or an enzyme-based mismatch detection assay; A preferred polynucleotide may be used in a hybridization assay for determining the identity of the nucleotide at a *PCTA-1*-related biallelic marker. Another preferred polynucleotide may be used in a sequencing or microsequencing assay for determining the identity of the nucleotide at a *PCTA-1*-related biallelic marker. A third preferred polynucleotide may be used in an enzyme-based mismatch detection assay for determining the identity of the nucleotide at a *PCTA-1*-related biallelic marker. A fourth preferred polynucleotide may be used in amplifying a segment of polynucleotides comprising a *PCTA-1*-related biallelic marker. Optionally, any of the polynucleotides described above may be attached to a solid support, array, or addressable array; Optionally, said polynucleotide may be labeled.

The probes of the present invention may be designed from the disclosed sequences for any method known in the art, particularly methods which allow for testing if a marker disclosed herein is present. A preferred set of probes may be designed for use in the hybridization assays of the invention in any manner known in the art such that they selectively bind to one allele of a biallelic marker, but not the other under any particular set of assay conditions. Preferred hybridization probes comprise the polymorphic base of either allele 1 or allele 2 of the considered biallelic marker. Optionally, said biallelic marker may be within 6, 5, 4, 3, 2, or 1 nucleotides of the center of the hybridization probe or at the center of said probe. In a preferred embodiment, the probes are selected in the group consisting of the sequences P1 to P125 and the complementary sequence thereto.

It should be noted that the polynucleotides of the present invention are not limited to having the exact flanking sequences surrounding the polymorphic bases which are enumerated in Sequence Listing. Rather, it will be appreciated that the flanking sequences surrounding the biallelic markers may be lengthened or shortened to any extent compatible with their intended use and the present invention specifically contemplates such sequences. The flanking regions outside of the contiguous span need not be homologous to native flanking sequences which actually occur in human subjects. The addition of any nucleotide sequence which is compatible with the nucleotides intended use is specifically contemplated.

Primers and probes may be labeled or immobilized on a solid support as described in " Oligonucleotide probes and primers".

The polynucleotides of the invention which are attached to a solid support encompass polynucleotides with any further limitation described in this disclosure, or those following, specified alone or in any combination: Optionally, said polynucleotides may be specified as attached individually or in groups of at least 2, 5, 8, 10, 12, 15, 20, or 25 distinct polynucleotides of the invention to a single solid support. Optionally, polynucleotides other than those of the invention may attached to the same solid support as polynucleotides of the invention. Optionally, when multiple polynucleotides are attached to a solid support they may be attached at random locations, or in an ordered array. Optionally, said ordered array may be addressable.

The present invention also encompasses diagnostic kits comprising one or more polynucleotides of the invention with a portion or all of the necessary reagents and instructions for genotyping a test subject by determining the identity of a nucleotide at a *PCTA-1*-related biallelic marker. The polynucleotides of a kit may optionally be attached to a solid support, or be part of an array or addressable array of polynucleotides. The kit may provide for the determination of the identity of the nucleotide at a marker position by any method known in the art including, but not limited to, a sequencing assay method, a microsequencing assay method, a hybridization assay method, or an enzyme-based mismatch detection assay method.

### Methods For Genotyping An Individual For Biallelic Markers

Methods are provided to genotype a biological sample for one or more biallelic markers of the present invention, all of which may be performed *in vitro.* Such methods of genotyping comprise determining the identity of a nucleotide at a *PCTA-1* biallelic marker selected from the group consisting of A2, A30, A41, A55 and A57 site by any method known in the art. These methods find use in genotyping case-control populations in association studies as well as individuals in the context of detection of alleles of biallelic markers which are known to be associated with a given trait, in which case both copies of the biallelic marker present in individual's genome are determined so that an individual may be classified as homozygous or heterozygous for a particular allele.

These genotyping methods can be performed on nucleic acid samples derived from a single individual or pooled DNA samples.

Genotyping can be performed using similar methods as those described above for the identification of the biallelic markers, or using other genotyping methods such as those further described below. In preferred embodiments, the comparison of sequences of amplified genomic fragments from different individuals is used to identify new biallelic markers whereas microsequencing is used for genotyping known biallelic markers in diagnostic and association study applications.

In one embodiment the invention encompasses methods of genotyping comprising determining the identity of a nucleotide at a *PCTA-1-*related biallelic marker selected from the group consisting of A2, A30, A41, A55 and A57 or the complement thereof in a biological sample; optionally, wherein said biological sample is derived from a single subject; optionally, wherein the identity of the nucleotides at said biallelic marker is determined for both copies of said biallelic marker present in said individual's genome; optionally, wherein said biological sample is derived from multiple subjects; Optionally, the genotyping methods of the invention encompass methods with any further limitation described in this disclosure, or those following, specified alone or in any combination; Optionally, said method is performed *in vitro*; optionally, further comprising amplifying a portion of said sequence comprising the biallelic marker prior to said determining step; Optionally, wherein said amplifying is performed by PCR, LCR, or replication of a recombinant vector comprising an origin of replication and said fragment in a host cell; optionally, wherein said determining is performed by a hybridization assay, a sequencing assay, a microsequencing assay, or an enzyme-based mismatch detection assay.

### Source of Nucleic Acids for genotyping

Any source of nucleic acids, in purified or non-purified form, can be utilized as the starting nucleic acid, provided it contains or is suspected of containing the specific nucleic acid sequence desired. DNA or RNA may be extracted from cells, tissues, body fluids and the like as described above. While nucleic acids for use in the genotyping methods of the invention can be derived from any mammalian source, the test subjects and individuals from which nucleic acid samples are taken are generally understood to be human.

### Amplification Of DNA Fragments Comprising Biallelic Markers

Methods and polynucleotides are provided to amplify a segment of nucleotides comprising one or more biallelic marker of the present invention. It will be appreciated that amplification of DNA fragments comprising biallelic markers may be used in various methods and for various purposes and is not restricted to genotyping. Nevertheless, many genotyping methods, although not all, require the previous amplification of the DNA region carrying the biallelic marker of interest. Such methods specifically increase the concentration or total number of sequences that span the biallelic marker or include that site and sequences located either distal or proximal to it. Diagnostic assays may also rely on amplification of DNA segments carrying a biallelic marker of the present invention. Amplification of DNA may be achieved by any method known in the art. Amplification techniques are described above in the section entitled, "DNA amplification."

The invention also concerns a method for the amplification of a *PCTA-1* gene region, preferably containing at least one of the polymorphic bases identified in the context of the present invention, or a fragment or variant thereof, in a test sample. The method comprises the step of contacting a test sample suspected of containing the targeted *PCTA-1* gene sequence or a fragment thereof with amplification reaction reagents comprising a pair of amplification primers, preferably located on either side of the polymorphic base. The method may further comprise the step of detecting the amplification product. For example, the amplification product may be detected using a detection probe that can hybridize with an internal region of the amplicon sequences.

Some of these amplification methods are particularly suited for the detection of single nucleotide polymorphisms and allow the simultaneous amplification of a target sequence and the identification of the polymorphic nucleotide as it is further described below.

The identification of biallelic markers as described above allows the design of appropriate oligonucleotides, which can be used as primers to amplify DNA fragments comprising the biallelic markers of the present invention. Amplification can be performed using the primers initially used to discover new biallelic markers which are described herein or any set of primers allowing the amplification of a DNA fragment comprising a biallelic marker of the present invention.

In some embodiments the present invention provides primers for amplifying a DNA fragment containing one or more biallelic markers of the present invention. Preferred amplification primers are listed in Example 2. It will be appreciated that the primers listed are merely exemplary and that any other set of primers which produce amplification products containing one or more biallelic markers of the present invention are also of use.

The spacing of the primers determines the length of the segment to be amplified. In the context of the present invention, amplified segments carrying biallelic markers can range in size from at least about 25 bp to 35 kbp. Amplification fragments from 25-3000 bp are typical, fragments from 50-1000 bp are preferred and fragments from 100-600 bp are highly preferred. It will be appreciated that amplification primers for the biallelic markers may be any sequence which allow the specific amplification of any DNA fragment carrying the markers. Amplification primers may be labeled or immobilized on a solid support as described in "Oligonucleotide probes and primers".

### Methods of Genotyping DNA samples for Biallelic Markers

Any method known in the art can be used to identify the nucleotide present at a biallelic marker site. Since the biallelic marker allele to be detected has been identified and specified in the present invention, detection will prove simple for one of ordinary skill in the art by employing any of a number of techniques. Many genotyping methods require the previous amplification of the DNA region carrying the biallelic marker of interest. While the amplification of target or signal is often preferred at present, ultrasensitive detection methods which do not require amplification are also encompassed by the present genotyping methods. Methods well-known to those skilled in the art that can be used to detect biallelic polymorphisms include methods such as, conventional dot blot analyzes, single strand conformational polymorphism analysis (SSCP) described by Orita et al.(1989), denaturing gradient gel electrophoresis (DGGE), heteroduplex analysis, mismatch cleavage detection, and other conventional techniques as described in Sheffield et al.(1991), White et al.(1992), Grompe et al.(1989 and 1993). Another method for determining the identity of the nucleotide present at a particular polymorphic site employs a specialized exonuclease-resistant nucleotide derivative as described in US patent 4,656,127.

Preferred methods involve directly determining the identity of the nucleotide present at a biallelic marker site by sequencing assay, enzyme-based mismatch detection assay, or hybridization assay. The following is a description of some preferred methods. A highly preferred method is the microsequencing technique. The term "sequencing" is generally used herein to refer to polymerase extension of duplex primer/template complexes and includes both traditional sequencing and microsequencing.

### 1) Sequencing Assays

The nucleotide present at a polymorphic site can be determined by sequencing methods. In a preferred embodiment, DNA samples are subjected to PCR amplification before sequencing as described above. DNA sequencing methods are described in "Sequencing Of Amplified Genomic DNA And Identification Of Single Nucleotide Polymorphisms".

Preferably, the amplified DNA is subjected to automated dideoxy terminator sequencing reactions using a dye-primer cycle sequencing protocol. Sequence analysis allows the identification of the base present at the biallelic marker site.

### 2) Microsequencing Assays

In microsequencing methods, the nucleotide at a polymorphic site in a target DNA is detected by a single nucleotide primer extension reaction. This method involves appropriate microsequencing primers which, hybridize just upstream of the polymorphic base of interest in the target nucleic acid. A polymerase is used to specifically extend the 3' end of the primer with one single ddNTP (chain terminator) complementary to the nucleotide at the polymorphic site. Next the identity of the incorporated nucleotide is determined in any suitable way.

Typically, microsequencing reactions are carried out using fluorescent ddNTPs and the extended microsequencing primers are analyzed by electrophoresis on ABI 377 sequencing machines to determine the identity of the incorporated nucleotide as described in EP 412 883, the disclosure of which is incorporated herein by reference in its entirety. Alternatively capillary electrophoresis can be used in order to process a higher number of assays simultaneously. An example of a typical microsequencing procedure that can be used in the context of the present invention is provided in Example 4.

Different approaches can be used for the labeling and detection of ddNTPs. A homogeneous phase detection method based on fluorescence resonance energy transfer has been described by Chen and Kwok (1997) and Chen et al.(1997). In this method, amplified genomic DNA fragments containing polymorphic sites are incubated with a 5'-fluorescein-labeled primer in the presence of allelic dye-labeled dideoxyribonucleoside triphosphates and a modified Taq polymerase. The dye-labeled primer is extended one base by the dye-terminator specific for the allele present on the template. At the end of the genotyping reaction, the fluorescence intensities of the two dyes in the reaction mixture are analyzed directly without separation or purification. All these steps can be performed in the same tube and the fluorescence changes can be monitored in real time. Alternatively, the extended primer may be analyzed by MALDI-TOF Mass Spectrometry. The base at the polymorphic site is identified by the mass added onto the microsequencing primer (see Haff and Smirnov, 1997).

Microsequencing may be achieved by the established microsequencing method or by developments or derivatives thereof. Alternative methods include several solid-phase microsequencing techniques. The basic microsequencing protocol is the same as described previously, except that the method is conducted as a heterogeneous phase assay, in which the primer or the target molecule is immobilized or captured onto a solid support. To simplify the primer separation and the terminal nucleotide addition analysis, oligonucleotides are attached to solid supports or are modified in such ways that permit affinity separation as well as polymerase extension. The 5' ends and internal nucleotides of synthetic oligonucleotides can be modified in a number of different ways to permit different affinity separation approaches, e.g., biotinylation. If a single affinity group is used on the oligonucleotides, the oligonucleotides can be separated from the incorporated terminator regent. This eliminates the need of physical or size separation. More than one oligonucleotide can be separated from the terminator reagent and analyzed simultaneously if more than one affinity group is used. This permits the analysis of several nucleic acid species or more nucleic acid sequence information per extension reaction. The affinity group need not be on the priming oligonucleotide but could alternatively be present on the template. For example, immobilization can be carried out via an interaction between biotinylated DNA and streptavidin-coated microtitration wells or avidin-coated polystyrene particles. In the same manner, oligonucleotides or templates may be attached to a solid support in a high-density format. In such solid phase microsequencing reactions, incorporated ddNTPs can be radiolabeled (Syvänen, 1994) or linked to fluorescein (Livak and Hainer, 1994). The detection of radiolabeled ddNTPs can be achieved through scintillation-based techniques. The detection of fluorescein-linked ddNTPs can be based on the binding of antifluorescein antibody conjugated with alkaline phosphatase, followed by incubation with a chromogenic substrate (such as *p*-nitrophenyl phosphate). Other possible reporter-detection pairs include: ddNTP linked to dinitrophenyl (DNP) and anti-DNP alkaline phosphatase conjugate (Harju et al., 1993) or biotinylated ddNTP and horseradish peroxidase-conjugated streptavidin with *o*-phenylenediamine as a substrate (WO 92/15712). As yet another alternative solid-phase microsequencing procedure, Nyren et al.(1993) described a method relying on the detection of DNA polymerase activity by an enzymatic luminometric inorganic pyrophosphate detection assay (ELIDA).

Pastinen et al.(1997) describe a method for multiplex detection of single nucleotide polymorphism in which the solid phase minisequencing principle is applied to an oligonucleotide array format. High-density arrays of DNA probes attached to a solid support (DNA chips) are further described below.

In one aspect the present invention provides polynucleotides and methods to genotype one or more biallelic markers of the present invention by performing a microsequencing assay. It will be appreciated that the microsequencing primers listed in Example 4 are merely exemplary and that, any primer having a 3' end immediately adjacent to the polymorphic nucleotide may be used. Similarly, it will be appreciated that microsequencing analysis may be performed for any biallelic marker or any combination of biallelic markers of the present invention. One aspect of the present invention is a solid support which includes one or more microsequencing primers listed in Example 4, or fragments comprising at least 8, 12, 15, 20, 25, 30, 40, or 50 consecutive nucleotides thereof, to the extent that such lengths are consistent with the primer described, and having a 3' terminus immediately upstream of the corresponding biallelic marker, for determining the identity of a nucleotide at a biallelic marker site.

### 3) Mismatch detection assays based on polymerases and ligases

In one aspect the present invention provides polynucleotides and methods to determine the allele of one or more biallelic markers of the present invention in a biological sample, by mismatch detection assays based on polymerases and/or ligases. These assays are based on the specificity of polymerases and ligases. Polymerization reactions places particularly stringent requirements on correct base pairing of the 3' end of the amplification primer and the joining of two oligonucleotides hybridized to a target DNA sequence is quite sensitive to mismatches close to the ligation site, especially at the 3' end. Methods, primers and various parameters to amplify DNA fragments comprising biallelic markers of the present invention are further described above in "Amplification Of DNA Fragments Comprising Biallelic Marker".

### Allele Specific Amplification Primers

Discrimination between the two alleles of a biallelic marker can also be achieved by allele specific amplification, a selective strategy, whereby one of the alleles is amplified without amplification of the other allele. For allele specific amplification, at least one member of the pair of primers is sufficiently complementary with a region of a *PCTA-1* gene comprising the polymorphic base of a biallelic marker of the present invention to hybridize therewith and to initiate the amplification. Such primers are able to discriminate between the two alleles of a biallelic marker.

This is accomplished by placing the polymorphic base at the 3' end of one of the amplification primers. Because the extension forms from the 3'end of the primer, a mismatch at or near this position has an inhibitory effect on amplification. Therefore, under appropriate amplification conditions, these primers only direct amplification on their complementary allele. Determining the precise location of the mismatch and the corresponding assay conditions are well within the ordinary skill in the art.

### Ligation/Amplification Based Methods

The "Oligonucleotide Ligation Assay" (OLA) uses two oligonucleotides which are designed to be capable of hybridizing to abutting sequences of a single strand of a target molecules. One of the oligonucleotides is biotinylated, and the other is detectably labeled. If the precise complementary sequence is found in a target molecule, the oligonucleotides will hybridize such that their termini abut, and create a ligation substrate that can be captured and detected. OLA is capable of detecting single nucleotide polymorphisms and may be advantageously combined with PCR as described by Nickerson et al.(1990). In this method, PCR is used to achieve the exponential amplification of target DNA, which is then detected using OLA.

Other amplification methods which are particularly suited for the detection of single nucleotide polymorphism include LCR (ligase chain reaction), Gap LCR (GLCR) which are described above in "DNA Amplification". LCR uses two pairs of probes to exponentially amplify a specific target. The sequences of each pair of oligonucleotides, is selected to permit the pair to hybridize to abutting sequences of the same strand of the target. Such hybridization forms a substrate for a template-dependant ligase. In accordance with the present invention, LCR can be performed with oligonucleotides having the proximal and distal sequences of the same strand of a biallelic marker site. In one embodiment, either oligonucleotide will be designed to include the biallelic marker site. In such an embodiment, the reaction conditions are selected such that the oligonucleotides can be ligated together only if the target molecule either contains or lacks the specific nucleotide that is complementary to the biallelic marker on the oligonucleotide. In an alternative embodiment, the oligonucleotides will not include the biallelic marker, such that when they hybridize to the target molecule, a "gap" is created as described in WO 90/01069. This gap is then "filled" with complementary dNTPs (as mediated by DNA polymerase), or by an additional pair of oligonucleotides. Thus at the end of each cycle, each single strand has a complement capable of serving as a target during the next cycle and exponential allele-specific amplification of the desired sequence is obtained.

Ligase/Polymerase-mediated Genetic Bit Analysis^{™} is another method for determining the identity of a nucleotide at a preselected site in a nucleic acid molecule (WO 95/21271). This method involves the incorporation of a nucleoside triphosphate that is complementary to the nucleotide present at the preselected site onto the terminus of a primer molecule, and their subsequent ligation to a second oligonucleotide. The reaction is monitored by detecting a specific label attached to the reaction's solid phase or by detection in solution.

### 4) Hybridization Assay Methods

A preferred method of determining the identity of the nucleotide present at a biallelic marker site involves nucleic acid hybridization. The hybridization probes, which can be conveniently used in such reactions, preferably include the probes defined herein. Any hybridization assay may be used including Southern hybridization, Northern hybridization, dot blot hybridization and solid-phase hybridization (see Sambrook et al., 1989).

Hybridization refers to the formation of a duplex structure by two single stranded nucleic acids due to complementary base pairing. Hybridization can occur between exactly complementary nucleic acid strands or between nucleic acid strands that contain minor regions of mismatch. Specific probes can be designed that hybridize to one form of a biallelic marker and not to the other and therefore are able to discriminate between different allelic forms. Allele-specific probes are often used in pairs, one member of a pair showing perfect match to a target sequence containing the original allele and the other showing a perfect match to the target sequence containing the alternative allele. Hybridization conditions should be sufficiently stringent that there is a significant difference in hybridization intensity between alleles, and preferably an essentially binary response, whereby a probe hybridizes to only one of the alleles. Stringent, sequence specific hybridization conditions, under which a probe will hybridize only to the exactly complementary target sequence are well known in the art (Sambrook et al., 1989). Stringent conditions are sequence dependent and will be different in different circumstances. Generally, stringent conditions are selected to be about 5°C lower than the thermal melting point (Tm) for the specific sequence at a defined ionic strength and pH. Although such hybridization can be performed in solution, it is preferred to employ a solid-phase hybridization assay. The target DNA comprising a biallelic marker of the present invention may be amplified prior to the hybridization reaction. The presence of a specific allele in the sample is determined by detecting the presence or the absence of stable hybrid duplexes formed between the probe and the target DNA. The detection of hybrid duplexes can be carried out by a number of methods. Various detection assay formats are well known which utilize detectable labels bound to either the target or the probe to enable detection of the hybrid duplexes. Typically, hybridization duplexes are separated from unhybridized nucleic acids and the labels bound to the duplexes are then detected. Those skilled in the art will recognize that wash steps may be employed to wash away excess target DNA or probe as well as unbound conjugate. Further, standard heterogeneous assay formats are suitable for detecting the hybrids using the labels present on the primers and probes.

Two recently developed assays allow hybridization-based allele discrimination with no need for separations or washes (see Landegren U. et al., 1998). The TaqMan assay takes advantage of the 5' nuclease activity of Taq DNA polymerase to digest a DNA probe annealed specifically to the accumulating amplification product. TaqMan probes are labeled with a donor-acceptor dye pair that interacts via fluorescence energy transfer. Cleavage of the TaqMan probe by the advancing polymerase during amplification dissociates the donor dye from the quenching acceptor dye, greatly increasing the donor fluorescence. All reagents necessary to detect two allelic variants can be assembled at the beginning of the reaction and the results are monitored in real time (see Livak et al., 1995). In an alternative homogeneous hybridization based procedure, molecular beacons are used for allele discriminations. Molecular beacons are hairpin-shaped oligonucleotide probes that report the presence of specific nucleic acids in homogeneous solutions. When they bind to their targets they undergo a conformational reorganization that restores the fluorescence of an internally quenched fluorophore (Tyagi et al., 1998).

The polynucleotides provided herein can be used to produce probes which can be used in hybridization assays for the detection of biallelic marker alleles in biological samples. These probes are characterized in that they preferably comprise between 8 and 50 nucleotides, and in that they are sufficiently complementary to a sequence comprising a biallelic marker of the present invention to hybridize thereto and preferably sufficiently specific to be able to discriminate the targeted sequence for only one nucleotide variation. A particularly preferred probe is 25 nucleotides in length. Another particularly preferred probe is 47 nucleotides in length. Preferably the biallelic marker is within 4 nucleotides of the center of the polynucleotide probe. In particularly preferred probes, the biallelic marker is at the center of said polynucleotide. Preferred probes comprise a nucleotide sequence selected from the group consisting of amplicons listed in Table I and the sequences complementary thereto, or a fragment thereof, said fragment comprising at least about 8 consecutive nucleotides, preferably 10, 15, 20, more preferably 25, 30, 40, 47, or 50 consecutive nucleotides and containing a polymorphic base. In preferred embodiments the polymorphic base(s) are within 5, 4, 3, 2, 1, nucleotides of the center of the said polynucleotide, more preferably at the center of said polynucleotide.

Preferably the probes of the present invention are labeled or immobilized on a solid support. Labels and solid supports are further described in "Oligonucleotide Probes and Primers". The probes can be non-extendable as described in "Oligonucleotide Probes and Primers".

By assaying the hybridization to an allele specific probe, one can detect the presence or absence of a biallelic marker allele in a given sample. High-Throughput parallel hybridization in array format is specifically encompassed within "hybridization assays" and are described below.

### 5) Hybridization To Addressable Arrays Of Oligonucleotides

Hybridization assays based on oligonucleotide arrays rely on the differences in hybridization stability of short oligonucleotides to perfectly matched and mismatched target sequence variants. Efficient access to polymorphism information is obtained through a basic structure comprising high-density arrays of oligonucleotide probes attached to a solid support (e.g., the chip) at selected positions. Each DNA chip can contain thousands to millions of individual synthetic DNA probes arranged in a grid-like pattern and miniaturized to the size of a dime.

The chip technology has already been applied with success in numerous cases. For example, the screening of mutations has been undertaken in the BRCA1 gene, in *S. cerevisiae* mutant strains, and in the protease gene of HIV-1 virus (Hacia et al., 1996; Shoemaker et al., 1996; Kozal et al., 1996). Chips of various formats for use in detecting biallelic polymorphisms can be produced on a customized basis by Affymetrix (GeneChip^{™}), Hyseq (HyChip and HyGnostics), and Protogene Laboratories.

In general, these methods employ arrays of oligonucleotide probes that are complementary to target nucleic acid sequence segments from an individual which, target sequences include a polymorphic marker. EP 785280 describes a tiling strategy for the detection of single nucleotide polymorphisms. Briefly, arrays may generally be "tiled" for a large number of specific polymorphisms. By "tiling" is generally meant the synthesis of a defined set of oligonucleotide probes which is made up of a sequence complementary to the target sequence of interest, as well as preselected variations of that sequence, e.g., substitution of one or more given positions with one or more members of the basis set of nucleotides. Tiling strategies are further described in PCT application No. WO 95/11995. In a particular aspect, arrays are tiled for a number of specific, identified biallelic marker sequences. In particular, the array is tiled to include a number of detection blocks, each detection block being specific for a specific biallelic marker or a set of biallelic markers. For example, a detection block may be tiled to include a number of probes, which span the sequence segment that includes a specific polymorphism. To ensure probes that are complementary to each allele, the probes are synthesized in pairs differing at the biallelic marker. In addition to the probes differing at the polymorphic base, monosubstituted probes are also generally tiled within the detection block. These monosubstituted probes have bases at and up to a certain number of bases in either direction from the polymorphism, substituted with the remaining nucleotides (selected from A, T, G, C and U). Typically the probes in a tiled detection block will include substitutions of the sequence positions up to and including those that are 5 bases away from the biallelic marker. The monosubstituted probes provide internal controls for the tiled array, to distinguish actual hybridization from artefactual cross-hybridization. Upon completion of hybridization with the target sequence and washing of the array, the array is scanned to determine the position on the array to which the target sequence hybridizes. The hybridization data from the scanned array is then analyzed to identify which allele or alleles of the biallelic marker are present in the sample. Hybridization and scanning may be carried out as described in PCT application No. WO 92/10092 and WO 95/11995 and US patent No. 5,424,186.

Thus, in some embodiments, the chips may comprise an array of nucleic acid sequences of fragments of about 15 nucleotides in length. In further embodiments, the chip may comprise an array including at least one of the sequences selected from the group consisting of amplicons listed in table 1 and the sequences complementary thereto, or a fragment thereof, said fragment comprising at least about 8 consecutive nucleotides, preferably 10, 15, 20, more preferably 25, 30, 40, 47, or 50 consecutive nucleotides and containing a polymorphic base. In preferred embodiments the polymorphic base is within 5, 4, 3, 2, 1, nucleotides of the center of the said polynucleotide, more preferably at the center of said polynucleotide. In some embodiments, the chip may comprise an array of at least 2, 3, 4, 5, 6, 7, 8 or more of these polynucleotides of the invention. Solid supports and polynucleotides of the present invention attached to solid supports are further described in "Oligonucleotide Probes And Primers".

### 6) Integrated Systems

Another technique, which may be used to analyze polymorphisms, includes multicomponent integrated systems, which miniaturize and compartmentalize processes such as PCR and capillary electrophoresis reactions in a single functional device. An example of such technique is disclosed in US patent 5,589,136, which describes the integration of PCR amplification and capillary electrophoresis in chips.

Integrated systems can be envisaged mainly when microfluidic systems are used. These systems comprise a pattern of microchannels designed onto a glass, silicon, quartz, or plastic wafer included on a microchip. The movements of the samples are controlled by electric, electroosmotic or hydrostatic forces applied across different areas of the microchip to create functional microscopic valves and pumps with no moving parts.

For genotyping biallelic markers, the microfluidic system may integrate nucleic acid amplification, microsequencing, capillary electrophoresis and a detection method such as laser-induced fluorescence detection.

### Methods Of Genetic Analysis Using The Biallelic Markers Of The Present Invention

Different methods are available for the genetic analysis of complex traits (see Lander and Schork, 1994). The search for disease-susceptibility genes is conducted using two main methods: the linkage approach in which evidence is sought for cosegregation between a locus and a putative trait locus using family studies, and the association approach in which evidence is sought for a statistically significant association between an allele or a trait causing allele and a trait (Khoury et al., 1993). In general, the biallelic markers of the present invention find use in any method known in the art to demonstrate a statistically significant correlation between a genotype and a phenotype. The biallelic markers may be used in parametric and non-parametric linkage analysis methods. Preferably, the biallelic markers of the present invention are used to identify genes associated with detectable traits using association studies, an approach which does not require the use of affected families and which permits the identification of genes associated with complex and sporadic traits.

The genetic analysis using the biallelic markers of the present invention may be conducted on any scale. The whole set of biallelic markers of the present invention or any subset of biallelic markers of the present invention corresponding to the candidate gene may be used. Further, any set of genetic markers including a biallelic marker of the present invention may be used. A set of biallelic polymorphisms that could be used as genetic markers in combination with the biallelic markers of the present invention has been described in WO 98/20165. As mentioned above, it should be noted that the biallelic markers of the present invention may be included in any complete or partial genetic map of the human genome. These different uses are specifically contemplated in the present invention and claims.

### Linkage Analysis

Linkage analysis is based upon establishing a correlation between the transmission of genetic markers and that of a specific trait throughout generations within a family. Thus, the aim of linkage analysis is to detect marker loci that show cosegregation with a trait of interest in pedigrees.

### Parametric Methods

When data are available from successive generations there is the opportunity to study the degree of linkage between pairs of loci. Estimates of the recombination fraction enable loci to be ordered and placed onto a genetic map. With loci that are genetic markers, a genetic map can be established, and then the strength of linkage between markers and traits can be calculated and used to indicate the relative positions of markers and genes affecting those traits (Weir, 1996). The classical method for linkage analysis is the logarithm of odds (lod) score method (see Morton, 1955; Ott, 1991). Calculation of lod scores requires specification of the mode of inheritance for the disease (parametric method). Generally, the length of the candidate region identified using linkage analysis is between 2 and 20Mb. Once a candidate region is identified as described above, analysis of recombinant individuals using additional markers allows further delineation of the candidate region. Linkage analysis studies have generally relied on the use of a maximum of 5,000 microsatellite markers, thus limiting the maximum theoretical attainable resolution of linkage analysis to about 600 kb on average.

Linkage analysis has been successfully applied to map simple genetic traits that show clear Mendelian inheritance patterns and which have a high penetrance (i.e., the ratio between the number of trait positive carriers of allele a and the total number of a carriers in the population). However, parametric linkage analysis suffers from a variety of drawbacks. First, it is limited by its reliance on the choice of a genetic model suitable for each studied trait. Furthermore, as already mentioned, the resolution attainable using linkage analysis is limited, and complementary studies are required to refine the analysis of the typical 2Mb to 20Mb regions initially identified through linkage analysis. In addition, parametric linkage analysis approaches have proven difficult when applied to complex genetic traits, such as those due to the combined action of multiple genes and/or environmental factors. It is very difficult to model these factors adequately in a lod score analysis. In such cases, too large an effort and cost are needed to recruit the adequate number of affected families required for applying linkage analysis to these situations, as recently discussed by Risch, N. and Merikangas, K. (1996).

### Non-Parametric Methods

The advantage of the so-called non-parametric methods for linkage analysis is that they do not require specification of the mode of inheritance for the disease, they tend to be more useful for the analysis of complex traits. In non-parametric methods, one tries to prove that the inheritance pattern of a chromosomal region is not consistent with random Mendelian segregation by showing that affected relatives inherit identical copies of the region more often than expected by chance. Affected relatives should show excess "allele sharing" even in the presence of incomplete penetrance and polygenic inheritance. In non-parametric linkage analysis the degree of agreement at a marker locus in two individuals can be measured either by the number of alleles identical by state (IBS) or by the number of alleles identical by descent (IBD). Affected sib pair analysis is a well-known special case and is the simplest form of these methods.

The biallelic markers of the present invention may be used in both parametric and non-parametric linkage analysis. Preferably biallelic markers may be used in non-parametric methods which allow the mapping of genes involved in complex traits. The biallelic markers of the present invention may be used in both IBD- and IBS- methods to map genes affecting a complex trait. In such studies, taking advantage of the high density of biallelic markers, several adjacent biallelic marker loci may be pooled to achieve the efficiency attained by multi-allelic markers (Zhao et al., 1998).

### Population Association Studies

The present invention comprises methods for identifying if the *PCTA-1* gene is associated with a detectable trait using the biallelic markers of the present invention. In one embodiment the present invention comprises methods to detect an association between a biallelic marker allele or a biallelic marker haplotype and a trait. Further, the invention comprises methods to identify a trait causing allele in linkage disequilibrium with any biallelic marker allele of the present invention.

Alternative approaches can be employed to perform association studies: genome-wide association studies, candidate region association studies and candidate gene association studies. In a preferred embodiment, the biallelic markers of the present invention are used to perform candidate gene association studies. The candidate gene analysis clearly provides a short-cut approach to the identification of genes and gene polymorphisms related to a particular trait when some information concerning the biology of the trait is available. Further, the biallelic markers of the present invention may be incorporated in any map of genetic markers of the human genome in order to perform genome-wide association studies. Methods to generate a high-density map of biallelic markers has been described in US Provisional Patent application serial number 60/082,614. The biallelic markers of the present invention may further be incorporated in any map of a specific candidate region of the genome (a specific chromosome or a specific chromosomal segment for example).

As mentioned above, association studies may be conducted within the general population and are not limited to studies performed on related individuals in affected families. Association studies are extremely valuable as they permit the analysis of sporadic or multifactor traits. Moreover, association studies represent a powerful method for fine-scale mapping enabling much finer mapping of trait causing alleles than linkage studies. Studies based on pedigrees often only narrow the location of the trait causing allele. Association studies using the biallelic markers of the present invention can therefore be used to refine the location of a trait causing allele in a candidate region identified by Linkage Analysis methods. Moreover, once a chromosome segment of interest has been identified, the presence of a candidate gene such as a candidate gene of the present invention, in the region of interest can provide a shortcut to the identification of the trait causing allele. Biallelic markers of the present invention can be used to demonstrate that a candidate gene is associated with a trait. Such uses are specifically contemplated in the present invention.

### Determining The Frequency Of A Biallelic Marker Allele Or Of A Biallelic Marker Haplotype In A Population

Association studies explore the relationships among frequencies for sets of alleles between loci.

### Determining The Frequency Of An Allele In A Population

Allelic frequencies of the biallelic markers in a populations can be determined using one of the methods described above under the heading "Methods For Genotyping An Individual For Biallelic Markers", or any genotyping procedure suitable for this intended purpose. Genotyping pooled samples or individual samples can determine the frequency of a biallelic marker allele in a population. One way to reduce the number of genotypings required is to use pooled samples. A major obstacle in using pooled samples is in terms of accuracy and reproducibility for determining accurate DNA concentrations in setting up the pools. Genotyping individual samples provides higher sensitivity, reproducibility and accuracy and; is the preferred method used in the present invention. Preferably, each individual is genotyped separately and simple gene counting is applied to determine the frequency of an allele of a biallelic marker or of a genotype in a given population.

The invention also relates to methods of estimating the frequency of a *PCTA-1*-related biallelic marker allele in a population comprising: a) genotyping individuals from said population for said biallelic marker according to the method of the present invention; and b) determining the proportional representation of said biallelic marker in said population. In addition, the methods of estimating the frequency of an allele in a population of the invention encompass methods with any further limitation described in this disclosure, or those following, specified alone or in any combination, wherein said *PCTA-1*-related biallelic marker is selected from the group consisting of A2, A30, A41, A55 and/or A57, and the complements thereof. Optionally, determining the frequency of a biallelic marker allele in a population may be accomplished by determining the identity of the nucleotides for both copies of said biallelic marker present in the genome of each individual in said population and calculating the proportional representation of said nucleotide at said *PCTA-1*-related biallelic marker for the population; Optionally, determining the proportional representation may be accomplished by performing a genotyping method of the invention on a pooled biological sample derived from a representative number of individuals, or each individual, in said population, and calculating the proportional amount of said nucleotide compared with the total.

### Determining The Frequency Of A Haplotype In A Population

The gametic phase of haplotypes is unknown when diploid individuals are heterozygous at more than one locus. Using genealogical information in families gametic phase can sometimes be inferred (Perlin et al., 1994). When no gencalogical information is available different strategies may be used. One possibility is that the multiple-site heterozygous diploids can be eliminated from the analysis, keeping only the homozygotes and the single-site heterozygote individuals, but this approach might lead to a possible bias in the sample composition and the underestimation of low-frequency haplotypes. Another possibility is that single chromosomes can be studied independently, for example, by asymmetric PCR amplification (see Newton et al, 1989; Wu et al., 1989) or by isolation of single chromosome by limit dilution followed by PCR amplification (see Ruano et al., 1990). Further, a sample may be haplotyped for sufficiently close biallelic markers by double PCR amplification of specific alleles (Sarkar, G. and Sommer S. S., 1991). These approaches are not entirely satisfying either because of their technical complexity, the additional cost they entail, their lack of generalization at a large scale, or the possible biases they introduce. To overcome these difficulties, an algorithm to infer the phase of PCR-amplified DNA genotypes introduced by Clark, A.G.(1990) may be used. Briefly, the principle is to start filling a preliminary list of haplotypes present in the sample by examining unambiguous individuals, that is, the complete homozygotes and the single-site heterozygotes. Then other individuals in the same sample are screened for the possible occurrence of previously recognized haplotypes. For each positive identification, the complementary haplotype is added to the list of recognized haplotypes, until the phase information for all individuals is either resolved or identified as unresolved. This method assigns a single haplotype to each multiheterozygous individual, whereas several haplotypes are possible when there are more than one heterozygous site. Alternatively, one can use methods estimating haplotype frequencies in a population without assigning haplotypes to each individual. Preferably, a method based on an expectation-maximization (EM) algorithm (Dempster et al., 1977) leading to maximum-likelihood estimates of haplotype frequencies under the assumption of Hardy-Weinberg proportions (random mating) is used (see Excoffier L. and Slatkin M., 1995). The EM algorithm is a generalized iterative maximum-likelihood approach to estimation that is useful when data are ambiguous and/or incomplete. The EM algorithm is used to resolve heterozygotes into haplotypes. Haplotype estimations are further described below under the heading "Statistical Methods." Any other method known in the art to determine or to estimate the frequency of a haplotype in a population may be used.

The invention also discloses methods of estimating the frequency of a haplotype for a set of biallelic markers selected from the group consisting of A2, A30, A41, A55 and A57 in a population, comprising the steps of: a) genotyping at least one *PCTA-1-*related biallelic marker according to a method of the invention for each individual in said population; b) genotyping a second biallelic marker by determining the identity of the nucleotides at said second biallelic marker for both copies of said second biallelic marker present in the genome of each individual in said population; and c) applying a haplotype determination method to the identities of the nucleotides determined in steps a) and b) to obtain an estimate of said frequency. In addition, the methods of estimating the frequency of a haplotype of the invention encompass methods with any further limitation described in this disclosure, or those following, specified alone or in any combination: wherein said *PCTA-1*-related biallelic marker is selected from the group consisting of A2, A30, A41, A55 and/or A57, and the complements thereof. Optionally, said haplotype determination method is performed by asymmetric PCR amplification, double PCR amplification of specific alleles, the Clark algorithm, or an expectation-maximization algorithm.

### Linkage Disequilibrium Analysis

Linkage disequilibrium is the non-random association of alleles at two or more loci and represents a powerful tool for mapping genes involved in disease traits (see Ajioka R.S. et al., 1997). Biallelic markers, because they are densely spaced in the human genome and can be genotyped in greater numbers than other types of genetic markers (such as RFLP or VNTR markers), are particularly useful in genetic analysis based on linkage disequilibrium.

When a disease mutation is first introduced into a population (by a new mutation or the immigration of a mutation carrier), it necessarily resides on a single chromosome and thus on a single "background" or "ancestral" haplotype of linked markers. Consequently, there is complete disequilibrium between these markers and the disease mutation: one finds the disease mutation only in the presence of a specific set of marker alleles. Through subsequent generations recombination events occur between the disease mutation and these marker polymorphisms, and the disequilibrium gradually dissipates. The pace of this dissipation is a function of the recombination frequency, so the markers closest to the disease gene will manifest higher levels of disequilibrium than those that are further away. When not broken up by recombination, "ancestral" haplotypes and linkage disequilibrium between marker alleles at different loci can be tracked not only through pedigrees but also through populations. Linkage disequilibrium is usually seen as an association between one specific allele at one locus and another specific allele at a second locus.

The pattern or curve of disequilibrium between disease and marker loci is expected to exhibit a maximum that occurs at the disease locus. Consequently, the amount of linkage disequilibrium between a disease allele and closely linked genetic markers may yield valuable information regarding the location of the disease gene. For fine-scale mapping of a disease locus, it is useful to have some knowledge of the patterns of linkage disequilibrium that exist between markers in the studied region. As mentioned above the mapping resolution achieved through the analysis of linkage disequilibrium is much higher than that of linkage studies. The high density of biallelic markers combined with linkage disequilibrium analysis provides powerful tools for fine-scale mapping. Different methods to calculate linkage disequilibrium are described below under the heading "Statistical Methods".

### Population-Based Case-Control Studies Of Trait-Marker Associations

As mentioned above, the occurrence of pairs of specific alleles at different loci on the same chromosome is not random and the deviation from random is called linkage disequilibrium. Association studies focus on population frequencies and rely on the phenomenon of linkage disequilibrium. If a specific allele in a given gene is directly involved in causing a particular trait, its frequency will be statistically increased in an affected (trait positive) population, when compared to the frequency in a trait negative population or in a random control population. As a consequence of the existence of linkage disequilibrium, the frequency of all other alleles present in the haplotype carrying the trait-causing allele will also be increased in trait positive individuals compared to trait negative individuals or random controls. Therefore, association between the trait and any allele (specifically a biallelic marker allele) in linkage disequilibrium with the trait-causing allele will suffice to suggest the presence of a trait-related gene in that particular region. Case-control populations can be genotyped for biallelic markers to identify associations that narrowly locate a trait causing allele. As any marker in linkage disequilibrium with one given marker associated with a trait will be associated with the trait. Linkage disequilibrium allows the relative frequencies in case-control populations of a limited number of genetic polymorphisms (specifically biallelic markers) to be analyzed as an alternative to screening all possible functional polymorphisms in order to find trait-causing alleles. Association studies compare the frequency of marker alleles in unrelated case-control populations, and represent powerful tools for the dissection of complex traits.

### Case-Control Populations (Inclusion Criteria)

Population-based association studies do not concern familial inheritance but compare the prevalence of a particular genetic marker, or a set of markers, in case-control populations. They are case-control studies based on comparison of unrelated case (affected or trait positive) individuals and unrelated control (unaffected, trait negative or random) individuals. Preferably the control group is composed of unaffected or trait negative individuals. Further, the control group is ethnically matched to the case population. Moreover, the control group is preferably matched to the case-population for the main known confusion factor for the trait under study (for example age-matched for an age-dependent trait). Ideally, individuals in the two samples are paired in such a way that they are expected to differ only in their disease status. The terms "trait positive population", "case population" and "affected population" are used interchangeably herein.

An important step in the dissection of complex traits using association studies is the choice of case-control populations (see Lander and Schork, 1994). A major step in the choice of case-control populations is the clinical definition of a given trait or phenotype. Any genetic trait may be analyzed by the association method proposed here by carefully selecting the individuals to be included in the trait positive and trait negative phenotypic groups. Four criteria are often useful: clinical phenotype, age at onset, family history and severity. The selection procedure for continuous or quantitative traits (such as blood pressure for example) involves selecting individuals at opposite ends of the phenotype distribution of the trait under study, so as to include in these trait positive and trait negative populations individuals with non-overlapping phenotypes. Preferably, cause-control populations consist of phenotypically homogeneous populations. Trait positive and trait negative populations consist of phenotypically uniform populations of individuals representing each between 1 and 98%, preferably between 1 and 80%, more preferably between 1 and 50%, and more preferably between 1 and 30%, most preferably between 1 and 20% of the total population under study, and preferably selected among individuals exhibiting non-overlapping phenotypes. The clearer the difference between the two trait phenotypes, the greater the probability of detecting an association with biallelic markers. The selection of those drastically different but relatively uniform phenotypes enables efficient comparisons in association studies and the possible detection of marked differences at the genetic level, provided that the sample sizes of the populations under study are significant enough.

In preferred embodiments, a first group of between 50 and 300 trait positive individuals, preferably about 100 individuals, are recruited according to their phenotypes. A similar number of control individuals are included in such studies.

In the present invention, typical examples of inclusion criteria include, but are not restricted to, prostate cancer or aggressiveness of prostate cancer tumors. In one preferred embodiment of the present invention, association studies are carried out on the basis of a presence (trait positive) or absence (trait negative) of prostate cancer.

Associations studies can be carried out by the skilled technician using the biallelic markers of the invention defined above, with different trait positive and trait negative populations. Suitable further examples of association studies using biallelic markers of the *PCTA-1* gene, including the biallelic markers A2, A30, A41, A55 and/or A57 involve studies on the following populations:
- a trait positive population suffering from a cancer and a healthy unaffected population, or
- a trait positive population suffering from prostate cancer treated with agents acting against prostate cancer and suffering from side-effects resulting from this treatment and an trait negative population suffering from prostate cancer treated with same agents without any substantial side-effects, or
- a trait positive population suffering from prostate cancer treated with agents acting against prostate cancer showing a beneficial response and a trait negative population suffering from prostate cancer treated with same agents without any beneficial response, or
- a trait positive population suffering from prostate cancer presenting highly aggressive prostate cancer tumors and a trait negative population suffering from prostate cancer with prostate cancer tumors devoid of aggressiveness.

### Association Analysis

The general strategy to perform association studies using biallelic markers derived from a region carrying a candidate gene is to scan two groups of individuals (case-control populations) in order to measure and statistically compare the allele frequencies of the biallelic markers of the present invention in both groups.

If a statistically significant association with a trait is identified for at least one or more of the analyzed biallelic markers, one can assume that: either the associated allele is directly responsible for causing the trait (i.e. the associated allele is the trait causing allele), or more likely the associated allele is in linkage disequilibrium with the trait causing allele. The specific characteristics of the associated allele with respect to the candidate gene function usually give further insight into the relationship between the associated allele and the trait (causal or in linkage disequilibrium). If the evidence indicates that the associated allele within the candidate gene is most probably not the trait causing allele but is in linkage disequilibrium with the real trait causing allele, then the trait causing allele can be found by sequencing the vicinity of the associated marker, and performing further association studies with the polymorphisms that are revealed in an iterative manner.

Association studies are usually run in two successive steps. In a first phase, the frequencies of a reduced number of biallelic markers from the candidate gene are determined in the trait positive and control populations. In a second phase of the analysis, the position of the genetic loci responsible for the given trait is further refined using a higher density of markers from the relevant region. However, if the candidate gene under study is relatively small in length, as is the case for *PCTA-1,* a single phase may be sufficient to establish significant associations.

It is another object of the present invention to provide a method for the identification and characterization of an association between an allele of one or more biallelic markers of a *PCTA-1* gene and a trait. The method comprises the steps of:
- genotyping a marker or a group of biallelic markers selected from the group consisting of A2, A30, A41, A55 and A57 in trait positive and control individuals; and
- establishing a statistically significant association between one allele of at least one marker and the trait.

The control individuals can be random or trait negative populations. Preferably, the trait positive and trait negative individuals are selected from non-overlapping phenotypes relating trait under study. In some embodiments, the biallelic marker is comprised in one or more of the sequences of P1 to P125, and the complementary sequences thereof.

The invention also comprises methods of detecting an association between a genotype and a phenotype, comprising the steps of a) determining the frequency of at least one *PCTA-1*-related biallelic marker selected from the group consisting of A2, A30, A41, A55 and A57 in a trait positive population according to a genotyping method of the invention; b) determining the frequency of said *PCTA-1*-related biallelic marker in a control population according to a genotyping method of the invention; and c) determining whether a statistically significant association exists between said genotype and said phenotype. In addition, the methods of detecting an association between a genotype and a phenotype of the invention encompass methods with any further limitation described in this disclosure, or those following, specified alone or in any combination: wherein said *PCTA-1*-related biallelic marker is selected from the group consisting of A2, A30, A41, A55 and/or A57, and the complements thereof. Optionally, said control population may be a trait negative population, or a random population; Optionally, each of said genotyping steps a) and b) may be performed on a pooled biological sample derived from each of said populations; Optionally, each of said genotyping of steps a) and b) is performed separately on biological samples derived from each individual in said population or a subsample thereof; Optionally, the identity of the nucleotides at the biallelic markers of the *PCTA-1* gene is determined in steps a) and b). Optionally, said phenotype is symptoms of, or susceptibility to cancer, preferably prostate cancer, the level of aggressiveness of prostate cancer tumors, an early onset of prostate cancer, a beneficial response to or side effects related to treatment against prostate cancer.

If the trait is a beneficial response or inversely a side effect to a treatment of prostate cancer, the method of the invention referred to above further comprises some or all of the following steps:
- selecting a population or cohort of subjects diagnosed as suffering from prostate cancer;
- administering a specified treatment of prostate cancer to said cohort of subjects;
- monitoring the outcome of drug administration and identifying those individuals that are trait positive or trait negative relative to the treatment;
- taking from said cohort biological samples containing DNA and testing this DNA for the presence of a specific allele or of a set of alleles of biallelic markers of the *PCTA-1* gene selected from the group consisting of A2, A30, A41, A55 and A57;
- analyzing the distribution of alleles of biallelic markers between trait positive and trait negative individuals; and
- performing a statistical analysis to determine a statistically significant association between the presence or absence of alleles of biallelic markers of the *PCTA-1* gene selected from the group consisting of A2, A30, A41, A55 and A57 and the treatment related trait.

### Haplotype Analysis

As described above, when a chromosome carrying a disease allele first appears in a population as a result of either mutation or migration, the mutant allele necessarily resides on a chromosome having a set of linked markers: the ancestral haplotype. This haplotype can be tracked through populations and its statistical association with a given trait can be analyzed. Complementing single point (allelic) association studies with multi-point association studies also called haplotype studies increases the statistical power of association studies. Thus, a haplotype association study allows one to define the frequency and the type of the ancestral carrier haplotype. A haplotype analysis is important in that it increases the statistical power of an analysis involving individual markers.

In a first stage of a haplotype frequency analysis, the frequency of the possible haplotypes based on various combinations of the identified biallelic markers of the invention is determined. The haplotype frequency is then compared for distinct populations of trait positive and control individuals. The number of trait positive individuals, which should be, subjected to this analysis to obtain statistically significant results usually ranges between 30 and 300, with a preferred number of individuals ranging between 50 and 150. The same considerations apply to the number of unaffected individuals (or random control) used in the study. The results of this first analysis provide haplotype frequencies in case-control populations, for each evaluated haplotype frequency a p-value and an odd ratio are calculated. If a statistically significant association is found the relative risk for an individual carrying the given haplotype of being affected with the trait under study can be approximated.

The present invention also provides a method for the identification and characterization of an association between a haplotype comprising alleles of several biallelic markers of the genomic sequence of the *PCTA-1* gene and a trait. The method comprises the steps of:
- genotyping a group of biallelic markers selected from the group consisting of A2, A30, A41, A55 and A57 in trait positive and control individuals; and
- establishing a statistically significant association between a haplotype and the trait.

Preferably, the control individuals can be random or trait negative populations.

An additional embodiment of the present invention encompasses methods of detecting an association between a haplotype and a phenotype, comprising the steps of: a) estimating the frequency of at least one haplotype in a trait positive population, according to a method of the invention for estimating the frequency of a haplotype; b) estimating the frequency of said haplotype in a control population, according to a method of the invention for estimating the frequency of a haplotype; and c) determining whether a statistically significant association exists between said haplotype and said phenotype. In addition, the methods of detecting an association between a haplotype and a phenotype of the invention encompass methods with any further limitation described in this disclosure, or those following: wherein said *PCTA-1-*related biallelic marker is selected from the group consisting of A2, A3, A41, A55 and/or A57, and the complements thereof. Optionally, said control population is a trait negative population, or a random population. Optionally, said phenotype is symptoms of, or susceptibility to cancer, preferably prostate cancer, the level of aggressiveness of prostate cancer tumors, an early onset of prostate cancer, a beneficial response to or side effects related to treatment against prostate cancer; Optionally, said method comprises the additional steps of determining the phenotype in said trait positive and said control populations prior to step c).

### Interaction Analysis

The biallelic markers of the present invention may also be used to identify patterns of biallelic markers associated with detectable traits resulting from polygenic interactions. The analysis of genetic interaction between alleles at unlinked loci requires individual genotyping using the techniques described herein. The analysis of allelic interaction among a selected set of biallelic markers with appropriate level of statistical significance can be considered as a haplotype analysis. Interaction analysis consists in stratifying the case-control populations with respect to a given haplotype for the first loci and performing a haplotype analysis with the second loci with each subpopulation.

Statistical methods used in association studies are further described below.

### Testing For Linkage In The Presence Of Association

The biallelic markers of the present invention may further be used in TDT (transmission/disequilibrium test). TDT tests for both linkage and association and is not affected by population stratification. TDT requires data for affected individuals and their parents or data from unaffected sibs instead of from parents (see Spielmann S. et al., 1993; Schaid D.J. et al., 1996, Spielmann S. and Ewens W.J., 1998). Such combined tests generally reduce the false - positive errors produced by separate analyses.

### Association OF Biallelic Markers Of The Invention With Prostate Cancer

### Trait Positive And Control Populations

Two groups of independent individuals were used: the overall trait positive and the trait negative populations included 491 individuals suffering from prostate cancer and 313 individuals without any sign of prostate cancer. A specific protocol for the collection of DNA samples from trait positive and trait negative individuals is described in Example 5. The 491 individuals suffering from prostate cancer can be subdivided into a population of individuals who developed prostate cancer under 65 years-old and a population of individuals who developed prostate cancer after the age of 65. The population of individuals who are less than 65 years-old was used to determine an association with an early onset of prostate cancer. The affected individuals can also be subdivided in familial cases and sporadic cases.

In order to have as much certainty as possible on the absence of prostate cancer in trait negative individuals, it is preferred to conduct a PSA dosage analysis on this population. Several commercial assays can be used (WO 96/21042, herein by reference). In one preferred embodiment, a Hybritech assay is used and trait negative individuals must have a level of PSA less than 2.8 ng/ml of serum in order to be selected as such. In a preferred embodiment, the Yang assay is used and trait negative individuals must have a level of PSA of less than 4 ng/ml of serum in order to be included in the population under study.

### Association Analysis

In one preferred embodiment of the invention in which a correlation was found between biallelic markers of the *PCTA-1* gene selected from the group consisting of A2, A30, A41, A55 and A57 and prostate cancer, results of the association study, further details of which are provided in example 5, seem to indicate that prostate cancer, preferably familial prostate cancer, more preferably early onset familial prostate cancer, is associated most strongly with the biallelic markers A30 (99-1572/440) and A41 (5-171/204) which present a particular interest. These association results constitute new elements for studying the genetic susceptibility of individuals to prostate cancer, preferably to familial prostate cancer, more preferably familial early onset prostate cancer. Further details concerning this association study are provided below.

The biallelic markers most strongly associated with prostate cancer, namely A30 and A41, are located in the regulatory region of the *PCTA-1* gene, more particularly in the promoter region. The consequences of the presence of these markers in these regions are discussed below.

Furthermore, the biallelic marker A2 (99-1601/402) was found to be also associated with prostate cancer, more particularly with sporadic prostate cancer. This biallelic marker is localized in the 5' regulatory region of the *PCTA-1* gene.

Similar association studies can also be carried out with other biallelic markers within the scope of the invention, preferably with biallelic markers in linkage disequilibrium with the markers associated with prostate cancer as described above, including the biallelic markers A1 to A125.

### Analysis Of Biallelic Marker Associations

Even though polymorphisms associated with prostate cancer have been identified in the coding region of the *PCTA-1* gene, these polymorphisms do not appear to be as significant as those found in the upstream regulatory region of the *PCTA-1* gene. The results further suggest that a trait-causing mutation is likely to be located within the 5' regulatory region of the *PCTA-1* gene. The extent to which the markers found within the coding region of *PCTA-1* are significant in relation to cancer can be determined using haplotype analyses involving at least two of the biallelic markers of the present invention.

Six of the biallelic markers of the present invention result in a change in the amino acid sequence of a PCTA-1 protein. These are biallelic markers A54, A56, A60, A75, A76 and A85. These mutations may change the function and/or the stability of the PCTA-1 protein. An amino acid change in a PCTA-1 protein can lead to alterations in PCTA-1 biological activity. Either a modified function or an increased stability can be involved in prostate cancer appearance.

Furthermore, as the expression of the *PCTA-1* gene has mainly been reported in prostate cancer cells, one can assume that its expression is closely linked to the development of cancer, particularly prostate cancer. Generally, a major control of gene expression proceeds at the level of the initiation of the transcription. This initiation involves the promoter which can be considered as a concentration of transcription factor binding sites. The initiation of the transcription also involves enhancers which modulate the efficiency of the initiation and consist of DNA binding sites which are located in regulatory regions of the considered gene which may be at a certain distance in 3' or 5' of the gene.

Most of the biallelic polymorphisms of the *PCTA-1* gene associated with prostate cancer according to the present invention are located in the regulatory region upstream of the transcription start site of the *PCTA-1* gene and particularly in the promoter. Biallelic marker A41, which is located about 120 bp upstream of the beginning of the first exon (exon 0), may be comprised in the proximal promoter of the *PCTA-1* gene. This biallelic marker could be a trait causing mutation of prostate cancer. Biallelic marker A30, which is located about 1.5 kb upstream the beginning of the first exon (exon 0), may be comprised in the distal promoter of the *PCTA-1* gene. Biallelic marker A2 is located in the 5' regulatory region of the *PCTA-1* gene.

As the expression of the *PCTA-1* gene has mainly been reported in prostate cancer cells, the expression of *PCTA-1* gene is modified during the carcinogenesis. The exact mechanism through which PCTA expression is modified is not understood. However, it is possible that the polymorphisms A41, A30, and A2 modulate *PCTA-1* expression by modulating *PCTA-1* transcription through DNA binding proteins, which will be explained in further detail below.

The regulation of *PCTA-1* expression is a key factor in the onset and for development of cancer and particularly prostate cancer. In this regard, the polymorphisms located in the 5' regulatory region of the *PCTA-1* gene appear to play the most significant role in the association of *PCTA-1* with cancer. It appears clear that the polymorphisms found in the promoter region adjacent to the transcription initiation site, and particularly those located in the proximal *PCTA-1* promoter, are more strongly associated with prostate cancer than polymorphisms of the other promoter elements located further upstream of this site. Furthermore, some polymorphisms, such as the biallelic marker A41, are clearly associated with early onset prostate cancer. The polymorphisms found in the proximal 2000 to 3000 bp of the 5' regulatory region are associated with early onset prostate cancer. The inventors have also shown an association between some of the biallelic markers of the present invention located at the 3' end of the *PCTA-1* genomic DNA and prostate cancer.

The involvement of the associated polymorphisms in the modification of the *PCTA-1* expression in prostate cancer cells can be confirmed through the assays described below.

The expression levels of a *PCTA-1* gene, preferably a gene comprising at least one biallelic marker according to the invention, in different tissues, can be determined by analyses of tissue samples from individuals typed for the presence or absence of a specific polymorphism. Any convenient method can be used such as Northern, or Dot blot or other hybridization analyses, and quantitative RT-PCR for mRNA quantitation, Western blot ELISA, RIA for protein quantitation. The tissue specific expression can then be correlated with the genotype. More details on some of these methods are provided below under the heading "Method For Screening".

The effects of modifications in the regulatory regions of the *PCTA-1* gene, and particularly in the sequence of its promoter, can be studied through the determination of expression levels by expression assays for the particular promoter sequence. The assays are performed with the *PCTA-1* coding sequence or with a detectable marker sequence using a reporter gene. To determine tissue specificity, the assay is performed in cells from different sources. Preferably the assay is performed on normal tissue cells and cancerous cells of the same tissue type (e.g. prostate cells and on prostate cancer cells). More preferably, the assay is performed on a large range of cell lines with an increasing level of malignancy. Some methods are discussed in more detail below under the heading "Method For Screening".

An assay to determine the effect of a sequence polymorphism on *PCTA-1* expression may be performed in cell-free extracts, or in cell-culture assays, such as transient or stable transfection assays. This assay is also within the scope of the present invention. Alterations in expression may be correlated to decreases or increases in the basic amounts of *PCTA-1* mRNA and/or protein that are expressed in one or more cell types. Expression levels of different alleles are compared using various methods known in the art. Methods for determining whether the level of expression triggered by promoter or enhancer sequences is increased or decreased depending on the studied allele of said sequence include the insertion into a vector of said sequence upstream a reporter gene such as β-galactosidase, luciferase, green fluorescent protein or chloramphenicol acetyltransferase. Expression levels are assessed by quantitation of expressed reporter proteins that provides for convenient quantitation.

The changes in *PCTA-1* expression can be the result of modifications in the modulation of *PCTA-1* transcription by DNA binding proteins, which are able to activate or inhibit the initiation of the transcription of the *PCTA-1* gene. The term "DNA binding protein" is intended to encompass more particularly transcriptional factors. The binding of these proteins on the sites located in the promoter is critical for a correct binding of polymerases and consequently for the initiation of transcription. The binding of these proteins on the sites located in the 5' upstream regulatory regions modulates transcription.

The binding sites of DNA binding proteins, preferably transcription factors, are generally 6-20 nucleotides in length. A polymorphic site located in a transcription factor binding site may result in a difference of binding affinity of the said transcription factor between the two allele of the polymorphism. This difference of affinity could explain the changes of expression of the *PCTA-1* gene.

When one or more alleles of the biallelic markers of the *PCTA-1* gene associated with cancer are present in the genome of an individual since conception, there would be an event which provokes a drastic increase in the expression of *PCTA-1.* There are at least two possible hypotheses that can be formulated to explain this event. Firstly, as cancer is the result of a succession of mutations, one mutation could lead to either the expression of a new DNA binding activity, or the overexpression of a DNA binding factor which binds to the site containing the polymorphism and which is involved in the transcription of the *PCTA-1* gene. Secondly the DNA binding factor readily binds to the site containing the polymorphism in normal cells where it is either unable to activate the transcription of *PCTA-1* or repressor of the *PCTA-1* transcription initiation. A mutation in the transcription factor can make the transcription factor either functional in the case of an activator or unfunctional in the case of a repressor. Likewise, a mutation in an additional protein can induce the binding of this protein which is needed by the DNA binding factor for activating the transcription of the *PCTA-1* gene.

In order to confirm the capacity of transcription factors to bind sites containing the biallelic markers of the present invention, so as to assess the difference in affinity between the two alleles of the considered biallelic marker and to discriminate between these hypotheses, a gel retardation assay or DNA mobility shift assay can be carried out. This type of assay is well-known to those skilled in the art and is described in US 5,698,389, US 5,502,176, Fried and Crothers (1981), Garner and Revzin (1981) and Dent and Latchman (1993).

This type of method relies on the principle that a fragment of DNA to which a protein has bound will move more slowly in gel electrophoresis than the same DNA fragment without the bound protein. The DNA mobility shift assay is carried out, therefore, by first labeling the specific DNA segment whose protein-binding properties are being investigated. The labeled DNA is then incubated with a nuclear (Dignam *et al*., 1983; Schreiber et al., 1989; Muller et al., 1989; Mizokami et al., 1994) or whole cell (Manley *et al*., 1980) extract of cells prepared in such a way as to contain DNA-binding proteins. DNA-protein complexes are then allowed to form. The complexes are then electrophoresed on a non-denaturing polyacrylamide gel and the position of the labeled DNA is visualized by suitable techniques. Various types of suitable labels can be selected by the person skilled in the art. Notably, the radioactive labeling is appropriate. If no protein has bound to the DNA, all the label is free to migrate quickly, whereas labeled protein-DNA complexes migrate more slowly and hence give a different signal from that of the unbound DNA near the top of the gel. The interaction specificity can be estimated by carrying out a gel retardation assay with increasing amount of unlabeled DNA segment which can compete with the labeled one. A positive control can be realized with an oligonucleotide containing the androgene responsive element.

The investigated DNA segment comprises the sequence of a potential binding site containing an allele of a polymorphism selected from the group consisting of A2, A30, A41, A55 and A57, more preferably a sequence comprising a sequence selected from P1 to P125 and the complementary sequences thereto, still more preferably a sequence comprising a sequence selected from P1 to P43 and the complementary sequences thereto. In an embodiment, the polymorphism site is located in the middle of the DNA fragment. In an other embodiment, the polymorphism site can be located close to an end of the DNA fragment, for example at 6 nucleotides away from the end. The DNA fragment has a sufficient length to hybridize with the complementary strand and to form a stable double strand. For example, the DNA fragment comprises at least 8 nucleotides, preferably at least 20 nucleotides, more preferable 30 nucleotides. In a specific embodiment, the DNA fragment comprises the sequence of interest at the middle of the fragment and some poly G, poly C, or poly GC at its 5' and/or 3' ends.

In a preferred embodiment, the DNA segment consists of an oligonucleotide selected from the group consisting of Oligo1 to Oligo60 which are described in Table C and detailed as feature in SEQ ID No 1. For each polymorphic site, 4 oligonucleotides are generated and correspond to the two complementary strands of the DNA for each of the two alleles of the considered polymorphism. The DNA segments are designed such as the polymorphic base is surrounded with 14 nucleotides on each side.

**Table C**

| Biallelic marker | All | Oligonucleotide name | Position range of the oligonucleotide in SEQ ID No 1 | | Olignonucleotide name | Complementary position range of the oligonucleotide in SEQ ID No 1 | |
|---|---|---|---|---|---|---|---|
| | | | Beginning | End | | Beginning | End |
| 5-169-208 | A | Oligo1 | 67820 | 67848 | Oligo31 | 67820 | 67850 |
| 5-169-208 | G | Oligo2 | 67820 | 67848 | Oligo32 | 67820 | 67850 |
| 5-169-331 | C | Oligo3 | 67940 | 67969 | Oligo33 | 67941 | 67969 |
| 5-169-331 | T | Oligo4 | 67940 | 67969 | Oligo34 | 67941 | 67969 |
| 5-169-97 | C | Oligo5 | 67707 | 67737 | Oligo35 | 67709 | 67738 |
| 5-169-97 | G | Oligo6 | 67707 | 67737 | Oligo36 | 67709 | 67738 |
| 5-170-238 | A | Oligo7 | 68198 | 68227 | Oligo37 | 68199 | 68228 |
| 5-170-238 | G | Oligo8 | 68198 | 68227 | Oligo38 | 68199 | 68228 |
| 5-170-288 | A | Oligo9 | 68247 | 68277 | Oligo39 | 68249 | 68277 |
| 5-170-288 | C | Oligo10 | 68247 | 68277 | Oligo40 | 68249 | 68277 |
| 5-171-156 | G | Oligo11 | 68463 | 68491 | Oligo41 | 68463 | 68492 |
| 5-171-156 | T | Oligo12 | 68463 | 68491 | Oligo42 | 68463 | 68492 |
| 5-171-204 | C | Oligo13 | 68511 | 68539 | Oligo43 | 68511 | 68539 |
| 5-171-204 | T | Oligo14 | 68511 | 68539 | Oligo44 | 68511 | 68539 |
| 5-171-273 | A | Oligo15 | 68580 | 68608 | Oligo45 | 68580 | 68608 |
| 5-171-273 | G | Oligo16 | 68580 | 68608 | Oligo46 | 68580 | 68608 |
| 5-171-289 | C | Oligo17 | 68596 | 68624 | Oligo47 | 68596 | 68626 |
| 5-171-289 | T | Oligo18 | 68596 | 68624 | Oligo48 | 68596 | 68626 |
| 5-171-54 | C | Oligo19 | 68360 | 68389 | Oligo49 | 68361 | 68389 |
| 5-171-54 | G | Oligo20 | 68360 | 68389 | Oligo50 | 68361 | 68389 |
| 99-1572-315 | C | Oligo21 | 66951 | 66981 | Oligo51 | 66953 | 66983 |
| 99-1572-315 | T | Oligo22 | 66951 | 66981 | Oligo52 | 66953 | 66983 |
| 99-1572-335 | A | Oligo23 | 66973 | 67001 | Oligo53 | 66973 | 67002 |
| 99-1572-335 | G | Oligo24 | 66973 | 67001 | Oligo54 | 66973 | 67002 |
| 99-1572-440 | C | Oligo25 | 67078 | 67106 | Oligo55 | 67078 | 67106 |
| 99-1572-440 | T | Oligo26 | 67078 | 67106 | Oligo56 | 67078 | 67106 |
| 99-1572-477 | A | Oligo27 | 67113 | 67143 | Oligo57 | 67115 | 67144 |
| 99-1572-477 | T | Oligo28 | 67113 | 67143 | Oligo58 | 67115 | 67144 |
| 99-1572-578 | C | Oligo29 | 67212 | 67243 | Oligo59 | 67215 | 67247 |
| 99-1572-578 | T | Oligo30 | 67212 | 67243 | Oligo60 | 67215 | 67247 |

Each oligonucleotide selected from Oligol to Oligo60 comprises 4 additional bases, namely GATC, at its 5' end.

In a preferred embodiment, either the nuclear or whole cell extracts are provided from normal and cancer cells, particularly from normal prostate cells and prostate cancer cells. For example, suitable cell extracts can be provided from PZ-HPV-7 (ATCC : CRL-2221), CA-HPV-10 (ATCC: CRL-2220), PC-3 (ATCC : CRL-1435), DU 145 (ATCC : HTB-81), LNCaP-FGC (ATCC : CRL-10995 and CRL-1740), or NCI-H660 (ATCC : CRL-5813) cells. In a more preferred embodiment, the cell extracts are provided form PNT1A, PNT2, LNCaP-JMV, DU 145 (ATCC Nr : HTB-81) or PC3 (ATCC Nr : CRL-1435) cells.

In case a new transcription factor is specifically expressed in cancer cells, a gel retardation assay will show a retarded or shifted band only when the DNA was incubated with cell extracts from prostate cancer cells. If the DNA binding activity already exists in normal cells, the gel retardation assay will show a shifted band with cell extracts from normal prostate cells and prostate cancer cells. Gel retardation assays will also allow to show a significant difference in affinity between a DNA binding factor and binding sites containing the two alleles of the considered polymorphism.

The interaction of the DNA segment described above with transcription factors can also be studied with an optical biosensor such as BIACORE. This technology is well-known to those skilled in the art and is described in Szabo et al. (1995) and Edwards et al. (1997). The main advantage of this method is that it allows the determination of the association rate between the DNA fragment which is investigated and the DNA binding protein. Typically, a DNA segment such as those defined above is biotinylated at its 5' or 3' ends and is immobilized on a streptavidin-coated sensor chip. Then, a whole or a nuclear extract of cells is placed in contact with the DNA segment. The binding of DNA binding proteins to the DNA fragment causes a change in the refractive index and/or thickness. This change is detected by the Biosensor provided it occurs in the evanescent field. The affinity of the DNA binding protein to the DNA fragment can then be measured.

In order to precisely localize the binding site of the transcription factors, DNAse I footprinting or DMS protection footprinting assays can also be carried out with DNA fragments which contain the sequence of a potential binding site containing an allele of a polymorphism of the present invention, preferably a sequence comprising a sequence selected from P1 to P125 and the complementary sequences thereto, more preferably a sequence comprising a sequence selected from P1 to P43 and the complementary sequences thereto. This type of assay is well-known to those skilled in the art and is described in Galas and Schmitz (1978), and Dynan and Tjian (1983). Briefly, in the DNAse I footprinting assay, end-labeled DNA is incubated with protein extract and then partially digested with DNAse I. Specific binding of proteins to DNA will modify nuclease digestion at the site of interaction relative to free DNA, leaving an "imprint" which can be visualized after extraction of the labeled DNA and electrophoresis in a sequence gel.

The interaction with transcription factors can also be studied with the methylation interference assay which is well-known to those skilled in the art and is described in Siebenlist and Gilbert (1980) and Maxam and Gilbert (1980). Briefly, this method relies on the ability of DMS to methylate G residues, which can be cleaved with piperidine. The target DNA is partially methylated so that, on average, only one G residue per DNA molecule is methylated. These partially methylated molecules is used in a DNA mobility shift experiment with an appropriate cell extract containing transcription factors. After electrophoresis, the band produced by the DNA which has bound protein and that produced by the unbound DNA are excised from the gel and treated with piperidine to cleave the DNA at the methylated G residues and not at unmethylated G residues. If methylation of a particular G residue prevents transcription factors binding, then cleavage at this methylated G residue will be observed only in the DNA that failed to bind the protein.

In order to confirm the implication of a particular *PCTA-1* derived sequence containing the biallelic marker as a binding site for a transcription regulator of *PCTA-1* in cancer cells, a transient expression assay can be carried out in which a vector comprising the considered binding site upstream of the HSV1 thymidine kinase promoter operably linked to a reporter gene such as chloramphenicol acetyltransferase is transfected in appropriate cell lines. This assay is well-known to those skilled in the art and is described in Doucas et al. (1991). This assay can also be realized by cloning the considered binding site upstream the SV40 promoter into the pGL3-promoter luciferase vector (Promega) as described in Coles et al. (1998). Both normal and cancer cells, more particularly normal and cancer cells from prostate, are transfected with said vector. The effect of the binding site and more particularly of the alleles comprised in the binding site can be assessed through the expression level of the reporter gene.

The inventors believe that these polymorphisms, particularly the polymorphisms located on or close to polyadenylation sites have a direct although somewhat milder effect on prostate cancer development.

### Haplotype Analysis

In the context of the present invention, a haplotype can be defined as a combination of biallelic markers found in a given individual and which may be associated more or less significantly, as a result of appropriate statistical analyses, with the expression of a given trait.

A two-marker haplotype including markers A30 and A41 (TT alleles respectively) was shown to be significantly associated with prostate cancer, preferably with a familial prostate cancer, more preferably with a familial early onset prostate cancer. As shown in Table 8, the "TT" haplotype present a p-value of 2.5 x 10⁻⁶ for the familial early onset prostate cancer (see Example 5).

A three-marker haplotype including markers A2, A30, and A41 (ATT alleles respectively) was shown to be significantly associated with prostate cancer, preferably with a familial prostate cancer, more preferably with a familial early onset prostate cancer. As shown in table 8, the "ATT" haplotype present a p-value of 2.5 x 10⁻⁷ for the familial early onset prostate cancer (see Example 5).

A first two-marker haplotype including markers A2 and A57 (99-1605/112) (TA alleles, respectively) was shown to be significantly associated with prostate cancer, preferably with a sporadic prostate cancer. As shown in table 8, the "TA" haplotype present a p-value of 3.4 x 10⁻⁵ for the sporadic informative prostate cancer (see Example 5). A second two-marker haplotype including markers A2 and A55 (5-2/178) (TT alleles, respectively) was shown to be significantly associated with prostate cancer, preferably with a sporadic prostate cancer. As shown in table 8, the "TT" haplotype present a p-value of 1 x 10⁻⁵ for the sporadic informative prostate cancer (see Example 5).

Therefore, one preferred haplotype of the present invention associated with a familial prostate cancer comprises a biallelic marker selected from the group consisting of A30 (allele T), A41 (allele T), A2 (allele A), A55 (allele C) and A57 (allele G). One more preferred haplotype of the present invention associated with a familial prostate cancer comprises a biallelic marker selected from the group consisting of A30 (allele T), A41 (allele T), and A2 (allele A). One still more haplotype of the present invention associated with a familial prostate cancer comprises a biallelic marker selected from the group consisting of A30 (allele T), and A41 (allele T).

Furthermore, one preferred haplotype of the present invention associated with a sporadic prostate cancer comprises a biallelic marker selected from the group consisting of A2 (allele T), A55 (allele T), A57 (allele A), A30 (allele T) and A41 (allele T). One more preferred haplotype of the present invention associated with a sporadic prostate cancer comprises a biallelic marker selected from the group consisting of A2 (allele T), A41 (allele T), A55 (allele T), A57 (allele A).

The permutation tests clearly validated the statistical significance of the association between these haplotypes and the prostate cancer (see Example 5). All these haplotypes can be used in diagnostic of prostate cancer, more particularly either familial prostate cancer or sporadic prostate cancer.

One can observe that the haplotypes associated to familial cases of prostate cancer are not associated with the sporadic cases of prostate cancer and that the haplotypes associated to the sporadic cases are not associated with the familial cases (see Table 7 of Example 5). Moreover, except the biallelic markers A2, the familial and sporadic cases haplotypes do not present any common biallelic marker. Therefore, the ancestral haplotypes would be different and the causing trait allele would not be the same.

This information is extremely valuable. The knowledge of a potential genetic predisposition to prostate cancer, even if this predisposition is not absolute, might contribute in a very significant manner to treatment efficacy of prostate cancer and to the development of new therapeutic and diagnostic tools.

### Statistical methods

In general, any method known in the art to test whether a trait and a genotype show a statistically significant correlation may be used.

### 1) Methods In Linkage Analysis

Statistical methods and computer programs useful for linkage analysis are well-known to those skilled in the art (see Terwilliger J.D. and Ott J., 1994; Ott J., 1991).

### 2) Methods To Estimate Haplotype Frequencies In A Population

As described above, when genotypes are scored, it is often not possible to distinguish heterozygotes so that haplotype frequencies cannot be easily inferred. When the gametic phase is not known, haplotype frequencies can be estimated from the multilocus genotypic data. Any method known to person skilled in the art can be used to estimate haplotype frequencies (see Lange K., 1997; Weir, B.S., 1996) Preferably, maximum-likelihood haplotype frequencies are computed using an Expectation- Maximization (EM) algorithm (see Dempster et al., 1977; Excoffier L. and Slatkin M., 1995). This procedure is an iterative process aiming at obtaining maximum-likelihood estimates of haplotype frequencies from multi-locus genotype data when the gametic phase is unknown. Haplotype estimations are usually performed by applying the EM algorithm using for example the EM-HAPLO program (Hawley M. E. et al., 1994) or the Arlequin program (Schneider et al., 1997). The EM algorithm is a generalized iterative maximum likelihood approach to estimation and is briefly described below.

Please note that in the present section, "Methods To Estimate Haplotype Frequencies In A Population, " of this text, phenotypes will refer to multi-locus genotypes with unknown phase. Genotypes will refer to known-phase multi-locus genotypes.

A sample of N unrelated individuals is typed for K markers. The data observed are the unknown-phase K-locus phenotypes that can categorized in F different phenotypes. Suppose that we have H underlying possible haplotypes (in case of K biallelic markers, H=2^{K}).

For phenotype j, suppose that cⱼ genotypes are possible. We thus have the following equation ${P}_{j} = {\sum }_{i = 1}^{{c}_{j}} pr \left({genotype}_{i}\right) = {\sum }_{i = 1}^{{c}_{j}} pr \left({h}_{k}, ⁢, {h}_{l}\right)$
where *Pj* is the probability of the phenotype *j*, *hₖ* and *hₗ* are the two haplotypes constituent the genotype i. Under the Hardy-Weinberg equilibrium, *pr(hₖ,hₗ)* becomes: $pr \left({h}_{k}, ⁢, {h}_{l}\right) = pr ( {h}_{k} ⁢ {)}^{2} if {h}_{k} = {h}_{l} , pr \left({h}_{k}, ⁢, {h}_{l}\right) = 2 ⁢ pr \left({h}_{k}\right) . pr \left({h}_{l}\right) if {h}_{k} \neq {h}_{l} .$

### The successive steps of the E-M algorithm can be described as follows:

Starting with initial values of the of haplotypes frequencies, noted *p*₁⁽⁰⁾ *p*₂⁽⁰⁾*,.....p_{H}⁽⁰⁾*, these initial values serve to estimate the genotype frequencies (Expectation step) and then estimate another set of haplotype frequencies (Maximization step), noted *p*₁⁽¹⁾, *p*₂⁽¹⁾,.....p_{H}⁽¹⁾, these two steps are iterated until changes in the sets of haplotypes frequency are very small.

A stop criterion can be that the maximum difference between haplotype frequencies between two iterations is less than 10⁻⁷. These values can be adjusted according to the desired precision of estimations.

At a given iteration s, the Expectation step consists in calculating the genotypes frequencies by the following equation: $\begin{matrix}pr ( {genotype}_{i} ⁢ {)}^{\left(s\right)} & = pr \left({phenotype}_{j}\right) . pr ( {genotype}_{i} | {phenotype}_{j} ⁢ {)}^{\left(s\right)} \\ & = \frac{{n}_{j}}{N} ⋅ \frac{pr ( {h}_{k} , {h}_{l} ⁢ {)}^{\left(s\right)}}{{P}_{j}^{\left(s\right)}}\end{matrix}$
where genotype i occurs in phenotype j, and where *hₖ* and *hₗ* constitute genotype *i*. Each probability is derived according to eq. 1, and eq. 2 described above.

Then the Maximization step simply estimates another set of haplotype frequencies given the genotypes frequencies. This approach is also known as the gene-counting method (Smith, 1957). ${p}_{t}^{\left(s+1\right)} = \frac{1}{2} {\sum }_{j = 1}^{F} {\sum }_{i = 1}^{{c}_{j}} {δ}_{it} . pr ( {genotype}_{i} ⁢ {)}^{\left(s\right)}$

Where δ*ᵢₜ* is an indicator variable which count the number of time haplotype *t* in genotype *i*. It takes the values of 0, 1 or 2.

To ensure that the estimation finally obtained is the maximum-likelihood estimation several values of departures are required. The estimations obtained are compared and if they are different the estimations leading to the best likelihood are kept.

### 3) Methods To Calculate Linkage Disequilibrium Between Markers

A number of methods can be used to calculate linkage disequilibrium between any two genetic positions, in practice linkage disequilibrium is measured by applying a statistical association test to haplotype data taken from a population.

Linkage disequilibrium between any pair of biallelic markers comprising at least one of the biallelic markers of the present invention (Mᵢ, Mⱼ) having alleles (aᵢ/bᵢ) at marker Mᵢ and alleles (aⱼ/bⱼ) at marker Mⱼ can be calculated for every allele combination (aᵢ,aⱼ: aᵢ,bⱼ; bᵢ,aⱼ and bᵢ,bⱼ), according to the Piazza formula: ${Δ}_{aiaj} = √θ ⁢ 4 - √ ⁢ \left(θ⁢4+θ⁢3\right) ⁢ \left(θ⁢4+θ⁢2\right) ,$
where:
θ4= - - = frequency of genotypes not having allele aᵢ at Mᵢ and not having allele aⱼ at Mⱼ
θ3= - + = frequency of genotypes not having allele aᵢ at Mᵢ and having allele aⱼ at Mⱼ
θ2= + - = frequency of genotypes having allele aᵢ at Mᵢ and not having allele aⱼ at Mⱼ

Linkage disequilibrium (LD) between pairs of biallelic markers (Mᵢ, Mⱼ) can also be calculated for every allele combination (ai,aj_{;} ai,bj; bᵢ,aⱼ and bᵢ,bⱼ), according to the maximum-likelihood estimate (MLE) for delta (the composite genotypic disequilibrium coefficient), as described by Weir (Weir B. S., 1996). The MLE for the composite linkage disequilibrium is: ${D}_{aiaj} = \left(2⁢{n}_{1}+{n}_{2}+{n}_{3}+{n}_{4}/2\right) / N - 2 \left(pr\left({a}_{i}\right).pr\left({a}_{j}\right)\right)$

Where n₁ = E phenotype (aᵢ/aᵢ, aⱼ/aⱼ), n₂ = E phenotype (aᵢ/aᵢ, aⱼ/aⱼ), n₃= E phenotype (aᵢ/bᵢ, aⱼ/aⱼ), n4= Σ phenotype (aᵢ/aᵢ, aⱼ/aⱼ) and N is the number of individuals in the sample.

This formula allows linkage disequilibrium between alleles to be estimated when only genotype, and not haplotype, data are available.

Another means of calculating the linkage disequilibrium between markers is as follows. For a couple of biallelic markers, *Mᵢ* (*aᵢ*/*bᵢ*) and *Mⱼ*(*aⱼ*/*bⱼ*), fitting the Hardy-Weinberg equilibrium, one can estimate the four possible haplotype frequencies in a given population according to the approach described above.

The estimation of gametic disequilibrium between *ai* and *aj* is simply: ${D}_{aiaj} = pr \left(haplotype\left({a}_{i}, ⁢, {a}_{j}\right)\right) - pr \left({a}_{i}\right) . pr \left({a}_{j}\right) .$

Where *pr(aᵢ)* is the probability of allele α*ᵢ* and pr(a) is the probability of allele α*ⱼ* and where *pr*(*haplotype (*α*ᵢ*, α*ⱼ))* is estimated as in Equation 3 above.

For a couple of biallelic marker only one measure of disequilibrium is necessary to describe the association between *Mᵢ* and *Mⱼ*.

Then a normalized value of the above is calculated as follows: ${Dʹ}_{aiaj} = {D}_{aiaj} / max ⁢ \left(-pr\left({a}_{i}\right).pr\left({a}_{j}\right),-pr\left({b}_{i}\right).pr\left({b}_{j}\right)\right) with {D}_{aiaj} < 0$ ${Dʹ}_{aiaj} = {D}_{aiaj} / max \left(pr\left({b}_{i}\right).pr\left({a}_{j}\right),pr\left({a}_{i}\right).pr\left({b}_{j}\right)\right) with {D}_{aiaj} > 0$

The skilled person will readily appreciate that other linkage disequilibrium calculation methods can be used.

Linkage disequilibrium among a set of biallelic markers having an adequate heterozygosity rate can be determined by genotyping between 50 and 1000 unrelated individuals, preferably between 75 and 200, more preferably around 100.

### 4) Testing For Association

Methods for determining the statistical significance of a correlation between a phenotype and a genotype, in this case an allele at a biallelic marker or a haplotype made up of such alleles, may be determined by any statistical test known in the art and with any accepted threshold of statistical significance being required. The application of particular methods and thresholds of significance are well with in the skill of the ordinary practitioner of the art.

Testing for association is performed by determining the frequency of a biallelic marker allele in case and control populations and comparing these frequencies with a statistical test to determine if their is a statistically significant difference in frequency which would indicate a correlation between the trait and the biallelic marker allele under study. Similarly, a haplotype analysis is performed by estimating the frequencies of all possible haplotypes for a given set of biallelic markers in case and control populations, and comparing these frequencies with a statistical test to determine if their is a statistically significant correlation between the haplotype and the phenotype (trait) under study. Any statistical tool useful to test for a statistically significant association between a genotype and a phenotype may be used. Preferably the statistical test employed is a chi-square test with one degree of freedom. A P-value is calculated (the P-value is the probability that a statistic as large or larger than the observed one would occur by chance).

### Statistical Significance

In preferred embodiments, significance for diagnosis purposes, either as a positive basis for further diagnostic tests or as a preliminary starting point for early preventive therapy, the p value related to a biallelic marker association is preferably about 1 x 10⁻² or less, more preferably about 1 x 10⁻⁴ or less, for a single biallelic marker analysis and about 1 x 10⁻³ or less, still more preferably 1 x 10⁻⁶ or less and most preferably of about 1 x 10⁻⁸ or less, for a haplotype analysis involving two or more markers. These values are believed to be applicable to any association studies involving single or multiple marker combinations.

The skilled person can use the range of values set forth above as a starting point in order to carry out association studies with biallelic markers of the present invention. In doing so, significant associations between the biallelic markers of the present invention and prostate cancer, the level of aggressiveness of prostate cancer tumors, an early onset of prostate cancer, or a beneficial response to or side effects related to treatment against prostate cancer can be revealed and used for diagnosis and drug screening purposes.

### Phenotypic Permutation

In order to confirm the statistical significance of the first stage haplotype analysis described above, it might be suitable to perform further analyses in which genotyping data from case-control individuals are pooled and randomized with respect to the trait phenotype. Each individual genotyping data is randomly allocated to two groups, which contain the same number of individuals as the case-control populations used to compile the data obtained in the first stage. A second stage haplotype analysis is preferably run on these artificial groups, preferably for the markers included in the haplotype of the first stage analysis showing the highest relative risk coefficient. This experiment is reiterated preferably at least between 100 and 10000 times. The repeated iterations allow the determination of the probability to obtain by chance the tested haplotype.

### Assessment Of Statistical Association

To address the problem of false positives similar analysis may be performed with the same case-control populations in random genomic regions. Results in random regions and the candidate region are compared as described in a co-pending US Provisional Patent Application entitled "Methods, Software And Apparati For Identifying Genomic Regions Harboring A Gene Associated With A Detectable Trait," U.S. Serial Number 60/107,986, filed November 10, 1998.

### 5) Evaluation Of Risk Factors

The association between a risk factor (in genetic epidemiology the risk factor is the presence or the absence of a certain allele or haplotype at marker loci) and a disease is measured by the odds ratio (OR) and by the relative risk (RR). If P(R⁺) is the probability of developing the disease for individuals with R and P(R⁻) is the probability for individuals without the risk factor, then the relative risk is simply the ratio of the two probabilities, that is: $RR = P ⁢ \left({R}^{+}\right) / P ⁢ \left({R}^{-}\right)$

In case-control studies, direct measures of the relative risk cannot be obtained because of the sampling design. However, the odds ratio allows a good approximation of the relative risk for low-incidence diseases and can be calculated: $OR = \left({R}^{+}/\left(1-{F}^{+}\right)\right) / \left({F}^{-}/\left(1-{F}^{-}\right)\right)$

F⁺ is the frequency of the exposure to the risk factor in cases and F⁻ is the frequency of the exposure to the risk factor in controls. F⁺ and F are calculated using the allelic or haplotype frequencies of the study and further depend on the underlying genetic model (dominant, recessive, additive...).

One can further estimate the attributable risk (AR) which describes the proportion of individuals in a population exhibiting a trait due to a given risk factor. This measure is important in quantifying the role of a specific factor in disease etiology and in terms of the public health impact of a risk factor. The public health relevance of this measure lies in estimating the proportion of cases of disease in the population that could be prevented if the exposure of interest were absent. AR is determined as follows: $AR = {P}_{E} ⁢ \left(RR-1\right) / \left({P}_{E}⁢\left(RR-1\right)+1\right)$

AR is the risk attributable to a biallelic marker allele or a biallelic marker haplotype. P_{E} is the frequency of exposure to an allele or a haplotype within the population at large; and RR is the relative risk which, is approximated with the odds ratio when the trait under study has a relatively low incidence in the general population.

### Biallelic Markers Of The Invention In Methods Of Genetic Diagnostics

The biallelic markers of the present invention can also be used to develop diagnostics tests capable of identifying individuals who express a detectable trait as the result of a specific genotype or individuals whose genotype places them at risk of developing a detectable trait at a subsequent time. The trait analyzed using the present diagnostics may be any detectable trait, including susceptibility to cancer, preferably prostate cancer, the level of aggressiveness of prostate cancer tumors, an early onset of prostate cancer, a beneficial response to or side effects related to treatment against prostate cancer.

Information resulting from single marker association and for haplotype analyses is extremely valuable as it can, in certain circumstances, be used to initiate preventive treatments or to allow an individual carrying a significant haplotype to foresee warning signs such as minor symptoms. In diseases such as prostate cancer, in which metastasis can be fatal if not stopped in time, the knowledge of a potential predisposition, might contribute in a very significant manner to treatment efficacy. Similarly, a diagnosed predisposition to a potential side-effect could immediately direct the physician toward a treatment for which such side-effects have not been observed during clinical trials.

The invention concerns a method for the detection in an individual of alleles of *PCTA-1-*related biallelic markers associated with prostate cancer, an early onset of prostate cancer, a susceptibility to prostate cancer, the level of aggressiveness of prostate cancer tumors, or the level of expression of the *PCTA-1* gene. The information obtained using this method is useful in the diagnosis, staging, monitoring, prognosis and/or prophylactic or curative therapy of prostate cancer. The method also concerns the detection of specific alleles present within the *PCTA-1* gene expressing a modified level of *PCTA-1* mRNA or an altered *PCTA-1* mRNA, coding for an altered PCTA-1 protein. The identities of the polymorphic bases may be determined using any of the genotyping procedures described above in "Method For Genotyping An Individual For Biallelic Markers". More particularly, the invention concerns the detection of a *PCTA-1* nucleic acid comprising at least one of the nucleotide sequences of P1 to P125 and the complementary sequence thereof. This method comprises the following steps:
- obtaining a nucleic acid sample from the individual to be tested; and
- determining the presence in the sample of an allele of a biallelic marker selected from the group consisting of A2, A30, A41, A55 and A57.

In preferred embodiments of the detection method described above, the presence of alleles of one or more biallelic markers of the *PCTA-1* gene is determined through microsequencing reactions. Optionally, the microsequencing primers are selected from the group consisting of D1 to D125 and E1 to E125. Optionally, the microsequencing primers can be bound to a solid support, preferably in the form of arrays of primers attached to appropriate chips or be used in microfluidic devices. Such arrays are described in further detail in the "Oligonucleotide arrays" section. Optionally, the microsequencing primers can be labeled.

In additional preferred embodiments of the detection method, the presence of alleles of one or more biallelic markers of the *PCTA-1* gene selected from the group consisting of A2, A30, A41, A55 and A57 is determined through an allele specific amplification assay or an enzyme based mismatch detection assay. Optionally, the allele specific amplification assay comprises a step of detecting the presence of the amplification product.

In further preferred embodiments of the detection method, the presence of alleles of one or more biallelic markers of the *PCTA-1* gene selected from the group consisting of A2, A30, A41, A55 and A57 is determined through a hybridization assay. Preferably, the probe is labeled.

A diagnostic method according to the present invention can also consist on the detection of an allele of the *PCTA-1* gene comprising a trait causing mutation.

The invention also specifically relates to a method of determining whether an individual suffering from prostate cancer or susceptible of developing prostate cancer is likely to respond positively to treatment with a selected medicament acting against prostate cancer.

The method comprises the following steps:
- obtaining a DNA sample from the individual to be tested; and
- analyzing said DNA sample to determine whether it comprises alleles of one or more bialielic markers associated with a positive response to treatment with the medicament and/or alleles of one or more biallelic markers associated with a negative response to treatment with the medicament.

The biallelic marker is selected from the group consisting of A2, A30, A41, A55 and/or A57, and the complements thereof.

The detection methods of the present invention can be applied to, for example, the preliminary screening of patient populations suffering from prostate cancer. This preliminary screening is useful to initiate adequate treatment when needed or to determine and select appropriate patient populations for clinical trials on new compounds in order to avoid the potential occurrence of specific side effects or to enhance the probability of beneficial patient response. By establishing in advance a homogeneous genotype selection for the population to be tested, the assessment of drug efficacy and/or toxicity can be more readily achieved and less hampered by divergences in population response. This approach can yield better therapeutic approaches based on patient population targeting resulting from pharmacogenomics studies.

The invention also relates to diagnostic kits useful for determining the presence in a DNA sample of alleles associated with the trait, preferably with prostate cancer, with an early onset of prostate cancer, with the level of aggressiveness of prostate cancer tumors, with a modified or forthcoming expression of the *PCTA-1* gene, with a modified or forthcoming production of the PCTA-1 protein, or with the production of a modified PCTA-1 protein. Diagnostic kits can comprise any of the polynucleotides of the present invention.

In a first embodiment, the kit comprises primers such as those described above, preferably forward and reverse primers which are used to amplify the *PCTA-1* gene or a fragment thereof. In some embodiments, at least one of the primers is complementary to a nucleotide sequence of the *PCTA-1* gene comprising a biallelic marker associated with prostate cancer, with an early onset of prostate cancer, with the level of aggressiveness of prostate cancer tumors, with a modified or forthcoming expression of the *PCTA-1* gene, with a modified or forthcoming production of the PCTA-1 protein, or with the production of a modified PCTA-1 protein. Optionally, the kit comprises an amplification primer which includes a polymorphic base of at least one biallelic marker selected from the group consisting of A 1 to A 125 and the complements thereof.

In a second embodiment, the kit comprises microsequencing primers, wherein at least one of said primers is an oligonucleotide capable of hybridizing, either with the coding or with the non-coding strand, immediately upstream of the polymorphic base of a biallelic marker selected from the group consisting of A2, A30, A41, A55 and/or A57 and the complements thereof.

In a third embodiment, the kit comprises a hybridization DNA probe, that is or eventually becomes immobilized on a solid support, which is capable of hybridizing with the *PCTA-1* gene or fragment thereof, preferably which is capable of hybridizing with a region of the *PCTA-1* gene which comprises an allele of a biallelic marker of the present invention, more preferably an allele associated with prostate cancer, with an early onset of prostate cancer, with a susceptibility to prostate cancer, with the level of aggressiveness of prostate cancer tumors, with a modified or forthcoming expression of the *PCTA-1* gene, with a modified or forthcoming production of the PCTA-1 protein, or with the production of a modified PCTA-1 protein.

The kits of the present invention can also comprise optional elements including appropriate amplification reagents such as DNA polymerases when the kit comprises primers, reagents useful in hybridization reactions and reagents useful to reveal the presence of a hybridization reaction between a labeled hybridization probe and the *PCTA-1* gene containing at least one biallelic marker.

### Recombinant Vectors

The term "vector" is used herein to designate either a circular or a linear DNA or RNA molecule, which is either double-stranded or single-stranded, and which comprise at least one polynucleotide of interest that is sought to be transferred in a cell host or in a unicellular or multicellular host organism.

The present invention discloses a recombinant vector. This recombinant vector comprises a nucleotide sequence encoding a regulatory region of the *PCTA-1* gene, the promoter region of the *PCTA-1* gene, an intron of the *PCTA-1* gene, exon 0 and/or exon 1 of the *PCTA-1* gene, exon 6bis of the *PCTA-1* gene, exon 9bis of the *PCTA-1* gene, the genomic sequence of the *PCTA-1* gene, a cDNA sequence of the *PCTA-1* gene, or combinations of such sequences, or complementary sequences thereto or fragments or variants thereof. Preferred nucleotide sequences included in such an expression vector include at least one nucleotide sequence selected from the group consisting of SEQ ID Nos 1, 2, 3, 4, 8 or fragments or variants thereof or a complementary sequence thereto.

Generally, a recombinant vector of the invention may comprise any of the polynucleotides described herein, including regulatory sequences and coding sequences, as well as any *PCTA-1* primer or probe as defined above. More particularly, the recombinant vectors of the present invention can comprise any of the polynucleotides described in the "*PCTA-1* cDNA Sequences" section, the "Coding Regions" section, and the "Oligonucleotide Probes And Primers" section.

The vector includes a *PCTA-1* gene or cDNA or a fragment thereof comprising at least one of the biallelic markers A2, A30, A41, A55 and/or A57, and more preferably a mutated *PCTA-1* gene or cDNA comprising a trait causing mutation, particularly a mutation determined using the method described above.

### Cell Hosts

The invention also discloses host cells transformed by one of the vectors described above that produce either a heterologous protein, a PCTA-1 protein or fragments thereof encoded by the *PCTA-1* gene, preferably comprising at least one of the biallelic polymorphisms described herein, and more preferably a mutated *PCTA-1* gene comprising the trait causing mutation determined using the above-noted method.

The invention discloses of a host cell that has been transformed or transfected with one of the polynucleotides described herein. Also included are host cells that are transformed (prokaryotic cells) or that are transfected (eukaryotic cells) with a recombinant vector such as one of those described above. More particularly, the cell hosts of the present invention can comprise any of the polynucleotides described in the "*PCTA-1* cDNA Sequences" section, the "Coding Regions" section, and the "Oligonucleotide Probes And Primers" section.

A further recombinant cell host according to the invention comprises a polynucleotide containing a biallelic marker selected from the group consisting of A2, A30, A41, A55 and/or A57, and the complements thereof.

Generally, a recombinant host cell of the invention comprises any one of the polynucleotides or the recombinant vectors described herein.

Preferred host cells used as recipients for the expression vectors of the invention are the following:
a) Prokaryotic host cells: *Escherichia coli* strains (I.E.DF15-α strain), *Bacillus subtilis*, *Salmonella typhimurium*, and strains from species like *Pseudomonas*, *Streptomyces* and *Staphylococcus.*
b) Eukaryotic host cells: HeLa cells (ATCC N°CCL2; N°CCL2.1; N°CCL2.2), Cv I cells (ATCC N°CCL70), COS cells (ATCC N°CRL1650; N°CRL1651), Sf-9 cells (ATCC N°CRL1711), C127 cells (ATCC N° CRL-1804), 3T3 (ATCC N° CRL-6361), CHO (ATCC N° CCL-61), human kidney 293. (ATCC N° 45504; N° CRL-1573) and BHK (ECACC N° 84100501; N° 84111301).
c) Other mammalian host cells.

The *PCTA-1* gene expression in mammalian, and typically human, cells may be rendered defective, or alternatively it may be proceeded with the insertion of a *PCTA-1* genomic or cDNA sequence with the replacement of the *PCTA-1* gene counterpart in the genome of an animal cell by a *PCTA-1* polynucleotide according to the invention. These genetic alterations may be generated by homologous recombination events using specific DNA constructs that have been previously described.

One kind of cell hosts that may be used are mammal zygotes, such as murine zygotes. For example, murine zygotes may undergo microinjection with a purified DNA molecule of interest, for example a purified DNA molecule that has previously been adjusted to a concentration range from I ng/ml -for BAC inserts- 3 ng/µl -for PI bacteriophage inserts- in 10 mM Tris-HCl, pH 7.4, 250 µM EDTA containing 100 mM NaCl, 30 µM spermine, and70 µM spermidine. When the DNA to be microinjected has a large size, polyamines and high salt concentrations can be used in order to avoid mechanical breakage of this DNA, as described by Sched1 et al (1993b).

Anyone of the polynucleotides of the invention, including the DNA constructs described herein, may be introduced in an embryonic stem (ES) cell line, preferably a mouse ES cell line. ES cell lines are derived from pluripotent, uncommitted cells of the inner cell mass of pre-implantation blastocysts. Preferred ES cell lines are the following: ES-E14TG2a (ATCC n° CRL-1821), ES-D3 (ATCC n° CRL1934 and n° CRL-11632), YS001 (ATCC n° CURL-11776), 36.5 (ATCC n° CRL-11116). To maintain ES cells in an uncommitted state, they are cultured in the presence of growth inhibited feeder cells which provide the appropriate signals to preserve this embryonic phenotype and serve as a matrix for ES cell adherence. Preferred feeder cells consist of primary embryonic fibroblasts that are established from tissue of day 13- day 14 embryos of virtually any mouse strain, that are maintained in culture, such as described by Abbondanzo et al.(1993) and are inhibited in growth by irradiation, such as described by Robertson (1987), or by the presence of an inhibitory concentration of LIF, such as described by Pease and Williams (1990).

The constructs in the host cells can be used in a conventional manner to produce the gene product encoded by the recombinant sequence.

Following transformation of a suitable host and growth of the host to an appropriate cell density, the selected promoter is induced by appropriate means, such as temperature shift or chemical induction, and cells are cultivated for an additional period.

Cells are typically harvested by centrifugation, disrupted by physical or chemical means, and the resulting crude extract retained for further purification.

Microbial cells employed in the expression of proteins can be disrupted by any convenient method, including freeze-thaw cycling, sonication, mechanical disruption, or use of cell lysing agents. Such methods are well known by the skill artisan.

### EXAMPLES

### Example 1

### Detection Of PCTA-1 Biallelic Markers: DNA Extraction

Blood donors were from French Caucasian origin. They presented a sufficient diversity for being representative of a French heterogeneous population. The DNA from 100 unrelated and healthy individuals was extracted, pooled and tested for the detection of biallelic markers. The pool was constituted by mixing equivalent quantities of DNA from each individual.

30 ml of peripheral venous blood were taken from each donor in the presence of EDTA. Cells (pellet) were collected after centrifugation for 10 minutes at 2000 rpm. Red cells were lysed by a lysis solution (50 ml final volume : 10 mM Tris pH7.6; 5 mM MgCl₂; 10 mM NaCl). The solution was centrifuged (10 minutes, 2000 rpm) as many times as necessary to eliminate the residual red cells present in the supernatant, after resuspension of the pellet in the lysis solution.

The pellet of white cells was lysed overnight at 42°C with 3.7 ml of lysis solution composed of:
- 3 ml TE 10-2 (Tris-HCl 10 mM, EDTA 2 mM) / NaCl 0.4 M
- 200 µl SDS 10%
- 500 µl K-proteinase (2 mg K-proteinase in TE 10-2 / NaCl 0.4 M).

For the extraction of proteins, 1 ml saturated NaCl (6M) (1/3.5 v/v) was added. After vigorous agitation, the solution was centrifuged for 20 minutes at 10000 rpm.

For the precipitation of DNA, 2 to 3 volumes of 100% ethanol were added to the previous supernatant, and the solution was centrifuged for 30 minutes at 2000 rpm. The DNA solution was rinsed three times with 70% ethanol to eliminate salts, and centrifuged for 20 minutes at 2000 rpm. The pellet was dried at 37°C, and resuspended in I ml TE 10-1 or I ml water. The DNA concentration was evaluated by measuring the OD at 260 nm (1 unit OD = 50 µg/ml DNA).

To determine the presence of proteins in the DNA solution, the OD 260 / OD 280 ratio was determined. Only DNA preparations having a OD 260 / OD 280 ratio between 1.8 and 2 were used in the subsequent examples described below.

### Example 2

### Detection Of The Biallelic Markers: Amplification Of Genomic DNA By PCR

The amplification of specific genomic sequences of the DNA samples of example 1 was carried out on the pool of DNA obtained previously. In addition, 10 individual samples were similarly amplified.

PCR assays were performed using the following protocol:

| | |
|---|---|
| Final volume | 25 µl |
| DNA | 2 ng/µl |
| MgCl₂ | 2 mM |
| dNTP (each) | 200 µM |
| primer (each) | 2.9 ng/µl |
| Ampli Taq Gold DNA polymerase | 0.05 unit/µl |
| PCR buffer (10x = 0.1 M TrisHCl pH8.3 0.5M KCl | 1x |

Each pair of primers was designed using the sequence information of our total genomic sequence (SEQ ID No 1) and the OSP software (Hillier & Green, 1991). These primers had about 20 nucleotide in length and their respective sequences are disclosed in Table I and had the positions disclosed in Table 1 in the columns labeled "Position range of amplification primer in SEQ ID No 1" and "Complementary position range of amplification primer in SEQ ID No 1".

The primers contained a common oligonucleotide tail upstream of the specific bases targeted for amplification which was useful for sequencing.

Primers from the columns labeled "Position range of amplification primer in SEQ ID No 1," contain the following additional PU 5' sequence: TGTAAAACGACGGCCAGT; arid primers from the columns labeled "Complementary position range of amplification primer in SEQ ID No 1," contain the following RP 5' sequence: CAGGAAACAGCTATGACC. The primer containing the additional PU 5' sequence is listed in SEQ ID No 10. The primer containing the additional RP 5' sequence is listed in SEQ ID No 11.

The synthesis of these primers was performed following the phosphoramidite method, on a GENSET UFPS 24.1 synthesizer.

DNA amplification was performed on a Genius II thermocycler. After heating at 94°C for 10 min, 40 cycles were performed. Each cycle comprised: 30 sec at 94°C, 55°C for 1 min, and 30 sec at 72°C. For final elongation, 7 min at 72°C end the amplification. The quantities of the amplification products obtained were determined on 96-well microtiter plates, using a fluorometer and Picogreen as intercalant agent (Molecular Probes).

**Table 1**

| **Amplicon** | **Position range of the amplicon in SEQ ID 1** | | **Primer name** | **Position range of amplification primer in SEQ ID No 1** | | **primer name** | **Complementary position range of amplification primer in SEQ ID No 1** | |
|---|---|---|---|---|---|---|---|---|
| 99-1601 | 1 | 506 | B1 | 1 | 18 | C1 | 486 | 506 |
| 99-13801 | 2607 | 3054 | B2 | 2607 | 2627 | C2 | 3035 | 3054 |
| 99-13806 | 11883 | 12331 | B3 | 11883 | 11902 | C3 | 12313 | 12331 |
| 99-13799 | 12379 | 12909 | B4 | 12379 | 12399 | C4 | 12889 | 12909 |
| 99-13798 | 17442 | 17887 | B5 | 17442 | 17462 | C5 | 17868 | 17887 |
| 99-1602 | 21881 | 22506 | B6 | 21881 | 21899 | C6 | 22487 | 22506 |
| 99-13794 | 28669 | 29149 | B7 | 28669 | 28689 | C7 | 29131 | 29149 |
| 99-13812 | 30941 | 31457 | B8 | 30941 | 30961 | C8 | 31437 | 31457 |
| 99-13805 | 31560 | 32075 | B9 | 31560 | 31579 | C9 | 32057 | 32075 |
| 99-1587 | 34515 | 34909 | B10 | 34515 | 34535 | C10 | 34890 | 34909 |
| 99-1582 | 45325 | 46018 | B11 | 45325 | 45343 | C11 | 46000 | 46018 |
| 99-1585 | 49765 | 50310 | B12 | 49765 | 49784 | C12 | 50291 | 50310 |
| 99-1607 | 54726 | 55325 | B13 | 54726 | 54746 | C13 | 55307 | 55325 |
| 99-1577 | 64135 | 64536 | B14 | 64135 | 64153 | C14 | 64518 | 64536 |
| 99-1591 | 65202 | 65834 | B15 | 65202 | 65219 | C15 | 65815 | 65834 |
| 99-1572 | 66653 | 67295 | B16 | 66653 | 66671 | C16 | 67275 | 67295 |
| 5-169 | 67627 | 68043 | B17 | 67627 | 67646 | C17 | 68024 | 68043 |
| 5-264 | 67246 | 67696 | B18 | 67246 | 67263 | C18 | 67678 | 67696 |
| 5-170 | 67977 | 68424 | B 19 | 67977 | 67994 | C19 | 68406 | 68424 |
| 5-171 | 68322 | 68742 | B20 | 68322 | 68340 | C20 | 68725 | 68742 |
| 5-1 | 70507 | 70928 | B21 | 70507 | 70524 | C21 | 70909 | 70928 |
| 99-1578 | 79940 | 80575 | B22 | 79940 | 79957 | C22 | 80557 | 80575 |
| 99-1605 | 82057 | 82504 | B23 | 82057 | 82077 | C23 | 82484 | 82504 |
| 5-2 | 82058 | 82492 | B24 | 82058 | 82077 | C24 | 82473 | 82492 |
| 5-3 | 83561 | 83982 | B25 | 83561 | 83578 | C25 | 83965 | 83982 |
| 5-4 | 83597 | 84017 | B26 | 83597 | 83616 | C26 | 83999 | 84017 |
| 5-260 | 83793 | 84167 | B27 | 83793 | 83812 | C27 | 84148 | 84167 |
| 5-9 | 85153 | 85576 | B28 | 85153 | 85170 | C28 | 85559 | 85576 |
| 5-5 | 86239 | 86539 | B29 | 86239 | 86257 | C29 | 86519 | 86539 |
| 5-202 | 87619 | 88050 | B30 | 87619 | 87638 | C30 | 88033 | 88050 |
| 5-7 | 88104 | 88536 | B31 | 88104 | 88122 | C31 | 88519 | 88536 |
| 5-181 | 89338 | 89758 | B32 | 89338 | 89357 | C32 | 89739 | 89758 |
| 5-10 | 92722 | 93142 | B33 | 92722 | 92741 | C33 | 93124 | 93142 |
| 5-11 | 93090 | 93509 | B34 | 93090 | 93108 | C34 | 93490 | 93509 |
| 5-12 | 93460 | 93881 | B35 | 93460 | 93478 | C35 | 93862 | 93881 |
| 5-13 | 93759 | 94192 | B36 | 93759 | 93776 | C36 | 94175 | 94192 |
| 5-14 | 94127 | 94554 | B37 | 94127 | 94144 | C37 | 94535 | 94554 |
| 5-15 | 94504 | 94921 | B38 | 94504 | 94521 | C38 | 94904 | 94921 |
| 5-16 | 94833 | 95251 | B39 | 94833 | 94850 | C39 | 95232 | 95251 |
| 5-17 | 95124 | 95561 | B40 | 95124 | 95142 | C40 | 95542 | 95561 |
| 5-18 | 95290 | 95708 | B41 | 95290 | 95308 | C41 | 95689 | 95708 |
| 5-300 | 95533 | 95952 | B42 | 95533 | 95551 | C42 | 95934 | 95952 |
| 5-262 | 96097 | 96591 | B43 | 96097 | 96115 | C43 | 96574 | 96591 |
| 5-263 | 96548 | 97001 | B44 | 96548 | 96565 | C44 | 96982 | 97001 |
| 5-265 | 96901 | 97309 | B45 | 96901 | 96918 | C45 | 97292 | 97309 |
| 99-7183 | 102156 | 102604 | B46 | 102156 | 102176 | C46 | 102584 | 102604 |
| 99-7207 | 105570 | 106074 | B47 | 105570 | 105588 | C47 | 106056 | 106074 |

### Example 3

### Detection Of The Biallelic Markers: Sequencing Of Amplified Genomic DNA And Identification Of Polymorphisms

The sequencing of the amplified DNA obtained in example 2 was carried out on ABI 377 sequencers. The sequences of the amplification products were determined using automated dideoxy terminator sequencing reactions with a dye terminator cycle sequencing protocol. The products of the sequencing reactions were run on sequencing gels and the sequences were determined using gel image analysis (ABI Prism DNA Sequencing Analysis software (2.1.2 version)).

The sequence data were further evaluated using the above mentioned polymorphism analysis software designed to detect the presence of biallelic markers among the pooled amplified fragments. The polymorphism search was based on the presence of superimposed peaks in the electrophoresis pattern resulting from different bases occurring at the same position as described previously.

47 fragments of amplification were analyzed. In these segments, 125 markers were detected. The localization of the biallelic markers was as shown in Table 2. Table 3 comprises the polynucleotides defining the *PCTA-1*-related biallelic markers. They could be used as probes and their sequence are disclosed in Table 3 in "Position range of probes in SEQ ID No 1".

**Table 2**

| **Amplicon** | **BM** | **Marker Name** | **Localization in *PCTA-1* gene** | **Polymorphism (frequency %)** | | **BM position in SEQ ID** | | | |
|---|---|---|---|---|---|---|---|---|---|
| | | | | **all1** | **all2** | **No 1** | **No 2** | **No 3** | **No 4** |
| 99-1601 | A1 | 99-1601-278 | 5'regulatory | A | C | 278 | | | |
| 99-1601 | A2 | 99-1601-402 | 5'regulatory | A (66) | T | 402 | | | |
| 99-1601 | A3 | 99-1601-472 | 5'regulatory | A | T | 472 | | | |
| 99-13801 | A4 | 99-13801-100 | 5'regulatory | T | C | 2955 | | | |
| 99-13806 | A5 | 99-13806-166 | 5'regulatory | G | A | 12167 | | | |
| 99-13799 | A6 | 99-13799-376 | 5'regulatory | T | G | 12536 | | | |
| 99-13798 | A7 | 99-13798-297 | 5'regulatory | T | C | 17593 | | | |
| 99-13798 | A8 | 99-13798-284 | 5'regulatory | T | C | 17606 | | | |
| 99-1602 | A9 | 99-1602-200 | 5'regulatory | C | G | 22079 | | | |
| 99-13794 | A10 | 99-13794-186 | 5'regulatory | T | C | 28964 | | | |
| 99-13794 | A11 | 99-13794-147 | 5'regulatory | C | G | 29003 | | | |
| 99-13812 | A12 | 99-13812-384 | 5'regulatory | T | C | 31077 | | | |
| 99-13805 | A13 | 99-13805-313 | 5'regulatory | T | C | 31766 | | | |
| 99-1587 | A14 | 99-1587-281 | 5'regulatory | A | G | 34791 | | | |
| 99-1582 | A15 | 99-1582-430 | 5'regulatory | C | T | 45751 | | | |
| 99-1585 | A16 | 99-1585-465 | 5'regulatory | T | C | 49847 | | | |
| 99-1585 | A17 | 99-1585-457 | 5'regulatory | T | C | 49855 | | | |
| 99-1585 | A18 | 99-1585-426 | 5'regulatory | G | A | 49886 | | | |
| 99-1585 | A19 | 99-1585-412 | 5'regulatory | G | A | 49900 | | | |
| 99-1585 | A20 | 99-1585-406 | 5'regulatory | C | A | 49906 | | | |
| 99-1585 | A21 | 99-1585-391 | 5'regulatory | C | A | 49921 | | | |
| 99-1585 | A22 | 99-1585-373 | 5'regulatory | G | A | 49939 | | | |
| 99-1585 | A23 | 99-1585-55 | 5'regulatory | C | A | 50256 | | | |
| 99-1607 | A24 | 99-1607-373 | 5'regulatory | T | C | 54955 | | | |
| 99-1577 | A25 | 99-1577-105 | 5'regulatory | A (54) | G | 64239 | | | |
| 99-1591 | A26 | 99-1591-235 | 5'regulatory | A | G | 65436 | | | |
| 99-1591 | A27 | 99-1591-295 | 5'regulatory | G | T | 65496 | | | |
| 99-1572 | A28 | 99-1572-315 | Promoter | C | T | 66967 | | | |
| 99-1572 | A29 | 99-1572-335 | Promoter | A | G | 66987 | | | |
| 99-1572 | A30 | 99-1572-440 | Promoter | C (32) | T | 67092 | | | |
| 99-1572 | A31 | 99-1572-477 | Promoter | A | T | 67129 | | | |
| 99-1572 | A32 | 99-1572-578 | Promoter | C | T | 67229 | | | |
| 5-264 | A33 | 5-264-188 | Promoter | A | G | 67433 | | | |
| 5-169 | A34 | 5-169-97 | Promoter | G (18) | C | 67723 | | | |
| 5-169 | A35 | 5-169-208 | Promoter | A (<1) | G | 67834 | | | |
| 5-169 | A36 | 5-169-331 | Promoter | C (99) | T | 67955 | | | |
| 5-170 | A37 | 5-170-238 | Promoter | A | G | 68213 | | | |
| 5-170 | A38 | 5-170-288 | Promoter | A (1) | C | 68263 | | | |
| 5-170 | A39 | 5-170-400 | Promoter | G | C | 68375 | | | |
| 5-171 | A40 | 5-171-156 | Promoter | G | T | 68477 | | | |
| 5-171 | A41 | 5-171-204 | Promoter | C (30) | T | 68525 | | | |
| 5-171 | A42 | 5-171-273 | Promoter | A | G | 68594 | | | |
| 5-171 | A43 | 5-171-289 | Promoter | C | T | 68610 | | | |
| 5-1 | A44 | 5-1-60 | Intron 0 | C (1) | T | 70566 | | | |
| 5-1 | A45 | 5-1-222 | Exon 1 | A | G | 70728 | 176 | 176 | 176 |
| 99-1578 | A46 | 99-1578-99 | Intron 1 | G | T | 80038 | | | |
| 99-1578 | A47 | 99-1578-179 | Intron 1 | A | T | 80118 | | | |
| 99-1578 | A48 | 99-1578-231 | Intron 1 | Ins AC | | 80170 | | | |
| 99-1578 | A49 | 99-1578-245 | Intron 1 | del AT | | 80183 | | | |
| 99-1578 | A50 | 99-1578-496 | Intron 1 | C | T | 80435 | | | |
| 5-2 | A51 | 5-2-30 | Intron 1 | Ins CAG | | 82090 | | | |
| 5-2 | A52 | 5-2-109 | Intron 1 | G | T | 82165 | | | |
| 5-2 | A53 | 5-2-113 | Intron 1 | Del GTTT | | 82169 | | | |
| 5-2 | A54 | 5-2-162 | Exon 2 | A (67) | T | 82218 | 253 | 253 | 253 |
| 5-2 | A55 | 5-2-178 | Exon 2 | C (67) | T | 82234 | 269 | 269 | 269 |
| 5-2 | A56 | 5-2-213 | Exon 2 | C (33) | T | 82268 | 303 | 303 | 303 |
| 99-1605 | A57 | 99-1605-112 | Intron 2 | T (67) | C | 82393 | | | |
| 5-3 | A58 | 5-3-27 | Intron 2 | A | G | 83587 | | | |
| 5-3 | A59 | 5-3-83 | Exon 3 | C (39) | T | 83643 | 362 | 362 | 362 |
| 5-3 | A60 | 5-3-84 | Exon 3 | A (29) | G | 83644 | 363 | 363 | 363 |
| 5-3 | A61 | 5-3-248 | Exon 3 | A | G | 83808 | 527 | 527 | 527 |
| 5-3 | A62 | 5-3-321 | Intron 3 | G | T | 83881 | | | |
| 5-3 | A63 | 5-3-324 | Intron 3 | C | T | 83884 | | | |
| 5-4 | A64 | 5-4-313 | Intron 3 | A | G | 83909 | | | |
| 5-3 | A65 | 5-3-377 | Intron 3 | ins TTTG | | 83937 | | | |
| 5-4 | A66 | 5-4-351 | Intron 3 | C | T | 83947 | | | |
| 5-4 | A67 | 5-4-386 | Intron 3 | A | G | 83982 | | | |
| 5-4 | A68 | 5-4-392 | Intron 3 | GGG | TA | 83988 | | | |
| 5-260 | A69 | 5-260-255 | Intron 3 | C | T | 84047 | | | |
| 5-260 | A70 | 5-260-300 | Intron 3 | C | T | 84092 | | | |
| 5-260 | A71 | 5-260-353 | Intron 3 | C | T | 84145 | | | |
| 5-9 | A72 | 5-9-50 | Intron 3 | C | T | 85202 | | | |
| 5-5 | A73 | 5-5-21 | Intron 4 | A | G | 86259 | | | |
| 5-5 | A74 | 5-5-85 | Intron 4 | TATA AAAT ATT | ACAG GTTA TATA | 86323 | | | |
| 5-202 | A75 | 5-202-95 | Exon 6bis | G | T (<1) | 87713 | | 810 | |
| 5-202 | A76 | 5-202-117 | Exon 6bis | A (<1) | T | 87735 | | 832 | |
| 5-202 | A77 | 5-202-169 | Intron 6bis | A | C | 87787 | | | |
| 5-202 | A78 | 5-202-188 | Intron 6bis | A | G | 87806 | | | |
| 5-202 | A79 | 5-202-242 | Intron 6bis | A | G | 87860 | | | |
| 5-202 | A80 | 5-202-284 | Intron 6bis | C | T | 87902 | | | |
| 5-202 | A81 | 5-202-362 | Intron 6bis | del CC | | 87980 | | | |
| 5-202 | A82 | 5-202-394 | Intron 6bis | C | T | 88012 | | | |
| 5-7 | A83 | 5-7-113 | Intron 6bis | C | T | 88215 | | | |
| 5-7 | A84 | 5-7-181 | Intron 6bis | G | C | 88283 | | | |
| 5-7 | A85 | 5-7-195 | Exon 7 | G (25) | C | 88297 | 749 | 875 | 749 |
| 5-7 | A86 | 5-7-340 | Intron 7 | C | T | 88442 | | | |
| 5-7 | A87 | 5-7-369 | Intron 7 | A | T | 88471 | | | |
| 5-7 | A88 | 5-7-378 | Intro 7 | C | T | 88480 | | | |
| 5-181 | A89 | 5-181-57 | Intron 7 | A | G | 89394 | | | |
| 5-181 | A90 | 5-181-127 | Intron 7 | C | T | 89464 | | | |
| 5-181 | A91 | 5-181-134 | Intron 7 | C | T | 89471 | | | |
| 5-181 | A92 | 5-181-321 | Intron 8 | A | C | 89658 | | | |
| 5-10 | A93 | 5-10-39 | Exon 9 exon 9bis | C | T | 92760 | 1013 | 1139 | 1013 |
| 5-10 | A94 | 5-10-302 | Exon 9 Intron 9bis | A | G | 93023 | 1276 | 1402 | |
| 5-10 | A95 | 5-10-334 | Exon 9 Intron 9bis | A | C | 93055 | 1308 | 1434 | |
| 5-11 | A96 | 5-11-158 | Exon 9 Intron 9bis | A (22) | G | 93247 | 1500 | 1626 | |
| 5-11 | A97 | 5-11-230 | Exon 9 Intron 9bis | G | T | 93319 | 1572 | 1698 | |
| 5-11 | A98 | 5-11-234 | Exon 9 Intron 9bis | C | T | 93323 | 1576 | 1702 | |
| 5-11 | A99 | 5-11-299 | Exon 9 Intron 9bis | A | T | 93388 | 1641 | 1767 | |
| 5-11 | A100 | 5-11-304 | Exon 9 Intron 9bis | A | C | 93393 | 1646 | 1772 | |
| 5-11 | A101 | 5-11-329 | Exon 9 Intron 9bis | C | T | 93418 | 1671 | 1797 | |
| 5-12 | A102 | 5-12-56 | Exon 9 Intron 9bis | ins CTTT | | 93515 | 1768 | 1894 | |
| 5-12 | A103 | 5-12-267 | Exon 9 Intron 9bis | A | C | 93726 | 1979 | 2105 | |
| 5-13 | A104 | 5-13-145 | Exon 9 Intron 9bis | C | T | 93903 | 2156 | 2282 | |
| 5-14 | A105 | 5-14-44 | Exon 9 Intron 9bis | C | T | 94170 | 2423 | 2549 | |
| 5-14 | A106 | 5-14-93 | Exon 9 Intron 9bis | A | T | 94218 | 2471 | 2597 | |
| 5-14 | A107 | 5-14-144 | Exon 9 Intron 9bis | ins | T | 94269 | 2522 | 2648 | |
| 5-14 | A108 | 5-14-165 | Exon 9 Intron 9bis | C | T | 94290 | 2543 | 2669 | |
| 5-14 | A109 | 5-14-297 | Exon 9 Intron 9bis | A | C | 94422 | 2675 | 2801 | |
| 5-14 | A110 | 5-14-307 | Exon 9 Intron 9bis | G | T | 94432 | 2685 | 2811 | |
| 5-15 | A111 | 5-15-219 | Exon 9 Intron 9bis | A | T | 94720 | 2973 | 3099 | |
| 5-16 | A112 | 5-16-157 | Exon 9 Intron 9bis | A | G | 94989 | 3242 | 3368 | |
| 5-17 | A113 | 5-17-140 | Exon 9 Intron 9bis | A | G | 95261 | 3514 | 3640 | |
| 5-18 | A114 | 5-18-51 | Exon 9 Intron 9bis | G | T | 95340 | 3593 | 3719 | |
| 5-18 | A115 | 5-18-208 | Exon 9 Intron 9bis | A | C | 95497 | 3750 | 3876 | |
| 5-300 | A116 | 5-300-238 | Exon 9 Intron 9bis | C | T | 95770 | 4023 | 4149 | |
| 5-300 | A117 | 5-300-287 | Exon 9 Intron 9bis | A | G | 95819 | 4072 | 4198 | |
| 5-262 | A118 | 5-262-49 | Exon 9 Exon 9ter | ins | C | 96145 | 4398 | 4524 | 1461 |
| 5-262 | A119 | 5-262-85 | Exon 9 Exon 9ter | C | T | 96181 | 4434 | 4560 | 1497 |
| 5-262 | A120 | 5-262-254 | Exon 9 Exon 9ter | C | T | 96350 | 4603 | 4729 | 1666 |
| 5-263 | A121 | 5-263-404 | Exon 9 Exon 9ter | C | T | 96951 | 5204 | 5330 | 2267 |
| 5-265 | A122 | 5-265-244 | Exon 9 Exon 9ter | A | G | 97144 | 5397 | 5523 | 2460 |
| 5-265 | A123 | 5-265-376 | 3'regualtory | A | G | 97276 | | | |
| 99-7183 | A124 | 99-7183-338 | 3'regualtory | C | T | 102267 | | | |
| 99-7207 | A125 | 99-7207-138 | 3'regualtory | A | G | 105937 | | | |

BM refers to "biallelic marker". All1 and all2 refer respectively to allele I and allele 2 of the biallelic marker. "Frequency%" refers to the frequency of the allele in percentage in control population. Frequencies corresponded to a population of random blood donors of French Caucasian origin.

**Table 3**

| **BM** | **Marker Name** | **Position range of probes in SEQ ID No 1** | | **Probes** |
|---|---|---|---|---|
| A1 | 99-1601-278 | 255 | 301 | P1 |
| A2 | 99-1601-402 | 379 | 425 | P2 |
| A3 | 99-1601-472 | 449 | 495 | P3 |
| A4 | 99-13801-100 | 2932 | 2978 | P4 |
| A5 | 99-13806-166 | 12144 | 12190 | P5 |
| A6 | 99-13799-376 | 12513 | 12559 | P6 |
| A7 | 99-13798-297 | 17570 | 17616 | P7 |
| A8 | 99-13798-284 | 17583 | 17629 | P8 |
| A9 | 99-1602-200 | 22056 | 22102 | P9 |
| A10 | 99-13794-186 | 28941 | 28987 | P10 |
| A11 | 99-13794-147 | 28980 | 29026 | P11 |
| A12 | 99-13812-384 | 31054 | 31100 | P12 |
| A13 | 99-13805-313 | 31743 | 31789 | P13 |
| A14 | 99-1587-281 | 34768 | 34814 | P14 |
| A15 | 99-1582-430 | 45728 | 45774 | P15 |
| A16 | 99-1585-465 | 49824 | 49870 | P16 |
| A17 | 99-1585-457 | 49832 | 49878 | P17 |
| A18 | 99-1585-426 | 49863 | 49909 | P18 |
| A19 | 99-1585-412 | 49877 | 49923 | P19 |
| A20 | 99-1585-406 | 49883 | 49929 | P20 |
| A21 | 99-1585-391 | 49898 | 49944 | P21 |
| A22 | 99-1585-373 | 49916 | 49962 | P22 |
| A23 | 99-1585-55 | 50233 | 50279 | P23 |
| A24 | 99-1607-373 | 54932 | 54978 | P24 |
| A25 | 99-1577-105 | 64216 | 64262 | P25 |
| A26 | 99-1591-235 | 65413 | 65459 | P26 |
| A27 | 99-1591-295 | 65473 | 65519 | P27 |
| A28 | 99-1572-315 | 66944 | 66990 | P28 |
| A29 | 99-1572-335 | 66964 | 67010 | P29 |
| A30 | 99-1572-440 | 67069 | 67115 | P30 |
| A31 | 99-1572-477 | 67106 | 67152 | P31 |
| A32 | 99-1572-578 | 67206 | 67252 | P32 |
| A33 | 5-264-188 | 67410 | 67456 | P33 |
| A34 | 5-169-97 | 67700 | 67746 | P34 |
| A35 | 5-169-208 | 67811 | 67857 | P35 |
| A36 | 5-169-331 | 67932 | 67978 | P36 |
| A37 | 5-170-238 | 68190 | 68236 | P37 |
| A38 | 5-170-288 | 68240 | 68286 | P38 |
| A39 | 5-170-400 | 68352 | 68398 | P39 |
| A40 | 5-171-156 | 68454 | 68500 | P40 |
| A41 | 5-171-204 | 68502 | 68548 | P41 |
| A42 | 5-171-273 | 68571 | 68617 | P42 |
| A43 | 5-171-289 | 68587 | 68633 | P43 |
| A44 | 5-1-60 | 70543 | 70589 | P44 |
| A45 | 5-1-222 | 70705 | 70751 | P45 |
| A46 | 99-1578-99 | 80015 | 80061 | P46 |
| A47 | 99-1578-179 | 80095 | 80141 | P47 |
| A48 | 99-1578-231 | 80147 | 80193 | P48 |
| A49 | 99-1578-245 | 80160 | 80206 | P49 |
| A50 | 99-1578-496 | 80412 | 80458 | P50 |
| A51 | 5-2-30 | 82067 | 82113 | P51 |
| A52 | 5-2-109 | 82142 | 82188 | P52 |
| A53 | 5-2-113 | 82146 | 82192 | P53 |
| A54 | 5-2-162 | 82195 | 82241 | P54 |
| A55 | 5-2-178 | 82211 | 82257 | P55 |
| A56 | 5-2-213 | 82245 | 82291 | P56 |
| A57 | 99-1605-112 | 82370 | 82416 | P57 |
| A58 | 5-3-27 | 83564 | 83610 | P58 |
| A59 | 5-3-83 | 83620 | 83666 | P59 |
| A60 | 5-3-84 | 83621 | 83667 | P60 |
| A61 | 5-3-248 | 83785 | 83831 | P61 |
| A62 | 5-3-321 | 83858 | 83904 | P62 |
| A63 | 5-3-324 | 83861 | 83907 | P63 |
| A64 | 5-4-313 | 83886 | 83932 | P64 |
| A65 | 5-3-377 | 83914 | 83960 | P65 |
| A66 | 5-4-351 | 83924 | 83970 | P66 |
| A67 | 5-4-386 | 83959 | 84005 | P67 |
| A68 | 5-4-392 | 83965 | 84011 | P68 |
| A69 | 5-260-255 | 84024 | 84070 | P69 |
| A70 | 5-260-300 | 84069 | 84115 | P70 |
| A71 | 5-260-353 | 84122 | 84168 | P71 |
| A72 | 5-9-50 | 85179 | 85225 | P72 |
| A73 | 5-5-21 | 86236 | 86282 | P73 |
| A74 | 5-5-85 | 86300 | 86346 | P74 |
| A75 | 5-202-95 | 87690 | 87736 | P75 |
| A76 | 5-202-117 | 87712 | 87758 | P76 |
| A77 | 5-202-169 | 87764 | 87810 | P77 |
| A78 | 5-202-188 | 87783 | 87829 | P78 |
| A79 | 5-202-242 | 87837 | 87883 | P79 |
| A80 | 5-202-284 | 87879 | 87925 | P80 |
| A81 | 5-202-362 | 87957 | 88003 | P81 |
| A82 | 5-202-394 | 87989 | 88035 | P82 |
| A83 | 5-7-113 | 88192 | 88238 | P83 |
| A84 | 5-7-181 | 88260 | 88306 | P84 |
| A85 | 5-7-195 | 88274 | 88320 | P85 |
| A86 | 5-7-340 | 88419 | 88465 | P86 |
| A87 | 5-7-369 | 88448 | 88494 | P87 |
| A88 | 5-7-378 | 88457 | 88503 | P88 |
| A89 | 5-181-57 | 89371 | 89417 | P89 |
| A90 | 5-181-127 | 89441 | 89487 | P90 |
| A91 | 5-181-134 | 89448 | 89494 | P91 |
| A92 | 5-181-321 | 89635 | 89681 | P92 |
| A93 | 5-10-39 | 92737 | 92783 | P93 |
| A94 | 5-10-302 | 93000 | 93046 | P94 |
| A95 | 5-10-334 | 93032 | 93078 | P95 |
| A96 | 5-11-158 | 93224 | 93270 | P96 |
| A97 | 5-11-230 | 93296 | 93342 | P97 |
| A98 | 5-11-234 | 93300 | 93346 | P98 |
| A99 | 5-11-299 | 93365 | 93411 | P99 |
| A100 | 5-11-304 | 93370 | 93416 | P100 |
| A101 | 5-11-329 | 93395 | 93441 | P101 |
| A102 | 5-12-56 | 93492 | 93538 | P102 |
| A103 | 5-12-267 | 93703 | 93749 | P103 |
| A104 | 5-13-145 | 93880 | 93926 | P104 |
| A105 | 5-14-44 | 94147 | 94193 | P105 |
| A106 | 5-14-93 | 94195 | 94241 | P106 |
| A107 | 5-14-144 | 94246 | 94292 | P107 |
| A108 | 5-14-165 | 94267 | 94313 | P108 |
| A109 | 5-14-297 | 94399 | 94445 | P109 |
| A110 | 5-14-307 | 94409 | 94455 | P110 |
| A111 | 5-15-219 | 94697 | 94743 | P111 |
| A112 | 5-16-157 | 94966 | 95012 | P112 |
| A113 | 5-17-140 | 95238 | 95284 | P113 |
| A114 | 5-18-51 | 95317 | 95363 | P114 |
| A115 | 5-18-208 | 95474 | 95520 | P115 |
| A116 | 5-300-238 | 95747 | 95793 | P116 |
| A117 | 5-300-287 | 95796 | 95842 | P117 |
| A118 | 5-262-49 | 96122 | 96168 | P118 |
| A119 | 5-262-85 | 96158 | 96204 | P119 |
| A120 | 5-262-254 | 96327 | 96373 | P120 |
| A121 | 5-263-404 | 96928 | 96974 | P121 |
| A122 | 5-265-244 | 97121 | 97167 | P122 |
| A123 | 5-265-376 | 97253 | 97299 | P123 |
| A124 | 99-7183-338 | 102244 | 102290 | P124 |
| A125 | 99-7207-138 | 105914 | 105960 | P125 |

### Example 4

### Validation Of The Polymorphisms Through Microsequencing

The biallelic markers identified in example 3 were further confirmed and their respective frequencies were determined through microsequencing. Microsequencing was carried out for each individual DNA sample described in Example 1.

Amplification from genomic DNA of individuals was performed by PCR as described above for the detection of the biallelic markers with the same set of PCR primers (Table 1).

The preferred primers used in microsequencing had about 19 nucleotides in length and hybridized just upstream of the considered polymorphic base. Their sequences are disclosed in Table 4 in columns labeled " Position range of microsequencing primer mis. 1 in SEQ ID No 1" and " Complementary position range of microsequencing primer mis. 2 in SEQ ID No 1".

Mis 1 and Mis 2 respectively refer to microsequencing primers which hybridized with the non-coding strand of the *PCTA-1* gene or with the coding strand of the *PCTA-1* gene.

The microsequencing reaction was performed as follows :

10 µl of PCR products were added to 20 µl of microsequencing reaction mixture containing : 10 pmol microsequencing oligonucleotide (crude synthesis, 5 OD), 1 U Thermosequenase (Amersham E79000G), 1.25 µl Thermosequenase buffer (260 mM Tris HCl pH 9.5, 65 mM MgCl₂), and the appropriate fluorescent ddNTPs complementary to the nucleotides at the polymorphic site corresponding to the polymorphic bases (11.25 nM TAMRA-ddTTP ; 16.25 nM ROX-ddCTP ; 1.675 nM REG-ddATP ; 1.25 nM RHO-ddGTP ; Perkin Elmer, Dye Terminator Set 401095). After 4 minutes at 94°C, 20 PCR cycles of 15 sec at 55°C, 5 sec at 72°C, and 10 sec at 94°C were carried out in a thermocycler. After amplification, the unincorporated dye terminators were removed by ethanol precipitation. After discarding the supernatants, the microplate was evaporated to dryness under reduced pressure (Speed Vac) ; samples were resuspended in 2.5 µl formamide EDTA loading buffer and heated for 2 min at 95°C. 0.8 µl microsequencing reaction were loaded on a 10 % (19:1) polyacrylamide sequencing gel. The data were collected by an ABI PRISM 377 DNA sequencer and processed using the GENESCAN software (Perkin Elmer).

Following gel analysis, data were automatically processed with software that allows the determination of the alleles of biallelic markers present in each amplified fragment.

The software evaluates such factors as whether the intensities of the signals resulting from the above microsequencing procedures are weak, normal, or saturated, or whether the signals are ambiguous. In addition, the software identifies significant peaks (according to shape and height criteria). Among the significant peaks, peaks corresponding to the targeted site are identified based on their position. When two significant peaks are detected for the same position, each sample is categorized classification as homozygous or heterozygous type based on the height ratio.

**Table 4**

| **Marker Name** | **BM** | **Mis. 1** | **Position range of microsequencing primer mis. 1 in SEQ ID No 1** | | **Mis. 2** | **Complementary position range of microsequencing primer mis. 2 in SEQ ID No 1** | |
|---|---|---|---|---|---|---|---|
| 99-1601-278 | A1 | D1 | 258 | 277 | E1 | 279 | 298 |
| 99-1601-402 | A2 | **D2** | 382 | 401 | E2 | 403 | 422 |
| 99-1601-472 | A3 | D3 | 452 | 471 | E3 | 473 | 492 |
| 99-13801-100 | A4 | D4 | 2935 | 2954 | E4 | 2956 | 2975 |
| 99-13806-166 | A5 | D5 | 12147 | 12166 | E5 | 12168 | 12187 |
| 99-13799-376 | A6 | D6 | 12516 | 12535 | E6 | 12537 | 12556 |
| 99-13798-297 | A7 | D7 | 17573 | 17592 | E7 | 17594 | 17613 |
| 99-13798-284 | A8 | D8 | 17586 | 17605 | E8 | 17607 | 17626 |
| 99-1602-200 | A9 | D9 | 22059 | 22078 | E9 | 22080 | 22099 |
| 99-13794-186 | A10 | D10 | 28944 | 28963 | E10 | 28965 | 28984 |
| 99-13794-147 | A11 | D11 | 28983 | 29002 | **E11** | 29004 | 29023 |
| 99-13812-384 | A12 | D12 | 31057 | 31076 | E12 | 31078 | 31097 |
| 99-13805-313 | A13 | D13 | 31746 | 31765 | E13 | 31767 | 31786 |
| 99-1587-281 | A14 | D14 | 34771 | 34790 | **E14** | 34792 | 34811 |
| 99-1582-430 | A15 | **D15** | 45731 | 45750 | E15 | 45752 | 45771 |
| 99-1585-465 | A16 | D16 | 49827 | 49846 | E16 | 49848 | 49867 |
| 99-1585-457 | A17 | D17 | 49835 | 49854 | E17 | 49856 | 49875 |
| 99-1585-426 | A18 | D18 | 49866 | 49885 | E18 | 49887 | 49906 |
| 99-1585-412 | A19 | D19 | 49880 | 49899 | E19 | 49901 | 49920 |
| 99-1585-406 | A20 | D20 | 49886 | 49905 | E20 | 49907 | 49926 |
| 99-1585-391 | A21 | D21 | 49901 | 49920 | E21 | 49922 | 49941 |
| 99-1585-373 | A22 | D22 | 49919 | 49938 | **E22** | 49940 | 49959 |
| 99-1585-55 | A23 | D23 | 50236 | 50255 | E23 | 50257 | 50276 |
| 99-1607-373 | A24 | **D24** | 54935 | 54954 | E24 | 54956 | 54975 |
| 99-1577-105 | A25 | D25 | 64219 | 64238 | **E25** | 64240 | 64259 |
| 99-1591-235 | A26 | D26 | 65416 | 65435 | **E26** | 65437 | 65456 |
| 99-1591-295 | A27 | D27 | 65476 | 65495 | E27 | 65497 | 65516 |
| 99-1572-315 | A28 | D28 | 66947 | 66966 | E28 | 66968 | 66987 |
| 99-1572-335 | A29 | D29 | 66967 | 66986 | E29 | 66988 | 67007 |
| 99-1572-440 | A30 | **D30** | 67072 | 67091 | E30 | 67093 | 67112 |
| 99-1572-477 | A31 | D31 | 67109 | 67128 | E31 | 67130 | 67149 |
| 99-1572-578 | A32 | D32 | 67209 | 67228 | E32 | 67230 | 67249 |
| 5-264-188 | A33 | D33 | 67413 | 67432 | E33 | 67434 | 67453 |
| 5-169-97 | A34 | **D34** | 67703 | 67722 | E34 | 67724 | 67743 |
| 5-169-208 | A35 | D35 | 67814 | 67833 | **E35** | 67835 | 67854 |
| 5-169-331 | A36 | **D36** | 67935 | 67954 | E36 | 67956 | 67975 |
| 5-170-238 | A37 | D37 | 68193 | 68212 | E37 | 68214 | 68233 |
| 5-170-288 | A38 | **D38** | 68243 | 68262 | E38 | 68264 | 68283 |
| 5-170-400 | A39 | D39 | 68355 | 68374 | E39 | 68376 | 68395 |
| 5-171-156 | A40 | D40 | 68457 | 68476 | E40 | 68478 | 68497 |
| 5-171-204 | A41 | **D41** | 68505 | 68524 | E41 | 68526 | 68545 |
| 5-171-273 | A42 | D42 | 68574 | 68593 | **E42** | 68595 | 68614 |
| 5-171-289 | A43 | D43 | 68590 | 68609 | E43 | 68611 | 68630 |
| 5-1-60 | A44 | **D44** | 70546 | 70565 | E44 | 70567 | 70586 |
| 5-1-222 | A45 | D45 | 70708 | 70727 | E45 | 70729 | 70748 |
| 99-1578-99 | A46 | D46 | 80018 | 80037 | E46 | 80039 | 80058 |
| 99-1578-179 | A47 | D47 | 80098 | 80117 | E47 | 80119 | 80138 |
| 99-1578-231 | A48 | D48 | 80150 | 80169 | E48 | 80171 | 80190 |
| 99-1578-245 | A49 | D49 | 80163 | 80182 | E49 | 80184 | 80203 |
| 99-1578-496 | A50 | **D50** | 80415 | 80434 | E50 | 80436 | 80455 |
| 5-2-30 | A51 | D51 | 82070 | 82089 | E51 | 82091 | 82110 |
| 5-2-109 | A52 | D52 | 82145 | 82164 | **E52** | 82166 | 82185 |
| 5-2-113 | A53 | **D53** | 82149 | 82168 | **E53** | 82170 | 82189 |
| 5-2-162 | A54 | **D54** | 82198 | 82217 | E54 | 82219 | 82238 |
| 5-2-178 | A55 | D55 | 82214 | 82233 | **E55** | 82235 | 82254 |
| 5-2-213 | A56 | **D56** | 82248 | 82267 | **E56** | 82269 | 82288 |
| 99-1605-112 | A57 | **D57** | 82373 | 82392 | E57 | 82394 | 82413 |
| 5-3-27 | A58 | D58 | 83567 | 83586 | E58 | 83588 | 83607 |
| 5-3-83 | A59 | **D59** | 83623 | 83642 | E59 | 83644 | 83663 |
| 5-3-84 | A60 | D60 | 83624 | 83643 | **E60** | 83645 | 83664 |
| 5-3-248 | A61 | D61 | 83788 | 83807 | **E61** | 83809 | 83828 |
| 5-3-321 | A62 | D62 | 83861 | 83880 | E62 | 83882 | 83901 |
| 5-3-324 | A63 | D63 | 83864 | 83883 | E63 | 83885 | 83904 |
| 5-4-313 | A64 | D64 | 83889 | 83908 | **E64** | 83910 | 83929 |
| 5-3-377 | A65 | D65 | 83917 | 83936 | E65 | 83938 | 83957 |
| 5-4-351 | A66 | D66 | 83927 | 83946 | E66 | 83948 | 83967 |
| 5-4-386 | A67 | D67 | 83962 | 83981 | E67 | 83983 | 84002 |
| 5-4-392 | A68 | D68 | 83968 | 83987 | E68 | 83989 | 84008 |
| 5-260-255 | A69 | D69 | 84027 | 84046 | E69 | 84048 | 84067 |
| 5-260-300 | A70 | D70 | 84072 | 84091 | E70 | 84093 | 84112 |
| 5-260-353 | A71 | D71 | 84125 | 84144 | E71 | 84146 | 84165 |
| 5-9-50 | A72 | D72 | 85182 | 85201 | E72 | 85203 | 85222 |
| 5-5-21 | A73 | D73 | 86239 | 86258 | E73 | 86260 | 86279 |
| 5-5-85 | A74 | D74 | 86303 | 86322 | E74 | 86324 | 86343 |
| 5-202-95 | A75 | D75 | 87693 | 87712 | **E75** | 87714 | 87733 |
| 5-202-117 | A76 | **D76** | 87715 | 87734 | E76 | 87736 | 87755 |
| 5-202-169 | A77 | D77 | 87767 | 87786 | E77 | 87788 | 87807 |
| 5-202-188 | A78 | D78 | 87786 | 87805 | E78 | 87807 | 87826 |
| 5-202-242 | A79 | D79 | 87840 | 87859 | E79 | 87861 | 87880 |
| 5-202-284 | A80 | D80 | 87882 | 87901 | E80 | 87903 | 87922 |
| 5-202-362 | A81 | D81 | 87960 | 87979 | E81 | 87981 | 88000 |
| 5-202-394 | A82 | D82 | 87992 | 88011 | E82 | 88013 | 88032 |
| 5-7-113 | A83 | D83 | 88195 | 88214 | E83 | 88216 | 88235 |
| 5-7-181 | A84 | D84 | 88263 | 88282 | E84 | 88284 | 88303 |
| 5-7-195 | A85 | **D85** | 88277 | 88296 | E85 | 88298 | 88317 |
| 5-7-340 | A86 | D86 | 88422 | 88441 | E86 | 88443 | 88462 |
| 5-7-369 | A87 | D87 | 88451 | 88470 | E87 | 88472 | 88491 |
| 5-7-378 | A88 | D88 | 88460 | 88479 | E88 | 88481 | 88500 |
| 5-181-57 | A89 | D89 | 89374 | 89393 | E89 | 89395 | 89414 |
| 5-181-127 | A90 | D90 | 89444 | 89463 | E90 | 89465 | 89484 |
| 5-181-134 | A91 | D91 | 89451 | 89470 | E91 | 89472 | 89491 |
| 5-181-321 | A92 | D92 | 89638 | 89657 | E92 | 89659 | 89678 |
| 5-10-39 | A93 | **D93** | 92740 | 92759 | **E93** | 92761 | 92780 |
| 5-10-302 | A94 | D94 | 93003 | 93022 | E94 | 93024 | 93043 |
| 5-10-334 | A95 | D95 | 93035 | 93054 | E95 | 93056 | 93075 |
| 5-11-158 | A96 | D96 | 93227 | 93246 | **E96** | 93248 | 93267 |
| 5-11-230 | A97 | D97 | 93299 | 93318 | E97 | 93320 | 93339 |
| 5-11-234 | A98 | D98 | 93303 | 93322 | E98 | 93324 | 93343 |
| 5-11-299 | A99 | D99 | 93368 | 93387 | E99 | 93389 | 93408 |
| 5-11-304 | A100 | D100 | 93373 | 93392 | E100 | 93394 | 93413 |
| 5-11-329 | A101 | D101 | 93398 | 93417 | E101 | 93419 | 93438 |
| 5-12-56 | A102 | D102 | 93495 | 93514 | E102 | 93516 | 93535 |
| 5-12-267 | A103 | D103 | 93706 | 93725 | E103 | 93727 | 93746 |
| 5-13-145 | A104 | D104 | 93883 | 93902 | E104 | 93904 | 93923 |
| 5-14-44 | A105 | D105 | 94150 | 94169 | E105 | 94171 | 94190 |
| 5-14-93 | A106 | D106 | 94198 | 94217 | E106 | 94219 | 94238 |
| 5-14-144 | A107 | D107 | 94249 | 94268 | E107 | 94270 | 94289 |
| 5-14-165 | A108 | **D108** | 94270 | 94289 | E108 | 94291 | 94310 |
| 5-14-297 | A109 | D109 | 94402 | 94421 | E109 | 94423 | 94442 |
| 5-14-307 | A110 | D110 | 94412 | 94431 | E110 | 94433 | 94452 |
| 5-15-219 | A111 | **D111** | 94700 | 94719 | E111 | 94721 | 94740 |
| 5-16-157 | A112 | D112 | 94969 | 94988 | E112 | 94990 | 95009 |
| 5-17-140 | A113 | D113 | 95241 | 95260 | E113 | 95262 | 95281 |
| 5-18-51 | A114 | D114 | 95320 | 95339 | E114 | 95341 | 95360 |
| 5-18-208 | A115 | **D115** | 95477 | 95496 | E115 | 95498 | 95517 |
| 5-300-238 | A116 | D116 | 95750 | 95769 | E116 | 95771 | 95790 |
| 5-300-287 | A117 | D117 | 95799 | 95818 | E117 | 95820 | 95839 |
| 5-262-49 | A118 | D118 | 96125 | 96144 | E118 | 96146 | 96165 |
| 5-262-85 | A119 | D119 | 96161 | 96180 | E119 | 96182 | 96201 |
| 5-262-254 | A120 | D120 | 96330 | 96349 | E120 | 96351 | 96370 |
| 5-263-404 | A121 | D121 | 96931 | 96950 | E121 | 96952 | 96971 |
| 5-265-244 | A122 | D122 | 97124 | 97143 | E122 | 97145 | 97164 |
| 5-265-376 | A123 | D123 | 97256 | 97275 | E123 | 97277 | 97296 |
| 99-7183-338 | A124 | **D124** | 102247 | 102266 | E124 | 102268 | 102287 |
| 99-7207-138 | A125 | D125 | 105917 | 105936 | E125 | 105938 | 105957 |

### Example 5

### Association Study Between Prostate Cancer And The Biallelic Markers Of The PCTA-1 Gene

### Collection Of DNA Samples From Affected And Non-Affected Individuals

### Affected population :

The positive trait followed in this association study was prostate cancer. Prostate cancer patients were recruited according to a combination of clinical, histological and biological inclusion criteria. Clinical criteria can include rectal examination and prostate biopsies. Biological criteria can include PSA assays. The affected individuals were recorded as familial forms when at least two persons affected by prostate cancer have been diagnosed in the family. Remaining cases were classified as non-familial informative cases (at least two sibs of the case both aged over 50 years old are unaffected), or non-familial uniformative cases (no information about sibs over 50 years old is available). All affected individuals included in the statistical analysis of this patent were unrelated. Cases were also separated following the criteria of diagnosis age : early onset prostate cancer (under 65 years old) and late onset prostate cancer (65 years old or more).

### Unaffected population :

Control individuals included in this study were checked for both the absence of all clinical and biological criteria defining the presence or the risk of prostate cancer (PSA < 4) (WO 96/21042), and for their age (aged 65 years old or more). All unaffected individuals included in the statistical analysis of this patent were unrelated.

The affected group was composed by 491 unrelated individuals, comprising:
- 197 familial cases among which 91 individuals were under 65 years old and 106 individuals were 65 years old or more; and
- 294 sporadic cases.

The unaffected group contained 313 individuals which were 65 years or older.

As used herein, the term "early onset cancer" refers to a cancer in which the individuals are under 65 years old.

### Genotyping Of Affected And Control Individuals

The general strategy to perform the association studies was to individually scan the DNA samples from all individuals in each of the populations described above in order to establish the allele frequencies of the above described biallelic markers in each of these populations. More particularly, the biallelic markers used in the present association study are A2, A9, A15, A22, A24, A25, A26, A30, A34, A35, A36, A38, A41, A42, A44, A51, A52, A54, A55, A56, A57, A59, A60, A64, A73, A75, A76, A85, A93, A96, A108, A111, A115.

Allelic frequencies of the above-described biallelic markers in each population were determined by performing microsequencing reactions on amplified fragments obtained by genomic PCR performed on the DNA samples from each individual. Genomic PCR and microsequencing were performed as detailed above in examples 2 and 4 using the described PCR and microsequencing primers.

### Association Study Between Prostate Cancer And The Biallelic Markers Of The PCTA-1 Gene

The alleles of two biallelic markers, namely (T) A30 and (T) A41, have been shown to be significantly associated to familial prostate cancer, more particularly early onset familial prostate cancer. Indeed, the allele T of the biallelic marker A30 showed a p-value of 1.08 x 10⁻² for the early onset familial prostate cancer and of 3.39 x 10⁻² for the familial prostate cancer. The allele T of the biallelic marker A41 presented a p-value of 4.04 x 10⁻² for the early onset familial prostate cancer. These two markers could be then used in diagnostics.

Some other biallelic markers, namely A54, A55, A56, A57, A59, A60, A61, A85, A96, A108, A115, showed a moderate association. These biallelic markers are localized in the exons and introns of the PCTA-1 gene.

The inventors observed that all the *PCTA-1*-related biallelic markers were in linkage disequilibrium with each other in the controls individuals. In the familial cases of prostate cancer, the biallelic markers localized in the promoter did not show a linkage disequilibrium with those localized in exonic and intronic region of the *PCTA-1* gene and were not in linkage disequilibrium with each other. This lack of linkage disequilibrium for the promoter biallelic markers suggests that this region comprises a trait causing mutation and could explain the cases haplotypes.

A strong association has been observed between the allele A of the biallelic marker and sporadic cases of prostate cancer. This association is highly significant with a pvalue of 7.71 x 10⁻³. The marker A2 can be then used in diagnostics.

### Haplotype Frequency Analysis

One way of increasing the statistical power of individual markers, is by performing haplotype association analysis.

Haplotype analysis for association of *PCTA-1* markers and prostate cancer was performed by estimating the frequencies of all possible haplotypes comprising biallelic markers selected from the group consisting of A2, A9, A15, A22, A24, A25, A26, A30, A34, A35, A36, A38, A41, A42, A44, A51, A52, A54, A55, A56, A57, A59, A60, A64, A73, A75, A76, A85, A93, A96, A108, A111, A115 in the cases and control populations described in Example 5, and comparing these frequencies by means of a chi square statistical test (one degree of freedom). Haplotype estimations were performed by applying the Expectation-Maximization (EM) algorithm (Excoffier L & Slatkin M, 1995), using the EM-HAPLO program (Hawley ME, Pakstis AJ & Kidd KK, 1994).

### Haplotype frequency analysis for familial cases of prostate cancer

The most significant haplotypes obtained with the familial cases of prostate cancer are shown in Table 5. These haplotypes comprise the biallelic markers A2, A30, A41, A55, A57, and 5-202/95.

The preferred two-markers haplotypes are described in Table 5 as H1 to H7 of PT2. The more preferred haplotype is the haplotype H1/PT2 and comprises the biallelic markers A30 (99-1572/440 allele T) and A41 (allele T). This haplotype presented a p-value of 1.1 x 10⁻⁴ and an odd-ratio of 1.67. Estimated haplotype frequencies were 57.2% in the cases and 44.4% in the controls.

The preferred three-markers haplotypes are described in Table 5 as H1, H2, H3, H7, H8, H9, H10, H11, and H12 of PT3. The more preferred haplotype is the haplotype H1/PT3 and comprises the biallelic markers A2 (allele A), A30 (99-1572/440 allele T) and A41 (allele T). This haplotype presented a p-value of 1.1 x 10⁻⁵ and an odd-ratio of 1.84. Estimated haplotype frequencies were 42.9% in the cases and 29% in the controls.

The preferred four-markers haplotypes are described in Table 5 as H1, H2, H4, H5, H7, H9, H16, H17, H18 and H19 of PT4.

In conclusion, most preferred haplotypes for the familial cases of prostate cancer comprise the biallelic markers A30 (99-1572/440 allele T) and/or A41 (allele T). Some other preferred haplotypes for the familial cases of prostate cancer comprise the biallelic marker A2 (allele A). Optionally, preferred haplotypes for the familial cases of prostate cancer comprise the biallelic markers A55 (allege C) and/or A57 (allele G). These haplotypes can be used in diagnostic of prostate cancer susceptibility.

### Haplotype frequency analysis for sporadic cases of prostate cancer

The most significant haplotypes obtained with the sporadic cases of prostate cancer are shown in Table 6. These haplotypes comprise the biallelic markers A2, A30, A41, A55, A57, and 5-202/95.

The preferred two-markers haplotypes are described in Table 6 as H1 to H4, H6, and H7 of PT2. The first more preferred haplotype is the haplotype H1/PT2 and comprises the biallelic markers A2 (allele T) and A55 (allele T). This haplotype presented a p-value of 2.4 x 10⁻⁴ and an odd-ratio of 1.94. Estimated haplotype frequencies were 16.2% in the cases and 9% in the controls. The second more preferred haplotype is the haplotype H2/PT2 and comprises the biallelic markers A2 (allele T) and A57 (allele A). This haplotype presented a p-value of 5.3 x 10⁻⁴ and an odd-ratio of 1.84. Estimated haplotype frequencies were 16.3% in the cases and 9.5% in the controls.

The preferred three-markers haplotypes are described in Table 6 as H1, H2, H3, H4, H6, H7, and H8 of PT3. The more preferred haplotype is the haplotype H2/PT3 and comprises the biallelic markers A2 (allele T), A55 (allele T), and A57 (allele A). This haplotype presented a p-value of 2.3 x 10⁻³ and an odd-ratio of 1.75. Estimated haplotype frequencies were 15% in the cases and 9.2% in the controls.

The preferred four-markers haplotypes are described in Table 6 as H1, H2, H3, H4, and H6 of PT4.

In conclusion, most preferred haplotypes for the sporadic cases of prostate cancer comprise a biallelic marker selected in the group consisting of A2 (allele T), A55 (allele T), and A57 (allele A). Optionally, preferred haplotypes for the sporadic cases of prostate cancer comprise the biallelic markers A30 (allele T) and/or A41 (allele T). These haplotypes can be used in diagnostic of prostate cancer.

### Summary of haplotype frequency analysis

The most preferred two- and three-biallelic markers haplotypes for the familial and sporadic prostate cancer are summarized in Table 7. These haplotypes can be used in diagnostic of prostate cancer susceptibility.

The statistical significance of the results obtained for the haplotype analysis was evaluated by a phenotypic permutation test reiterated 1000 or 10,000 times on a computer. For this computer simulation, data from the cases and control individuals were pooled and randomly allocated to two groups which contained the same number of individuals as the case-control populations used to produce the haplotype frequency analysis data. A haplotype analysis was then run on these artificial groups for the five haplotypes of the Table 7 which presented a strong association with prostate cancer. This experiment was reiterated 1000 times and the results are shown in Table 8.

The haplotypes 1 and 2 of the Table 7 are clearly associated with familial prostate cancer and more particularly with familial cases which were under 65 years and with >3caP familial cases. The permutation test clearly validate the statistical significance of the association between these haplotypes and familial prostate cancer since, among 1000 iterations, none of the obtained haplotypes had a p-value comparable to the one obtained for the haplotypes 1 and 2 of Table 7 for the familial cases, the familial cases under 65 years and the >3caP familial cases.

The haplotypes 3, 4, and 5 of the Table 7 are clearly associated with the sporadic prostate cancer. The permutation test clearly validate the statistical significance of the association between these haplotypes and sporadic prostate cancer since, among 1000 iterations, less than 6 of the obtained haplotypes had a p-value comparable to the one obtained for the haplotypes 3, 4 and 5 of Table 7 for the sporadic cases. Moreover, among 1000 iterations, none of the obtained haplotypes had a p-value comparable to the one obtained for the haplotypes 3, 4 and 5 of Table 7 for the informative sporadic cases.

### Attributable Risk

The attributable risk has been calculated as described in the "Evaluation of risk factors" of the part entitled "Statistic method". The results are disclosed in Table 9.

These results show that the preferred haplotypes disclosed in the present invention are highly significant for the prostate cancer. Indeed, 16.92 % of the sporadic prostate cancer cases carried the haplotype 4 of the Table 7 considering a dominant model which is the more relevant model for prostate cancer. Moreover, 60.77 % of the familial early onset prostate cancer cases carried the haplotype 1 of the Table 7 considering a dominant model.

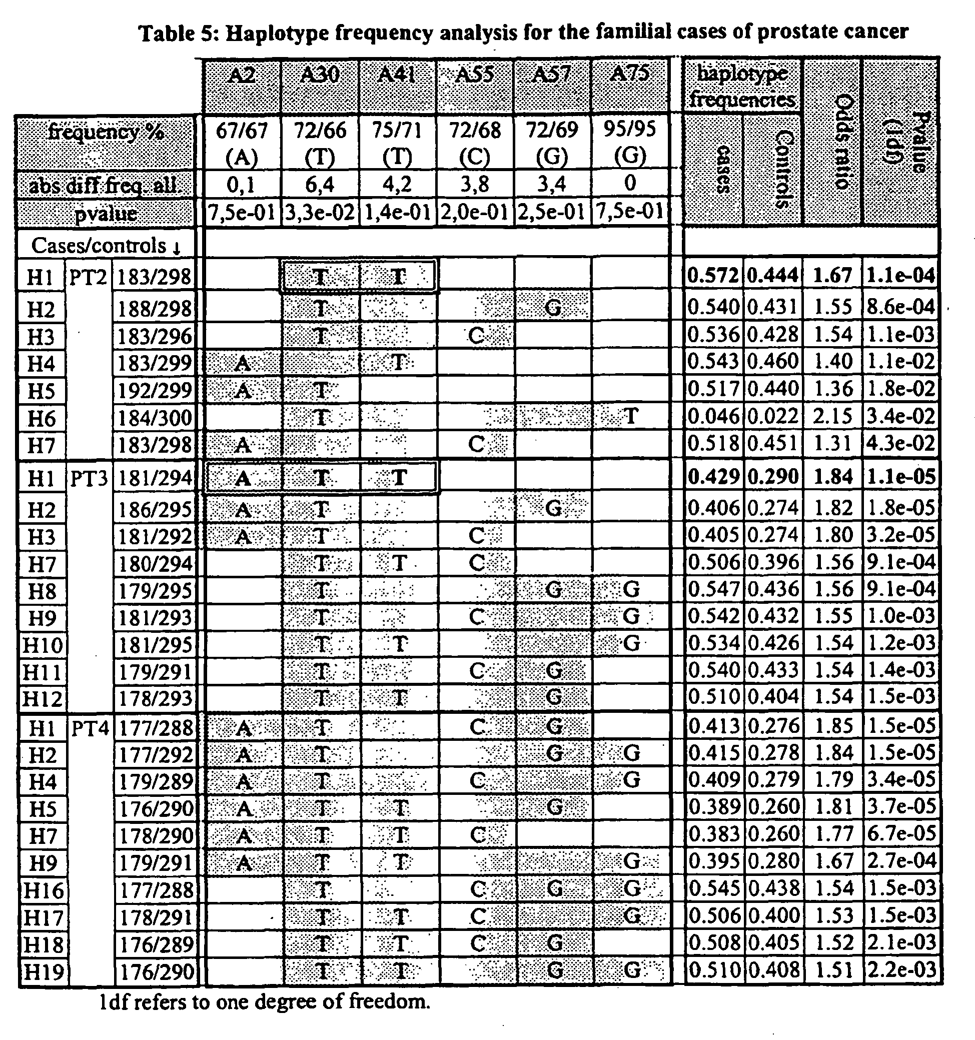

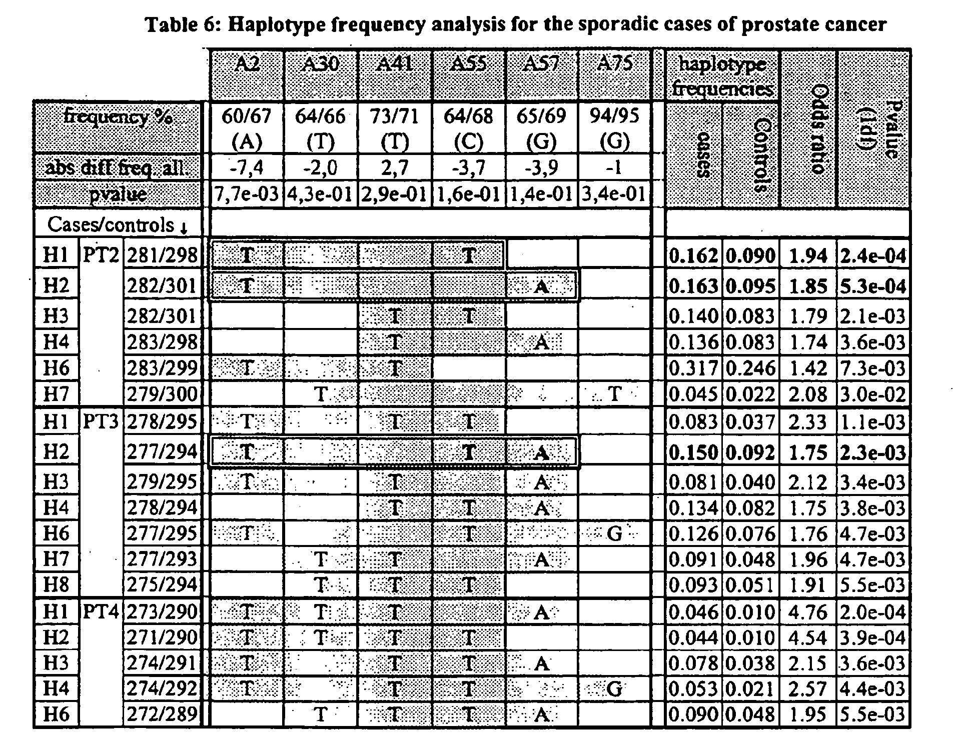

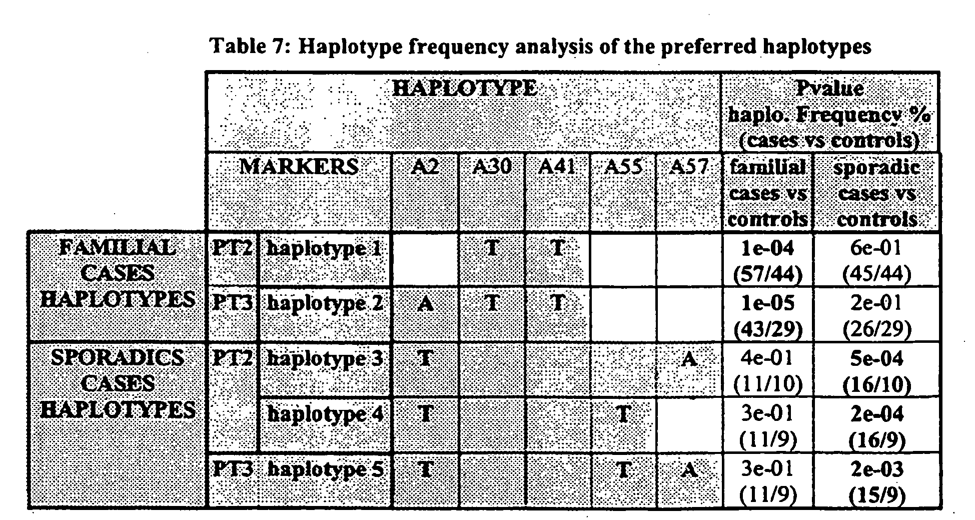

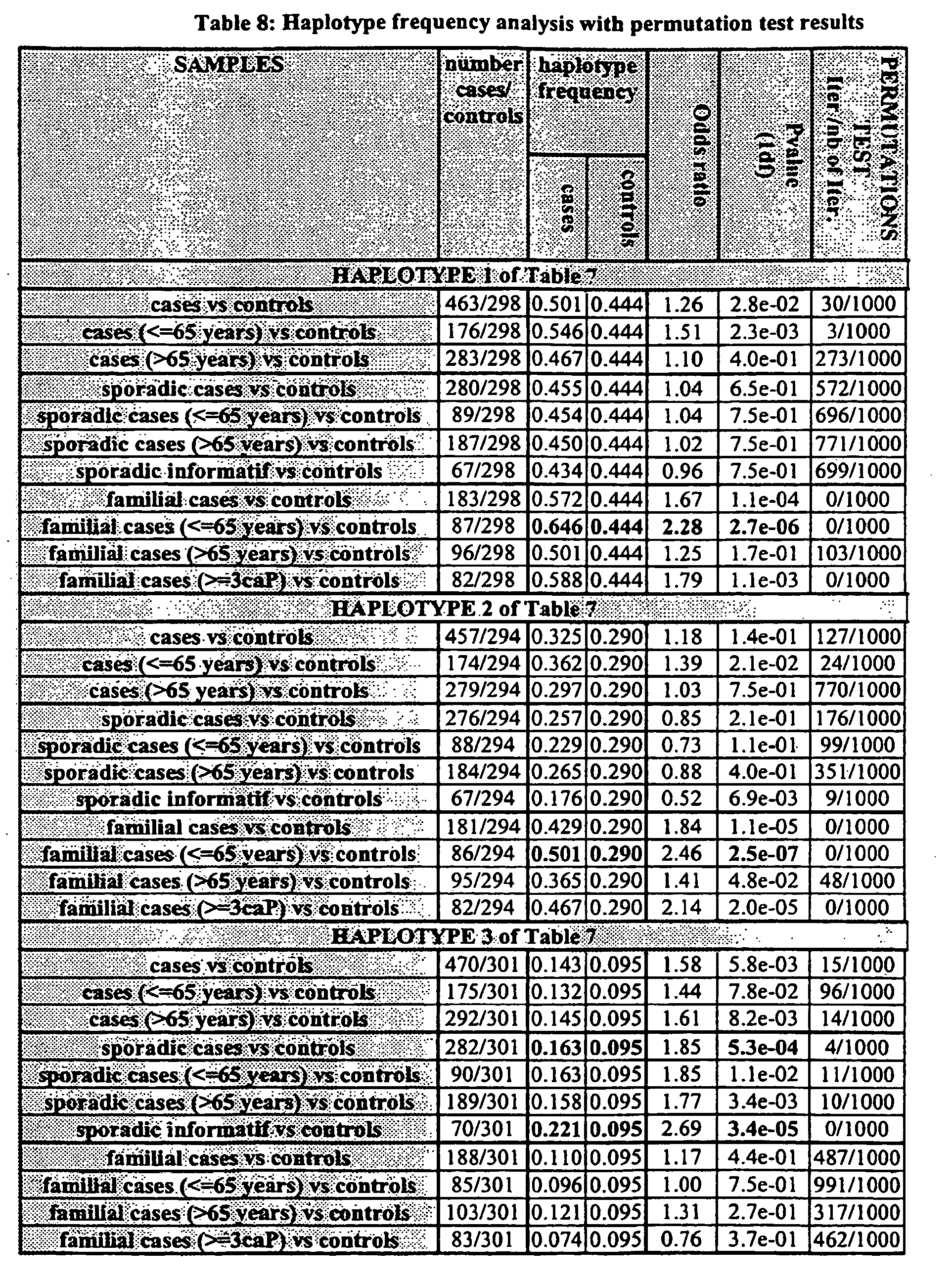

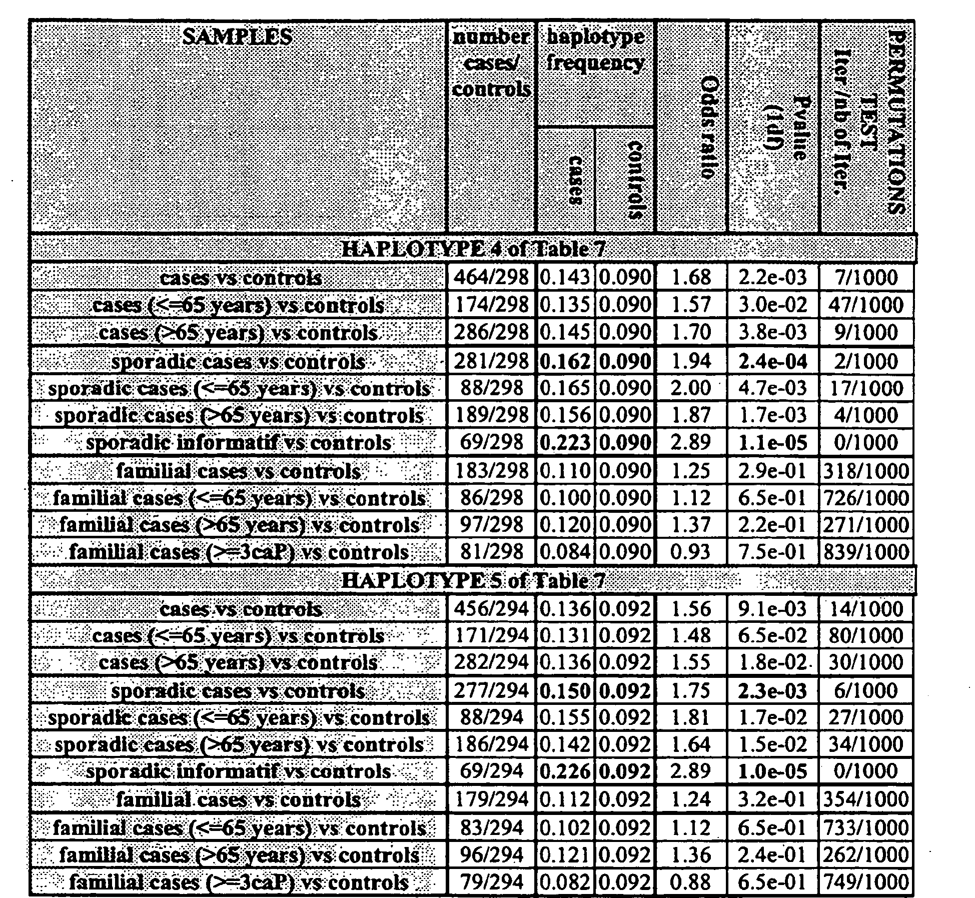

Familial forms in which at least three persons are affected by prostate cancer in the family are described in the present application as >3CaP. Sporadic cases were classified as informative sporadic cases when at least two sibs of the case both aged over 50 years old are unaffected.

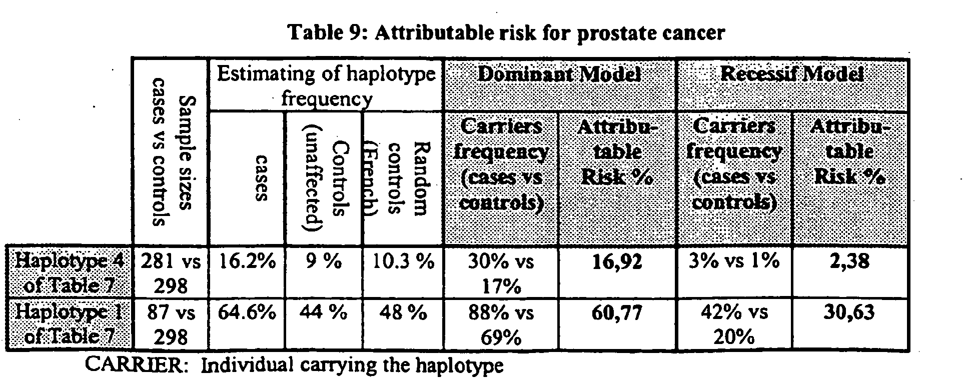

ODD RATIO of Carrier (OR): Carrier of cases* (1-Carrier of controls) / Carrier of controls * (1-Carrier of cases)
ATTRIBUTABLE RISK (RR): Carrier of Randoms controls * (OR -1) / (Carrier of Randoms controls* (OR -1) + 1)
(ref: Epidémiologie - Principes et méthodes quantitatives - J. Bouyer, D. Hémon, S. Cordier 1995)

### Example 6

### Mouse PCTA-1 protein

The inventors have cloned a cDNA molecule encoding a mouse homologue of the PCTA-1 protein (SEQ ID No 8). The deduce amino acid sequence is provided in SEQ ID No 9. Figure 3 shows alignments between the human and mouse PCTA-1 protein sequences of the inventions, as well as that of GenBank L78132. It shows an 80 % homology between the human and mouse homologues.

Further comparisons between these mouse and human cDNA and protein sequences, taking into consideration the position of significant polymorphisms in relation with potentially conserved motifs, should allow the person skilled in the art to identify regions of specific physiological interest, in the design of suitable vaccine or therapeutic candidates. Two galactoside binding sites shown in figure 7 are of special interest. These sites are conserved among the PCTA-1 proteins and the galectins, and seem to be involved in the cell-cell and cell-matrix interactions which are of high relevance to cancer. Two other sites, HFNPRF and VVCN, are also highly conserved among all these proteins.

### References

Abbondanzo SJ et al., 1993, Methods in Enzymology, Academic Press, New York, pp 803-823
Ajioka R.S. et al., Am. J. Hum. Genet., 60:1439-1447, 1997
Altschul et al., 1990, J. Mol. Biol. 215(3):403-410
Altschul et al., 1993, Nature Genetics 3:266-272
Altschul et al., 1997, Nuc. Acids Res. 25:3389-3402
Anton M. et al., 1995, J. Virol., 69 : 4600-4606
Araki K et al. (1995) Proc. Natl. Acad. Sci. U S A. 92(1):160-4.
Asz6di et al., Proteins:Structure, Function, and Genetics, Supplement 1:38-42 (1997)
Ausubel et al. (1989)Current Protocols in Molecular Biology, Green Publishing Associates and Wiley Interscience, N.Y.
Baubonis W. (1993) Nucleic Acids Res. 21(9):2025-9.
Beaucage et al., Tetrahedron Lett 1981, 22: 1859-1862
Bram RJ et al., 1993, Mol. Cell Biol., 13 : 4760-4769
Brown EL, Belagaje R, Ryan MJ, Khorana HG, Methods Enzymol 1979;68:109-151
Brutlag et al. Comp. App. Biosci. 6:237-245, 1990
Bush et al., 1997, J. Chromatogr., 777 : 311-328.
Chai H. et al. (1993) Biotechnol. Appl. Biochem. 18:259-273.
Chee et al. (1996) Science. 274:610-614.
Chen and Kwok Nucleic Acids Research 25:347-353 1997
Chen et al. (1987) Mol. Cell. Biol. 7:2745-2752.
Chen et al. Proc. Natl. Acad. Sci. USA 94/20 10756-10761,1997
Cho RJ et al., 1998, Proc. Natl. Acad. Sci. USA, 95(7) : 3752-3757.
Chou J.Y., 1989, Mol. Endocrinol., 3: 1511-1514.
Clark A.G. (1990) Mol. Biol. Evol. 7:111-122.
Coles R, Caswell R, Rubinsztein DC, Hum Mol Genet 1998;7:791-800
Compton J. (1991) Nature. 350(6313):91-92.
Davis L.G., M.D. Dibner, and J.F. Battey, Basic Methods in Molecular Biology, ed., Elsevier Press, NY, 1986
Dempster et al., (1977) J. R. Stat. Soc., 39B:1-38.
Dent DS & Latchman DS (1993) The DNA mobility shift assay. In: Transcription Factors: A Practical Approach (Latchman DS, ed.) pp1-26. Oxford: IRL Press
Dignam JD, et al, Nucleic Acids Res. 1983 Mar 11; 11(5): 1475-1489.
Doucas V, et al, EMBO J. 1991 Aug; 10(8): 2237-2245
Dynan WS, Tjian R, Cell 1983;35:79-87
Edwards et Leatherbarrow, Analytical Biochemistry, 246, 1-6 (1997)
Engvall, E., Meth. Enzymol. 70:419 (1980)
Excoffier L. and Slatkin M. (1995) Mol. Biol. Evol., 12(5): 921-927.
Feldman and Steg, 1996, Medecine/Sciences, synthese, 12:47-55
Felici F., 1991, J. Mol. Biol., Vol. 222:301-310
Fields and Song, 1989, Nature, 340 : 245-246
Fisher, D., Chap. 42 in: Manual of Clinical Immunology, 2d Ed. (Rose and Friedman, Eds.) Amer. Soc. For Microbiol., Washington, D.C. (1980)
Flotte et al. (1992) Am. J. Respir. Cell Mol. Biol. 7:349-356.
Fodor et al. (1991) Science 251:767-777.
Fraley et al. (1979) Proc. Natl. Acad. Sci. USA. 76:3348-3352.
Fried M, Crothers DM, Nucleic Acids Res 1981;9:6505-6525
Fromont-Racine M. et al., 1997, Nature Genetics, 16(3) : 277-282.
Fuller S. A. et al. (1996) Immunology in Current Protocols in Molecular Biology, Ausubel et al.Eds, John Wiley & Sons, Inc., USA.
Furth P.A. et al. (1994) Proc. Natl. Acad. Sci USA. 91:9302-9306.
Galas DJ, Schmitz A, Nucleic Acids Res 1978;5:3157-3170
Garner MM, Revzin A, Nucleic Acids Res 1981;9:3047-3060
Geysen H. Mario et al. 1984. Proc. Natl. Acad. Sci. U.S.A. 81:3998-4002
Ghosh and Bacchawat, 1991, Targeting of liposomes to hepatocytes, IN: Liver Diseases, Targeted diagnosis and therapy using specific rceptors and ligands. Wu et al. Eds., Marcel Dekeker, New York, pp. 87-104.
Gonnet et al., 1992, Science 256:1443-1445
Gopal (1985) Mol. Cell. Biol., 5:1188-1190.
Gossen M. et al. (1992) Proc. Natl. Acad. Sci. USA. 89:5547-5551.
Gossen M. et al. (1995) Science. 268:1766-1769.
Graham et al. (1973) Virology 52:456-457.
Green et al., Ann. Rev. Biochem. 55:569-597 (1986)
Griffin et al. Science 245:967-971 (1989)
Grompe, M. (1993) Nature Genetics. 5:111-117.
Grompe, M. et al. (1989) Proc. Natl. Acad. Sci. U.S.A. 86:5855-5892.
Gu H. et al. (1993) Cell 73:1155-1164.
Gu H. et al. (1994) Science 265:103-106.
Guatelli J C et al. Proc. Natl. Acad. Sci. USA. 35:273-286.
Hacia JG, Brody LC, Chee MS, Fodor SP, Collins FS, Nat Genet 1996;14(4):441-447
HaffL. A. and Smimov I. P. (1997) Genome Research, 7:378-388.
Hames B.D. and Higgins S.J. (1985) Nucleic Acid Hybridization: A Practical Approach. Hames and Higgins Ed., IRL Press, Oxford.
Harju L, Weber T, Alexandrova L, Lukin M, Ranki M, Jalanko A, Clin Chem 1993;39(11Pt 1):2282-2287
Harland et al. (1985) J. Cell. Biol. 101:1094-1095.
Harlow, E., and D. Lane. 1988. Antibodies A Laboratory Manual. Cold Spring Harbor Laboratory. pp. 53-242
Harper JW et al., 1993, Cell, 75 : 805-816
Hawley M.E. et al. (1994) Am. J. Phys. Anthropol. 18:104.
Henikoff and Henikoff, 1993, Proteins 17:49-61
Higgins et al., 1996, Methods Enzymol. 266:383-402
Hillier L. and Green P. Methods Appl., 1991, 1: 124-8.
Hoess et al. (1986) Nucleic Acids Res. 14:2287-2300.
Huang L. et al. (1996) Cancer Res 56(5):1137-1141.
Huygen et al. (1996) Nature Medicine. 2(8):893-898.
Izant JG, Weintraub H, Cell 1984 Apr;36(4):1007-15
Julan et al. (1992) J. Gen. Virol. 73:3251-3255.
Kanegae Y. et al., Nucl. Acids Res. 23:3816-3821.
Karlin and Altschul, 1990, Proc. Natl. Acad. Sci. USA 87:2267-2268
Khoury J. et al., Fundamentals of Genetic Epidemiology, Oxford University Press, NY, 1993
Kim U-J. et al. (1996) Genomics 34:213-218.
Klein et al. (1987) Nature. 327:70-73.
Kohler, G. and Milstein, C., Nature 256:495 (1975)
Koller et al. (1992) Annu. Rev. Immunol. 10:705-730.
Kozal MJ, Shah N, Shen N, Yang R, Fucini R, Merigan TC, Richman DD, Morris D, Hubbell E, Chee M, Lander and Schork, Science, 265, 2037-2048, 1994
Landegren U. et al. (1998) Genome Research, 8:769-776.
Lange K. (1997) Mathematical and Statistical Methods for Genetic Analysis. Springer, New York.
Lenhard T. et al. (1996) Gene. 169:187-190.
Linton M.F. et al. (1993) J. Clin. Invest. 92:3029-3037.
Livak et al., Nature Genetics, 9:341-342, 1995
Livak KJ, Hainer JW, Hum Mutat 1994;3(4):379-385
Lockhart et al. Nature Biotechnology 14: 1675-1680, 1996
Lucas A.H., 1994, In : Development and Clinical Uses of Haempophilus b Conjugate;
Manley JL, et al, Proc Natl Acad Sci USA. 1980 Jul; 77(7): 3855-3859
Mansour S.L. et al. (1988) Nature. 336:348-352.
Marshall R. L. et al. (1994) PCR Methods and Applications. 4:80-84.
Maxam AM, Gilbert W, Methods Enzymol 1980;65:499-560
McCormick et al. (1994) Genet. Anal. Tech. Appl. 11:158-164.
McLaughlin B.A. et al. (1996) Am. J. Hum. Genet. 59:561-569.
Mizokami A, Yeh SY, Chang C, Mol Endocrinol 1994;8:77-88
Morton N.E., Am.J. Hum. Genet., 7:277-318, 1955
Muller MM, Schreiber E, Schaffner W, Matthias P, Nucleic Acids Res 1989;17:6420
Muzyczka et al. (1992) Curr. Topics in Micro. and Immunol. 158:97-129.
Nada S. et al. (1993) Cell 73:1125-1135.
Nagy A. et al., 1993, Proc. Natl. Acad. Sci. USA, 90: 8424-8428.
Narang SA, Hsiung HM, Brousseau R, Methods Enzymol 1979;68:90-98
Neda et al. (1991) J. Biol. Chem. 266:14143-14146.
Newton et al. (1989) Nucleic Acids Res. 17:2503-2516.
Nickerson D.A. et al. (1990) Proc. Natl. Acad. Sci. U.S.A. 87:8923-8927.
Nicolau C. et al., 1987, Methods Enzymol., 149:157-76.
Nicolau et al. (1982) Biochim. Biophys. Acta. 721:185-190.
Nihei et al, Genes Chromosomes Cancer 1995;14:112-119
Nyren P, Pettersson B, Uhlen M, Anal Biochem 1993;208(1):171-175
O'Reilly et al. (1992) Baculovirus Expression Vectors: A Laboratory Manual. W. H. Freeman and Co., New York.
Ohno et al. (1994) Science. 265:781-784.
Oldenburg K.R. et al., 1992, Proc. Natl. Acad. Sci., 89:5393-5397.
Orita et al. (1989) Proc. Natl. Acad. Sci. U.S.A. 86: 2776-2770.
Ott J., Analysis of Human Genetic Linkage, John Hopkins University Press, Baltimore, 1991
Ouchterlony, O. et al., Chap. 19 in: Handbook of Experimental Immunology D. Wier (ed) Blackwell (1973)
Parmley and Smith, Gene, 1988, 73:305-318
Pastinen et al., Genome Research 1997; 7:606-614
Pearson and Lipman, 1988, Proc. Natl. Acad. Sci. USA 85(8):2444-2448
Pease S. ans William R.S., 1990, Exp. Cell. Res., 190: 209-211.
Perlin et al. (1994) Am. J. Hum. Genet. 55:777-787.
Peterson et al., 1993, Proc. Natl. Acad. Sci. USA, 90 : 7593-7597.
Pietu et al. Genome Research 6:492-503, 1996
Potter et al. (1984) Proc. Natl. Acad. Sci. U.S.A. 81(22):7161-7165.
Ramunsen et al., 1997, Electrophoresis, 18 : 588-598.
Reid L.H. et al. (1990) Proc. Natl. Acad. Sci. U.S.A. 87:4299-4303.
Risch, N. and Merikangas, K. (Science, 273:1516-1517, 1996
Robertson E., 1987, Embryo-derived stem cell lines. In: E.J. Robertson Ed. Teratocarcinomas and embrionic stem cells: a practical approach. IRL Press, Oxford, pp. 71.
Rossi et al., Pharmacol. Ther. 50:245-254, (1991)
Roth J.A. et al. (1996) Nature Medicine. 2(9):985-991.
Roux et al. (1989) Proc. Natl. Acad. Sci. U.S.A. 86:9079-9083.
Ruano et al. (1990) Proc. Natl. Acad. Sci. U.S.A. 87:6296-6300.
Sambrook, J., Fritsch, E.F., and T. Maniatis. (1989) Molecular Cloning: A Laboratory Manual. 2ed. Cold Spring Harbor Laboratory, Cold Spring Harbor, New York.
Samson M, et al. (1996) Nature, 382(6593):722-725.
Samulski et al. (1989) J. Virol. 63:3822-3828.
Sanchez-Pescador R. (1988) J. Clin. Microbiol. 26(10):1934-1938.
Sarkar, G. and Sommer S.S. (1991) Biotechniques.
Sauer B. et al. (1988) Proc. Natl. Acad. Sci. U.S.A. 85:5166-5170.
Saunders AM, et al, Neurology 1993;43;1467-1472
Schaid D.J. et al., Genet. Epidemiol.,13:423-450, 1996
Schedl A. et al., 1993a, Nature, 362: 258-261.
Schedl et al., 1993b, Nucleic Acids Res., 21: 4783-4787.
Schena et al. Science 270:467-470, 1995
Schena et al., 1996, Proc Natl Acad Sci U S A,.93(20):10614-10619.
Schneider et al.(1997) Arlequin: A Software For Population Genetics Data Analysis. University of Geneva.
Schreiber E, Matthias P, Muller MM, Schaffner W, Nucleic Acids Res 1989 ;17:6419
Schwartz and Dayhoff, eds., 1978, Matrices for Detecting Distance Relationships: Atlas of Protein Sequence and Structure, Washington: National Biomedical Research Foundation
Sczakiel G. et al. (1995) Trends Microbiol. 3(6):213-217.
Shay J.W. et al., 1991, Biochem. Biophys. Acta, 1072: 1-7.
Sheffield, V.C. et al. (1991) Proc. Natl. Acad. Sci. U.S.A. 49:699-706.
Shizuya et al. (1992) Proc. Natl. Acad. Sci. U.S.A. 89:8794-8797.
Shoemaker DD, et al., Nat Genet 1996;14(4):450-456
Siebenlist U, Gilbert W, Proc Natl Acad Sci U S A 1980;77:122-126
Smith (1957) Ann. Hum. Genet. 21:254-276.
Smith et al. (1983) Mol. Cell. Biol. 3:2156-2165.
Sosnowski RG, et al., Proc Natl Acad Sci USA 1997;94:1119-1123
Sowdhamini et al., Protein Engineering 10:207, 215 (1997)
Spielmann S. and Ewens W.J., Am. J. Hum. Genet., 62:450-458, 1998
Spielmann S. et al., Am. J. Hum. Genet., 52:506-516, 1993
Sternberg N.L. (1994) Mamm. Genome. 5:397-404.
Strittmatter WJ, et al., Proc Natl Acad Sci U S A 1993 ;90:1977-1981
Stryer, L., Biochemistry, 4th edition, 1995
Syvanen AC, Clin Chim Acta 1994;226(2):225-236
Szabo A. et al. Curr Opin Struct Biol 5, 699-705 (1995)
Szabo et al., 1995, Curr Opin Struct Biol., 5(5):699-705
Tacson et al. (1996) Nature Medicine. 2(8):888-892.
Te Riele et al. (1990) Nature. 348:649-651.
Terwilliger J.D. and Ott J., Handbook of Human Genetic Linkage, John Hopkins University Press, London, 1994
Thomas K.R. et al. (1986) Cell. 44:419-428.
Thomas K.R. et al. (1987) Cell. 51:503-512.
Thompson et al., 1994, Nucleic Acids Res. 22(2):4673-4680
Tur-Kaspa et al. (1986) Mol. Cell. Biol. 6:716-718.
Tyagi et al. (1998) Nature Biotechnology. 16:49-53.
Urdea M.S. (1988) Nucleic Acids Research. 11:4937-4957.
Urdea M.S. et al.(1991) Nucleic Acids Symp. Ser. 24:197-200.
Vaitukaitis, J. et al. J. Clin. Endocrinol. Metab. 33:988-991 (1971)
Valadon P., et al., 1996, J. Mol. Biol., 261:11-22.
Van der Lugt et al. (1991) Gene. 105:263-267.
Vlasak R. et al. (1983) Eur. J. Biochem. 135:123-126.
Wabiko et al. (1986) DNA.5(4):305-314.
Walker et al. (1996) Clin. Chem. 42:9-13.
Wang et al., 1997, Chromatographia, 44 : 205-208.
Weir, B.S. (1996) Genetic data Analysis II: Methods for Discrete population genetic Data, Sinauer Assoc., Inc., Sunderland, MA, U.S.A.
Westerink M.A.J., 1995, Proc. Natl. Acad. Sci., 92:4021-4025
White, M.B. et al. (1992) Genomics. 12:301-306.
White, M.B. et al. (1997) Genomics. 12:301-306.
Wong et al. (1980) Gene. 10:87-94.
Wood S.A. et al., 1993, Proc. Natl. Acad. Sci. USA, 90: 4582-4585.
Wu and Wu (1987) J. Biol. Chem. 262:4429-4432.
Wu and Wu (1988) Biochemistry. 27:887-892.
Wu et al. (1989) Proc. Natl. Acad. Sci. U.S.A. 86:2757.
Yagi T. et al. (1990) Proc. Natl. Acad. Sci. U.S.A. 87:9918-9922.
Zhao et al., Am. J. Hum. Genet., 63:225-240, 1998
Zou Y. R. et al. (1994) Curr. Biol. 4:1099-1103.

### SEQUENCE LISTING FREE TEXT

The following free text appears in the accompanying Sequence Listing :
5' regulatory region
3'regulatory region
Stop
Homology genset EST
Homology with sequence in ref
Diverging nucleotide in ref
Of
or
Deletion
insertion
Polymorphic base
Potential
complement
Probe
Polymorphic amino acid
Sequencing oligonucleotide primerPU
Sequencing oligonucleotide primer RP
   <110> Genset SA
      <120> Polymorphic markers of prostate carcinoma antigen-1 PCTA-1
      <130> D18248
      <150> US 60/088,187
      <151> 1998-06-05
      <150> US 60/102,324
      <151> 1998-09-28
      <160> 11
      <170> Patent.pm
   <210> 1
      <211> 106746
      <212> DNA
      <213> Homo sapiens
   <220>
      <221> misc_feature
      <222> 1..68647
      <223> 5'regulation region
   <220>
      <221> misc_feature
      <222> 66647..68647
      <223> promoter
   <220>
   <221> misc_feature
      <222> 97156..106746
      <223> 3'regulation region
   <220>

   <221> exon
   <222> 68648..68741
   <223> exon0
   <220>
      <221> exon
      <222> 70647..70794
      <223> exon1
   <220>
      <221> exon
      <222> 82208..82296
      <223> exon2
   <220>
      <221> exon
      <222> 83613..83823
      <223> exon3
   <220>
      <221> exon
      <222> 85298..85417
      <223> exon4
   <220>
      <221> exon
      <222> 86389..86445
      <223> exon5
   <220>
      <221> exon
      <222> 87496..87522
      <223> exon6
   <220>
      <221> exon
      <222> 87650..87775
      <223> exon6bis
   <220>
      <221> exon
      <222> 88295..88383
      <223> exon7
   <220>
      <221> exon
      <222> 89484..89649
      <223> exon8
   <220>
      <221> exon
      <222> 92749..97155
      <223> exon9
   <220>
      <221> exon
      <222> 92749..92883
      <223> exon9bis
   <220>
      <221> exon
      <222> 95821..97155
      <223> exon9ter
   <220>
      <221> misc_feature
      <222> 70647..70794
      <223> homology with genset EST : A241850
   <220>
      <221> misc_feature
      <222> 68648..68741
      <223> homology with genset EST : A241850

   <220>
      <221> misc_feature
      <222> 82208..82229
      <223> homology with genset EST : A241850
   <220>
      <221> allele
      <222> 278
      <223> 99-1601-278 : polymorphic base A or C
   <220>
      <221> allele
      <222> 402
      <223> 99-1601-402 : polymorphic base A or T
   <220>
      <221> allele
      <222> 472
      <223> 99-1601-472 : polymorphic base A or T
   <220>
      <221> allele
      <222> 2955
      <223> 99-13801-100 : polymorphic base T or C
   <220>
      <221> allele
      <222> 12167
      <223> 99-13806-166 : polymorphic base G or A
   <220>
      <221> allele
      <222> 12536
      <223> 99-13799-376 : polymorphic base T or G
   <220>
      <221> allele
      <222> 17593
      <223> 99-13798-297 : polymorphic base T or C
   <220>
      <221> allele
      <222> 17606
      <223> 99-13798-284 : polymorphic base T or C
   <220>
      <221> allele
      <222> 22079
      <223> 99-1602-200 : polymorphic base G or C
   <220>
      <221> allele
      <222> 28964
      <223> 99-13794-186 : polymorphic base T or C
   <220>
      <221> allele
      <222> 29003
      <223> 99-13794-147 polymorphic base C or G
   <220>
      <221> allele
      <222> 31077
      <223> 99-13812-384 : polymorphic base T or C

   <220>
      <221> allele
      <222> 31766
      <223> 99-13805-313 : polymorphic base T or C
   <220>
      <221> allele
      <222> 34791
      <223> 99-1587-281 : polymorphic base A or G
   <220>
      <221> allele
      <222> 45751
      <223> 99-1582-430 : polymorphic base C or T
   <220>
      <221> allele
      <222> 49847
      <223> 99-1585-465 : polymorphic base T or C
   <220>
      <221> allele
      <222> 49855
      <223> 99-1585-457 : polymorphic base T or C
   <220>
      <221> allele
      <222> 49886
      <223> 99-1585-426 : polymorphic base G or A
   <220>
      <221> allele
      <222> 49900
      <223> 99-1585-412 : polymorphic base G or A
   <220>
      <221> allele
      <222> 49906
      <223> 99-1585-406 : polymorphic base C or A
   <220>
      <221> allele
      <222> 49921
      <223> 99-1585-391 : polymorphic base C or A
   <220>
      <221> allele
      <222> 49939
      <223> 99-1585-373 : polymorphic base G or A
   <220>
      <221> allele
      <222> 50256
      <223> 99-1585-55 polymorphic base C or A
   <220>
      <221> allele
      <222> 54955
      <223> 99-1607-373 : polymorphic base T or C
   <220>
      <221> allele
      <222> 64239
      <223> 99-1577-105 : polymorphic base A or G

   <220>
      <221> allele
      <222> 65436
      <223> 99-1591-235 : polymorphic base A or G
   <220>
      <221> allele
      <222> 65496
      <223> 99-1591-295 : polymorphic base G or T
   <220>
      <221> allele
      <222> 66967
      <223> 99-1572-315 : polymorphic base C or T
   <220>
      <221> allele
      <222> 66987
      <223> 99-1572-335 : polymorphic base A or G
   <220>
      <221> allele
      <222> 67092
      <223> 99-1572-440 : polymorphic base C or T
   <220>
      <221> allele
      <222> 67129
      <223> 99-1572-477 : polymorphic base A or T
   <220>
      <221> allele
      <222> 67229
      <223> 99-1572-578 : polymorphic base C or T
   <220>
      <221> allele
      <222> 67433
      <223> 5-264-188 : polymorphic base A or G
   <220>
      <221> allele
      <222> 67723
      <223> 5-169-97 : polymorphic base G or C
   <220>
      <221> allele
      <222> 67834
      <223> 5-169-208 : polymorphic base A or G
   <220>
      <221> allele
      <222> 67955
      <223> 5-169-331 : polymorphic base C or T
   <220>
      <221> allele
      <222> 68213
      <223> 5-170-238 : polymorphic base A or G
   <220>
      <221> allele
      <222> 68263
      <223> 5-170-288 : polymorphic base A or C
   <220>
      <221> allele
      <222> 68375
      <223> 5-170-400 : polymorphic base G or C

   <220>
      <221> allele
      <222> 68477
      <223> 5-171-156 : polymorphic base G or T
   <220>
      <221> allele
      <222> 68525
      <223> 5-171-204 : polymorphic base C or T
   <220>
      <221> allele
      <222> 68594
      <223> 5-171-273 : polymorphic base A or G
   <220>
      <221> allele
      <222> 68610
      <223> 5-171-289 : polymorphic base C or T
   <220>
      <221> allele
      <222> 70566
      <223> 5-1-60 : polymorphic base C or T
   <220>
      <221> allele
      <222> 70728
      <223> 5-1-222 : polymorphic base A or G
   <220>
      <221> allele
      <222> 80038
      <223> 99-1578-99 : polymorphic base G or T
   <220>
      <221> allele
      <222> 80118
      <223> 99-1578-179 : polymorphic base A or T
   <220>
      <221> allele
      <222> 80170
      <223> 99-1578-231 : insertion AC
   <220>
      <221> allele
      <222> 80183
      <223> 99-1578-245 : deletion AT
   <220>
      <221> allele
      <222> 80435
      <223> 99-1578-496 : polymorphic base C or T
   <220>
      <221> allele
      <222> 82090
      <223> 5-2-30 : insertion CAG
   <220>
      <221> allele
      <222> 82165
      <223> 5-2-109 : polymorphic base G or T

   <220>
      <221> allele
      <222> 82169
      <223> 5-2-113 : deletion GTTT
   <220>
      <221> allele
      <222> 82218
      <223> 5-2-162 : polymorphic base A or T
   <220>
      <221> allele
      <222> 82234
      <223> 5-2-178 : polymorphic base C or T
   <220>
      <221> allele
      <222> 82268
      <223> 5-2-213 : polymorphic base C or T
   <220>
      <221> allele
      <222> 82393
      <223> 99-1605-112 : polymorphic base T or C
   <220>
      <221> allele
      <222> 83587
      <223> 5-3-27 : polymorphic base A or G
   <220>
      <221> allele
      <222> 83643
      <223> 5-3-83 : polymorphic base C or T
   <220>
      <221> allele
      <222> 83644
      <223> 5-3-84 : polymorphic base A or G
   <220>
      <221> allele
      <222> 83808
      <223> 5-3-248 : polymorphic base A or G
   <220>
      <221> allele
      <222> 83881
      <223> 5-3-321 : polymorphic base G or T
   <220>
      <221> allele
      <222> 83884
      <223> 5-3-324 : polymorphic base C or T
   <220>
      <221> allele
      <222> 83909
      <223> 5-4-313 : polymorphic base A or G

   <220>
      <221> allele
      <222> 83937
      <223> 5-3-377 : insertion TTTG
   <220>
      <221> allele
      <222> 83947
      <223> 5-4-351 : polymorphic base C or T
   <220>
      <221> allele
      <222> 83982
      <223> 5-4-386 : polymorphic base A or G
   <220>
      <221> allele
      <222> 83988
      <223> 5-4-392 : polymorphic base GGG or TA
   <220>
      <221> allele
      <222> 84047
      <223> 5-260-255 : polymorphic base C or T
   <220>
      <221> allele
      <222> 84092
      <223> 5-260-300 : polymorphic base C or T
   <220>
      <221> allele
      <222> 84145
      <223> 5-260-353 : polymorphic base C or T
   <220>
      <221> allele
      <222> 85202
      <223> 5-9-50 : polymorphic base C or T
   <220>
      <221> allele
      <222> 86259
      <223> 5-5-21 : polymorphic base A or G
   <220>
      <221> allele
      <222> 86323
      <223> 5-5-85 : polymorphic base TATAAAATATT or ACAGGTTATATA
   <220>
      <221> allele
      <222> 87713
      <223> 5-202-95 : polymorphic base G or T
   <220>
      <221> allele
      <222> 87735
      <223> 5-202-117 : polymorphic base A or T
   <220>
      <221> allele
      <222> 87787
      <223> 5-202-169 : polymorphic base A or C
   <220>
      <221> allele
      <222> 87806
      <223> 5-202-188 : polymorphic base A or G
   <220>
      <221> allele
      <222> 87860
      <223> 5-202-242 : polymorphic base A or G
   <220>
      <221> allele
      <222> 87902
      <223> 5-202-284 : polymorphic base C or T
   <220>
      <221> allele
      <222> 87980
      <223> 5-202-362 : deletion CC
   <220>
      <221> allele
      <222> 88012
      <223> 5-202-394 : polymorphic base C or T
   <220>
      <221> allele
      <222> 88215
      <223> 5-7-113 : polymorphic base C or T
   <220>
      <221> allele
      <222> 88283
      <223> 5-7-181 : polymorphic base G or C

   <220>
      <221> allele
      <222> 88297
      <223> 5-7-195 : polymorphic base G or C
   <220>
      <221> allele
      <222> 88442
      <223> 5-7-340 : polymorphic base C or T
   <220>
      <221> allele
      <222> 88471
      <223> 5-7-369 : polymorphic base A or T
   <220>
      <221> allele
      <222> 88480
      <223> 5-7-378 : polymorphic base C or T
   <220>
      <221> allele
      <222> 89394
      <223> 5-181-57 : polymorphic base A or G
   <220>
      <221> allele
      <222> 89464
      <223> 5-181-127 : polymorphic base C or T
   <220>
      <221> allele
      <222> 89471
      <223> 5-181-134 : polymorphic base C or T
   <220>
      <221> allele
      <222> 89658
      <223> 5-181-321 : polymorphic base A or C
   <220>
      <221> allele
      <222> 92760
      <223> 5-10-39 : polymorphic base C or T
   <220>
      <221> allele
      <222> 93023
      <223> 5-10-302 : polymorphic base A or G
   <220>
      <221> allele
      <222> 93055
      <223> 5-10-334 : polymorphic base A or C
   <220>
      <221> allele
      <222> 93247
      <223> 5-11-158 : polymorphic base A or G
   <220>
      <221> allele
      <222> 93319
      <223> 5-11-230 : polymorphic base G or T
   <220>
      <221> allele
      <222> 93323
      <223> 5-11-234 : polymorphic base C or T

   <220>
      <221> allele
      <222> 93388
      <223> 5-11-299 : polymorphic base A or T
      <220>
      <221> allele
      <222> 93393
      <223> 5-11-304 : polymorphic base A or C
   <220>
      <221> allele
      <222> 93418
      <223> 5-11-329 : polymorphic base C or T
   <220>
      <221> allele
      <222> 93515
      <223> 5-12-56 : insertion CTTT
   <220>
      <221> allele
      <222> 93726
      <223> 5-12-267 : polymorphic base A or C
   <220>
      <221> allele
      <222> 93903
      <223> 5-13-145 : polymorphic base C or T
   <220>
      <221> allele
      <222> 94170
      <223> 5-14-44 : polymorphic base C or T
   <220>
      <221> allele
      <222> 94218
      <223> 5-14-93 : polymorphic base A or T
   <220>
      <221> allele
      <222> 94269
      <223> 5-14-144 : insertion T
   <220>
      <221> allele
      <222> 94290
      <223> 5-14-165 : polymorphic base C or T
   <220>
      <221> allele
      <222> 94422
      <223> 5-14-297 : polymorphic base A or C
   <220>
      <221> allele
      <222> 94432
      <223> 5-14-307 : polymorphic base G or T
   <220>
      <221> allele
      <222> 94720
      <223> 5-15-219 : polymorphic base A or T
   <220>
      <221> allele
      <222> 94989
      <223> 5-16-157 : polymorphic base A or G

   <220>
      <221> allele
      <222> 95261
      <223> 5-17-140 : polymorphic base A or G
   <220>
      <221> allele
      <222> 95340
      <223> 5-18-51 : polymorphic base G or T
   <220>
      <221> allele
      <222> 95497
      <223> 5-18-208 : polymorphic base A or C
   <220>
      <221> allele
      <222> 95770
      <223> 5-300-238 : polymorphic base C or T
   <220>
      <221> allele
      <222> 95819
      <223> 5-300-287 : polymorphic base A or G
   <220>
      <221> allele
      <222> 96145
      <223> 5-262-49 : insertion C
   <220>
      <221> allele
      <222> 96181
      <223> 5-262-85 : polymorphic base C or T
   <220>
      <221> allele
      <222> 96350
      <223> 5-262-254 : polymorphic base C or T
   <220>
      <221> allele
      <222> 96951
      <223> 5-263-404 : polymorphic base C or T
   <220>
      <221> allele
      <222> 97144
      <223> 5-265-244 : polymorphic base A or G
   <220>
      <221> allele
      <222> 97276
      <223> 5-265-376 : polymorphic base A or G
   <220>
      <221> allele
      <222> 102267
      <223> 99-7183-338 : polymorphic base C or T

   <220>
      <221> allele
      <222> 105937
      <223> 99-7207-138 : polymorphic base A or G
   <220>
      <221> misc_binding
      <222> 258..277
      <223> 99-1601-278.mis1 potential
   <220>
      <221> misc_binding
      <222> 279..298
      <223> 99-1601-278.mis2 potential complement
   <220>
      <221> misc_binding
      <222> 382..401
      <223> 99-1601-402.mis1
   <220>
      <221> misc_binding
      <222> 403..422
      <223> 99-1601-402.mis2 potential complement
   <220>
      <221> misc_binding
      <222> 452..471
      <223> 99-1601-472.mis1 potential
   <220>
      <221> misc_binding
      <222> 473..492
      <223> 99-1601-472.mis2 potential complement
   <220>
      <221> misc_binding
      <222> 2935..2954
      <223> 99-13801-100.mis2 potential
   <220>
      <221> misc_binding
      <222> 2956..2975
      <223> 99-13801-100.mis1 potential complement
   <220>
      <221> misc_binding
      <222> 12147..12166
      <223> 99-13806-166.mis2 potential
   <220>
      <221> misc_binding
      <222> 12168..12187
      <223> 99-13806-166.mis1 potential complement
   <220>
      <221> misc_binding
      <222> 12516..12535
      <223> 99-13799-376.mis2 potential
   <220>
      <221> misc_binding
      <222> 12537..12556
      <223> 99-13799-376.mis1 potential complement
   <220>
      <221> misc_binding
      <222> 17573..17592
      <223> 99-13798-297.mis2 potential
   <220>
      <221> misc_binding
      <222> 17594..17613
      <223> 99-13798-297.mis1 potential complement
   <220>
      <221> misc_binding
      <222> 17586..17605
      <223> 99-13798-284.mis2 potential
   <220>
      <221> misc_binding
      <222> 17607..17626
      <223> 99-13798-284.mis1 potential complement

   <220>
      <221> misc_binding
      <222> 22059..22078
      <223> 99-1602-200.mis1 potential
   <220>
      <221> misc_binding
      <222> 22080..22099
      <223> 99-1602-200.mis2 potential complement
   <220>
      <221> misc_binding
      <222> 28944..28963
      <223> 99-13794-186.mis2 potential
   <220>
      <221> misc_binding
      <222> 28965..28984
      <223> 99-13794-186.mis1 potential complement
   <220>
      <221> misc_binding
      <222> 28983..29002
      <223> 99-13794-147.mis2 potential
   <220>
      <221> misc_binding
      <222> 29004..29023
      <223> 99-13794-147.mis1 complement
   <220>
      <221> misc_binding
      <222> 31057..31076
      <223> 99-13812-384.mis2 potential
   <220>
      <221> misc_binding
      <222> 31078..31097
      <223> 99-13812-384.mis1 potential complement
   <220>
      <221> misc_binding
      <222> 31746..31765
      <223> 99-13805-313.mis2 potential
   <220>
      <221> misc_binding
      <222> 31767..31786
      <223> 99-13805-313.mis1 potential complement
   <220>
      <221> misc_binding
      <222> 34771..34790
      <223> 99-1587-281.mis1 potential
   <220>
      <221> misc_binding
      <222> 34792..34811
      <223> 99-1587-281.mis2 complement
   <220>
      <221> misc_binding
      <222> 45731..45750
      <223> 99-1582-430.mis1
   <220>
      <221> misc_binding
      <222> 45752..45771
      <223> 99-1582-430.mis2 potential complement
   <220>
      <221> misc_binding
      <222> 49827..49846
      <223> 99-1585-465.mis2 potential
   <220>
      <221> misc_binding
      <222> 49848..49867
      <223> 99-1585-465.mis1 potential complement
   <220>
      <221> misc_binding
      <222> 49835..49854
      <223> 99-1585-457.mis2 potential
   <220>
      <221> misc_binding
      <222> 49856..49875
      <223> 99-1585-457.mis1 potential complement
   <220>
      <221> misc_binding
      <222> 49866..49885
      <223> 99-1585-426.mis2 potential
   <220>
      <221> misc_binding
      <222> 49887..49906
      <223> 99-1585-426.mis1 potential complement

   <220>
      <221> misc_binding
      <222> 49880..49899
      <223> 99-1585-412.mis2 potential
   <220>
      <221> misc_binding
      <222> 49901..49920
      <223> 99-1585-412.mis1 potential complement
   <220>
      <221> misc_binding
      <222> 49886..49905
      <223> 99-1585-406.mis2 potential
   <220>
      <221> misc_binding
      <222> 49907..49926
      <223> 99-1585-406.mis1 potential complement
   <220>
      <221> misc_binding
      <222> 49901..49920
      <223> 99-1585-391.mis2 potential
   <220>
      <221> misc_binding
      <222> 49922..49941
      <223> 99-1585-391.mis1 potential complement
   <220>
      <221> misc_binding
      <222> 49919..49938
      <223> 99-1585-373.mis2 potential
   <220>
      <221> misc_binding
      <222> 49940..49959
      <223> 99-1585-373.mis1 complement
   <220>
      <221> misc_binding
      <222> 50236..50255
      <223> 99-1585-55.mis2 potential
   <220>
      <221> misc_binding
      <222> 50257..50276
      <223> 99-1585-55.mis1 potential complement
   <220>
      <221> misc_binding
      <222> 54935..54954
      <223> 99-1607-373.mis2
   <220>
      <221> misc_binding
      <222> 54956..54975
      <223> 99-1607-373.mis1 potential complement
   <220>
      <221> misc_binding
      <222> 64219..64238
      <223> 99-1577-105.mis1 potential
   <220>
      <221> misc_binding
      <222> 64240..64259
      <223> 99-1577-105.mis2 complement
   <220>
      <221> misc_binding
      <222> 65416..65435
      <223> 99-1591-235.mis1 potential
   <220>
      <221> misc_binding
      <222> 65437..65456
      <223> 99-1591-235.mis2 complement
   <220>
      <221> misc_binding
      <222> 65476..65495
      <223> 99-1591-295.mis1 potential
   <220>
      <221> misc_binding
      <222> 65497..65516
      <223> 99-1591-295.mis2 potential complement

   <220>
      <221> misc_binding
      <222> 66947..66966
      <223> 99-1572-315.mis1 potential
   <220>
      <221> misc_binding
      <222> 66968..66987
      <223> 99-1572-315.mis2 potential complement
   <220>
      <221> misc_binding
      <222> 66967..66986
      <223> 99-1572-335.mis1 potential
   <220>
      <221> misc_binding
      <222> 66988..67007
      <223> 99-1572-335.mis2 potential complement
   <220>
      <221> misc_binding
      <222> 67072..67091
      <223> 99-1572-440.mis1
   <220>
      <221> misc_binding
      <222> 67093..67112
      <223> 99-1572-440.mis2 potential complement
   <220>
      <221> misc_binding
      <222> 67109..67128
      <223> 99-1572-477.mis1 potential
   <220>
      <221> misc_binding
      <222> 67130..67149
      <223> 99-1572-477.mis2 potential complement
   <220>
      <221> misc_binding
      <222> 67209..67228
      <223> 99-1572-578.mis1 potential
   <220>
      <221> misc_binding
      <222> 67230..67249
      <223> 99-1572-578.mis2 potential complement
   <220>
      <221> misc_binding
      <222> 67413..67432
      <223> 5-264-188.mis1 potential
   <220>
      <221> misc_binding
      <222> 67434..67453
      <223> 5-264-188.mis2 potential complement
   <220>
      <221> misc_binding
      <222> 67703..67722
      <223> 5-169-97.mis1
   <220>
      <221> misc_binding
      <222> 67724..67743
      <223> 5-169-97.mis2 potential complement
   <220>
      <221> misc_binding
      <222> 67814..67833
      <223> 5-169-208.mis1 potential
   <220>
      <221> misc_binding
      <222> 67835..67854
      <223> 5-169-208.mis2 complement

   <220>
      <221> misc_binding
      <222> 67935..67954
      <223> 5-169-331.mis1
   <220>
      <221> misc_binding
      <222> 67956..67975
      <223> 5-169-331.mis2 potential complement
   <220>
      <221> misc_binding
      <222> 68193..68212
      <223> 5-170-238.mis1 potential
   <220>
      <221> misc_binding
      <222> 68214..68233
      <223> 5-170-238.mis2 potential complement
   <220>
      <221> misc_binding
      <222> 68243..68262
      <223> 5-170-288.mis1
   <220>
      <221> misc_binding
      <222> 68264..68283
      <223> 5-170-288.mis2 potential complement
   <220>
      <221> misc_binding
      <222> 68355..68374
      <223> 5-170-400.mis1 potential
   <220>
      <221> misc_binding
      <222> 68376..68395
      <223> 5-170-400.mis2 potential complement
   <220>
      <221> misc_binding
      <222> 68457..68476
      <223> 5-171-156.mis1 potential
   <220>
      <221> misc_binding
      <222> 68478..68497
      <223> 5-171-156.mis2 potential complement
   <220>
      <221> misc_binding
      <222> 68505..68524
      <223> 5-171-204.mis1
   <220>
      <221> misc_binding
      <222> 68526..68545
      <223> 5-171-204.mis2 potential complement
   <220>
      <221> misc_binding
      <222> 68574..68593
      <223> 5-171-273.mis1 potential
   <220>
      <221> misc_binding
      <222> 68595..68614
      <223> 5-171-273.mis2 complement
   <220>
      <221> misc_binding
      <222> 68590..68609
      <223> 5-171-289.mis1 potential
   <220>
      <221> misc_binding
      <222> 68611..68630
      <223> 5-171-289.mis2 potential complement
   <220>
      <221> misc_binding
      <222> 70546..70565
      <223> 5-1-60.mis1
   <220>
      <221> misc_binding
      <222> 70567..70586
      <223> 5-1-60.mis2 potential complement
   <220>
      <221> misc_binding
      <222> 70708..70727
      <223> 5-1-222.mis1 potential
   <220>
      <221> misc_binding
      <222> 70729..70748
      <223> 5-1-222..mis2 potential complement
   <220>
      <221> misc_binding
      <222> 80018..80037
      <223> 99-1578-99.mis1 potential
   <220>
      <221> misc_binding
      <222> 80039..80058
      <223> 99-1578-99.mis2 potential complement
   <220>
      <221> misc_binding
      <222> 80098..80117
      <223> 99-1578-179.mis1 potential
   <220>
      <221> misc_binding
      <222> 80119..80138
      <223> 99-1578-179.mis2 potential complement
   <220>
      <221> misc_binding
      <222> 80150..80169
      <223> 99-1578-231.mis1 potential
   <220>
      <221> misc_binding
      <222> 80171..80190
      <223> 99-1578-231.mis2 potential complement

   <220>
      <221> misc_binding
      <222> 80163..80182
      <223> 99-1578-245.mis1 potential
   <220>
      <221> misc_binding
      <222> 80184..80203
      <223> 99-1578-245.mis2 potential complement
   <220>
      <221> misc_binding
      <222> 80415..80434
      <223> 99-1578-496.mis1
   <220>
      <221> misc_binding
      <222> 80436..80455
      <223> 99-1578-496.mis2 potential complement
   <220>
      <221> misc_binding
      <222> 82070..82089
      <223> 5-2-30.mis1 potential
   <220>
      <221> misc_binding
      <222> 82091..82110
      <223> 5-2-30.mis2 complement
   <220>
      <221> misc_binding
      <222> 82145..82164
      <223> 5-2-109.mis1 potential
   <220>
      <221> misc_binding
      <222> 82166..82185
      <223> 5-2-109.mis2 complement
   <220>
      <221> misc_binding
      <222> 82149..82168
      <223> 5-2-113.mis1
   <220>
      <221> misc_binding
      <222> 82170..82189
      <223> 5-2-113.mis2 potential complement
   <220>
      <221> misc_binding
      <222> 82198..82217
      <223> 5-2-162.mis1
   <220>
      <221> misc_binding
      <222> 82219..82238
      <223> 5-2-162.mis2 complement
   <220>
      <221> misc_binding
      <222> 82214..82233
      <223> 5-2-178.mis1 potential
   <220>
      <221> misc_binding
      <222> 82235..82254
      <223> 5-2-178.mis2 complement
   <220>
      <221> misc_binding
      <222> 82248..82267
      <223> 5-2-213.mis1
   <220>
      <221> misc_binding
      <222> 82269..82288
      <223> 5-2-213.mis2 potential complement
   <220>
      <221> misc_binding
      <222> 82373..82392
      <223> 99-1605-112.mis2
   <220>
      <221> misc_binding
      <222> 82394..82413
      <223> 99-1605-112.mis1 potential complement
   <220>
      <221> misc_binding
      <222> 83567..83586
      <223> 5-3-27.mis1 potential

   <220>
      <221> misc_binding
      <222> 83588..83607
      <223> 5-3-27.mis2 potential complement
   <220>
      <221> misc_binding
      <222> 83623..83642
      <223> 5-3-83.mis1
   <220>
      <221> misc_binding
      <222> 83644..83663
      <223> 5-3-83.mis2 potential complement
   <220>
      <221> misc_binding
      <222> 83624..83643
      <223> 5-3-84.mis1 potential
   <220>
      <221> misc_binding
      <222> 83645..83664
      <223> 5-3-84.mis2 complement
   <220>
      <221> misc_binding
      <222> 83788..83807
      <223> 5-3-248.mis1 potential
   <220>
      <221> misc_binding
      <222> 83809..83828
      <223> 5-3-248.mis2 complement
   <220>
      <221> misc_binding
      <222> 83861..83880
      <223> 5-3-321.mis1 potential
   <220>
      <221> misc_binding
      <222> 83882..83901
      <223> 5-3-321.mis2 potential complement
   <220>
      <221> misc_binding
      <222> 83864..83883
      <223> 5-3-324.mis1 potential
   <220>
      <221> misc_binding
      <222> 83885..83904
      <223> 5-3-324.mis2 potential complement
   <220>
      <221> misc_binding
      <222> 83889..83908
      <223> 5-4-313.mis1 potential
   <220>
      <221> misc_binding
      <222> 83910..83929
      <223> 5-4-313.mis2 complement
   <220>
      <221> misc_binding
      <222> 83917..83936
      <223> 5-3-377.mis1 potential
   <220>
      <221> misc_binding
      <222> 83938..83957
      <223> 5-3-377.mis2 potential complement
   <220>
      <221> misc_binding
      <222> 83927..83946
      <223> 5-4-351.mis1 potential
   <220>
      <221> misc_binding
      <222> 83948..83967
      <223> 5-4-351.mis2 potential complement
   <220>
      <221> misc_binding
      <222> 83962..83981
      <223> 5-4-386.mis1 potential
   <220>
      <221> misc_binding
      <222> 83983..84002
      <223> 5-4-386.mis2 potential complement
   <220>
      <221> misc_binding
      <222> 83968..83987
      <223> 5-4-392.mis1 potential
   <220>
      <221> misc_binding
      <222> 83989..84008
      <223> 5-4-392.mis2 potential complement
   <220>
      <221> misc_binding
      <222> 84027..84046
      <223> 5-260-255.mis1 potential
   <220>
      <221> misc_binding
      <222> 84048..84067
      <223> 5-260-255.mis2 potential complement
   <220>
      <221> misc_binding
      <222> 84072..84091
      <223> 5-260-300.mis1 potential
   <220>
      <221> misc_binding
      <222> 84093..84112
      <223> 5-260-300.mis2 potential complement

   <220>
      <221> misc_binding
      <222> 84125..84144
      <223> 5-260-353.mis1 potential
   <220>
      <221> misc_binding
      <222> 84146..84165
      <223> 5-260-353.mis2 potential complement
   <220>
      <221> misc_binding
      <222> 85182..85201
      <223> 5-9-50.mis1 potential
   <220>
      <221> misc_binding
      <222> 85203..85222
      <223> 5-9-50.mis2 potential complement
   <220>
      <221> misc_binding
      <222> 86239..86258
      <223> 5-5-21.mis1 potential
   <220>
      <221> misc_binding
      <222> 86260..86279
      <223> 5-5-21.mis2 complement
   <220>
      <221> misc_binding
      <222> 86303..86322
      <223> 5-5-85.mis1 potential
   <220>
      <221> misc_binding
      <222> 86324..86343
      <223> 5-5-85.mis2 potential complement
   <220>
      <221> misc_binding
      <222> 87693..87712
      <223> 5-202-95.mis1 potential
   <220>
      <221> misc_binding
      <222> 87714..87733
      <223> 5-202-95.mis2 complement
   <220>
      <221> misc_binding
      <222> 87715..87734
      <223> 5-202-117.mis1
   <220>
      <221> misc_binding
      <222> 87736..87755
      <223> 5-202-117.mis2 potential complement
   <220>
      <221> misc_binding
      <222> 87767..87786
      <223> 5-202-169.mis1 potential
   <220>
      <221> misc_binding
      <222> 87788..87807
      <223> 5-202-169.mis2 potential complement
   <220>
      <221> misc_binding
      <222> 87786..87805
      <223> 5-202-188.mis1 potential
   <220>
      <221> misc_binding
      <222> 87807..87826
      <223> 5-202-188.mis2 potential complement
   <220>
      <221> misc_binding
      <222> 87840..87859
      <223> 5-202-242.mis1 potential
   <220>
      <221> misc_binding
      <222> 87861..87880
      <223> 5-202-242.mis2 potential complement
   <220>
      <221> misc_binding
      <222> 87882..87901
      <223> 5-202-284.mis1 potential
   <220>
      <221> misc_binding
      <222> 87903..87922
      <223> 5-202-284.mis2 potential complement
   <220>
      <221> misc_binding
      <222> 87960..87979
      <223> 5-202-362.mis1 potential
   <220>
      <221> misc_binding
      <222> 87981..88000
      <223> 5-202-362.mis2 potential complement
   <220>
      <221> misc_binding
      <222> 87992..88011
      <223> 5-202-394.mis1 potential
   <220>
      <221> misc_binding
      <222> 88013..88032
      <223> 5-202-394.mis2 potential complement
   <220>
      <221> misc_binding
      <222> 88195..88214
      <223> 5-7-113.mis1 potential
   <220>
      <221> misc_binding
      <222> 88216..88235
      <223> 5-7-113.mis2 potential complement

   <220>
      <221> misc_binding
      <222> 88263..88282
      <223> 5-7-181.mis1 potential
   <220>
      <221> misc_binding
      <222> 88284..88303
      <223> 5-7-181.mis2 potential complement
   <220>
      <221> misc_binding
      <222> 88277..88296
      <223> 5-7-195.mis1
   <220>
      <221> misc_binding
      <222> 88298..88317
      <223> 5-7-195.mis2 potential complement
   <220>
      <221> misc_binding
      <222> 88422..88441
      <223> 5-7-340.mis1 potential
   <220>
      <221> misc_binding
      <222> 88443..88462
      <223> 5-7-340.mis2 potential complement
   <220>
      <221> misc_binding
      <222> 88451..88470
      <223> 5-7-369.mis1 potential
   <220>
      <221> misc_binding
      <222> 88472..88491
      <223> 5-7-369.mis2 potential complement
   <220>
      <221> misc_binding
      <222> 88460..88479
      <223> 5-7-378.mis1 potential
   <220>
      <221> misc_binding
      <222> 88481..88500
      <223> 5-7-378.mis2 potential complement
   <220>
      <221> misc_binding
      <222> 89374..89393
      <223> 5-181-57.mis1 potential
   <220>
      <221> misc_binding
      <222> 89395..89414
      <223> 5-181-57.mis2 potential complement
   <220>
      <221> misc_binding
      <222> 89444..89463
      <223> 5-181-127.mis1 potential
   <220>
      <221> misc_binding
      <222> 89465..89484
      <223> 5-181-127.mis2 potential complement
   <220>
      <221> misc_binding
      <222> 89451..89470
      <223> 5-181-134.mis1 potential
   <220>
      <221> misc_binding
      <222> 89472..89491
      <223> 5-181-134.mis2 potential complement
   <220>
      <221> misc_binding
      <222> 89638..89657
      <223> 5-181-321.mis1 potential
   <220>
      <221> misc_binding
      <222> 89659..89678
      <223> 5-181-321.mis2 potential complement
   <220>
      <221> misc_binding
      <222> 92740..92759
      <223> 5-10-39.mis1
   <220>
      <221> misc_binding
      <222> 92761..92780
      <223> 5-10-39.mis2 complement
   <220>
      <221> misc_binding
      <222> 93003..93022
      <223> 5-10-302.mis1 potential
   <220>
      <221> misc_binding
      <222> 93024..93043
      <223> 5-10-302.mis2 potential complement
   <220>
      <221> misc_binding
      <222> 93035..93054
      <223> 5-10-334.mis1 potential
   <220>
      <221> misc_binding
      <222> 93056..93075
      <223> 5-10-334.mis2 potential complement
   <220>
      <221> misc_binding
      <222> 93227..93246
      <223> 5-11-158.mis1 potential
   <220>
      <221> misc_binding
      <222> 93248..93267
      <223> 5-11-158.mis2 complement

   <220>
      <221> misc_binding
      <222> 93299..93318
      <223> 5-11-230.mis1 potential
   <220>
      <221> misc_binding
      <222> 93320..93339
      <223> 5-11-230.mis2 potential complement
   <220>
      <221> misc_binding
      <222> 93303..93322
      <223> 5-11-234.mis1 potential
   <220>
      <221> misc_binding
      <222> 93324..93343
      <223> 5-11-234.mis2 potential complement
   <220>
      <221> misc_binding
      <222> 93368..93387
      <223> 5-11-299.mis1 potential
   <220>
      <221> misc_binding
      <222> 93389..93408
      <223> 5-11-299.mis2 potential complement
   <220>
      <221> misc_binding
      <222> 93373..93392
      <223> 5-11-304.mis1 potential
   <220>
      <221> misc_binding
      <222> 93394..93413
      <223> 5-11-304.mis2 potential complement
   <220>
      <221> misc_binding
      <222> 93398..93417
      <223> 5-11-329.mis1 potential
   <220>
      <221> misc_binding
      <222> 93419..93438
      <223> 5-11-329.mis2 potential complement
   <220>
      <221> misc_binding
      <222> 93495..93514
      <223> 5-12-56.mis1 potential
   <220>
      <221> misc_binding
      <222> 93516..93535
      <223> 5-12-56.mis2 potential complement
   <220>
      <221> misc_binding
      <222> 93706..93725
      <223> 5-12-267.mis1 potential
   <220>
      <221> misc_binding
      <222> 93727..93746
      <223> 5-12-267.mis2 potential complement
   <220>
      <221> misc_binding
      <222> 93883..93902
      <223> 5-13-145.mis1 potential
   <220>
      <221> misc_binding
      <222> 93904..93923
      <223> 5-13-145.mis2 potential complement
   <220>
      <221> misc_binding
      <222> 94150..94169
      <223> 5-14-44.mis1 potential
   <220>
      <221> misc_binding
      <222> 94171..94190
      <223> 5-14-44.mis2 potential complement
   <220>
      <221> misc_binding
      <222> 94198..94217
      <223> 5-14-93.mis1 potential
   <220>
      <221> misc_binding
      <222> 94219..94238
      <223> 5-14-93.mis2 potential complement
   <220>
      <221> misc_binding
      <222> 94249..94268
      <223> 5-14-144.mis1 potential
   <220>
      <221> misc_binding
      <222> 94270..94289
      <223> 5-14-144.mis2 potential complement
   <220>
      <221> misc_binding
      <222> 94270..94289
      <223> 5-14-165.mis1
   <220>
      <221> misc_binding
      <222> 94291..94310
      <223> 5-14-165.mis2 potential complement
   <220>
      <221> misc_binding
      <222> 94402..94421
      <223> 5-14-297.mis1 potential

   <220>
      <221> misc_binding
      <222> 94423..94442
      <223> 5-14-297.mis2 potential complement
   <220>
      <221> misc_binding
      <222> 94412..94431
      <223> 5-14-307.mis1 potential
   <220>
      <221> misc_binding
      <222> 94433..94452
      <223> 5-14-307.mis2 potential complement
   <220>
      <221> misc_binding
      <222> 94700..94719
      <223> 5-15-219.mis1
   <220>
      <221> misc_binding
      <222> 94721..94740
      <223> 5-15-219.mis2 potential complement
   <220>
      <221> misc_binding
      <222> 94969..94988
      <223> 5-16-157.mis1 potential
   <220>
      <221> misc_binding
      <222> 94990..95009
      <223> 5-16-157.mis2 potential complement
   <220>
      <221> misc_binding
      <222> 95241..95260
      <223> 5-17-140.mis1 potential
   <220>
      <221> misc_binding
      <222> 95262..95281
      <223> 5-17-140.mis2 potential complement
   <220>
      <221> misc_binding
      <222> 95320..95339
      <223> 5-18-51.mis1 potential
   <220>
      <221> misc_binding
      <222> 95341..95360
      <223> 5-18-51.mis2 potential complement
   <220>
      <221> misc_binding
      <222> 95477..95496
      <223> 5-18-208.mis1
   <220>
      <221> misc_binding
      <222> 95498..95517
      <223> 5-18-208.mis2 potential complement
   <220>
      <221> misc_binding
      <222> 95750..95769
      <223> 5-300-238.mis1 potential
   <220>
      <221> misc_binding
      <222> 95771..95790
      <223> 5-300-238.mis2 potential complement
   <220>
      <221> misc_binding
      <222> 95799..95818
      <223> 5-300-287.mis1 potential
   <220>
      <221> misc_binding
      <222> 95820..95839
      <223> 5-300-287.mis2 potential complement
   <220>
      <221> misc_binding
      <222> 96125..96144
      <223> 5-262-49.mis1 potential
   <220>
      <221> misc_binding
      <222> 96146..96165
      <223> 5-262-49.mis2 potential complement
   <220>
      <221> misc_binding
      <222> 96161..96180
      <223> 5-262-85.mis1 potential
   <220>
      <221> misc_binding
      <222> 96182..96201
      <223> 5-262-85.mis2 potential complement
   <220>
      <221> misc_binding
      <222> 96330..96349
      <223> 5-262-254.mis1 potential
   <220>
      <221> misc_binding
      <222> 96351..96370
      <223> 5-262-254.mis2 potential complement
   <220>
      <221> misc_binding
      <222> 96931..96950
      <223> 5-263-404.mis1 potential

   <220>
      <221> misc_binding
      <222> 96952..96971
      <223> 5-263-404.mis2 potential complement
   <220>
      <221> misc_binding
      <222> 97124..97143
      <223> 5-265-244.mis1 potential
   <220>
      <221> misc_binding
      <222> 97145..97164
      <223> 5-265-244.mis2 potential complement
   <220>
      <221> misc_binding
      <222> 97256..97275
      <223> 5-265-376.mis1 potential
   <220>
      <221> misc_binding
      <222> 97277..97296
      <223> 5-265-376.mis2 potential complement
   <220>
      <221> misc_binding
      <222> 102247..102266
      <223> 99-7183-338.mis2
   <220>
      <221> misc_binding
      <222> 102268..102287
      <223> 99-7183-338.mis1 potential complement
   <220>
      <221> misc_binding
      <222> 105917..105936
      <223> 99-7207-138.mis2 potential
   <220>
      <221> misc_binding
      <222> 105938..105957
      <223> 99-7207-138.mis1 potential complement
   <220>
      <221> misc_binding
      <222> 255..301
      <223> 99-1601-278.probe potential
   <220>
      <221> misc_binding
      <222> 379..425
      <223> 99-1601-402.probe potential
   <220>
      <221> misc_binding
      <222> 449..495
      <223> 99-1601-472.probe potential
   <220>
      <221> misc_binding
      <222> 2932..2978
      <223> 99-13801-100.probe potential
   <220>
      <221> misc_binding
      <222> 12144..12190
      <223> 99-13806-166.probe potential
   <220>
      <221> misc_binding
      <222> 12513..12559
      <223> 99-13799-376.probe potential
   <220>
      <221> misc_binding
      <222> 17570..17616
      <223> 99-13798-297.probe potential
   <220>
      <221> misc_binding
      <222> 17583..17629
      <223> 99-13798-284.probe potential
   <220>
      <221> misc_binding
      <222> 22056..22102
      <223> 99-1602-200.probe potential
   <220>
      <221> misc_binding
      <222> 28941..28987
      <223> 99-13794-186.probe potential
   <220>
      <221> misc_binding
      <222> 28980..29026
      <223> 99-13794-147.probe potential
   <220>
      <221> misc_binding
      <222> 31054..31100
      <223> 99-13812-384.probe potential
   <220>
      <221> misc_binding
      <222> 31743..31789
      <223> 99-13805-313.probe potential
   <220>
      <221> misc_binding
      <222> 34768..34814
      <223> 99-1587-281.probe potential
   <220>
      <221> misc_binding
      <222> 45728..45774
      <223> 99-1582-430.probe potential
   <220>
      <221> misc_binding
      <222> 49824..49870
      <223> 99-1585-465.probe potential
   <220>
      <221> misc_binding
      <222> 49832..49878
      <223> 99-1585-457.probe potential
   <220>
      <221> misc_binding
      <222> 49863..49909
      <223> 99-1585-426.probe potential
   <220>
      <221> misc_binding
      <222> 49877..49923
      <223> 99-1585-412.probe potential
   <220>
      <221> misc_binding
      <222> 49883..49929
      <223> 99-1585-406.probe potential
   <220>
      <221> misc_binding
      <222> 49898..49944
      <223> 99-1585-391.probe potential

   <220>
      <221> misc_binding
      <222> 49916..49962
      <223> 99-1585-373.probe potential
   <220>
      <221> misc_binding
      <222> 50233..50279
      <223> 99-1585-55.probe potential
   <220>
      <221> misc_binding
      <222> 54932..54978
      <223> 99-1607-373.probe potential
   <220>
      <221> misc_binding
      <222> 64216..64262
      <223> 99-1577-105.probe potential
   <220>
      <221> misc_binding
      <222> 65413..65459
      <223> 99-1591-235.probe potential
   <220>
      <221> misc_binding
      <222> 65473..65519
      <223> 99-1591-295.probe potential
   <220>
      <221> misc_binding
      <222> 66944..66990
      <223> 99-1572-315.probe potential
   <220>
      <221> misc_binding
      <222> 66964..67010
      <223> 99-1572-335.probe potential
   <220>
      <221> misc_binding
      <222> 67069..67115
      <223> 99-1572-440.probe potential
   <220>
      <221> misc_binding
      <222> 67106..67152
      <223> 99-1572-477.probe potential
   <220>
      <221> misc_binding
      <222> 67206..67252
      <223> 99-1572-578.probe potential
   <220>
      <221> misc_binding
      <222> 67410..67456
      <223> 5-264-188.probe potential
   <220>
      <221> misc_binding
      <222> 67700..67746
      <223> 5-169-97.probe potential
   <220>
      <221> misc_binding
      <222> 67811..67857
      <223> 5-169-208.probe potential
   <220>
      <221> misc_binding
      <222> 67932..67978
      <223> 5-169-331.probe potential
   <220>
      <221> misc_binding
      <222> 68190..68236
      <223> 5-170-238.probe potential
   <220>
      <221> misc_binding
      <222> 68240..68286
      <223> 5-170-288.probe potential
   <220>
      <221> misc_binding
      <222> 68352..68398
      <223> 5-170-400.probe potential
   <220>
      <221> misc_binding
      <222> 68454..68500
      <223> 5-171-156.probe potential
   <220>
      <221> misc_binding
      <222> 68502..68548
      <223> 5-171-204.probe potential
   <220>
      <221> misc_binding
      <222> 68571..68617
      <223> 5-171-273.probe potential
   <220>
      <221> misc_binding
      <222> 68587..68633
      <223> 5-171-289.probe potential
   <220>
      <221> misc_binding
      <222> 70543..70589
      <223> 5-1-60.probe potential
   <220>
      <221> misc_binding
      <222> 70705..70751
      <223> 5-1-222.probe potential
   <220>
      <221> misc_binding
      <222> 80015..80061
      <223> 99-1578-99.probe potential
   <220>
      <221> misc_binding
      <222> 80095..80141
      <223> 99-1578-179.probe potential
   <220>
      <221> misc_binding
      <222> 80147..80193
      <223> 99-1578-231.probe potential
   <220>
      <221> misc_binding
      <222> 80160..80206
      <223> 99-1578-245.probe potential
   <220>
      <221> misc_binding
      <222> 80412..80458
      <223> 99-1578-496.probe potential
   <220>
      <221> misc_binding
      <222> 82067..82113
      <223> 5-2-30.probe potential
   <220>
      <221> misc_binding
      <222> 82142..82188
      <223> 5-2-109.probe potential

   <220>
      <221> misc_binding
      <222> 82146..82192
      <223> 5-2-113.probe potential
   <220>
      <221> misc_binding
      <222> 82195..82241
      <223> 5-2-162.probe potential
   <220>
      <221> misc_binding
      <222> 82211..82257
      <223> 5-2-178.probe potential
   <220>
      <221> misc_binding
      <222> 82245..82291
      <223> 5-2-213.probe potential
   <220>
      <221> misc_binding
      <222> 82370..82416
      <223> 99-1605-112.probe potential
   <220>
      <221> misc_binding
      <222> 83564..83610
      <223> 5-3-27.probe potential
   <220>
      <221> misc_binding
      <222> 83620..83666
      <223> 5-3-83.probe potential
   <220>
      <221> misc_binding
      <222> 83621..83667
      <223> 5-3-84.probe potential
   <220>
      <221> misc_binding
      <222> 83785..83831
      <223> 5-3-248.probe potential
   <220>
      <221> misc_binding
      <222> 83858..83904
      <223> 5-3-321.probe potential
   <220>
      <221> misc_binding
      <222> 83861..83907
      <223> 5-3-324.probe potential
   <220>
      <221> misc_binding
      <222> 83886..83932
      <223> 5-4-313.probe potential
   <220>
      <221> misc_binding
      <222> 83914..83960
      <223> 5-3-377.probe potential
   <220>
      <221> misc_binding
      <222> 83924..83970
      <223> 5-4-351.probe potential
   <220>
      <221> misc_binding
      <222> 83959..84005
      <223> 5-4-386.probe potential
   <220>
      <221> misc_binding
      <222> 83965..84011
      <223> 5-4-392.probe potential
   <220>
      <221> misc_binding
      <222> 84024..84070
      <223> 5-260-255.probe potential
   <220>
      <221> misc_binding
      <222> 84069..84115
      <223> 5-260-300.probe potential
   <220>
      <221> misc_binding
      <222> 84122..84168
      <223> 5-260-353.probe potential
   <220>
      <221> misc_binding
      <222> 85179..85225
      <223> 5-9-50.probe potential
   <220>
      <221> misc_binding
      <222> 86236..86282
      <223> 5-5-21.probe potential
   <220>
      <221> misc_binding
      <222> 86300..86346
      <223> 5-5-85.probe potential
   <220>
      <221> misc_binding
      <222> 87690..87736
      <223> 5-202-95.probe potential
   <220>
      <221> misc_binding
      <222> 87712..87758
      <223> 5-202-117.probe potential
   <220>
      <221> misc_binding
      <222> 87764..87810
      <223> 5-202-169.probe potential
   <220>
      <221> misc_binding
      <222> 87783..87829
      <223> 5-202-188.probe potential
   <220>
      <221> misc_binding
      <222> 87837..87883
      <223> 5-202-242.probe potential
   <220>
      <221> misc_binding
      <222> 87879..87925
      <223> 5-202-284.probe potential
   <220>
      <221> misc_binding
      <222> 87957..88003
      <223> 5-202-362.probe potential
   <220>
      <221> misc_binding
      <222> 87989..88035
      <223> 5-202-394.probe potential
   <220>
      <221> misc_binding
      <222> 88192..88238
      <223> 5-7-113.probe potential

   <220>
      <221> misc_binding
      <222> 88260..88306
      <223> 5-7-181.probe potential
   <220>
      <221> misc_binding
      <222> 88274..88320
      <223> 5-7-195.probe potential
   <220>
      <221> misc_binding
      <222> 88419..88465
      <223> 5-7-340.probe potential
   <220>
      <221> misc_binding
      <222> 88448..88494
      <223> 5-7-369.probe potential
   <220>
      <221> misc_binding
      <222> 88457..88503
      <223> 5-7-378.probe potential
   <220>
      <221> misc_binding
      <222> 89371..89417
      <223> 5-181-57.probe potential
   <220>
      <221> misc_binding
      <222> 89441..89487
      <223> 5-181-127.probe potential
   <220>
      <221> misc_binding
      <222> 89448..89494
      <223> 5-181-134.probe potential
   <220>
      <221> misc_binding
      <222> 89635..89681
      <223> 5-181-321.probe potential
   <220>
      <221> misc_binding
      <222> 92737..92783
      <223> 5-10-39.probe potential
   <220>
      <221> misc_binding
      <222> 93000..93046
      <223> 5-10-302.probe potential
   <220>
      <221> misc_binding
      <222> 93032..93078
      <223> 5-10-334.probe potential
   <220>
      <221> misc_binding
      <222> 93224..93270
      <223> 5-11-158.probe potential
   <220>
      <221> misc_binding
      <222> 93296..93342
      <223> 5-11-230.probe potential
   <220>
      <221> misc_binding
      <222> 93300..93346
      <223> 5-11-234.probe potential
   <220>
      <221> misc_binding
      <222> 93365..93411
      <223> 5-11-299.probe potential
   <220>
      <221> misc_binding
      <222> 93370..93416
      <223> 5-11-304.probe potential
   <220>
      <221> misc_binding
      <222> 93395..93441
      <223> 5-11-329.probe potential
   <220>
      <221> misc_binding
      <222> 93492..93538
      <223> 5-12-56.probe potential
   <220>
      <221> misc_binding
      <222> 93703..93749
      <223> 5-12-267.probe potential
   <220>
      <221> misc_binding
      <222> 93880..93926
      <223> 5-13-145.probe potential
   <220>
      <221> misc_binding
      <222> 94147..94193
      <223> 5-14-44.probe potential
   <220>
      <221> misc_binding
      <222> 94195..94241
      <223> 5-14-93.probe potential
   <220>
      <221> misc_binding
      <222> 94246..94292
      <223> 5-14-144.probe potential
   <220>
      <221> misc_binding
      <222> 94267..94313
      <223> 5-14-165.probe potential

   <220>
      <221> misc_binding
      <222> 94399..94445
      <223> 5-14-297.probe potential
   <220>
      <221> misc_binding
      <222> 94409..94455
      <223> 5-14-307.probe potential
   <220>
      <221> misc_binding
      <222> 94697..94743
      <223> 5-15-219.probe potential
   <220>
      <221> misc_binding
      <222> 94966..95012
      <223> 5-16-157.probe potential
   <220>
      <221> misc_binding
      <222> 95238..95284
      <223> 5-17-140.probe potential
   <220>
      <221> misc_binding
      <222> 95317..95363
      <223> 5-18-51.probe potential
   <220>
      <221> misc_binding
      <222> 95474..95520
      <223> 5-18-208.probe potential
   <220>
      <221> misc_binding
      <222> 95747..95793
      <223> 5-300-238.probe potential
   <220>
      <221> misc_binding
      <222> 95796..95842
      <223> 5-300-287.probe potential
   <220>
      <221> misc_binding
      <222> 96122..96168
      <223> 5-262-49.probe potential
   <220>
      <221> misc_binding
      <222> 96158..96204
      <223> 5-262-85.probe potential
   <220>
      <221> misc_binding
      <222> 96327..96373
      <223> 5-262-254.probe potential
   <220>
      <221> misc_binding
      <222> 96928..96974
      <223> 5-263-404.probe potential
   <220>
      <221> misc_binding
      <222> 97121..97167
      <223> 5-265-244.probe potential
   <220>
      <221> misc_binding
      <222> 97253..97299
      <223> 5-265-376.probe potential
   <220>
      <221> misc_binding
      <222> 102244..102290
      <223> 99-7183-338.probe potential
   <220>
      <221> misc_binding
      <222> 105914..105960
      <223> 99-7207-138.probe potential
   <220>
      <221> misc_binding
      <222> 1..18
      <223> 99-1601.pu
   <220>
      <221> misc_binding
      <222> 486..506
      <223> 99-1601.rp complement
   <220>
      <221> misc_binding
      <222> 2607..2627
      <223> 99-13801.rp
   <220>
      <221> misc_binding
      <222> 3035..3054
      <223> 99-13801.pu complement
   <220>
      <221> misc_binding
      <222> 11883..11902
      <223> 99-13806.rp
   <220>
      <221> misc_binding
      <222> 12313..12331
      <223> 99-13806.pu complement
   <220>
      <221> misc_binding
      <222> 12379..12399
      <223> 99-13799.rp
   <220>
      <221> misc_binding
      <222> 12889..12909
      <223> 99-13799.pu complement
   <220>
      <221> misc_binding
      <222> 17442..17462
      <223> 99-13798.rp
   <220>
      <221> misc_binding
      <222> 17868..17887
      <223> 99-13798.pu complement
   <220>
      <221> misc_binding
      <222> 21881..21899
      <223> 99-1602.pu
   <220>
      <221> misc_binding
      <222> 22487..22506
      <223> 99-1602.rp complement
   <220>
      <221> misc_binding
      <222> 28669..28689
      <223> 99-13794.rp
   <220>
      <221> misc_binding
      <222> 29131..29149
      <223> 99-13794.pu complement
   <220>
      <221> misc_binding
      <222> 30941..30961
      <223> 99-13812.rp
   <220>
      <221> misc_binding
      <222> 31437..31457
      <223> 99-13812.pu complement
   <220>
      <221> misc_binding
      <222> 31560..31579
      <223> 99-13805.rp
   <220>
      <221> misc_binding
      <222> 32057..32075
      <223> 99-13805.pu complement
   <220>
      <221> misc_binding
      <222> 34515..34535
      <223> 99-1587.pu
   <220>
      <221> misc_binding
      <222> 34890..34909
      <223> 99-1587.rp complement
   <220>
      <221> misc_binding
      <222> 45325..45343
      <223> 99-1582.pu
   <220>
      <221> misc_binding
      <222> 46000..46018
      <223> 99-1582.rp complement
   <220>
      <221> misc_binding
      <222> 49765..49784
      <223> 99-1585.rp
   <220>
      <221> misc_binding
      <222> 50291..50310
      <223> 99-1585.pu complement
   <220>
      <221> misc_binding
      <222> 54726..54746
      <223> 99-1607.rp
   <220>
      <221> misc_binding
      <222> 55307..55325
      <223> 99-1607.pu complement
   <220>
      <221> misc_binding
      <222> 64135..64153
      <223> 99-1577.pu

   <220>
      <221> misc_binding
      <222> 64518..64536
      <223> 99-1577.rp complement
   <220>
      <221> misc_binding
      <222> 65202..65219
      <223> 99-1591.pu
   <220>
      <221> misc_binding
      <222> 65815..65834
      <223> 99-1591.rp complement
   <220>
      <221> misc_binding
      <222> 66653..66671
      <223> 99-1572.pu
   <220>
      <221> misc_binding
      <222> 67275..67295
      <223> 99-1572.rp complement
   <220>
      <221> misc_binding
      <222> 67627..67646
      <223> 5-169.pu
   <220>
      <221> misc_binding
      <222> 68024..68043
      <223> 5-169.rp complement
   <220>
      <221> misc_binding
      <222> 67246..67263
      <223> 5-264.pu
   <220>
      <221> misc_binding
      <222> 67678..67696
      <223> 5-264.rp complement
   <220>
      <221> misc_binding
      <222> 67977..67994
      <223> 5-170.pu
   <220>
      <221> misc_binding
      <222> 68406..68424
      <223> 5-170.rp complement
   <220>
      <221> misc_binding
      <222> 68322..68340
      <223> 5-171.pu
   <220>
      <221> misc_binding
      <222> 68725..68742
      <223> 5-171.rp complement
   <220>
      <221> misc_binding
      <222> 70507..70524
      <223> 5-1.pu
   <220>
      <221> misc_binding
      <222> 70909..70928
      <223> 5-1.rp complement
   <220>
      <221> misc_binding
      <222> 79940..79957
      <223> 99-1578.pu
   <220>
      <221> misc_binding
      <222> 80557..80575
      <223> 99-1578.rp complement
   <220>
      <221> misc_binding
      <222> 82057..82077
      <223> 99-1605.rp
   <220>
      <221> misc_binding
      <222> 82484..82504
      <223> 99-1605.pu complement
   <220>
      <221> misc_binding
      <222> 82058..82077
      <223> 5-2.pu
   <220>
      <221> misc_binding
      <222> 82473..82492
      <223> 5-2.rp complement
   <220>
      <221> misc_binding
      <222> 83561..83578
      <223> 5-3.pu
   <220>
      <221> misc_binding
      <222> 83965..83982
      <223> 5-3.rp complement
   <220>
      <221> misc_binding
      <222> 83597..83616
      <223> 5-4.pu
   <220>
      <221> misc_binding
      <222> 83999..84017
      <223> 5-4.rp complement
   <220>
      <221> misc_binding
      <222> 83793..83812
      <223> 5-260.pu
   <220>
      <221> misc_binding
      <222> 84148..84167
      <223> 5-260.rp complement
   <220>
      <221> misc_binding
      <222> 85153..85170
      <223> 5-9.pu
   <220>
      <221> misc_binding
      <222> 85559..85576
      <223> 5-9.rp complement
   <220>
      <221> misc_binding
      <222> 86239..86257
      <223> 5-5.pu
   <220>
      <221> misc_binding
      <222> 86519..86539
      <223> 5-5.rp complement
   <220>
      <221> misc_binding
      <222> 87619..87638
      <223> 5-202.pu
   <220>
      <221> misc_binding
      <222> 88033..88050
      <223> 5-202.rp complement
   <220>
      <221> misc_binding
      <222> 88104..88122
      <223> 5-7.pu
   <220>
      <221> misc_binding
      <222> 88519..88536
      <223> 5-7.rp complement
   <220>
      <221> misc_binding
      <222> 89338..89357
      <223> 5-181.pu

   <220>
      <221> misc_binding
      <222> 89739..89758
      <223> 5-181.rp complement
   <220>
      <221> misc_binding
      <222> 92722..92741
      <223> 5-10.pu
   <220>
      <221> misc_binding
      <222> 93124..93142
      <223> 5-10.rp complement
   <220>
      <221> misc_binding
      <222> 93090..93108
      <223> 5-11.pu
   <220>
      <221> misc_binding
      <222> 93490..93509
      <223> 5-11.rp complement
   <220>
      <221> misc_binding
      <222> 93460..93478
      <223> 5-12.pu
   <220>
      <221> misc_binding
      <222> 93862..93881
      <223> 5-12.rp complement
   <220>
      <221> misc_binding
      <222> 93759..93776
      <223> 5-13.pu
   <220>
      <221> misc_binding
      <222> 94175..94192
      <223> 5-13.rp complement
   <220>
      <221> misc_binding
      <222> 94127..94144
      <223> 5-14.pu
   <220>
      <221> misc_binding
      <222> 94535..94554
      <223> 5-14.rp complement
   <220>
      <221> misc_binding
      <222> 94504..94521
      <223> 5-15.pu
   <220>
      <221> misc_binding
      <222> 94904..94921
      <223> 5-15.rp complement
   <220>
      <221> misc_binding
      <222> 94833..94850
      <223> 5-16.pu
   <220>
      <221> misc_binding
      <222> 95232..95251
      <223> 5-16.rp complement
   <220>
      <221> misc_binding
      <222> 95124..95142
      <223> 5-17.pu
   <220>
      <221> misc_binding
      <222> 95542..95561
      <223> 5-17.rp complement
   <220>
      <221> misc_binding
      <222> 95290..95308
      <223> 5-18.pu
   <220>
      <221> misc_binding
      <222> 95689..95708
      <223> 5-18.rp complement
   <220>
      <221> misc_binding
      <222> 95533..95551
      <223> 5-300.pu
   <220>
      <221> misc_binding
      <222> 95934..95952
      <223> 5-300.rp complement
   <220>
      <221> misc_binding
      <222> 96097..96115
      <223> 5-262.pu
   <220>
      <221> misc_binding
      <222> 96574..96591
      <223> 5-262.rp complement
   <220>
      <221> misc_binding
      <222> 96548..96565
      <223> 5-263.pu
   <220>
      <221> misc_binding
      <222> 96982..97001
      <223> 5-263.rp complement
   <220>
      <221> misc_binding
      <222> 96901..96918
      <223> 5-265.pu
   <220>
      <221> misc_binding
      <222> 97292..97309
      <223> 5-265.rp complement
   <220>
      <221> misc_binding
      <222> 102156..102176
      <223> 99-7183.rp
   <220>
      <221> misc_binding
      <222> 102584..102604
      <223> 99-7183.pu complement
   <220>
      <221> misc_binding
      <222> 105570..105588
      <223> 99-7207.rp
   <220>
      <221> misc_binding
      <222> 106056..106074
      <223> 99-7207.pu complement
   <220>
      <221> misc_feature
      <222> 86434
      <223> diverging nucleotide G in ref genbank : L78132
   <220>
      <221> misc_feature
      <222> 86435
      <223> diverging nucleotide T in ref genbank : L78132
   <220>
      <221> misc_feature
      <222> 88355
      <223> diverging nucleotide C in ref genbank : L78132
   <220>
      <221> misc_feature
      <222> 92976
      <223> insertion of G in ref genbank : L78132
   <220>
      <221> misc_feature
      <222> 93240
      <223> diverging nucleotide T in ref genbank : L78132
   <220>
      <221> misc_feature
      <222> 93471
      <223> diverging nucleotide G in ref genbank : L78132

   <220>
      <221> misc_feature
      <222> 93592
      <223> diverging nucleotide C in ref genbank : L78132
   <220>
      <221> misc_feature
      <222> 93680
      <223> diverging nucleotide C in ref genbank : L78132
   <220>
      <221> misc_feature
      <222> 93681
      <223> diverging nucleotide C in ref genbank : L78132
   <220>
      <221> misc feature
      <222> 93682
      <223> diverging nucleotide C in ref genbank : L78132
   <220>
      <221> misc_feature
      <222> 93683
      <223> diverging nucleotide G in ref genbank : L78132
   <220>
      <221> misc_feature
      <222> 93712
      <223> deletion of A in ref genbank : L78132
   <220>
      <221> misc_feature
      <222> 93728
      <223> diverging nucleotide C in ref genbank : L78132
   <220>
      <221> misc_feature
      <222> 93747
      <223> diverging nucleotide T in ref genbank : L78132
   <220>
      <221> misc_feature
      <222> 93761
      <223> diverging nucleotide C in ref genbank : L78132
   <220>
      <221> misc_feature
      <222> 94151
      <223> deletion of TTA in ref genbank : L78132
   <220>
      <221> misc_feature
      <222> 94154
      <223> diverging nucleotide C in ref genbank : L78132
   <220>
      <221> misc_feature
      <222> 94241
      <223> insertion of G in ref genbank : L78132
   <220>
      <221> misc_feature
      <222> 94430
      <223> diverging nucleotide C in ref genbank : L78132
   <220>
      <221> misc_feature
      <222> 94771
      <223> insertion of A in ref genbank : L78132
   <220>
      <221> misc_feature
      <222> 94805
      <223> insertion of T in ref genbank : L78132
   <220>
      <221> misc_feature
      <222> 95121
      <223> deletion of AG in ref genbank : L78132
   <220>
      <221> misc_feature
      <222> 95126
      <223> diverging nucleotide A in ref genbank : L78132
   <220>
      <221> misc_feature
      <222> 95130
      <223> deletion of G in ref genbank : L78132
   <220>
      <221> misc_feature
      <222> 95134
      <223> deletion of G in ref genbank : L78132
   <220>
      <221> misc_feature
      <222> 95149
      <223> deletion of A in ref genbank : L78132
   <220>
      <221> misc_feature
      <222> 95155
      <223> deletion of A in ref genbank : L78132
   <220>
      <221> misc_feature
      <222> 95174
      <223> deletion of AA in ref genbank : L78132
   <220>
      <221> misc_feature
      <222> 95368
      <223> deletion of A in ref genbank : L78132
   <220>
      <221> misc_feature
      <222> 95411
      <223> deletion of C in ref genbank : L78132
   <220>
      <221> misc_feature
      <222> 95419
      <223> deletion of C in ref genbank : L78132
   <220>
      <221> misc_feature
      <222> 95431
      <223> insertion of TG in ref genbank : L78132
   <220>
      <221> misc_feature
      <222> 95435
      <223> insertion of C in ref genbank : L78132
   <220>
      <221> misc_feature
      <222> 95444
      <223> diverging nucleotide G in ref genbank : L78132
   <220>
      <221> misc_feature
      <222> 95445
      <223> diverging nucleotide C in ref genbank : L78132
   <220>
      <221> misc_feature
      <222> 95534
      <223> insertion of A in ref genbank : L78132
   <220>
      <221> misc_feature
      <222> 95678
      <223> insertion of G in ref genbank : L78132
   <220>
      <221> primer_bind
      <222> 67820..67850
      <223> 5-169-208_A_AS complement
   <220>
      <221> primer_bind
      <222> 67820..67848
      <223> 5-169-208_A_S
   <220>
      <221> primer_bind
      <222> 67820..67850
      <223> 5-169-208_G_AS complement
   <220>
      <221> primer_bind
      <222> 67820..67848
      <223> 5-169-208_G_S
   <220>
      <221> primer_bind
      <222> 67941..67969
      <223> 5-169-331_C_AS complement
   <220>
      <221> primer_bind
      <222> 67940..67969
      <223> 5-169-331_C_S

   <220>
      <221> primer_bind
      <222> 67941..67969
      <223> 5-169-331_T_AS complement
   <220>
      <221> primer_bind
      <222> 67940..67969
      <223> 5-169-331_T_S
   <220>
      <221> primer_bind
      <222> 67709..67738
      <223> 5-169-97_C_AS complement
   <220>
      <221> primer_bind
      <222> 67707..67737
      <223> 5-169-97_C_S
   <220>
      <221> primer_bind
      <222> 67709..67738
      <223> 5-169-97_G_AS complement
   <220>
      <221> primer_bind
      <222> 67707..67737
      <223> 5-169-97-G-S
   <220>
      <221> primer_bind
      <222> 68199..68228
      <223> 5-170-238_A_AS complement
   <220>
      <221> primer_bind
      <222> 68198..68227
      <223> 5-170-238_A_S
   <220>
      <221> primer_bind
      <222> 68199..68228
      <223> 5-170-238_G_AS complement
   <220>
      <221> primer_bind
      <222> 68198..68227
      <223> 5-170-238_G_S
   <220>
      <221> primer_bind
      <222> 68249..68277
      <223> 5-170-288_A_AS complement
   <220>
      <221> primer_bind
      <222> 68247..68277
      <223> 5-170-288_A_S
   <220>
      <221> primer_bind
      <222> 68249..68277
      <223> 5-170-288_C_AS complement
   <220>
      <221> primer_bind
      <222> 68247..68277
      <223> 5-170-288_C_S
   <220>
      <221> primer_bind
      <222> 68463..68492
      <223> 5-171-156_G_AS complement
   <220>
      <221> primer_bind
      <222> 68463..68491
      <223> 5-171-156_G_S
   <220>
      <221> primer_bind
      <222> 68463..68492
      <223> 5-171-156_T_AS complement
   <220>
      <221> primer_bind
      <222> 68463..68491
      <223> 5-171-156_T_S
   <220>
      <221> primer_bind
      <222> 68511..68539
      <223> 5-171-204_C_AS complement
   <220>
      <221> primer_bind
      <222> 68511..68539
      <223> 5-171-204_C_S
   <220>
      <221> primer_bind
      <222> 68511..68539
      <223> 5-171-204_T_AS complement
   <220>
      <221> primer_bind
      <222> 68511..68539
      <223> 5-171-204_T_S
   <220>
      <221> primer_bind
      <222> 68580..68608
      <223> 5-171-273_A_AS complement
   <220>
      <221> primer_bind
      <222> 68580..68608
      <223> 5-171-273_A_S
   <220>
      <221> primer_bind
      <222> 68580..68608
      <223> 5-171-273_G_AS complement
   <220>
      <221> primer_bind
      <222> 68580..68608
      <223> 5-171-273_G_S
   <220>
      <221> primer_bind
      <222> 68596..68626
      <223> 5-171-289_C_AS complement
   <220>
      <221> primer_bind
      <222> 68596..68624
      <223> 5-171-289_C_S
   <220>
      <221> primer_bind
      <222> 68596..68626
      <223> 5-171-289_T_AS complement
   <220>
      <221> primer_bind
      <222> 68596..68624
      <223> 5-171-289_T_S
   <220>
      <221> primer_bind
      <222> 68361..68389
      <223> 5-171-54_C_AS complement
   <220>
      <221> primer_bind
      <222> 68360..68389
      <223> 5-171-54_C_S
   <220>
      <221> primer_bind
      <222> 68361..68389
      <223> 5-171-54_G_AS complement
   <220>
      <221> primer_bind
      <222> 68360..68389
      <223> 5-171-54_G_S
   <220>
      <221> primer_bind
      <222> 66953..66983
      <223> 99-1572-315_C_AS complement
   <220>
      <221> primer_bind
      <222> 66951..66981
      <223> 99-1572-315_C_S
   <220>
      <221> primer_bind
      <222> 66953..66983
      <223> 99-1572-315_T_AS complement
   <220>
      <221> primer_bind
      <222> 66951..66981
      <223> 99-1572-315_T_S
   <220>
      <221> primer_bind
      <222> 66973..67002
      <223> 99-1572-335_A_AS complement
   <220>
      <221> primer_bind
      <222> 66973..67001
      <223> 99-1572-335_A_S
   <220>
      <221> primer_bind
      <222> 66973..67002
      <223> 99-1572-335_G_AS complement
   <220>
      <221> primer_bind
      <222> 66973..67001
      <223> 99-1572-335_G_S

   <220>
      <221> primer_bind
      <222> 67078..67106
      <223> 99-1572-440_C_AS complement
   <220>
      <221> primer_bind
      <222> 67078..67106
      <223> 99-1572-440_C_S
   <220>
      <221> primer_bind
      <222> 67078..67106
      <223> 99-1572-440_T_AS complement
   <220>
      <221> primer_bind
      <222> 67078..67106
      <223> 99-1572-440_T_S
   <220>
      <221> primer_bind
      <222> 67115..67144
      <223> 99-1572-477_A_AS complement
   <220>
      <221> primer_bind
      <222> 67113..67143
      <223> 99-1572-477_A_S
   <220>
      <221> primer_bind
      <222> 67115..67144
      <223> 99-1572-477_T_AS complement
   <220>
      <221> primer_bind
      <222> 67113..67143
      <223> 99-1572-477_T_S
   <220>
      <221> primer_bind
      <222> 67215..67247
      <223> 99-1572-578_C_AS complement
   <220>
      <221> primer_bind
      <222> 67212..67243
      <223> 99-1572-578_C_S
   <220>
      <221> primer_bind
      <222> 67215..67247
      <223> 99-1572-578_T_AS complement
   <220>
      <221> primer_bind
      <222> 67212..67243
      <223> 99-1572-578_T_S
   <220>
      <221> misc_feature
      <222> 8187,14867,14970,29204,29487,34266
      <223> n=a, g, c or t
   <400> 1
   <210> 2
      <211> 5408
      <212> DNA
      <213> Homo sapiens
   <220>
      <221> misc_feature
      <222> 201..203
      <223> ATG
   <220>
      <221> misc_feature
      <222> 1149..1151
      <223> stop :TAG
   <220>
      <221> polyA_signal
      <222> 1773..1778
      <223> AATAAA
   <220>
      <221> polyA_signal
      <222> 3624..3629
      <223> AATAAA
   <220>
      <221> polyA_signal
      <222> 3828..3833
      <223> AATAAA
   <220>
      <221> polyA_signal
      <222> 5119..5124
      <223> AATAAA
   <220>
      <221> polyA_signal
      <222> 5381..5386
      <223> AATAAA
   <220>
      <221> polyA_signal
      <222> 5386..5391
      <223> AATAAA
   <220>
      <221> allele
      <222> 176
      <223> 5-1-222 : polymorphic base A or G
   <220>
      <221> allele
      <222> 253
      <223> 5-2-162 : polymorphic base A or T
   <220>
      <221> allele
      <222> 269
      <223> 5-2-178 : polymorphic base C or T
   <220>
      <221> allele
      <222> 303
      <223> 5-2-213 : polymorphic base C or T
   <220>
      <221> allele
      <222> 362
      <223> 5-3-83 : polymorphic base C or T
   <220>
      <221> allele
      <222> 363
      <223> 5-3-84 : polymorphic base A or G
   <220>
      <221> allele
      <222> 527
      <223> 5-3-248 : polymorphic base A or G
   <220>
      <221> allele
      <222> 749
      <223> 5-7-195 : polymorphic base G or C
   <220>
      <221> allele
      <222> 1013
      <223> 5-10-39 : polymorphic base C or T
   <220>
      <221> allele
      <222> 1276
      <223> 5-10-302 : polymorphic base A or G
   <220>
      <221> allele
      <222> 1308
      <223> 5-10-334 : polymorphic base A or C
   <220>
      <221> allele
      <222> 1500
      <223> 5-11-158 : polymorphic base A or G
   <220>
      <221> allele
      <222> 1572
      <223> 5-11-230 : polymorphic base G or T
   <220>
      <221> allele
      <222> 1576
      <223> 5-11-234 : polymorphic base C or T
   <220>
      <221> allele
      <222> 1641
      <223> 5-11-299 : polymorphic base A or T
   <220>
      <221> allele
      <222> 1646
      <223> 5-11-304 : polymorphic base A or C
   <220>
      <221> allele
      <222> 1671
      <223> 5-11-329 : polymorphic base C or T
   <220>
      <221> allele
      <222> 1768
      <223> 5-12-56 : polymorphic base insertion of CTTT
   <220>
      <221> allele
      <222> 1979
      <223> 5-12-267 : polymorphic base A or C
   <220>
      <221> allele
      <222> 2156
      <223> 5-13-145 : polymorphic base C or T
   <220>
      <221> allele
      <222> 2423
      <223> 5-14-44 : polymorphic base C or T
   <220>
      <221> allele
      <222> 2471
      <223> 5-14-93 : polymorphic base A or T
   <220>
      <221> allele
      <222> 2522
      <223> 5-14-144 : polymorphic base insertion of T
   <220>
      <221> allele
      <222> 2543
      <223> 5-14-165 : polymorphic base C or T
   <220>
      <221> allele
      <222> 2675
      <223> 5-14-297 : polymorphic base A or C
   <220>
      <221> allele
      <222> 2685
      <223> 5-14-307 : polymorphic base G or T
   <220>
      <221> allele
      <222> 2973
      <223> 5-15-219 : polymorphic base A or T
   <220>
      <221> allele
      <222> 3242
      <223> 5-16-157 : polymorphic base A or G
   <220>
      <221> allele
      <222> 3514
      <223> 5-17-140 : polymorphic base A or G
   <220>
      <221> allele
      <222> 3593
      <223> 5-18-51 : polymorphic base G or T
   <220>
      <221> allele
      <222> 3750
      <223> 5-18-208 : polymorphic base A or C
   <220>
      <221> allele
      <222> 4023
      <223> 5-300-238 : polymorphic base C or T
   <220>
      <221> allele
      <222> 4072
      <223> 5-300-287 : polymorphic base A or G
   <220>
      <221> allele
      <222> 4398
      <223> 5-262-49 : polymorphic base insertion of C
   <220>
      <221> allele
      <222> 4434
      <223> 5-262-85 : polymorphic base C or T
   <220>
      <221> allele
      <222> 4603
      <223> 5-262-254 : polymorphic base C or T
   <220>
      <221> allele
      <222> 5204
      <223> 5-263-404 : polymorphic base C or T
   <220>
      <221> allele
      <222> 5397
      <223> 5-265-244 : polymorphic base A or G
   <220>
      <221> mise_feature
      <222> 708
      <223> diverging nucleotide G in ref genbank : L78132
   <220>
      <221> misc_feature
      <222> 709
      <223> diverging nucleotide T in ref genbank : L78132
   <220>
      <221> misc_feature
      <222> 807
      <223> diverging nucleotide C in ref genbank : L78132
   <220>
      <221> misc_feature
      <222> 1230
      <223> insertion of G in ref genbank : L78132
   <220>
      <221> misc_feature
      <222> 1493
      <223> diverging nucleotide T in ref genbank : L78132
   <220>
      <221> misc_feature
      <222> 1724
      <223> diverging nucleotide G in ref genbank : L78132
   <220>
      <221> misc_feature
      <222> 1845
      <223> diverging nucleotide C in ref genbank : L78132
   <220>
      <221> misc_feature
      <222> 1933
      <223> diverging nucleotide C in ref genbank : L78132
   <220>
      <221> misc_feature
      <222> 1934
      <223> diverging nucleotide C in ref genbank : L78132
   <220>
      <221> misc_feature
      <222> 1935
      <223> diverging nucleotide C in ref genbank : L78132
   <220>
      <221> misc_feature
      <222> 1936
      <223> diverging nucleotide G in ref genbank : L78132
   <220>
      <221> misc_feature
      <222> 1965
      <223> deletion of A in ref genbank : L78132
   <220>
      <221> misc_feature
      <222> 1981
      <223> diverging nucleotide C in ref genbank : L78132
   <220>
      <221> misc_feature
      <222> 2000
      <223> diverging nucleotide T in ref genbank : L78132
   <220>
      <221> misc_feature
      <222> 2014
      <223> diverging nucleotide C in ref genbank : L78132
   <220>
      <221> misc_feature
      <222> 2404
      <223> deletion of TTA in ref genbank : L78132
   <220>
      <221> misc_feature
      <222> 2407
      <223> diverging nucleotide C in ref genbank : L78132
   <220>
      <221> misc_feature
      <222> 2494
      <223> insertion of G in ref genbank : L78132
   <220>
      <221> misc_feature
      <222> 2683
      <223> diverging nucleotide C in ref genbank : L78132
   <220>
      <221> misc_feature
      <222> 3024
      <223> insertion of A in ref genbank : L78132
   <220>
      <221> misc_feature
      <222> 3058
      <223> insertion of T in ref genbank : L78132
   <220>
      <221> misc_feature
      <222> 3374
      <223> deletion of AG in ref genbank : L78132
   <220>
      <221> misc_feature
      <222> 3379
      <223> diverging nucleotide A in ref genbank : L78132
   <220>
      <221> misc_feature
      <222> 3383
      <223> deletion of G in ref genbank : L78132
   <220>
      <221> misc_feature
      <222> 3387
      <223> deletion of G in ref genbank : L78132
   <220>
      <221> misc_feature
      <222> 3402
      <223> deletion of A in ref genbank : L78132
   <220>
      <221> misc_feature
      <222> 3408
      <223> deletion of A in ref genbank : L78132
   <220>
      <221> misc_feature
      <222> 3427
      <223> deletion of AA in ref genbank : L78132
   <220>
      <221> misc_feature
      <222> 3621
      <223> deletion of A in ref genbank : L78132
   <220>
      <221> misc_feature
      <222> 3664
      <223> deletion of C in ref genbank : L78132
   <220>
      <221> misc_feature
      <222> 3672
      <223> deletion of C in ref genbank : L78132
   <220>
      <221> misc_feature
      <222> 3684
      <223> insertion of TG in ref genbank : L78132
   <220>
      <221> misc_feature
      <222> 3688
      <223> insertion of C in ref genbank : L78132
   <220>
      <221> misc_feature
      <222> 3697
      <223> diverging nucleotide G in ref genbank : L78132
   <220>
      <221> misc_feature
      <222> 3698
      <223> diverging nucleotide C in ref genbank : L78132
   <220>
      <221> misc_feature
      <222> 3787
      <223> insertion of A in ref genbank : L78132
   <220>
      <221> misc_feature
      <222> 3931
      <223> insertion of G in ref genbank : L78132
   <400> 2
   agccgcccac ggacgccaga gccgggaacc ctgacggcac ttagctgctg acaaacaacc      60
   tgctccgtgg agcgcctgaa acaccagtct ttggggccag tgcctcagtt tcaatccagg     120
   taacctttaa atgaaacttg cctaaaatct taggtcatac acagaagaga ctccaatcga     180
   caagaagctg gaaaagaatg atg ttg tcc tta aac aac cta cag aat atc atc     233
                         Met Leu Ser Leu Asn Asn Leu Gln Asn Ile Ile
                         1               5                   10
   tat aac ccg gta atc ccg tat gtt ggc acc att ccc gat cag ctg gat       281
   Tyr Asn Pro Val Ile Pro Tyr Val Gly Thr Ile Pro Asp Gln Leu Asp
               15                  20                  25
   cct gga act ttg att gtg ata tgt ggg cat gtt cct agt gac gca gac       329
   Pro Gly Thr Leu Ile Val Ile Cys Gly His Val Pro Ser Asp Ala Asp
           30                  35                  40
   aga ttc cag gtg gat ctg cag aat ggc agc agt gtg aaa cct cga gcc       377
   Arg Phe Gln Val Asp Leu Gln Asn Gly Ser Ser Val Lys Pro Arg Ala
       45                  50                  55
   gat gtg gcc ttt cat ttc aat cct cgt ttc aaa agg gcc ggc tgc att       425
   Asp Val Ala Phe His Phe Asn Pro Arg Phe Lys Arg Ala Gly Cys Ile
   60                  65                  70                  75
   gtt tgc aat act ttg ata aat gaa aaa tgg gga cgg gaa gag atc acc      473
   Val Cys Asn Thr Leu Ile Asn Glu Lys Trp Gly Arg Glu Glu Ile Thr
                   80                  85                  90
   tat gac acg cct ttc aaa aga gaa aag tct ttt gag atc gtg att atg      521
   Tyr Asp Thr Pro Phe Lys Arg Glu Lys Ser Phe Glu Ile Val Ile Met
               95                  100                 105
   gtg cta aag gac aaa ttc cag gtg gct gta aat gga aaa cat act ctg      569
   Val Leu Lys Asp Lys Phe Gln Val Ala Val Asn Gly Lys His Thr Leu
           110                 115                 120
   ctc tat ggc cac agg atc ggc cca gag aaa ata gac act ctg ggc att      617
   Leu Tyr Gly His Arg Ile Gly Pro Glu Lys Ile Asp Thr Leu Gly Ile
       125                 130                 135
   tat ggc aaa gtg aat att cac tca att ggt ttt agc ttc agc tcg gac      665
   Tyr Gly Lys Val Asn Ile His Ser Ile Gly Phe Ser Phe Ser Ser Asp
   140                 145                 150                 155
   tta caa agt acc caa gca tct agt ctg gaa ctg aca gag ata agt aga      713
   Leu Gln Ser Thr Gln Ala Ser Ser Leu Glu Leu Thr Glu Ile Ser Arg
                   160                 165                 170
   gaa aat gtt cca aag tct ggc acg ccc cag ctt agc ctg cca ttc gct      761
   Glu Asn Val Pro Lys Ser Gly Thr Pro Gln Leu Ser Leu Pro Phe Ala
               175                 180                 185
   gca agg ttg aac acc ccc atg ggc cct gga cga act gtc gtc gtt aaa      809
   Ala Arg Leu Asn Thr Pro Met Gly Pro Gly Arg Thr Val Val Val Lys
           190                 195                 200
   gga gaa gtg aat gca aat gcc aaa agc ttt aat gtt gac cta cta gca      857
   Gly Glu Val Asn Ala Asn Ala Lys Ser Phe Asn Val Asp Leu Leu Ala
       205                 210                 215
   gga aaa tca aag gat att gct cta cac ttg aac cca cgc ctg aat att      905
   Gly Lys Ser Lys Asp Ile Ala Leu His Leu Asn Pro Arg Leu Asn Ile
   220                 225                 230                 235
   aaa gca ttt gta aga aat tct ttt ctt cag gag tcc tgg gga gaa gaa      953
   Lys Ala Phe Val Arg Asn Ser Phe Leu Gln Glu Ser Trp Gly Glu Glu
                   240                 245                 250
   gag aga aat att acc tct ttc cca ttt agt cct ggg atg tac ttt gag     1001
   Glu Arg Asn Ile Thr Ser Phe Pro Phe Ser Pro Gly Met Tyr Phe Glu
               255                 260                 265
   atg ata att tac tgt gat gtt aga gaa ttc aag gtt gca gta aat ggc     1049
   Met Ile Ile Tyr Cys Asp Val Arg Glu Phe Lys Val Ala Val Asn Gly
           270                 275                 280
   gta cac agc ctg gag tac aaa cac aga ttt aaa gag ctc agc agt att     1097
   Val His Ser Leu Glu Tyr Lys His Arg Phe Lys Glu Leu Ser Ser Ile
       285                 290                 295
   gac acg ctg gaa att aat gga gac atc cac tta ctg gaa gta agg agc     1145
   Asp Thr Leu Glu Ile Asn Gly Asp Ile His Leu Leu Glu Val Arg Ser
   300                 305                 310                 315
   tgg tag cctacctaca cagctgctac aaaaaccaaa atacagaatg gcttctgtga      1201
   Trp  *
   tactggcctt gctgaaacgc atctcactgt cattctattg tttatattgt taaaatgagc   1261
   ttgtgcacca ttagatcctg ctgggtgttc tcagtccttg ccatgaagta tggtggtgtc   1321
   tagcactgaa tggggaaact gggggcagca acacttatag ccagttaaag ccactctgcc   1381
   ctctctccta ctttggctga ctcttcaaga atgccattca acaagtattt atggagtacc   1441
   tactataata cagtagctaa catgtattga gcacagattt tttttggtaa aactgtgagg   1501
   agctaggata tatacttggt gaaacaaacc agtatgttcc ctgttctctt gagcttcgac   1561
   tcttctgtgc tctattgctg cgcactgctt tttctacagg cattacatca actcctaagg   1621
   ggtcctctgg gattagttaa gcagctatta aatcacccga agacactaat ttacagaaga   1681
   cacaactcct tccccagtga tcactgtcat aaccagtgct ctaccgtatc ccatcactga   1741
   ggactgatgt tgactgacat cattttatcg taataaacat gtggctctat tagctgcaag   1801
   ctttaccaag taattggcat gacatctgag cacagaaatt aaggcaaaaa accaaagcaa   1861
   aacaaataca tggtgctgaa attaacttga tgccaagccc aaggcagctg atttctgtgt   1921
   atttgaactt agggcaaatc agagtctaca cagacgccta cagaaagttt caggaagagg   1981
   caagatgcat tcaatttgaa agatatttat gggcaacaaa gtaaggtcag gattagactt   2041
   caggcattca taaggcaggc actatcagaa agtgtacgcc aactaaggga cccacaaagc   2101
   aggcagaggt aatgcagaaa tctgttttgt tcccatgaaa tcaccaatca aggcctccgt   2161
   tcttctaaag attagtccat catcattagc aactgagatc aaagcactct tccactttac   2221
   gtgattaaaa tcaaacctgt atcagcaagt taaatggttc catttctgtg atttttctat   2281
   tatttgaggg gagttggcag aagttccatg tatatgggat ctttacaggt cagatcttgt   2341
   tacaggaaat ttcaaaggtt tgggagtggg gagggaaaaa agctcagtca gtgaggatca   2401
   ttttatcaca ttagactggg gcagaactct gccaggattt aggaatattt tcagaacaga   2461
   ttttagatat tatttctatc catatattga aaagaatacc attgtcaatc ttattttttt   2521
   aaaagtactc agtgtagaaa ttgctagccc ttaattcttt tccagctttt catattaatg   2581
   tatgcagagt ctcaccaagc tcaaagacac tggttggggg tggagggtgc cacagggaaa   2641
   gctgtagaag gcaagaagac tcgagaatcc cccagagtta tttttctcca taaagaccat   2701
   cagagtgctt aactgagctg ttggagactg tgaggcattt aggaaaaaaa tagcccactc   2761
   acatcattcc ttgtaagtct taagttcatt ttcattttac gtggaggaaa aaaatttaaa   2821
   aagctattag tatttattaa tgaattttac tgagacattt cttagaaata tgcacttcta   2881
   tactagcaag ctctgtctct aaaatgcaag ttggcctttt gcttgccaca tttctgcatt   2941
   aaacttctat attagcttca aaggctttta aactcaatgc gaacattcta cgggatgttc   3001
   ttagatgcct ttaaaaaggg ggcagatcta attttatttg aaccctcact ttccaacttc   3061
   accatgaccc agtactagag attagggcac ttcaaagcat tgaaaaaaat ctactgatac   3121
   ttactttctt agacaagtag ttcttagtta accaccaatg gaactgggtt cattctgaat   3181
   cctggaggag cttcctcgtg ccacccagtg tttctgggcc ctctgtgtga gcagccaggt   3241
   atgagctgtt ttagaagcag cgtgttgcct tcatctctcc cgtttcccaa aagaacaaag   3301
   gataaaggtg acagtcacac tcctgggtta aaaaaagcat tccagaacca cttctcttta   3361
   tgggcacaac aaagaaacga aggctgaagt tcgcctaccc aaaatgaaaa gtaggcttta   3421
   cagtcaaaag tacttctgtt gattgctaaa taacttcatt ttcttgaaat agagcaactt   3481
   tgagtgaaat ctgcaacatg gataccatgt atataagata ctgctgtaca gaagagttaa   3541
   ggcttacagt gcaaatgagg cgtcagcttt gggtgctaaa attaacaagt ctaatattat   3601
   taccatcaat caggaagaga ataataaatg tttaaacaaa cacagcagtc tgtataaaaa   3661
   taccgtgtat catttactct ttctgcagct ctatacgata ggcaggagag gcttatgtgg   3721
   cagcacaagc caggtgggga ttttgtaacg aagtgataaa acatttgtaa gtaatccaag   3781
   taggtgtatt aaggcaccaa aagtaacatg gcacccaaca cccaaaaata aaaatatgaa   3841
   atatgagtgt gaactctgag tagagtatga aacaccacag aaagtcttag aaatagctct   3901
   ggagtggctc tcccaggaca gtttccagtt gctgaatagt cttttggcac tgatgttcta   3961
   cttcttcaca ttcatctaaa aaaaaaaaaa aaaaaaatca aaattaaaat ctgagtcagt   4021
   ctgcctgcct cggttctcat tagtttaatt cttaatgcct tgcactttcc agcaatcatt   4081
   caatcaaaag agtgaaatga agcacattaa caaagcagga ggcgccacgg accgcctccc   4141
   tccacaccgc tccttccgcc ttcattcctt gcccacaggc ttgcactgga agctgaataa   4201
   gaatccccaa aactcaaact tcctagggat gccacccctt tagtagctca cacctccccc   4261
   ctccaagagc taagaaacaa aggagaatgt acttttgtag cttagataag caatgaatca   4321
   gtaaaggact gatctacttg ctccaccacc cctcccttaa taataacatt tactgttatt   4381
   tcctgggcct aagacttatg ttccagaact gtcacagctc cccatgtcac acccactagc   4441
   ttgtgatctt tgtcaaataa ctgaaatctt ttaagcctct agtttcttcc tttgtaaaac   4501
   agagataaaa tgttgtggtt tttaagtgag ataatccaag taaagcacct aacatggagt   4561
   agtgaatgaa catcggttgc tactaaaagt ggacatccta ccgcatcctt aatgccacta   4621
   ggcatttcca tacaatctgg ggaccaaaac ttcaatcata taaatgtatg aggttaatta   4681
   aaaacactac tgtaatctgc ttgtatgatc acaaaccacc acaaaagaaa agatcgtgaa   4741
   gattacactg taaacggact ctcaaatgat caggaggtgg tcacttcgca acttgctccc   4801
   tccacccaac tcaaaacagg agctcgagcc tgcctgtatt tgagactgga gctgcctgta   4861
   tgaggactgg atcaactgct agtcacgtta tatccaaatc tgcattatca ttgggcacat   4921
   tttcacagaa ttttactgaa ttattcctta attgtttaat ggttgggaat agtttgggaa   4981
   ttaccttcca tcaactctgc taagaaagga atggattctg gtagcaagac aatataattc   5041
   tcctttagtt tttcagccag tgctaacaca gtaatcaaag cagcaaatcg aacctgaaag   5101
   ggataaaaga gcaaagaaat aaaaagtagt gttactgtat ttattatctt aagagctgta   5161
   ctgacttgag acaagctcta actttttaaa cattagttca cacgcgttta ttcacttcat   5221
   tatgttcatt aagctttcat cttagaatac cagtttcacc atttgggagc tgtttgtaat   5281
   atgtgcaacc ttataaatag tgttttccaa actgtgtccc aggactgcaa atctttaatg   5341
   tgaaatgtct ttttataatc tcttccttta aaaaaaacca ataaaataaa atgccacatg   5401
   caaactc                                                              5408
   <210> 3
      <211> 5534
      <212> DNA
      <213> Homo sapiens
   <220>
      <221> misc_feature
      <222> 201..203
      <223> ATG
   <220>
      <221> misc_feature
      <222> 1275..1277
      <223> stop :TAG
   <220>
      <221> polyA_signal
      <222> 1899..1904
      <223> AATAAA
   <220>
      <221> polyA_signal
      <222> 3750..3755
      <223> AATAAA
   <220>
      <221> polyA_signal
      <222> 3954..3959
      <223> AATAAA
   <220>
      <221> polyA_signal
      <222> 5245..5250
      <223> AATAAA
   <220>
      <221> polyA_signal
      <222> 5507..5512
      <223> AATAAA
   <220>
      <221> polyA_signal
      <222> 5512..5517
      <223> AATAAA
   <220>
      <221> allele
      <222> 176
      <223> 5-1-222 : polymorphic base A or G
   <220>
      <221> allele
      <222> 253
      <223> 5-2-162 : polymorphic base A or T
   <220>
      <221> allele
      <222> 269
      <223> 5-2-178 : polymorphic base C or T
   <220>
      <221> allele
      <222> 303
      <223> 5-2-213 : polymorphic base C or T
   <220>
      <221> allele
      <222> 362
      <223> 5-3-83 : polymorphic base C or T
   <220>
      <221> allele
      <222> 363
      <223> 5-3-84 : polymorphic base A or G
   <220>
      <221> allele
      <222> 527
      <223> 5-3-248 : polymorphic base A or G
   <220>
      <221> allele
      <222> 810
      <223> 5-202-95 : polymorphic base G or T
   <220>
      <221> allele
      <222> 832
      <223> 5-202-117 : polymorphic base A or T
   <220>
      <221> allele
      <222> 875
      <223> 5-7-195 : polymorphic base G or C
   <220>
      <221> allele
      <222> 1139
      <223> 5-10-39 : polymorphic base C or T
   <220>
      <221> allele
      <222> 1402
      <223> 5-10-302 : polymorphic base A or G
   <220>
      <221> allele
      <222> 1434
      <223> 5-10-334 : polymorphic base A or C
   <220>
      <221> allele
      <222> 1626
      <223> 5-11-158 : polymorphic base A or G
   <220>
      <221> allele
      <222> 1698
      <223> 5-11-230 : polymorphic base G or T
   <220>
      <221> allele
      <222> 1702
      <223> 5-11-234 : polymorphic base C or T
   <220>
      <221> allele
      <222> 1767
      <223> 5-11-299 : polymorphic base A or T
   <220>
      <221> allele
      <222> 1772
      <223> 5-11-304 : polymorphic base A or C
   <220>
      <221> allele
      <222> 1797
      <223> 5-11-329 : polymorphic base C or T
   <220>
      <221> allele
      <222> 1894
      <223> 5-12-56 : polymorphic base insertion of CTTT
   <220>
      <221> allele
      <222> 2105
      <223> 5-12-267 : polymorphic base A or C
   <220>
      <221> allele
      <222> 2282
      <223> 5-13-145 : polymorphic base C or T
   <220>
      <221> allele
      <222> 2549
      <223> 5-14-44 : polymorphic base C or T
   <220>
      <221> allele
      <222> 2597
      <223> 5-14-93 : polymorphic base A or T
   <220>
      <221> allele
      <222> 2648
      <223> 5-14-144 : polymorphic base insertion of T
   <220>
      <221> allele
      <222> 2669
      <223> 5-14-165 : polymorphic base C or T
   <220>
      <221> allele
      <222> 2801
      <223> 5-14-297 : polymorphic base A or C
   <220>
      <221> allele
      <222> 2811
      <223> 5-14-307 : polymorphic base G or T
   <220>
      <221> allele
      <222> 3099
      <223> 5-15-219 : polymorphic base A or T
   <220>
      <221> allele
      <222> 3368
      <223> 5-16-157 : polymorphic base A or G
   <220>
      <221> allele
      <222> 3640
      <223> 5-17-140 : polymorphic base A or G
   <220>
      <221> allele
      <222> 3719
      <223> 5-18-51 : polymorphic base G or T
   <220>
      <221> allele
      <222> 3876
      <223> 5-18-208 : polymorphic base A or C
   <220>
      <221> allele
      <222> 4149
      <223> 5-300-238 : polymorphic base C or T
   <220>
      <221> allele
      <222> 4198
      <223> 5-300-287 : polymorphic base A or G
   <220>
      <221> allele
      <222> 4524
      <223> 5-262-49 : polymorphic base insertion of C
   <220>
      <221> allele
      <222> 4560
      <223> 5-262-85 : polymorphic base C or T
   <220>
      <221> allele
      <222> 4729
      <223> 5-262-254 : polymorphic base C or T
   <220>
      <221> allele
      <222> 5330
      <223> 5-263-404 : polymorphic base C or T
   <220>
      <221> allele
      <222> 5523
      <223> 5-265-244 : polymorphic base A or G
   <220>
      <221> misc_feature
      <222> 708
      <223> diverging nucleotide G in ref genbank : L78132
   <220>
      <221> misc_feature
      <222> 709
      <223> diverging nucleotide T in ref genbank : L78132
   <220>
      <221> misc_feature
      <222> 807
      <223> diverging nucleotide C in ref genbank : L78132
   <220>
      <221> misc_feature
      <222> 1356
      <223> insertion G in ref genbank : L78132
   <220>
      <221> misc_feature
      <222> 1619
      <223> diverging nucleotide T in ref genbank : L78132
   <220>
      <221> misc feature
      <222> 1850
      <223> diverging nucleotide G in ref genbank : L78132
   <220>
      <221> misc_feature
      <222> 1971
      <223> diverging nucleotide C in ref genbank : L78132
   <220>
      <221> misc_feature
      <222> 2059
      <223> diverging nucleotide C in ref genbank : L78132
   <220>
      <221> misc_feature
      <222> 2060
      <223> diverging nucleotide C in ref genbank : L78132
   <220>
      <221> misc_feature
      <222> 2061
      <223> diverging nucleotide C in ref genbank : L78132
   <220>
      <221> misc_feature
      <222> 2062
      <223> diverging nucleotide G in ref genbank : L78132
   <220>
      <221> misc_feature
      <222> 2091
      <223> deletion A in ref genbank : L78132
   <220>
      <221> misc_feature
      <222> 2107
      <223> diverging nucleotide C in ref genbank : L78132
   <220>
      <221> misc_feature
      <222> 2126
      <223> diverging nucleotide T in ref genbank : L78132
   <220>
      <221> misc_feature
      <222> 2140
      <223> diverging nucleotide C in ref genbank : L78132
   <220>
      <221> misc_feature
      <222> 2530
      <223> deletion TTA in ref genbank : L78132
   <220>
      <221> misc_feature
      <222> 2533
      <223> diverging nucleotide C in ref genbank : L78132
   <220>
      <221> misc_feature
      <222> 2620
      <223> isertion G in ref genbank : L78132
   <220>
      <221> misc_feature
      <222> 2809
      <223> diverging nucleotide C in ref genbank : L78132
   <220>
      <221> misc_feature
      <222> 3150
      <223> isertion A in ref genbank : L78132
   <220>
      <221> misc_feature
      <222> 3184
      <223> isertion T in ref genbank : L78132
   <220>
      <221> misc_feature
      <222> 3500
      <223> deletion AG in ref genbank : L78132
   <220>
      <221> misc_feature
      <222> 3505
      <223> diverging nucleotide A in ref genbank : L78132
   <220>
      <221> misc_feature
      <222> 3509
      <223> deletion G in ref genbank : L78132
   <220>
      <221> mise_feature
      <222> 3513
      <223> deletion G in ref genbank : L78132
   <220>
      <221> misc_feature
      <222> 3528
      <223> deletion A in ref genbank : L78132
   <220>
      <221> misc_feature
      <222> 3534
      <223> deletion A in ref genbank : L78132
   <220>
      <221> misc_feature
      <222> 3553
      <223> deletion AA in ref genbank : L78132
   <220>
      <221> misc_feature
      <222> 3747
      <223> deletion A in ref genbank : L78132
   <220>
      <221> misc_feature
      <222> 3790
      <223> deletion C in ref genbank : L78132
   <220>
      <221> misc_feature
      <222> 3798
      <223> deletion C in ref genbank : L78132
   <220>
      <221> misc_feature
      <222> 3810
      <223> isertion TG in ref genbank : L78132
   <220>
      <221> misc_feature
      <222> 3814
      <223> isertion C in ref genbank : L78132
   <220>
      <221> misc_feature
      <222> 3823
      <223> diverging nucleotide G in ref genbank : L78132
   <220>
      <221> misc_feature
      <222> 3824
      <223> diverging nucleotide C in ref genbank : L78132
   <220>
      <221> misc_feature
      <222> 3913
      <223> isertion A in ref genbank : L78132
   <220>
      <221> misc_feature
      <222> 4057
      <223> isertion G in ref genbank : L78132
   <400> 3
   agccgcccac ggacgccaga gccgggaacc ctgacggcac ttagctgctg acaaacaacc    60
   tgctccgtgg agcgcctgaa acaccagtct ttggggccag tgcctcagtt tcaatccagg   120
   taacctttaa atgaaacttg cctaaaatct taggtcatac acagaagaga ctccaatcga   180
   caagaagctg gaaaagaatg atg ttg tcc tta aac aac cta cag aat atc atc   233
   tat aac ccg gta atc ccg tat gtt ggc acc att ccc gat cag ctg gat     281
   cct gga act ttg att gtg ata tgt ggg cat gtt cct agt gac gca gac     329
   aga ttc cag gtg gat ctg cag aat ggc agc agt gtg aaa cct cga gcc     377
   gat gtg gcc ttt cat ttc aat cct cgt ttc aaa agg gcc ggc tgc att     425
   gtt tgc aat act ttg ata aat gaa aaa tgg gga cgg gaa gag atc acc     473
   tat gac acg cct ttc aaa aga gaa aag tct ttt gag atc gtg att atg     521
   gtg cta aag gac aaa ttc cag gtg gct gta aat gga aaa cat act ctg     569
   ctc tat ggc cac agg atc ggc cca gag aaa ata gac act ctg ggc att     617
   tat ggc aaa gtg aat att cac tca att ggt ttt agc ttc agc tcg gac     665
   tta caa agt acc caa gca tct agt ctg gaa ctg aca gag ata agt aga     713
   gaa aat gtt cca aag tct ggc acg ccc cag ctt cct agt aat aga gga     761
   gga gac att tct aaa atc gca ccc aga act gtc tac acc aag agc aaa     809
   gat tcg act gtc aat cac act ttg act tgc acc aaa ata cca cct atg     857
   aac tat gtg tca aag agc ctg cca ttc gct gca agg ttg aac acc ccc     905
   atg ggc cct gga cga act gtc gtc gtt aaa gga gaa gtg aat gca aat     953
   gcc aaa agc ttt aat gtt gac cta cta gca gga aaa tca aag gat att    1001
   gct cta cac ttg aac cca cgc ctg aat att aaa gca ttt gta aga aat    1049
   tct ttt ctt cag gag tcc tgg gga gaa gaa gag aga aat att acc tct    1097
   ttc cca ttt agt cct ggg atg tac ttt gag atg ata att tac tgt gat      1145
   gtt aga gaa ttc aag gtt gca gta aat ggc gta cac agc ctg gag tac      1193
   aaa cac aga ttt aaa gag ctc agc agt att gac acg ctg gaa att aat      1241
   gga gac atc cac tta ctg gaa gta agg agc tgg tag cctacctaca           1287
   cagctgctac aaaaaccaaa atacagaatg gcttctgtga tactggcctt gctgaaacgc   1347
   atctcactgt cattctattg tttatattgt taaaatgagc ttgtgcacca ttagatcctg   1407
   ctgggtgttc tcagtccttg ccatgaagta tggtggtgtc tagcactgaa tggggaaact   1467
   gggggcagca acacttatag ccagttaaag ccactctgcc ctctctccta ctttggctga   1527
   ctcttcaaga atgccattca acaagtattt atggagtacc tactataata cagtagctaa   1587
   catgtattga gcacagattt tttttggtaa aactgtgagg agctaggata tatacttggt   1647
   gaaacaaacc agtatgttcc ctgttctctt gagcttcgac tcttctgtgc tctattgctg   1707
   cgcactgctt tttctacagg cattacatca actcctaagg ggtcctctgg gattagttaa   1767
   gcagctatta aatcacccga agacactaat ttacagaaga cacaactcct tccccagtga   1827
   tcactgtcat aaccagtgct ctaccgtatc ccatcactga ggactgatgt tgactgacat   1887
   cattttatcg taataaacat gtggctctat tagctgcaag ctttaccaag taattggcat   1947
   gacatctgag cacagaaatt aaggcaaaaa accaaagcaa aacaaataca tggtgctgaa   2007
   attaacttga tgccaagccc aaggcagctg atttctgtgt atttgaactt agggcaaatc   2067
   agagtctaca cagacgccta cagaaagttt caggaagagg caagatgcat tcaatttgaa   2127
   agatatttat gggcaacaaa gtaaggtcag gattagactt caggcattca taaggcaggc   2187
   actatcagaa agtgtacgcc aactaaggga cccacaaagc aggcagaggt aatgcagaaa   2247
   tctgttttgt tcccatgaaa tcaccaatca aggcctccgt tcttctaaag attagtccat   2307
   catcattagc aactgagatc aaagcactct tccactttac gtgattaaaa tcaaacctgt   2367
   atcagcaagt taaatggttc catttctgtg atttttctat tatttgaggg gagttggcag   2427
   aagttccatg tatatgggat ctttacaggt cagatcttgt tacaggaaat ttcaaaggtt   2487
   tgggagtggg gagggaaaaa agctcagtca gtgaggatca ttttatcaca ttagactggg   2547
   gcagaactct gccaggattt aggaatattt tcagaacaga ttttagatat tatttctatc   2607
   catatattga aaagaatacc attgtcaatc ttattttttt aaaagtactc agtgtagaaa   2667
   ttgctagccc ttaattcttt tccagctttt catattaatg tatgcagagt ctcaccaagc   2727
   tcaaagacac tggttggggg tggagggtgc cacagggaaa gctgtagaag gcaagaagac   2787
   tcgagaatcc cccagagtta tttttctcca taaagaccat cagagtgctt aactgagctg   2847
   ttggagactg tgaggcattt aggaaaaaaa tagcccactc acatcattcc ttgtaagtct   2907
   taagttcatt ttcattttac gtggaggaaa aaaatttaaa aagctattag tatttattaa   2967
   tgaattttac tgagacattt cttagaaata tgcacttcta tactagcaag ctctgtctct   3027
   aaaatgcaag ttggcctttt gcttgccaca tttctgcatt aaacttctat attagcttca   3087
   aaggctttta aactcaatgc gaacattcta cgggatgttc ttagatgcct ttaaaaaggg   3147
   ggcagatcta attttatttg aaccctcact ttccaacttc accatgaccc agtactagag   3207
   attagggcac ttcaaagcat tgaaaaaaat ctactgatac ttactttctt agacaagtag   3267
   ttcttagtta accaccaatg gaactgggtt cattctgaat cctggaggag cttcctcgtg   3327
   ccacccagtg tttctgggcc ctctgtgtga gcagccaggt atgagctgtt ttagaagcag   3387
   cgtgttgcct tcatctctcc cgtttcccaa aagaacaaag gataaaggtg acagtcacac   3447
   tcctgggtta aaaaaagcat tccagaacca cttctcttta tgggcacaac aaagaaacga   3507
   aggctgaagt tcgcctaccc aaaatgaaaa gtaggcttta cagtcaaaag tacttctgtt   3567
   gattgctaaa taacttcatt ttcttgaaat agagcaactt tgagtgaaat ctgcaacatg   3627
   gataccatgt atataagata ctgctgtaca gaagagttaa ggcttacagt gcaaatgagg   3687
   cgtcagcttt gggtgctaaa attaacaagt ctaatattat taccatcaat caggaagaga   3747
   ataataaatg tttaaacaaa cacagcagtc tgtataaaaa taccgtgtat catttactct   3807
   ttctgcagct ctatacgata ggcaggagag gcttatgtgg cagcacaagc caggtgggga   3867
   ttttgtaacg aagtgataaa acatttgtaa gtaatccaag taggtgtatt aaggcaccaa   3927
   aagtaacatg gcacccaaca cccaaaaata aaaatatgaa atatgagtgt gaactctgag   3987
   tagagtatga aacaccacag aaagtcttag aaatagctct ggagtggctc tcccaggaca   4047
   gtttccagtt gctgaatagt cttttggcac tgatgttcta cttcttcaca ttcatctaaa   4107
   aaaaaaaaaa aaaaaaatca aaattaaaat ctgagtcagt ctgcctgcct cggttctcat 4167
   tagtttaatt cttaatgcct tgcactttcc agcaatcatt caatcaaaag agtgaaatga 4227
   agcacattaa caaagcagga ggcgccacgg accgcctccc tccacaccgc tccttccgcc 4287
   ttcattcctt gcccacaggc ttgcactgga agctgaataa gaatccccaa aactcaaact 4347
   tcctagggat gccacccctt tagtagctca cacctccccc ctccaagagc taagaaacaa 4407
   aggagaatgt acttttgtag cttagataag caatgaatca gtaaaggact gatctacttg 4467
   ctccaccacc cctcccttaa taataacatt tactgttatt tcctgggcct aagacttatg 4527
   ttccagaact gtcacagctc cccatgtcac acccactagc ttgtgatctt tgtcaaataa 4587
   ctgaaatctt ttaagcctct agtttcttcc tttgtaaaac agagataaaa tgttgtggtt 4647
   tttaagtgag ataatccaag taaagcacct aacatggagt agtgaatgaa catcggttgc 4707
   tactaaaagt ggacatccta ccgcatcctt aatgccacta ggcatttcca tacaatctgg 4767
   ggaccaaaac ttcaatcata taaatgtatg aggttaatta aaaacactac tgtaatctgc 4827
   ttgtatgatc acaaaccacc acaaaagaaa agatcgtgaa gattacactg taaacggact 4887
   ctcaaatgat caggaggtgg tcacttcgca acttgctccc tccacccaac tcaaaacagg 4947
   agctcgagcc tgcctgtatt tgagactgga gctgcctgta tgaggactgg atcaactgct 5007
   agtcacgtta tatccaaatc tgcattatca ttgggcacat tttcacagaa ttttactgaa 5067
   ttattcctta attgtttaat ggttgggaat agtttgggaa ttaccttcca tcaactctgc 5127
   taagaaagga atggattctg gtagcaagac aatataattc tcctttagtt tttcagccag 5187
   tgctaacaca gtaatcaaag cagcaaatcg aacctgaaag ggataaaaga gcaaagaaat 5247
   aaaaagtagt gttactgtat ttattatctt aagagctgta ctgacttgag acaagctcta 5307
   actttttaaa cattagttca cacgcgttta ttcacttcat tatgttcatt aagctttcat 5367
   cttagaatac cagtttcacc atttgggagc tgtttgtaat atgtgcaacc ttataaatag 5427
   tgttttccaa actgtgtccc aggactgcaa atctttaatg tgaaatgtct ttttataatc 5487
   tcttccttta aaaaaaacca ataaaataaa atgccacatg caaactc               5534
   <210> 4
      <211> 2471
      <212> DNA
      <213> Homo sapiens
   <220>
      <221> misc_feature
      <222> 201..203
      <223> ATG
   <220>
      <221> misc_feature
      <222> 1305..1307
      <223> stop :TAG
   <220>
      <221> polyA_signal
      <222> 2182..2187
      <223> AATAAA
   <220>
      <221> polyA_signal
      <222> 2444..2449
      <223> AATAAA
   <220>
      <221> polyA_signal
      <222> 2449..2454
      <223> AATAAA
   <220>
      <221> allele
      <222> 176
      <223> 5-1-222 : polymorphic base A or G
   <220>
      <221> allele
      <222> 253
      <223> 5-2-162 : polymorphic base A or T
   <220>
      <221> allele
      <222> 269
      <223> 5-2-178 : polymorphic base C or T
   <220>
      <221> allele
      <222> 303
      <223> 5-2-213 : polymorphic base C or T
   <220>
      <221> allele
      <222> 362
      <223> 5-3-83 : polymorphic base C or T
   <220>
      <221> allele
      <222> 363
      <223> 5-3-84 : polymorphic base A or G
   <220>
      <221> allele
      <222> 527
      <223> 5-3-248 : polymorphic base A or G
   <220>
      <221> allele
      <222> 749
      <223> 5-7-195 : polymorphic base G or C
   <220>
      <221> allele
      <222> 1013
      <223> 5-10-39 : polymorphic base C or T
   <220>
      <221> allele
      <222> 1461
      <223> 5-262-49 : polymorphic base insertion of C
   <220>
      <221> allele
      <222> 1497
      <223> 5-262-85 : polymorphic base C or T
   <220>
      <221> allele
      <222> 1666
      <223> 5-262-254 : polymorphic base C or T
   <220>
      <221> allele
      <222> 2267
      <223> 5-263-404 : polymorphic base C or T
   <220>
      <221> allele
      <222> 2460
      <223> 5-265-244 : polymorphic base A or G
   <220>
      <221> misc_feature
      <222> 708
      <223> diverging nucleotide G in ref genbank : L78132
   <220>
      <221> misc_feature
      <222> 709
      <223> diverging nucleotide T in ref genbank : L78132
   <220>
      <221> misc_feature
      <222> 807
      <223> diverging nucleotide C in ref genbank : L78132
   <220>
      <221> misc_feature
      <222> 1013
      <223> diverging nucleotide T in ref genbank : L78132
   <400> 4
   agccgcccac ggacgccaga gccgggaacc ctgacggcac ttagctgctg acaaacaacc     60
   tgctccgtgg agcgcctgaa acaccagtct ttggggccag tgcctcagtt tcaatccagg    120
   taacctttaa atgaaacttg cctaaaatct taggtcatac acagaagaga ctccaatcga    180
   caagaagctg gaaaagaatg atg ttg tcc tta aac aac cta cag aat atc atc    233
                         Met Leu Ser Leu Asn Asn Leu Gln Asn Ile Ile
                         1               5                   10
   tat aac ccg gta atc ccg tat gtt ggc acc att ccc gat cag ctg gat      281
   Tyr Asn Pro Val Ile Pro Tyr Val Gly Thr Ile Pro Asp Gln Leu Asp
               15                  20                  25
   cct gga act ttg att gtg ata tgt ggg cat gtt cct agt gac gca gac      329
   Pro Gly Thr Leu Ile Val Ile Cys Gly His Val Pro Ser Asp Ala Asp
           30                  35                  40
   aga ttc cag gtg gat ctg cag aat ggc agc agt gtg aaa cct cga gcc      377
   Arg Phe Gln Val Asp Leu Gln Asn Gly Ser Ser Val Lys Pro Arg Ala
       45                  50                  55
   gat gtg gcc ttt cat ttc aat cct cgt ttc aaa agg gcc ggc tgc att      425
   Asp Val Ala Phe His Phe Asn Pro Arg Phe Lys Arg Ala Gly Cys Ile
   60                  65                  70                  75
   gtt tgc aat act ttg ata aat gaa aaa tgg gga cgg gaa gag atc acc      473
   Val Cys Asn Thr Leu Ile Asn Glu Lys Trp Gly Arg Glu Glu Ile Thr
                   80                  85                  90
   tat gac acg cct ttc aaa aga gaa aag tct ttt gag atc gtg att atg      521
   Tyr Asp Thr Pro Phe Lys Arg Glu Lys Ser Phe Glu Ile Val Ile Met
               95                  100                 105
   gtg cta aag gac aaa ttc cag gtg gct gta aat gga aaa cat act ctg      569
   Val Leu Lys Asp Lys Phe Gln Val Ala Val Asn Gly Lys His Thr Leu
           110                 115                 120
   ctc tat ggc cac agg atc ggc cca gag aaa ata gac act ctg ggc att      617
   Leu Tyr Gly His Arg Ile Gly Pro Glu Lys Ile Asp Thr Leu Gly Ile
       125                 130                 135
   tat ggc aaa gtg aat att cac tca att ggt ttt agc ttc agc tcg gac      665
   Tyr Gly Lys Val Asn Ile His Ser Ile Gly Phe Ser Phe Ser Ser Asp
   140                 145                 150                 155
   tta caa agt acc caa gca tct agt ctg gaa ctg aca gag ata agt aga      713
   Leu Gln Ser Thr Gln Ala Ser Ser Leu Glu Leu Thr Glu Ile Ser Arg
                   160                 165                 170
   gaa aat gtt cca aag tct ggc acg ccc cag ctt agc ctg cca ttc gct      761
   Glu Asn Val Pro Lys Ser Gly Thr Pro Gln Leu Ser Leu Pro Phe Ala
               175                 180                 185
   gca agg ttg aac acc ccc atg ggc cct gga cga act gtc gtc gtt aaa      809
   Ala Arg Leu Asn Thr Pro Met Gly Pro Gly Arg Thr Val Val Val Lys
           190                 195                 200
   gga gaa gtg aat gca aat gcc aaa agc ttt aat gtt gac cta cta gca      857
   Gly Glu Val Asn Ala Asn Ala Lys Ser Phe Asn Val Asp Leu Leu Ala
       205                 210                 215
   gga aaa tca aag gat att gct cta cac ttg aac cca cgc ctg aat att      905
   Gly Lys Ser Lys Asp Ile Ala Leu His Leu Asn Pro Arg Leu Asn Ile
   220                 225                 230                 235
   aaa gca ttt gta aga aat tct ttt ctt cag gag tcc tgg gga gaa gaa      953
   Lys Ala Phe Val Arg Asn Ser Phe Leu Gln Glu Ser Trp Gly Glu Glu
                   240                 245                 250
   gag aga aat att acc tct ttc cca ttt agt cct ggg atg tac ttt gag     1001
   Glu Arg Asn Ile Thr Ser Phe Pro Phe Ser Pro Gly Met Tyr Phe Glu
               255                 260                 265
   atg ata att tac tgt gat gtt aga gaa ttc aag gtt gca gta aat ggc     1049
   Met Ile Ile Tyr Cys Asp Val Arg Glu Phe Lys Val Ala Val Asn Gly
           270                 275                 280
   gta cac agc ctg gag tac aaa cac aga ttt aaa gag ctc agc agt att     1097
   Val His Ser Leu Glu Tyr Lys His Arg Phe Lys Glu Leu Ser Ser Ile
       285                 290                 295
   gac acg ctg gaa att aat gga gac atc cac tta ctg gaa caa tca ttc     1145
   Asp Thr Leu Glu Ile Asn Gly Asp Ile His Leu Leu Glu Gln Ser Phe
   300                 305                 310                 315
   aat caa aag agt gaa atg aag cac att aac aaa gca gga ggc gcc acg     1193
   Asn Gln Lys Ser Glu Met Lys His Ile Asn Lys Ala Gly Gly Ala Thr
                   320                 325                 330
   gac cgc ctc cct cca cac cgc tcc ttc cgc ctt cat tcc ttg ccc aca     1241
   Asp Arg Leu Pro Pro His Arg Ser Phe Arg Leu His Ser Leu Pro Thr
               335                 340                 345
   ggc ttg cac tgg aag ctg aat aag aat ccc caa aac tca aac ttc cta     1289
   Gly Leu His Trp Lys Leu Asn Lys Asn Pro Gln Asn Ser Asn Phe Leu
           350                 355                 360
   ggg atg cca ccc ctt tag tagctcacac ctcccccctc caagagctaa            1337
   Gly Met Pro Pro Leu  *
       365
   gaaacaaagg agaatgtact tttgtagctt agataagcaa tgaatcagta aaggactgat   1397
   ctacttgctc caccacccct cccttaataa taacatttac tgttatttcc tgggcctaag   1457
   acttatgttc cagaactgtc acagctcccc atgtcacacc cactagcttg tgatctttgt   1517
   caaataactg aaatctttta agcctctagt ttcttccttt gtaaaacaga gataaaatgt   1577
   tgtggttttt aagtgagata atccaagtaa agcacctaac atggagtagt gaatgaacat   1637
   cggttgctac taaaagtgga catcctaccg catccttaat gccactaggc atttccatac   1697
   aatctgggga ccaaaacttc aatcatataa atgtatgagg ttaattaaaa acactactgt   1757
   aatctgcttg tatgatcaca aaccaccaca aaagaaaaga tcgtgaagat tacactgtaa   1817
   acggactctc aaatgatcag gaggtggtca cttcgcaact tgctccctcc acccaactca   1877
   aaacaggagc tcgagcctgc ctgtatttga gactggagct gcctgtatga ggactggatc   1937
   aactgctagt cacgttatat ccaaatctgc attatcattg ggcacatttt cacagaattt   1997
   tactgaatta ttccttaatt gtttaatggt tgggaatagt ttgggaatta ccttccatca   2057
   actctgctaa gaaaggaatg gattctggta gcaagacaat ataattctcc tttagttttt   2117
   cagccagtgc taacacagta atcaaagcag caaatcgaac ctgaaaggga taaaagagca   2177
   aagaaataaa aagtagtgtt actgtattta ttatcttaag agctgtactg acttgagaca   2237
   agctctaact ttttaaacat tagttcacac gcgtttattc acttcattat gttcattaag   2297
   ctttcatctt agaataccag tttcaccatt tgggagctgt ttgtaatatg tgcaacctta   2357
   taaatagtgt tttccaaact gtgtcccagg actgcaaatc tttaatgtga aatgtctttt  2417
   tataatctct tcctttaaaa aaaaccaata aaataaaatg ccacatgcaa actc         2471
   <210> 5
      <211> 316
      <212> PRT
      <213> Homo sapiens
   <220>
      <221> VARIANT
      <222> 18
      <223> 5-2-162 : polymorphic amino acid Tyr or Phe
   <220>
      <221> VARIANT
      <222> 35
      <223> 5-2-213 : polymorphic amino acid Cys or Arg
   <220>
      <221> VARIANT
      <222> 55
      <223> 5-3-84 : polymorphic amino acid Val or Met
   <220>
      <221> VARIANT
      <222> 183
      <223> 5-7-195 : polymorphic amino acid Ser or Arg
   <400> 5
   Met Leu Ser Leu Asn Asn Leu Gln Asn Ile Ile Tyr Asn Pro Val Ile
   1               5                   10                  15
   Pro Tyr Val Gly Thr Ile Pro Asp Gln Leu Asp Pro Gly Thr Leu Ile
               20                  25                  30
   Val Ile Cys Gly His Val Pro Ser Asp Ala Asp Arg Phe Gln Val Asp
           35                  40                  45
   Leu Gln Asn Gly Ser Ser Val Lys Pro Arg Ala Asp Val Ala Phe His
       50                  55                  60
   Phe Asn Pro Arg Phe Lys Arg Ala Gly Cys Ile Val Cys Asn Thr Leu
   65                  70                  75                  80
   Ile Asn Glu Lys Trp Gly Arg Glu Glu Ile Thr Tyr Asp Thr Pro Phe
                   85                  90                  95
   Lys Arg Glu Lys Ser Phe Glu Ile Val Ile Met Val Leu Lys Asp Lys
               100                 105                 110
   Phe Gln Val Ala Val Asn Gly Lys His Thr Leu Leu Tyr Gly His Arg
           115                 120                 125
   Ile Gly Pro Glu Lys Ile Asp Thr Leu Gly Ile Tyr Gly Lys Val Asn
       130                 135                 140
   Ile His Ser Ile Gly Phe Ser Phe Ser Ser Asp Leu Gln Ser Thr Gln
   145                 150                 155                 160
   Ala Ser Ser Leu Glu Leu Thr Glu Ile Ser Arg Glu Asn Val Pro Lys
                   165                 170                 175
   Ser Gly Thr Pro Gln Leu Ser Leu Pro Phe Ala Ala Arg Leu Asn Thr
               180                 185                 190
   Pro Met Gly Pro Gly Arg Thr Val Val Val Lys Gly Glu Val Asn Ala
           195                 200                 205
   Asn Ala Lys Ser Phe Asn Val Asp Leu Leu Ala Gly Lys Ser Lys Asp
       210                 215                 220
   Ile Ala Leu His Leu Asn Pro Arg Leu Asn Ile Lys Ala Phe Val Arg
   225                 230                 235                 240
   Asn Ser Phe Leu Gln Glu Ser Trp Gly Glu Glu Glu Arg Asn Ile Thr
                   245                 250                 255
   Ser Phe Pro Phe Ser Pro Gly Met Tyr Phe Glu Met Ile Ile Tyr Cys
               260                 265                 270
   Asp Val Arg Glu Phe Lys Val Ala Val Asn Gly Val His Ser Leu Glu
           275                 280                 285
   Tyr Lys His Arg Phe Lys Glu Leu Ser Ser Ile Asp Thr Leu Glu Ile
       290                 295                 300
   Asn Gly Asp Ile His Leu Leu Glu Val Arg Ser Trp
   305                 310                 315
   <210> 6
      <211> 358
      <212> PRT
      <213> Homo sapiens
   <220>
      <221> VARIANT
      <222> 18
      <223> 5-2-162 : polymorphic amino acid Tyr or Phe
   <220>
      <221> VARIANT
      <222> 35
      <223> 5-2-213 : polymorphic amino acid Cys or Arg
   <220>
      <221> VARIANT
      <222> 55
      <223> 5-3-84 : polymorphic amino acid Val or Met
   <220>
      <221> VARIANT
      <222> 204
      <223> 5-202-95 : polymorphic amino acid Asp or Tyr
   <220>
      <221> VARIANT
      <222> 211
      <223> 5-202-117 : polymorphic amino acid Leu or Stop
   <220>
      <221> VARIANT
      <222> 225
      <223> 5-7-195 : polymorphic amino acid Ser or Arg
   <400> 6
   <210> 7
      <211> 368
      <212> PRT
      <213> Homo sapiens
   <220>
      <221> VARIANT
      <222> 18
      <223> 5-2-162 : polymorphic amino acid Tyr or Phe
   <220>
      <221> VARIANT
      <222> 35
      <223> 5-2-213 : polymorphic amino acid Cys or Arg
   <220>
      <221> VARIANT
      <222> 55
      <223> 5-3-84 : polymorphic amino acid Val or Met
   <220>
      <221> VARIANT
      <222> 183
      <223> 5-7-195 : polymorphic amino acid Ser or Arg
   <400> 7
   Met Leu Ser Leu Asn Asn Leu Gln Asn Ile Ile Tyr Asn Pro Val Ile
   1               5                   10                  15
   Pro Tyr Val Gly Thr Ile Pro Asp Gln Leu Asp Pro Gly Thr Leu Ile
               20                  25                  30
   Val Ile Cys Gly His Val Pro Ser Asp Ala Asp Arg Phe Gln Val Asp
           35                  40                  45
   Leu Gln Asn Gly Ser Ser Val Lys Pro Arg Ala Asp Val Ala Phe His
       50                  55                  60
   Phe Asn Pro Arg Phe Lys Arg Ala Gly Cys Ile Val Cys Asn Thr Leu
   65                  70                  75                  80
   Ile Asn Glu Lys Trp Gly Arg Glu Glu Ile Thr Tyr Asp Thr Pro Phe
                   85                  90                  95
   Lys Arg Glu Lys Ser Phe Glu Ile Val Ile Met Val Leu Lys Asp Lys
               100                 105                 110
   Phe Gln Val Ala Val Asn Gly Lys His Thr Leu Leu Tyr Gly His Arg
           115                 120                 125
   Ile Gly Pro Glu Lys Ile Asp Thr Leu Gly Ile Tyr Gly Lys Val Asn
       130                 135                 140
   Ile His Ser Ile Gly Phe Ser Phe Ser Ser Asp Leu Gln Ser Thr Gln
   145                 150                 155                 160
   Ala Ser Ser Leu Glu Leu Thr Glu Ile Ser Arg Glu Asn Val Pro Lys
                   165                 170                 175
   Ser Gly Thr Pro Gln Leu Ser Leu Pro Phe Ala Ala Arg Leu Asn Thr
               180                 185                 190
   Pro Met Gly Pro Gly Arg Thr Val Val Val Lys Gly Glu Val Asn Ala
           195                 200                 205
   Asn Ala Lys Ser Phe Asn Val Asp Leu Leu Ala Gly Lys Ser Lys Asp
       210                 215                 220
   Ile Ala Leu His Leu Asn Pro Arg Leu Asn Ile Lys Ala Phe Val Arg
   225                 230                 235                 240
   Asn Ser Phe Leu Gln Glu Ser Trp Gly Glu Glu Glu Arg Asn Ile Thr
                   245                 250                 255
   Ser Phe Pro Phe Ser Pro Gly Met Tyr Phe Glu Met Ile Ile Tyr Cys
               260                 265                 270
   Asp Val Arg Glu Phe Lys Val Ala Val Asn Gly Val His Ser Leu Glu
           275                 280                 285
   Tyr Lys His Arg Phe Lys Glu Leu Ser Ser Ile Asp Thr Leu Glu Ile
       290                 295                 300
   Asn Gly Asp Ile His Leu Leu Glu Gln Ser Phe Asn Gln Lys Ser Glu
   305                 310                 315                 320
   Met Lys His Ile Asn Lys Ala Gly Gly Ala Thr Asp Arg Leu Pro Pro
                   325                 330                 335
   His Arg Ser Phe Arg Leu His Ser Leu Pro Thr Gly Leu His Trp Lys
               340                 345                 350
   Leu Asn Lys Asn Pro Gln Asn Ser Asn Phe Leu Gly Met Pro Pro Leu
           355                 360                 365
   <210> 8
      <211> 1738
      <212> DNA
      <213> Mus musculus
   <400> 8
   gagtgttact accaccgggg acaagttttt actttgagta atccttaaat gaagagtggg     60
   taaagtgtgt atacggaaga gagactccaa tcaacaatat caataagttg aaaaagaaaa    120
   atg ttg tcc tta aat aac cta caa aat atc atc tat aac ccg ata atc      168
   Met Leu Ser Leu Asn Asn Leu Gln Asn Ile Ile Tyr Asn Pro Ile Ile
   1               5                   10                  15
   ccc tat gtt ggc acc att act gag caa ttg aag cct ggc tct ctg att      216
   Pro Tyr Val Gly Thr Ile Thr Glu Gln Leu Lys Pro Gly Ser Leu Ile
               20                  25                  30
   gta atc cgt ggg cat gtc cct aaa gat tca gaa aga ttc cag gtt gac      264
   Val Ile Arg Gly His Val Pro Lys Asp Ser Glu Arg Phe Gln Val Asp
           35                  40                  45
   ttt cag ctg ggc aac agc ctg aag cca aga gca gac gtg gcc ttc cac      312
   Phe Gln Leu Gly Asn Ser Leu Lys Pro Arg Ala Asp Val Ala Phe His
       50                  55                  60
   ttt aac cct cgg ttc aaa agg tct agc tgc att gtt tgt aac aca ctg      360
   Phe Asn Pro Arg Phe Lys Arg Ser Ser Cys Ile Val Cys Asn Thr Leu
   65                  70                  75                  80
   aca cag gag aag tgg ggc tgg gag gag atc acc tac gac atg ccc ttc      408
   Thr Gln Glu Lys Trp Gly Trp Glu Glu Ile Thr Tyr Asp Met Pro Phe
                   85                  90                  95
   aga aaa gaa aag tcc ttt gag atc gtg ttc atg gtg ctc aag aac aaa       456
   Arg Lys Glu Lys Ser Phe Glu Ile Val Phe Met Val Leu Lys Asn Lys
               100                 105                 110
   ttc cag gtg gct gtg aac gga agg cat gtt ctg ctg tac gcc cac agg       504
   Phe Gln Val Ala Val Asn Gly Arg His Val Leu Leu Tyr Ala His Arg
           115                 120                 125
   atc agc ccg gag cag atc gac aca gtg ggc atc tac ggc aaa gtg aac       552
   Ile Ser Pro Glu Gln Ile Asp Thr Val Gly Ile Tyr Gly Lys Val Asn
       130                 135                 140
   atc cac tcc atc ggg ttc aga ttc agc tcg gat tta cag agt atg gaa       600
   Ile His Ser Ile Gly Phe Arg Phe Ser Ser Asp Leu Gln Ser Met Glu
   145                 150                 155                 160
   aca tct gct ctg gga ctg aca cag ata aac aga gag aat ata caa aag       648
   Thr Ser Ala Leu Gly Leu Thr Gln Ile Asn Arg Glu Asn Ile Gln Lys
                   165                 170                 175
   cca ggc aag ctc cag ctg agc ctg cca ttt gaa gca agg ttg aat gcc       696
   Pro Gly Lys Leu Gln Leu Ser Leu Pro Phe Glu Ala Arg Leu Asn Ala
               180                 185                 190
   tcc atg ggt cct gga cga acc gtt gtc att aaa ggg gaa gtg aac acc       744
   Ser Met Gly Pro Gly Arg Thr Val Val Ile Lys Gly Glu Val Asn Thr
           195                 200                 205
   aat gcc cga agc ttt aat gtt gac cta gtg gca gga aaa aca agg gat       792
   Asn Ala Arg Ser Phe Asn Val Asp Leu Val Ala Gly Lys Thr Arg Asp
       210                 215                 220
   atc gct ctg cac ttg aac cca cgc ctc aat gtg aaa gca ttt gta aga       840
   Ile Ala Leu His Leu Asn Pro Arg Leu Asn Val Lys Ala Phe Val Arg
   225                 230                 235                 240
   aat tcc ttt ctt cag gat gcc tgg gga gaa gag gag aga aat att acc       888
   Asn Ser Phe Leu Gln Asp Ala Trp Gly Glu Glu Glu Arg Asn Ile Thr
                   245                 250                 255
   tgc ttc cca ttt agt tct ggg atg tac ttt gag atg ata atc tac tgt       936
   Cys Phe Pro Phe Ser Ser Gly Met Tyr Phe Glu Met Ile Ile Tyr Cys
               260                 265                 270
   gat gtc cgg gaa ttc aag gtt gct ata aat ggt gtg cac agc ctg gag       984
   Asp Val Arg Glu Phe Lys Val Ala Ile Asn Gly Val His Ser Leu Glu
           275                 280                 285
   tac aaa cac aga ttt aaa gac cta agc agt att gat aca cta tca gtc      1032
   Tyr Lys His Arg Phe Lys Asp Leu Ser Ser Ile Asp Thr Leu Ser Val
       290                 295                 300
   gat ggt gat atc cgt ttg ctg gat gta agg agc tgg tag ctaccatgac       1081
   Asp Gly Asp Ile Arg Leu Leu Asp Val Arg Ser Trp  *
   305                 310                 315
   tgccaaaacc cccgaaatac aaaatggctt atccggtact ggccatgtca aatgcatctc    1141
   gctttcacca tattgtttat attgctaagt tgagctcctc caacatcaag tcctactggt    1201
   gttgtcaggt ctggccatgc agtacattca gaggaacaga gccggggcaa tcacagctca    1261
   ctgccagaga ggctctgcac actgggtccc tcttataaac cacactcagc aaatatttaa    1321
   gtgcctaata tactacatat actagctaat agggatggca agcatacttc ctttgtatat    1381
   tctctgagcc gggcacagac atggcagggc ccagaacttg tgtggtccat gttttctagc    1441
   acttcgtacc agtttctggc ctcctaatgt agggtcttct tgctggcatt gcattaaccc    1501
   cactaggggc ctttgcagtt aaggtcagaa aaatatacta atggatggca aacactactt    1561
   ccccagcaac ccttttcata atcagcattc tatcatatct cataattgaa gactgcatag    1621
   catttactta gctctcaccg ctttaaactt tataaaatgt atgatgctga acacagcaga    1681
   aaaactgagg ccaaaaccct gaattatgac aaaacaagtg ttctgctcca agcagat       1738
   <210> 9
      <211> 316
      <212> PRT
      <213> Mus musculus
   <400> 9
   Met Leu Ser Leu Asn Asn Leu Gln Asn Ile Ile Tyr Asn Pro Ile Ile
   1               5                   10                  15
   Pro Tyr Val Gly Thr Ile Thr Glu Gln Leu Lys Pro Gly Ser Leu Ile
               20                  25                  30
   Val Ile Arg Gly His Val Pro Lys Asp Ser Glu Arg Phe Gln Val Asp
           35                  40                  45
   Phe Gln Leu Gly Asn Ser Leu Lys Pro Arg Ala Asp Val Ala Phe His
       50                  55                  60
   Phe Asn Pro Arg Phe Lys Arg Ser Ser Cys Ile Val Cys Asn Thr Leu
   65                  70                  75                  80
   Thr Gln Glu Lys Trp Gly Trp Glu Glu Ile Thr Tyr Asp Met Pro Phe
                   85                  90                  95
   Arg Lys Glu Lys Ser Phe Glu Ile Val Phe Met Val Leu Lys Asn Lys
               100                 105                 110
   Phe Gln Val Ala Val Asn Gly Arg His Val Leu Leu Tyr Ala His Arg
           115                 120                 125
   Ile Ser Pro Glu Gln Ile Asp Thr Val Gly Ile Tyr Gly Lys Val Asn
       130                 135                 140
   Ile His Ser Ile Gly Phe Arg Phe Ser Ser Asp Leu Gln Ser Met Glu
   145                 150                 155                 160
   Thr Ser Ala Leu Gly Leu Thr Gln Ile Asn Arg Glu Asn Ile Gln Lys
                   165                 170                 175
   Pro Gly Lys Leu Gln Leu Ser Leu Pro Phe Glu Ala Arg Leu Asn Ala
               180                 185                 190
   Ser Met Gly Pro Gly Arg Thr Val Val Ile Lys Gly Glu Val Asn Thr
           195                 200                 205
   Asn Ala Arg Ser Phe Asn Val Asp Leu Val Ala Gly Lys Thr Arg Asp
       210                 215                 220
   Ile Ala Leu His Leu Asn Pro Arg Leu Asn Val Lys Ala Phe Val Arg
   225                 230                 235                 240
   Asn Ser Phe Leu Gln Asp Ala Trp Gly Glu Glu Glu Arg Asn Ile Thr
                   245                 250                 255
   Cys Phe Pro Phe Ser Ser Gly Met Tyr Phe Glu Met Ile Ile Tyr Cys
               260                 265                 270
   Asp Val Arg Glu Phe Lys Val Ala Ile Asn Gly Val His Ser Leu Glu
           275                 280                 285
   Tyr Lys His Arg Phe Lys Asp Leu Ser Ser Ile Asp Thr Leu Ser Val
       290                 295                 300
   Asp Gly Asp Ile Arg Leu Leu Asp Val Arg Ser Trp
   305                 310                 315
   <210> 10
      <211> 18
      <212> DNA
      <213> Homo sapiens
   <220>
      <221> misc_binding
      <222> 1..18
      <223> sequencing oligonucleotide PrimerPU
   <400> 10
      tgtaaaacga cggccagt 18
   <210> 11
      <211> 18
      <212> DNA
      <213> Homo sapiens
   <220>
      <221> misc_binding
      <222> 1..18
      <223> sequencing oligonucleotide PrimerRP
   <400> 11
      caggaaacag ctatgacc 18

## Claims

1. A method of determining whether an individual is at risk of developing prostate cancer, or whether said individual suffers from prostate cancer, comprising:
a) determining the identity of a nucleotide at a PCTA-1-related biallelic marker or the complement thereof in a biological sample derived from said individual, wherein the identity of the nucleotide at said biallelic marker is determined for both copies of said biallelic marker present in said individual's genome, and wherein said biallelic marker is selected from the group consisting of the PCTA-1-related biallelic markers shown in the table below:
| Biallelic marker | Alternative alleles | Position on SEQ ID NO: 1 |
|---|---|---|
| A2 | A/T | 402 |
| A30 | C /T | 67092 |
| A41 | C / T | 68525 |
| A55 | C / T | 82234 |
| A57 | T / C | 82393 |
and
b) correlating the result of step a) with a risk of developing prostate cancer, wherein:
(i) the A allele ofA2;
(ii) the C allele of A55;
(iii) the G allele ofA57; or
(iv) the T allele of A30 or A41;
indicates a risk of developing a familial case of prostate cancer; and wherein:
(v) the A allele of A57; or
(vi) the T allele of A2, A30, A41 or A55;
indicates a risk of developing a sporadic case of prostate cancer.

2. A method according to claim 1, further comprising amplifying a portion of said sequence comprising the biallelic marker prior to said determining step.

3. A method according to claim 2, wherein said amplifying is performed by PCR.

4. A method according TO claim 1, wherein said determining is performed by a hybridization assay.

5. A method according to claim 1, wherein said determining is performed by a sequencing assay.

6. A method according to claim 1, wherein determining is performed by a microsequencing assay.

7. A method according to claim 1, wherein said determining is performed by an enzyme-based mismatch detection assay.

8. The method according to any of claims 1 to 7, wherein said at least one PCTA-1-related biallelic marker is a set ofbiallelic markers comprising PCTA-1-related biallelic markers A30 and A41.

9. The method according to claim 8, wherein said set of biallelic markers further comprises PCTA-1-related biallelic marker A2.

10. The method according to claim 9, wherein said set of biallelic markers further comprises PCTA-1-related biallelic marker A55.

11. The method according to claim 9 or 10, wherein said set of biallelic markers further comprises PCTA-1-related biallelic marker A57.

12. The method according to any of claims 8 to 11, wherein the presence of a:
a) T at PCTA-1-related biallelic marker A30;
b) T at PCTA-1-related biallelic marker A41;
c) A at PCTA-1-related biallelic marker A2;
a) C at PCTA-1-related biallelic markerA55; and/or
d) G at PCTA-1-related biallelic marker A57;
indicates that said individual is at risk of developing prostate cancer, or suffers from prostate cancer.

13. The method according to any of claim 8 to 12, wherein said prostate cancer is a familial case of prostate cancer.

14. The method according to any of claims 1 to 7, wherein said at least one PCTA-1-related biallelic marker is a set of biallelic markers camprising PCTA-1-related biallelic markers A2 and A55.

15. The method according to any of claims 1 to 7, wherein said at least one PCTA-1-related biallelic marker is a set of biallelic markers comprising PCTA-1-related biallelic markers A2 and A57.

16. The method according to claim 14 or 15, wherein said set of biallelic markers comprises PCTA-1-related biallelic markers A2, A55 and A57.

17. The method according to claim 16, wherein said set of biallelic markers further comprises PCTA-1 -related biallelic marker A30.

18. The method according to claim 16 or 17, wherein said set of biallelic markers further comprises PCTA-1-related biallelic marker A41.

19. The method according to any of claims 14 to 18, wherein the presence of a:
a) T at PCTA-1-related biallelic marker A2;
b) Tat PCTA-1-related biallelic marker A55;
b) A at PCTA-1-related biallelic marker A57;
c) T at PCTA-1-related biallelic marker A30; and/or
d) T at PCTA-1-related biallelic marker A51;
indicates that said individual is at risk of developing prostate cancer, or suffers from prostate cancer.

20. The method according to any of claims 14 to 19, wherein said prostate cancer is a sporadic case of prostate cancer.

21. An isolated, purified or recombinant polynucleotide consisting of a contiguous span of 15 to 50 nucleotides of SEQ ID NO: 1 or the complements thereof, wherein said span includes a PCTA-1-related biallelic marker of the table set forth in claim 1 in said sequence.

22. A polynucleotide according to claim 21, wherein said contiguous span is 18 to 47 nucleotides in length and said biallelic marker is within 4 nucleotides of the center of said polynucleotide.

23. A polynucleotide according to claim 22, wherein said polynucleotide consists of said contiguous span and said contiguous span is 25 nucleotides in length and said biallelic marker is at the center of said polynucleotide.

24. A polynucleotide according to claim 23, wherein said biallelic marker is present at the 3' end of said polynucleotide.

25. A polynucleotide consisting of a contiguous span of 15 to 50 nucleotides of SEQ ID NO: 1 or the complements thereof, wherein said the 3' end of said polynucleotide is located 1 nucleotide upstream of a PCTA biallelic marker of the table set forth in claim 1.

26. A polynucleotide according to any one of claims 21 to 25 attached to a solid support.

27. A polynucleotide according to any one of claims 21 to 26 further comprising a label.

28. A diagnostic kit comprising a polynucleotide according to any of claims 21 to 27.

29. Use of a polynucleotide in a hybridization assay for determining the identity of a nucleotide at a PCTA-1-related biallelic marker of the table set forth in claim 1.

30. Use of a polynucleotide in a sequencing assay for determining the identity of a nucleotide at a PCTA-1-related biallelic marker of the table set forth in claim 1.

31. Use of a polynucleotide in an enzyme-based mismatch detection assay for determining the identity of a nucleotide at a PCTA-1-related biallelic marker of the table set forth in claim 1.

## Patentansprüche

1. Verfahren, um festzustellen, ob ein Individuum gefährdet ist, Prostatakrebs zu entwickeln oder ob das Individuum an Prostatakrebs leidet, umfassend:
a) Bestimmen der Identität eines Nukleotids aus einem *PCTA-1-*verwandten biallelen Marker oder Komplement davon in einer biologischen Probe, die aus dem Individuum stammt, wobei die Identität des Nukleotids aus dem biallelen Marker für beide Kopien des biallelen Markers, die im Genom des Individuums vorhanden sind, bestimmt wird und wobei der biallele Marker ausgewählt ist aus der Gruppe bestehend aus den *PCTA-1-*verwandten biallelen Markern, die in der Tabelle unten gezeigt sind:
| **Bialleler Marker** | **Alternative Allele** | **Position in der SEQ ID NO:1** |
|---|---|---|
| A2 | A/T | 402 |
| A30 | C/T | 67092 |
| A41 | C/T | 68525 |
| A55 | C/T | 82234 |
| A57 | T/C | 82393 |
und
b) Korrelieren des Ergebnis aus Schritt a) mit dem Risiko, Prostatakrebs zu entwickeln, wobei
(i) das A-Allel von A2;
(ii) das C-Allel von A55;
(iii) das G-Allel von A57 oder
(iv) das T-Allel von A30 oder A41;
das Risiko, einen familiär-bedingten Fall von Prostatakrebs zu entwickeln, anzeigt; und wobei:
(v) das A-Allel von A57 oder
(vi) das T-Allel von A2, A30, A41 oder A55;
das Risiko, einen sporadischen Fall von Prostatakrebs zu entwickeln, anzeigt.

2. Verfahren nach Anspruch 1, ferner umfassend die Amplifizierung eines Teilbereichs der Sequenz, die den biallelen Marker umfasst, vor dem Bestimmungsschritt.

3. Verfahren nach Anspruch 2, wobei die Amplifizierung mittels PCR durchgeführt wird.

4. Verfahren nach Anspruch 1, wobei die Bestimmung mittels eines Hybridisierungs-Assays durchgeführt wird.

5. Verfahren nach Anspruch 1, wobei die Bestimmung mittels eines Sequenzierungs-Assays durchgeführt wird.

6. Verfahren nach Anspruch 1, wobei die Bestimmung mittels eines Mikrosequenzierungs-Assays durchgeführt wird.

7. Verfahren nach Anspruch 1, wobei die Bestimmung mittels eines enzymbasierten Fehlpaarungs-Nachweis-Assays durchgeführt wird.

8. Verfahren nach einem der Ansprüche 1 bis 7, wobei der mindestens ein *PCTA-1-*verwandte biallele Marker ein Set an biallelen Markern ist,umfassend die *PCTA-1-*verwandten biallelen Marker A30 und A41.

9. Verfahren nach Anspruch 8, wobei das Set an biallelen Markern ferner den *PCTA-1-*verwandten biallelen Marker A2 enthält.

10. Verfahren nach Anspruch 9, wobei das Set an biallelen Markern ferner den *PCTA-1-*verwandten biallelen Marker A55 enthält.

11. Verfahren nach Anspruch 9 oder 10, wobei das Set an biallelen Markern ferner den PCTA-1-verwandten biallelen Marker A57 enthält.

12. Verfahren nach einem der Ansprüche 8 bis 11, wobei das Vorhandensein von einem
a) T im *PCTA-1-*verwandten biallelen Marker A30;
b) T im *PCTA-1-*verwandten biallelen Marker A41;
c) A im *PCTA*-*1*-verwandten biallelen Marker A2;
a) C im *PCTA-1-*verwandten biallelen MarkerA55 und/oder;
d) G im *PCTA*-*1*-verwandten biallelen Marker A57;
anzeigt, dass das Indivivuum gefährdet ist, Prostatakrebs zu entwickeln oder an Prostatakrebs leidet.

13. Verfahren nach einem der Ansprüche 8 bis 12, wobei der Prostatakrebs ein familiärbedingter Fall von Prostatakrebs ist.

14. Verfahren nach einem der Ansprüche 1 bis 7, wobei der mindestens ein *PCTA-1-*verwandte biallele Marker ein Set an biallelen Markern ist,umfassend die *PCTA-1-*verwandten biallelen Marker A2 und A55.

15. Verfahren nach einem der Ansprüche 1 bis 7, wobei der mindestens ein *PCTA-1-*verwandte biallele Marker ein Set an biallelen Markern ist,umfassend die *PCTA-1-*verwandten biallelen Marker A2 und A57.

16. Verfahren nach Anspruch 14 oder 15, wobei das Set an biallelen Markern die PCTA-1-verwandten biallelen Marker A2, A55 und A57 umfasst.

17. Verfahren nach Anspruch 16, wobei das Set an biallelen Markern ferner den *PCTA-*1-verwandten biallelen Marker A30 umfasst.

18. Verfahren nach Anspruch 16 oder 17, wobei das Set an biallelen Markern ferner den *PCTA*-*1*-verwandten biallelen Marker A41 umfasst.

19. Verfahren nach einem der Ansprüche 14 bis 18, wobei das Vorhandensein von einem
a) T im *PCTA*-*1*-verwandten biallelen Marker A2;
b) T im *PCTA-1*-verwandten biallelen Marker A55;
b) A im *PCTA-1-*verwandten biallelen Marker A57;
c) T im *PCTA*-*1*-verwandten biallelen Marker A30 und/oder;
d) T im *PCTA-1-*verwandten biallelen Marker A51;
anzeigt, dass das Indivivuum gefährdet ist, Prostatakrebs zu entwickeln oder an Prostatakrebs leidet.

20. Verfahren nach einem der Ansprüche 14 bis 19, wobei der Prostatakrebs ein sporadischer Fall von Prostatakrebs ist.

21. Ein isoliertes, gereinigtes oder rekombinantes Polynukleotid bestehend aus einem zusammenhängenden Bereich von 15 bis 50 Nukleotiden der SEQ ID NO:1 oder den Komplementen davon, wobei der Bereich einen *PCTA-1-*verwandten biallelen Marker aus der in Anspruch 1 dargelegten Tabelle in der Sequenz einschließt.

22. Polynukleotid nach Anspruch 21, wobei der zusammenhängenden Bereich 18 bis 47 Nukleotide lang ist und der biallele Marker innerhalb von 4 Nukleotiden von der Mitte des Polynukleotids ist.

23. Polynukleotid nach Anspruch 22, wobei das Polynukleotid aus dem zusammenhängenden Bereich besteht und der zusammenhängenden Bereich 25 Nukleotide lang ist und der biallele Marker in der Mitte des Polynukleotids ist.

24. Polynukleotid nach Anspruch 23, wobei der biallele Marker am 3'-Ende des Polynukleotids vorhanden ist.

25. Polynukleotid bestehend aus einem zusammenhängenden Bereich von 15 bis 50 Nukleotiden der SEQ ID NO:1 oder den Komplementen davon, wobei das 3' Ende des Polynukleotids 1 Nukleotid stromaufwärts von einem *PCTA* biallelen Marker aus der in Anspruch 1 dargelegten Tabelle lokalisiert ist.

26. Polynukleotid nach einem der Ansprüche 21 bis 25, angeheftet an einen festen Träger.

27. Polynukleotid nach einem der Ansprüche 21 bis 26, ferner eine Markierung umfassend.

28. Diagnostisches Kit umfassend ein Polynukleotid nach einem der Ansprüche 21 bis 27.

29. Verwendung eines Polynukleotids in einem Hybridisierungs-Assay zum Feststellen der Identität eines Nukleotids in einem *PCTA-1-*verwandten biallelen Marker aus der in Anspruch 1 dargelegten Tabelle.

30. Verwendung eines Polynukleotids in einem Sequenzierungs-Assay zum Feststellen der Identität eines Nukleotids in einem *PCTA-1-*verwandten biallelen Marker aus der in Anspruch 1 dargelegten Tabelle.

31. Verwendung eines Polynukleotids in einem enzymbasierten Fehlpaarungs-Nachweis-Assay zum Feststellen der Identität eines Nukleotids in einem *PCTA-1-*verwandten biallelen Marker aus der in Anspruch 1 dargelegten Tabelle.

## Revendications

1. Procédé de détermination si un individu est à risque de développer un cancer de la prostate, ou si ledit individu souffre d'un cancer de la prostate, comprenant :
a) la détermination de l'identité d'un nucléotide au niveau d'un marqueur biallélique lié au PCTA-1 ou du complémentaire de celui-ci dans un échantillon biologique dérivé dudit individu, où l'identité du nucléotide au niveau dudit marqueur biallélique est déterminée par les deux copies dudit marqueur biallélique présentes dans le génome dudit individu, et où ledit marqueur biallélique est choisi dans le groupe constitué des marqueurs bialléliques liés au PCTA-1 présentés dans le tableau ci-dessous :
| Marqueur biallélique | Allèles alternatifs | Position sur SEQ ID NO : 1 |
|---|---|---|
| A2 | A/T | 402 |
| A30 | C/T | 67092 |
| A41 | C/T | 68525 |
| A55 | C/T | 82234 |
| A57 | T/C | 82393 |
et
b) la corrélation du résultat de l'étape a) avec un risque de développement d'un cancer de la prostate, où
(i) l'allèle A de A2 ;
(ii) l'allèle C de A55 ;
(iii) l'allèle G de A57 ; ou
(iv) l'allèle T de A30 ou A41 ;
indique un risque de développement d'un cas familial de cancer de la prostate ; et où :
(v) l'allèle A de A57 ; ou
(vi) l'allèle T de A2, A30, A41 ou A55 ;
indique un risque de développement d'un cas sporadique de cancer de la prostate.

2. Procédé selon la revendication 1, comprenant en outre l'amplification d'une partie de ladite séquence comprenant le marqueur biallélique avant ladite étape de détermination.

3. Procédé selon la revendication 2, dans lequel ladite amplification est réalisée par PCR.

4. Procédé selon la revendication 1, dans lequel ladite détermination est réalisée par un test d'hybridation.

5. Procédé selon la revendication 1, dans lequel ladite détermination est réalisée par un test de séquençage.

6. Procédé selon la revendication 1, dans lequel ladite détermination est réalisée par un test de microséquençage.

7. Procédé selon la revendication 1, dans lequel ladite détermination est réalisée par un test enzymatique de détection de mésappariements.

8. Procédé selon l'une quelconque des revendications 1 à 7, dans lequel au moins un marqueur biallélique lié au PCTA-1 est un ensemble de marqueurs bialléliques comprenant les marqueurs bialléliques liés au PCTA-1 A30 et A41.

9. Procédé selon la revendication 8, dans lequel ledit ensemble de marqueurs bialléliques comprend en outre le marqueur biallélique lié au PCTA-1 A2.

10. Procédé selon la revendication 9, dans lequel ledit ensemble de marqueurs bialléliques comprend en outre le marqueur biallélique lié au PCTA-1 A55.

11. Procédé selon la revendication 9 ou 10, dans lequel ledit ensemble de marqueurs bialléliques comprend en outre le marqueur biallélique lié au PCTA-1 A57.

12. Procédé selon l'une quelconque des revendications 8 à 11, dans lequel la présence d'un :
a) T au niveau du marqueur biallélique lié au PCTA-1 A30 :
b) T au niveau du marqueur biallélique lié au PCTA-1 A41 ;
c) A au niveau du marqueur biallélique lié au PCTA-1 A2 ;
a) C au niveau du marqueur biallélique lié au PCTA-1 A55 ; et/ou
d) G au niveau du marqueur biallélique lié au PCTA-1 A57 ;
indique que ledit individu est à risque de développer un cancer de la prostate, ou souffre d'un cancer de la prostate.

13. Procédé selon l'une quelconque des revendications 8 à 12, dans lequel ledit cancer de la prostate est un cas familial de cancer de la prostate.

14. Procédé selon l'une quelconque des revendications 1 à 7, dans lequel ledit au moins un marqueur biallélique lié au PCTA-1 est un ensemble de marqueurs bialléliques comprenant les marqueurs bialléliques liés au PCTA-1 A2 et A55.

15. Procédé selon l'une quelconque des revendications 1 à 7, dans lequel ledit au moins un marqueur biallélique lié au PCTA-1 est un ensemble de marqueurs bialléliques comprenant les marqueurs bialléliques liés au PCTA-1 A2 et A57.

16. Procédé selon la revendication 14 ou 15, dans lequel ledit ensemble de marqueurs bialléliques comprend les marqueurs bialléliques liés au PCTA-1 A2, A55 et A57.

17. Procédé selon la revendication 16, dans lequel ledit ensemble de marqueurs bialléliques comprend en outre le marqueur biallélique lié au PCTA-1 A30.

18. Procédé selon la revendication 16 ou 17, dans lequel ledit ensemble de marqueurs bialléliques comprend en outre le marqueur biallélique lié au PCTA-1 A41.

19. Procédé selon l'une quelconque des revendications 14 à 18, dans lequel la présence d'un :
a) T au niveau du marqueur biallélique lié au PCTA-1 A2 ;
b) T au niveau du marqueur biallélique lié au PCTA-1 A55 ;
b) A au niveau du marqueur biallélique lié au PCTA-1 A57 ;
c) T au niveau du marqueur biallélique lié au PCTA-1 A30 ; et/ou
d) T au niveau du marqueur biallélique lié au PCTA-1 A51 ;
indique que ledit individu est à risque de développer un cancer de la prostate, ou souffre d'un cancer de la prostate.

20. Procédé selon l'une quelconque des revendications 14 à 19, dans lequel ledit cancer de la prostate est un cas sporadique de cancer de la prostate.

21. Polynucléotide isolé, purifié ou recombinant constitué d'une longueur contiguë de 15 à 50 nucléotides de SEQ ID NO : 1 ou des complémentaires de ceux-ci, où ladite longueur comprend un marqueur biallélique lié au PCTA-1 du tableau présenté dans la revendication 1 dans ladite séquence.

22. Polynucléotide selon la revendication 21, où ladite longueur contiguë est longue de 18 à 47 nucléotides et ledit marqueur biallélique est à 4 nucléotides du centre dudit polynucléotide.

23. Polynucléotide selon la revendication 22, dans lequel ledit polynucléotide est constitué de ladite longueur contiguë et ladite longueur contiguë est longue de 25 nucléotides et ledit marqueur biallélique est au centre dudit polynucléotide.

24. Polynucléotide selon la revendication 23, dans lequel ledit marqueur biallélique est présent à l'extrémité 3' dudit polynucléotide.

25. Polynucléotide constitué d'une longueur contiguë de 15 à 50 nucléotides de SEQ ID NO : 1 ou des complémentaires de ceux-ci, où ladite extrémité 3' dudit polynucléotide est localisée 1 nucléotide an amont d'un marqueur biallélique du PCTA du tableau présenté dans la revendication 1.

26. Polynucléotide selon l'une quelconque des revendications 21 à 25 fixé à un support solide.

27. Polynucléotide selon l'une quelconque des revendications 21 à 26 comprenant en outre un marqueur.

28. Kit de diagnostic comprenant un polynucléotide selon l'une quelconque des revendications 21 à 27.

29. Utilisation d'un polynucléotide dans un test d'hybridation pour déterminer l'identité d'un nucléotide au niveau d'un marqueur biallélique lié au PCTA-1 du tableau présenté dans la revendication 1.

30. Utilisation d'un polynucléotide dans un test de séquençage pour déterminer l'identité d'un nucléotide au niveau d'un marqueur biallélique lié au PCTA-1 du tableau présenté dans la revendication 1.

31. Utilisation d'un polynucléotide dans un test enzymatique de détection de mésappariements pour déterminer l'identité d'un nucléotide au niveau d'un marqueur biallélique lié au PCTA-1 du tableau présenté dans la revendication 1.
